# EUROPEAN PATENT APPLICATION

(11) **EP 4 763 287 A2**
(43) Date of publication of application: **24.06.2026**
(21) Application number: 26175905.4
(22) Date of filing: 02.09.2022
(51) Int. Cl.: A61P 35/00

(54) **NOVEL LIPID NANOPARTICLES FOR DELIVERY OF NUCLEIC ACIDS**

(30) Priority: 03.09.2021 WO PCT/EP2021/074344; 17.12.2021 WO PCT/EP2021/086516; 21.01.2022 WO PCT/EP2022/051320
(62) Divisional of application: 22773178.3
(71) Applicant: CureVac SE, 72076 Tübingen (DE)
(72) Inventor: BAUMHOF, Patrick, 72076 Tübingen (DE); NAVARRO, Gemma, 72076 Tübingen (DE); BOLLHORST, Tobias, 72076 Tübingen (DE); THIELE-SÜSS, Carolin, 72076 Tübingen (DE); PALMERSTON MENDES, Livia, 72076 Tübingen (DE)
(74) Representative: Pinsent Masons LLP

(57) **Abstract**

The invention relates to novel polymer conjugated lipids and to novel compositions comprising said novel polymer conjugated lipids useful for the delivery of nucleic acids into living cells.

## Description

### TECHNICAL FIELD

The present invention relates to lipid nanoparticles comprising polyoxazoline (POZ) or poly(2-methyl-2-oxazoline) (PMOZ)-lipids and nucleic acids, preferably mRNA, being useful as mRNA-based vaccines or mRNA-based therapeutics. The present invention also relates to compositions comprising lipid nanoparticles (LNPs) comprising lipids comprising POZ- or PMOZ-moieties (POZ-lipids, PMOZ-lipids) and mRNA and the use of said LNPs or the composition comprising POZ or PMOZ-lipids for the preparation of a pharmaceutical composition, especially a vaccine composition, e.g. for use in the prophylaxis or treatment of infectious diseases, tumor or cancer diseases, allergies or autoimmune diseases. The present invention also relates to mRNA-based therapeutics LNP compositions for mRNA-encoded protein replacement therapy, whereby the LNP compositions comprise POZ- or PMOZ-lipids. Examples for this are indications or illnesses that can be restored by liver transplantation (like OTC deficiency). The present invention further describes therapies using aforementioned LNPs or compositions comprising lipids comprising POZ- or PMOZ-moieties for protein replacement therapy. The present invention further describes methods of treatment or prophylaxis of the afore-mentioned diseases. The mRNA also may encode an antibody, an antibody fragment, variant, adduct or derivative of an antibody, such as a single-chain variable fragment, a diabody or a triabody.

### BACKGROUND OF THE INVENTION

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen in vivo. Genetic vaccines are expressed upon administration to a patient after uptake by target cells. Expression of the administered nucleic acids results in production of the encoded proteins. In the event these proteins are recognized as foreign by the patient's immune system, an immune response is triggered.

DNA as well as RNA may be used as nucleic acid molecules for administration in the context of genetic vaccination. DNA is known to be relatively stable and easy to handle. However, the use of DNA bears the risk of undesired insertion of the administered DNA-fragments into the patient's genome potentially resulting mutagenic events such as in loss of function of the impaired genes. As a further risk, the undesired generation of anti-DNA antibodies has emerged. Another drawback is the limited expression level of the encoded peptide or protein that is achievable upon DNA administration because the DNA must enter the nucleus in order to be transcribed before the resulting mRNA can be translated. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

The use of messenger RNA (mRNA) for delivery of genetic information into target cells offers an attractive alternative to DNA. The advantages of using mRNA include transient expression and a non-transforming character - mRNA does not need to enter the nucleus in order to be expressed and moreover cannot integrate into the host genome, thereby eliminating the risk of oncogenesis. I.e. by using RNA instead of DNA for genetic vaccination, the risk of undesired genomic integration and generation of anti-DNA antibodies is minimized or avoided. However, RNA is considered to be a rather unstable molecular species which may readily be degraded by ubiquitous RNAses. mRNA vaccines comprising antigen-encoding mRNA complexed to protamine are already described in the prior art (e.g. PMIDs 27336830 or 23159882, EP1083232, WO2010037539, WO2012116811, WO2012116810, and WO2015024665).

Two problems currently face the use of RNA or mRNA in therapeutic contexts. First, free RNAs are susceptible to nuclease digestion in plasma. Second, free RNAs have limited ability to gain access to the intracellular compartment where the relevant translation machinery resides. Lipid nanoparticles formed from cationic lipids with other lipid components, such as neutral lipids, cholesterol, PEGylated lipids, and RNA or mRNA have been used to block degradation of the RNAs in plasma and facilitate the cellular uptake of the oligonucleotides. WO2018078053 and WO2016176330 describe lipid nanoparticle compositions comprising unmodified and nucleoside-modified RNA encoding different antigens in this regard; both references are incorporated herein by reference in their entirety. In addition to molecular composition, parameters play a role in efficiency of delivery, such as particle size, charge, or grafting with molecular components such as polyethylene glycol (PEG) or ligands. Grafting with FDA approved PEG is considered to reduce interactions of the particles with serum, to increase serum stability and to extend the circulation time, being helpful for certain targeting approaches. Further, PEGylation can be used for particle engineering. For example, if lipid nanoparticles (LNP) are prepared by mixing an aqueous phase of RNA with an organic phase of lipids, a certain amount of PEG-conjugated lipid is required in the lipid mixture, otherwise the particles aggregate in the mixing step. By varying the molar fraction of PEG-lipid containing PEG at different molar masses, it could be shown that the particle size can be adjusted. Likewise, the particle size can be adjusted by varying the molar mass of the PEG fraction of the PEGylated lipids. Typical sizes that are accessible are in the range between 30 and 200 nm (Belliveau et al., 2012, Molecular Therapy-Nucleic Acids 1(8): e37, PMID: 23344179). Particles formed in this way have the additional advantage that they interact less with serum components due to the PEG fraction and have a longer circulation half-life, which is desirable in many drug delivery approaches. Summarized, without PEG lipids, discrete size particles cannot be formed; the particles would end up forming large aggregates and precipitate.

Thus, in techniques where LNPs are formed from an ethanolic and an aqueous phase, one of the main functions of the PEG lipids is to facilitate the self-assembly of the particles by forming a steric barrier on the surface of the resulting particles, which are formed when nucleic acids are rapidly mixed in ethanol solutions containing lipids to bind the RNA. The steric PEG barrier prevents the fusion between the particles and promotes the formation of a homogeneous population of LNPs with diameters <100 nm.

PEG is the most widely used "stealth" polymer for drug delivery and is considered to be the gold standard. To produce a homogeneous and colloidally stable nanoparticle population, PEG lipids are typically incorporated into systems due to their hydrophilic steric hindrance property (the PEG shell prevents electrostatic or van der Waals attraction leading to aggregation). PEGylation allows to attract a water envelope around the polymer, which shields the RNA complex from opsonization with serum proteins, increasing serum half-life and decreasing rapid renal clearance, resulting in an improvement of pharmacokinetic behavior. The variation of the length of the acyl chains of the lipids (C₁₈, C₁₆ or C₁₄) modifies the stability of the incorporation of the PEG-lipid into the particles, i.e. the PEG-shedding, leading to a modulation of the in vivo bioperformance and pharmacokinetics. The use of a PEG-lipid with short acyl chains (C₁₄) dissociating in vivo with a halftime of less than 30 min from LNPs leads to an optimal hepatocyte gene silencing potency (Chen et al., 2014, J Control Release 196:106-12; Ambegia et al., 2005, Biochimica et Biophysica Acta 1669:155- 163). Further, by varying the PEG-lipid parameter a strict control of particle size can be achieved, i.e. a higher PEG-MW or a higher molar fraction of PEG-lipids in the particles lead to smaller particles.

Despite these advantages, PEGylation of nanoparticles can also lead to various effects that may be detrimental to the intended use for drug delivery. It is known that PEGylation of liposomes and LNPs reduces cellular uptake and endosomal escape, ultimately reducing overall transfection efficiency. In fact, the PEG shell provides a steric barrier for efficient binding of particles to the cell and also hinders endosomal release by preventing membrane fusion between the liposome and the endosomal membrane. For this reason, the type of PEG-lipid and the amount of PEG-lipid used must always be carefully adjusted. On the one hand, it should provide a sufficient stealth effect for in vivo and stabilization aspects, on the other hand, it should not hinder transfection. This phenomenon is known as the "PEG dilemma".

Besides lowering transfection efficiency, PEGylation has been associated with accelerated blood clearance (ABC) phenomenon induced by anti-PEG antibodies and/or complement activation as well as storage diseases (Bendele A et al., 1998, Toxicolocical Sciences 42, 152-157; Young MA et al., 2007, Translational Research 149(6), 333-342; S.M. Moghimi, J. Szebeni, 2003, Progress in Lipid Research 42:463-478). Ishida et al and Laverman et al reported that intravenous injection in rats of PEG-grafted liposomes may significantly alter the pharmacokinetic behavior of a second dose when this second dose is administered after an interval of several days (Laverman P et al., 2001, J Pharmacol Exp Ther. 298(2), 607-12; Ishida et al., 2006, J Control Release 115(3), 251-8). The phenomenon of "accelerated blood clearance" (ABC) appears to be inversely related to the PEG content of liposomes. The presence of anti-PEG antibodies in the plasma induces a higher clearance of the particles by the Monophagocyte System (MPS) which at the end reduces the efficacy of the drug. This phenomenon even affects patients that have never been treated with drugs comprising PEG, due to ubiquity of PEG in e.g. cosmetics industry (ointments, creams, shampoos, toiletries, lotions) and agriculture chemicals.

PEG is also supposed to induce complement activation, which can lead to hypersensitivity reaction, also known as Complement-Activation Related Pseudo-Allergy (CARPA). It is still not clear from the literature if the activation of complement is due to the nanoparticle in general or to the presence of PEG in particular.

The presence of PEG in lipidic nanoparticles may also induce a specific immune response. Semple et al. reported that liposomes containing PEG-lipid derivatives and encapsulated antisense oligodeoxynucleotide or plasmid DNA elicit a strong immune response that results in the rapid blood clearance of subsequent doses in mice. The magnitude of this response was sufficient to induce significant morbidity and, in some instances, mortality. Rapid elimination of liposome-encapsulated ODN from blood depended on the presence of PEG-lipid in the membrane because the use of non-PEGylated liposomes or liposomes containing rapidly exchangeable PEG-lipid abrogated the response. The generation of anti-PEG antibody and the putative complement activation were a likely explanation for the rapid elimination of the vesicles from the blood. (Semple et al., 2005, J Pharmacol Exp Ther. 312(3), 1020-6).

As PEG may induce immune responses there is a need to avoid it for certain applications where multiple injections are needed. Examples are therapies using mRNA, for example for protein replacement therapy. Here, the risk can be particularly high due to the potential intrinsic immunogenicity of RNA.

Further, for storage and shipping purposes, LNPs are often stored at lower temperatures, e.g. frozen, or lyophilized (freeze-dryed). Frozen LNPs must be thawed to room temperature, while lyophilized LNPs must be reconstituted back into solution prior to patient administration. During thawing or reconstitution, respectively, an often observed problem is a change in physiochemical properties like e.g. an increase in size of the LNP and PDI. It was found, that standard LNP-formulations, comprising PEG-lipids, were highly sensitive against dilution and freeze/thaw cycles. This was reflected by a significant impact either on the size of LNPs, the mRNA encapsulation efficiency (EE) or both. It is crucial to address this because the freezing at low concentrations would allow the preparation of single-dose vials in contrast to the current presentation as concentrated multi-dose vials requiring a dilution step prior administration. In more detail, it was found that at concentrations below 1 g/L, a significant impact on the LNP size upon one freeze/thaw cycle was observed with nearly doubling of particle size for some of the dilutions.

Thus, there remains a need in the art for efficient methods and compositions for introducing RNA into cells which avoid the disadvantages accompanied by use of PEG. The object of the invention therefore is the provision of efficient methods and compositions for introducing RNA into cells avoiding the described disadvantages accompanied by use of PEG. The present invention solves this object and addresses these and other needs. There further remains a need for improved PEG-less lipid nanoparticles for the delivery of RNA. Preferably, these PEG-less lipid nanoparticles would provide optimal drug:lipid ratios, protect the nucleic acid from degradation and clearance in serum, be suitable for systemic or local delivery, and provide intracellular delivery of the nucleic acid. In addition, these PEG-less lipid nanoparticles comprising RNA or mRNA should be well-tolerated and provide an adequate therapeutic index, such that patient treatment at an effective dose of the nucleic acid is not associated with unacceptable toxicity and/or risk to the patient. The present invention provides these and related advantages. Thus, notwithstanding all the prior art, however, there is still a requirement for alternative polymer conjugated lipids, alternative cationic lipids and consequently alternative lipid nanoparticles comprising said alternative lipids, that offer one or more properties of reduced cell toxicity, better targeting ability, enhanced short-term and/or long-term immunity, or promotion of endosomal escape of molecules, e.g. nucleic acids. Despite the vast amount of work undertaken to date in the field of polymer conjugated lipids, lipid nanoparticles and cationic lipids, therefore, it is nevertheless desired to develop further polymer conjugated lipids, lipid nanoparticles and cationic lipids capable of ameliorating or obviating one or more of the problems described above or of in vivo efficacy of the transfection process, toxicity, cost and simplicity of design.

In detail, the inventors surprisingly found that the RNA particle formulations described herein fulfill the above mentioned requirements. In particular it is demonstrated that polyoxazoline (POZ) or poly(2-methyl-2-oxazoline) (PMOZ)-lipid conjugates are suitable components for assembly of RNA nanoparticles. Poly (2-oxazoline) is a class of polymers formed by cationic ring-opening that were first identified and synthesized over 50 years ago (Kagiya et al., J Polym Sci B Polym Lett 1966; 4:441-5). These polymers are nonionic, biostable, soluble in water and in some polar organic solvents, and can be synthesized from readily available nontoxic, nonexplosive starting materials. The N-carbonyl side chains on the polymer chain gives the appearance of a "pseudo-polypeptide". POZ with shorter side chains are generally more water-soluble than those with longer side chains. PMOZ e.g. is composed of repeated units of 2-methyl-2-oxazoline (CAS RN: 161358-46-9) and is rapidly excreted by the kidney with no significant accumulation in tissue (Gaertner et al., Journal of Controlled Release 119 (2007) 291-300). POZ/PMOZ-lipid conjugates enable manufacturing of RNA nanoparticles with different techniques, resulting in defined surface properties and controlled size ranges. Manufacturing can be done by robust processes, compliant with the requirements for pharmaceutical manufacturing. The particles can be end-group functionalized with different moieties to modulate charge or to introduce specific molecular moieties like ligands.

Here, the inventors found surprisingly, that the inventive LNPs comprising new polyoxazoline polymer conjugated lipids, preferably PMOZ-lipids, have advantageous physiochemical properties after being frozen and thawed or, upon lyophilization and reconstitution, measured with PDI and size measurements. The inventors found surprisingly, that PMOZ-LNPs were superior with regard to size (smaller size) and PDI after putting lipid nanoparticles of the invention under thermal stress, i.e. upon freeze/thaw cycles or lyophilization and reconstitution, respectively. Furthermore, a disadvantageous increase of size and PDI was found for PEG-LNPs for dilutions. I.e. PEG-LNPs had an increasing size and PDI when being diluted. The increase of size and PDI was not found, or respectively not found that pronounced, for LNPs comprising PMOZ as conjugated lipid. Even in cases, in which PMOZ-LNPs show an increase of size upon dilution or freeze/thaw in a similar range as PEG-LNPs, PMOZ-LNPs still were smaller than PEG-LNPs, which would be favorable as findings in that field indicate that smaller particles are more immunogenic (Li et al., 2014, Journal of controlled release, 173, 148-157; Ott et al., Vaccine, 1995 13(16), 1557-1562; Shah et al., 2014. Nanomedicine, 9(17), 2671-2681.

Thus, the object of the present invention can also be seen as to the provision of (i) novel polymer conjugated lipids, (ii) novel lipid nanoparticles comprising said novel polymer conjugated lipids, (iii) the use of said novel polymer conjugated lipids making the improved lipid nanoparticles with regards to the generation of anti-PEG antibodies (i.e. the novel lipid nanoparticles do not generate anti-PEG antibodies) and improved with regards to enhanced physiochemical properties upon (i) freezing and thawing or (ii) lyophilizing and reconstituting said lipid nanoparticles for e.g. storage or shipping. These objects and the further objects described under "Background of the Invention" are solved by the subject-matter of the present invention.

### SUMMARY OF THE INVENTION

In one aspect, the present invention relates to novel polymer conjugated lipids which are useful for the delivery of nucleic acids into living cells.

In a specific aspect, the polymer conjugated lipids are compounds according to formula (I):

[P]-[linker]-[L] formula (I)

or a pharmaceutically acceptable salt, prodrug, tautomer or stereoisomer thereof, wherein
- [P]: is a homopolymer moiety comprising at least one polyoxazoline (POZ) monomer unit 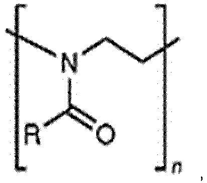 wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa
- [linker]: is an optional linker group, and
- [L]: is a lipid moiety.

In another embodiment, the polymer conjugated lipid comprises as [P] a heteropolymer moiety or homopolymer moiety comprising multiple monomer units selected from the group consisting of
poly(2-methyl-2-oxazoline) (PMOZ) 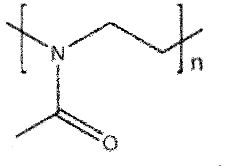
poly(2-ethyl-2-oxazoline) (PEOZ) 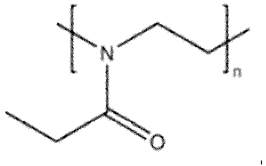
poly(2-propyl-2-oxazoline) (PPOZ) 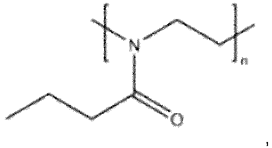
poly(2-butyl-2-oxazoline) (PBOZ) 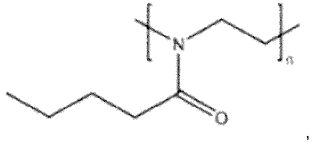
poly(2-isopropyl-2-oxazoline) (PIPOZ) 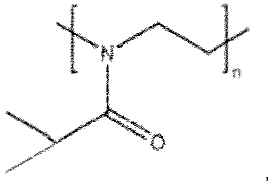
poly(2-methoxymethyl-2-oxazoline) (PMeOMeOx), and
poly(2-dimethylamino-2-oxazoline) (PDMAOx),
preferably wherein [P] is a homopolymer moiety comprising multiple PMOZ or PEOZ monomer units, more preferably wherein [P] comprises or preferably consists of multiple PMOZ monomer units,
wherein
   (i) n has a mean value ranging from about 45 to about 55, preferably n is about 50 or wherein
   (ii) n is selected such that the [P] moiety has an average molecular weight of about 4.2 kDa to about 4.4 kDa, or most preferably about 4.3 kDa.

In very preferred embodiments, the homopolymer moieties [P] are selected from the group consisting of PMeOz50 (polymethyloxazoline or poly(2-methyl-2-oxazoline) with 50 repeats), PEtOz50 (polyethyloxazoline with 50 repeats), PMeOz25 (polymethyloxazoline with 25 repeats) and PEtOz25 (polyethyloxazoline with 25 repeats), preferably PMeOz50 (polymethyloxazoline or poly(2-methyl-2-oxazoline) with 50 repeats).

In another embodiment, the polymer conjugated lipid is selected from the group consisting of a POZ-monoacylglycerol conjugate, POZ-diacylglycerol conjugate, a POZ-dialkyloxypropyl conjugate, a POZ-steroid or POZ-sterol conjugate, a POZ-phospholipid conjugate, a POZ-ceramide conjugate, and a mixture thereof. In a further embodiment, the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain, wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-),-C(R5)=N-, -N=C(R⁵)-, -C(R⁵)=N-O-, -O-N=C(R⁵)-, -O-C(O)O-, -C(O)N(R⁵), -N(R⁵)C(O)-,-C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl).

In a further embodiment, the lipid moiety [L] comprises two straight unsaturated alkyl chain containing from 6 to 30 carbon atoms, preferably wherein the lipid moiety [L] comprises at least one straight or branched saturated alkyl chain, wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or-OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), -C(R5)=N-,-N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, - C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl).

In a further embodiment, the lipid moiety [L] comprises two straight unsaturated alkyl chain each containing 14 carbon atoms.

In a further most preferred embodiment, the polymer conjugated lipid comprises a lipid moiety [L] comprising ditetradecylamin and a linker group [linker], preferably wherein the linker group [linker] is (-NHC(O)CH₂CH₂C(O)-). In a further preferred embodiment, the lipid moiety [L] comprises ditetradecylamin, wherein the linker moiety [linker], preferably (-NHC(O)CH₂CH₂C(O)-), is forming an amide connection by connection to the N-atom of ditetradecylamin. In most preferred embodiments, the polymer conjugated lipid comprises a linker (-NHC(O)CH₂CH₂C(O)-), wherein the linker is orientated in such way, that an carboxamide connection is formed through connection to the N-atom of ditetradecylamin.

In a further most preferred embodiment, the polymer conjugated lipid comprises a lipid moiety [L] comprising ditetradecylamin and a linker group [linker], preferably wherein the linker group [linker] is (C(O)CH2CH2C(O)NH). In a further preferred embodiment, the lipid moiety [L] comprises ditetradecylamin, wherein the linker moiety [linker], preferably (-NHC(O)CH₂CH₂C(O)-), is forming an amide connection by connection to the N-atom of ditetradecylamin.

In a further preferred embodiment, the lipid moiety [L] is the lipid moiety as used in "PMOZ 2".

In a further preferred embodiment, the linker moiety [linker] is the linker moiety as used in "PMOZ 2".

In further most preferred embodiment, the invention relates to a polymer conjugated lipid having a lipid moiety [L], being the lipid moiety as used in "PMOZ 4". In another most preferred embodiment, the invention relates to a polymer conjugated lipid having a linker moiety [linker], being the linker as used in "PMOZ 4".
comprises ditetradecylamin, preferably wherein the linker group [linker] is (-NHC(O)CH₂CH₂C(O)-). In a preferred embodiment, the lipid moiety [L] comprises ditetradecylamin, wherein the linker moiety [linker] is connected to the N-atom of ditetradecylamin.

In another aspect, the invention provides novel lipid nanoparticles comprising a homopolymer moiety comprising
at least one polyoxazoline (POZ) monomer unit 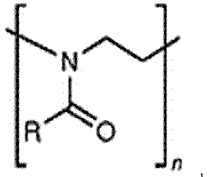
wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa, preferably, wherein the homopolymer moiety comprising multiple monomer units comprises poly(2-methyl-2-oxazoline) (PMOZ), poly(2-ethyl-2-oxazoline) (PEOZ), poly(2-propyl-2-oxazoline) (PPOZ), poly(2-butyl-2-oxazoline) (PBOZ), poly(2-isopropyl-2-oxazoline) (PIPOZ), poly(2-methoxymethyl-2-oxazoline) (PMeOMeOx), or poly(2-dimethylamino-2-oxazoline) (PDMAOx).

Most preferably, R is C₁ (i.e. CH₃ or methyl), yielding in polymethyloxazoline or poly(2-methyl-2-oxazoline) i.e. "PMOZ.

In further aspects, the invention provides vaccine compositions comprising the lipid nanoparticle of the invention, or a kit or kit of parts comprising the inventive polymer conjugated lipids for use as a medicament, and/or for prevention, prophylaxis, treatment and/or amelioration of a disease selected from infectious diseases including viral, bacterial or protozoological infectious diseases, cancer or tumor diseases.

In a further aspect, the invention provides methods of treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition comprising the steps:
a) providing a lipid nanoparticle, comprising a homopolymer moiety comprising at least one polyoxazoline (POZ) monomer, preferably the polymer conjugated lipid of the disclosure, the vaccine composition, or the kit or kit of parts of the disclosure; and
b) applying or administering the mRNA, the lipid nanoparticle, the vaccine composition or the kit or kit of parts to a tissue or an organism.

In another aspect of the present invention, the present invention also provides a pharmaceutical composition comprising a lipid nanoparticle of the disclosure, a kit or kit of parts of the disclosure, or the vaccine composition of the disclosure for use in vaccination of a subject comprising an effective dose of mRNA encoding a virus antigen.

In yet another aspect of the invention, the present invention provides improved lyophilizable lipid nanoparticles, which have advantageous physiochemical properties after being lyophilized and reconstituted.

In yet another aspect of the invention, the present invention provides improved lipid nanoparticles, which have advantageous physiochemical properties after being frozen and thawed.

### DEFINITIONS

For the sake of clarity and readability, the following scientific background information and definitions are provided. Any technical features mentioned herein or disclosed thereby can be part of or may be read on each and every embodiment of the invention. Additional definitions and explanations can be provided in the context of this disclosure.

Unless defined otherwise, or unless the specific context requires otherwise, all technical terms used herein have the same meaning as is commonly understood by a person skilled in the relevant technical field.

Unless the context indicates or requires otherwise, the words "comprise", "comprises" and "comprising" and similar expressions are to be construed in an open and inclusive sense, as "including, but not limited to" in this description and in the claims. It also needs to be understood that for the purposes of the present invention, the term "consisting of" is considered to be a preferred embodiment of the term "comprising". If hereinafter a group is defined to comprise at least a certain number of embodiments, this is also meant to encompass a group which preferably consists of these embodiments only.

The expressions, "one embodiment", "an embodiment", "a specific embodiment" and the like mean that a particular feature, property or characteristic, or a particular group or combination of features, properties or characteristics, as referred to in combination with the respective expression, is present in at least one of the embodiments of the invention. The occurrence of these expressions in various places throughout this description do not necessarily refer to the same embodiment. Moreover, the particular features, properties or characteristics may be combined in any suitable manner in one or more embodiments.

The singular forms "a", "an" and "the" should be understood as to include plural references unless the context clearly dictates otherwise.

Percentages in the context of numbers should be understood as relative to the total number of the respective items. In other cases, and unless the context dictates otherwise, percentages should be understood as percentages by weight (wt-%).

As used herein, a "compound" means a chemical substance, which is a material consisting of molecules having essentially the same chemical structure and properties. For a small molecular compound, the molecules are typically identical with respect to their atomic composition and structural configuration. For a macromolecular or polymeric compound, the molecules of a compound are highly similar but not all of them are necessarily identical. For example, a segment of a polymer that is designated to consist of 50 monomeric units may also contain individual molecules with e.g. 48 or 53 monomeric units.

The term "molecule" may either be used as a synonym for "compound" or for an individual (i.e. a single) molecule.

Any reference to a compound or moiety having a functional group which is ionizable under physiological conditions should be understood as including the ionized form of the respective compound or moiety. Vice versa, any reference to a compound or moiety having an ionized functional group which may also exist in the non-ionized form under physiological conditions should be understood as including the non-ionized form of the respective compound or moiety. For example, the disclosure of a compound having a carboxyl group should be interpreted as referring to the respective compound with non-ionized carboxyl group or with the ionized carboxylate group.

As used herein, "physiological conditions" refers to an aqueous environment having a pH that is within the pH range known from human physiology, including both extra- and intracellular conditions. An approximation of this pH range is from about pH 1 to about pH 9. Depending on the context, physiological conditions may also refer to approximately neutral conditions, such as from about pH 5 to about pH 8.5, or from about pH 5.5 to about pH 8.

A lipidoid compound, also simply referred to as lipidoid, is a lipid-like compound, i.e. an amphiphilic compound with lipid-like physical properties. In the context of the present invention, the term lipid is considered to encompass lipidoids.

In the context of the present invention, the term "selected from the group consisting of" followed by a certain group of elements (e.g. "A, B and C") is meant within the context of the invention to be not limited to said group. In other words, such a term does not indicate that the disclosure is closed to unrecited elements, i.e. also alternative meanings are comprised within the group following this term. Therefore, in the context of the present invention, the term "selected from the group consisting of" followed by a certain group of elements (i.e. "A, B and C") should be understood as "selected from A, B, and C" or alternatively "is A, B, or C" encompassing also other structurally and functionally related and unrelated but not mentioned elements.

The term "about" is used when parameters or values do not necessarily need to be identical, i.e. 100% the same. Accordingly, "about" means, that a parameter or values may diverge by 0.1% to 20%, preferably by 0.1% to 10%; in particular, by 0.5%, 1%, 2%, 3%, 4%, 5%, 6%, 7%, 8%, 9%, 10%, 11%, 12%, 13%, 14%, 15%, 16%, 17%, 18%, 19%, 20%. The skilled person will know that e.g. certain parameters or values may slightly vary based on the method how the parameter was determined. For example, if a certain parameter or value is defined herein to have e.g. a length of "about 1000 nucleotides", the length may diverge by 0.1% to 20%, preferably by 0.1% to 10%; in particular, by 0.5%, 1%, 2%, 3%, 4%, 5%, 6%, 7%, 8%, 9%, 10%, 11%, 12%, 13%, 14%, 15%, 16%, 17%, 18%, 19%, 20%. Accordingly, the skilled person will know that in that specific example, the length may diverge by 1 to 200 nucleotides, preferably by 1 to 100 nucleotides; in particular, by 5, 10, 20, 30, 40, 50, 60, 70, 80, 90, 100, 110, 120, 130, 140, 150, 160, 170, 180, 190, 200 nucleotides.

The term "cationic" means, unless a different meaning is clear from the specific context, that the respective structure bears a positive charge, either permanently or not permanently but in response to certain conditions such as e.g. pH. Thus, the term "cationic" covers both "permanently cationic" and "cationisable". The term "cationisable" as used herein means that a compound, or group or atom, is positively charged at a lower pH and uncharged at a higher pH of its environment. Also in non-aqueous environments where no pH value can be determined, a cationisable compound, group or atom is positively charged at a high hydrogen ion concentration and uncharged at a low concentration or activity of hydrogen ions. It depends on the individual properties of the cationisable or polycationisable compound, in particular the pKₐ of the respective cationisable group or atom, at which pH or hydrogen ion concentration it is charged or uncharged. In diluted aqueous environments, the fraction of cationisable compounds, groups or atoms bearing a positive charge may be estimated using the so-called Henderson-Hasselbalch equation which is well-known to a person skilled in the art. E.g., if a compound or moiety is cationisable, it is preferred that it is positively charged at a pH value of about 1 to 9, preferably 4 to 9, 5 to 8 or even 6 to 8, more preferably of a pH value of or below 9, of or below 8, of or below 7, most preferably at physiological pH values, e.g. about 7.3 to 7.4, i.e. under physiological conditions, particularly under physiological salt conditions of the cell in vivo. In embodiments, it is preferred that the cationisable compound or moiety is predominantly neutral at physiological pH values, e.g. about 7.0-7.4, but becomes positively charged at lower pH values. In some embodiments, the preferred range of pKₐ for the cationisable compound or moiety is about 5 to about 7. In some embodiments, the protonatable lipids have a pKₐ of the protonatable group in the range of about 4 to about 11, e.g., a pKₐ of about 5 to about 7.

Unless a different meaning is clear from the specific context, the term "cationic" means that the respective structure bears a positive charge, either permanently, or not permanently but in response to certain conditions such as pH. Thus, the term "cationic" covers both "permanently cationic" and "cationisable". For example, a compound or moiety with a primary, secondary or tertiary amino group is cationic, and more specifically, cationisable, as it may exist predominantly in the positively charged state under physiological conditions.

As used herein, "permanently cationic" means that the respective compound, or group or atom, is positively charged at any pH value or hydrogen ion activity of its environment. Very often, the positive charge results from the presence of a quaternary nitrogen atom. Where a compound carries a plurality of such positive charges, it may be referred to as permanently polycationic, which is a subcategory of permanently cationic.

Similarly, the terms "anionic", "anionizable" and "permanently anionic" are used to have the analog meaning as "cationic", "cationisable" and "permanently cationic", except that the charge of the respective compound, group or atom is negative rather than positive.

The expression "neutral", when applied to a compound such as a lipid or a steroid, or to a group or moiety, either means that it is neither cationic nor anionic, such as a compound having no functional groups that are ionizable under physiological conditions as, for example, like a hydrocarbon; or it is both cationic and anionic, i.e. zwitterionic, under typical physiological conditions, such as a typical native phosphatidylcholine.

A "lipid", as used herein, refers to a group of organic compounds that are derivatives of fatty acids (e.g., esters) and are generally characterized by being insoluble in water but soluble in many organic solvents. Lipids are usually divided in at least three classes: (1) "simple lipids" which include fats and oils as well as waxes; (2) "compound lipids" which include phospholipids and glycolipids; and (3) "derived lipids" such as steroids. Regarding glycolipids, in certain embodiments, the LNP comprises glycolipids (e.g., monosialoganglioside GM₁).

In this context, the prefix "poly-" refers to a plurality of atoms or groups having the respective property in a compound. If put in parenthesis, the presence of a plurality is optional. For example, (poly)cationic means cationic and/or polycationic. However, the absence of the prefix should not be interpreted such as to exclude a plurality. For example, a polycationic compound is also a cationic compound and may be referred to as such.

The term "nucleic acid" means any compound comprising, or consisting of, DNA or RNA. The term may be used for a polynucleotide and/or oligonucleotide. Wherever herein reference is made to a nucleic acid or nucleic acid sequence encoding a particular protein and/or peptide, said nucleic acid or nucleic acid sequence, respectively, preferably also comprises regulatory sequences allowing in a suitable host, e.g. a human being, its expression, i.e. transcription and/or translation of the nucleic acid sequence encoding the particular protein or peptide.

In particularly preferred embodiments, the artificial nucleic acid, nucleic acid or RNA is an mRNA, more preferably an isolated mRNA. mRNA technology is specifically preferred in the context of the invention because mRNA allows for regulated dosage, transient and controlled expression as when compared to viral systems, complete degradation of the mRNA after protein synthesis, and does not pose the risk of insertional mutations.

In the context of the present invention, the term "nucleoside modification" refers to nucleic acids such as mRNA compounds or molecules comprising nucleosides which do not normally occur in native mRNA, preferably non-natural nucleosides. In particular, the term preferably refers to mRNA nucleosides other than adenine, guanine, cytosine, uracil and thymine.

The term "nucleoside" generally refers to compounds consisting of a sugar, usually ribose or deoxyribose, and a purine or pyrimidine base. The term "nucleotide" generally refers to a nucleoside comprising a phosphate group attached to the sugar.

A "peptide" means an oligomer or polymer of at least two amino acid monomers linked by peptide bonds. The term does not limit the length of the polymer chain of amino acids. A peptide may, for example, contain less than 50 monomer units. Longer peptides are also called polypeptides, typically having 50 to 600 monomeric units, more specifically 50 to 300 monomeric units.

A "protein" comprises or consists of one or more polypeptides folded into a 3-dimensional form, facilitating a biological function.

An "influenza pandemic" or "pandemic flu" can occur when a non-human (novel) influenza virus gains the ability for efficient and sustained human-to-human transmission and then spreads globally. Influenza viruses that have the potential to cause a pandemic are referred to as "influenza viruses with pandemic potential" or "pandemic influenza virus".

Examples of influenza viruses with pandemic potential include avian influenza A (H5N1) and avian influenza A (H7N9), which are two different "bird flu" viruses. These are non-human viruses (i.e., they are novel among humans and circulate in birds in parts of the world) so there is little to no immunity against these viruses among people. Human infections with these viruses have occurred rarely, but if either of these viruses was to change in such a way that it was able to infect humans easily and spread easily from person to person, an influenza pandemic could result.

Vaccine for pandemic influenza/flu or pandemic influenza/flu vaccine: A vaccine directed against a pandemic influenza virus is called herein as a vaccine for pandemic influenza/flu or pandemic influenza/flu vaccine.

Flu/influenza season: Flu season is an annually recurring time period characterized by the prevalence of outbreaks of influenza (flu). The season occurs during the cold half of the year in each hemisphere. Influenza activity can sometimes be predicted and even tracked geographically. While the beginning of major flu activity in each season varies by location, in any specific location these minor epidemics usually take about 3 weeks to peak and another 3 weeks to significantly diminish. Flu vaccinations have been used to diminish the effects of the flu season; pneumonia vaccinations additionally diminishes the effects and complications of flu season. Since the Northern and Southern Hemisphere have winter at different times of the year, there are actually two flu seasons each year.

Vaccine for seasonal influenza/flu or seasonal influenza/flu vaccine: A vaccine directed against the seasonal occurring influenza viruses in a flu season is termed herein "vaccine for seasonal influenza/flu or seasonal influenza/flu vaccine".

Immune system: The immune system may protect organisms from infection. If a pathogen breaks through a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts contains so called humoral and cellular components.

Immune response: An immune response may typically either be a specific reaction of the adaptive immune system to a particular antigen (so called specific or adaptive immune response) or an unspecific reaction of the innate immune system (so called unspecific or innate immune response). The invention relates to the core to specific reactions (adaptive immune responses) of the adaptive immune system. Particularly, it relates to adaptive immune responses to infections by viruses like e.g. Influenza viruses. However, this specific response can be supported by an additional unspecific reaction (innate immune response). Therefore, the invention also relates to a compound for simultaneous stimulation of the innate and the adaptive immune system to evoke an efficient adaptive immune response.

Adaptive immune system: The adaptive immune system is composed of highly specialized, systemic cells and processes that eliminate or prevent pathogenic growth. The adaptive immune response provides the vertebrate immune system with the ability to recognize and remember specific pathogens (to generate immunity), and to mount stronger attacks each time the pathogen is encountered. The system is highly adaptable because of somatic hypermutation (a process of increased frequency of somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of that cell will then inherit genes encoding the same receptor specificity, including the Memory B cells and Memory T cells that are the keys to long-lived specific immunity. Immune network theory is a theory of how the adaptive immune system works, that is based on interactions between the variable regions of the receptors of T cells, B cells and of molecules made by T cells and B cells that have variable regions.

Adaptive immune response: The adaptive immune response is typically understood to be antigen-specific. Antigen specificity allows for the generation of responses that are tailored to specific antigens, pathogens or pathogen-infected cells. The ability to mount these tailored responses is maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naïve T cells are constantly passing. Cell types that can serve as antigen-presenting cells are inter alia dendritic cells, macrophages, and B cells. Each of these cells has a distinct function in eliciting immune responses. Dendritic cells take up antigens by phagocytosis and macropinocytosis and are stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. Presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind antigen directly, but instead recognize short peptide fragments e.g. of pathogen-derived protein antigens, which are bound to MHC molecules on the surfaces of other cells.

Cellular immunity/cellular immune response: Cellular immunity relates typically to the activation of macrophages, natural killer cells (NK), antigen-specific cytotoxic T-lymphocytes, and the release of various cytokines in response to an antigen. In a more general way, cellular immunity is not related to antibodies but to the activation of cells of the immune system. A cellular immune response is characterized e.g. by activating antigen-specific cytotoxic T-lymphocytes that are able to induce apoptosis in body cells displaying epitopes of an antigen on their surface, such as virus-infected cells, cells with intracellular bacteria, and cancer cells displaying tumor antigens; activating macrophages and natural killer cells, enabling them to destroy pathogens; and stimulating cells to secrete a variety of cytokines that influence the function of other cells involved in adaptive immune responses and innate immune responses.

Humoral immunity/humoral immune response: Humoral immunity refers typically to antibody production and the accessory processes that may accompany it. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Innate immune system: The innate immune system, also known as non-specific immune system, comprises the cells and mechanisms that defend the host from infection by other organisms in a non-specific manner. This means that the cells of the innate system recognize and respond to pathogens in a generic way, but unlike the adaptive immune system, it does not confer long-lasting or protective immunity to the host. The innate immune system may be e.g. activated by ligands of pathogen-associated molecular patterns (PAMP) receptors, e.g. Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. Typically a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system through a process known as antigen presentation; and/or acting as a physical and chemical barrier to infectious agents.

Adjuvant/adjuvant component: An adjuvant or an adjuvant component in the broadest sense is typically a (e.g. pharmacological or immunological) agent or composition that may modify, e.g. enhance, the efficacy of other agents, such as a drug or vaccine. Conventionally the term refers in the context of the invention to a compound or composition that serves as a carrier or auxiliary substance for immunogens and/or other pharmaceutically active compounds. It is to be interpreted in a broad sense and refers to a broad spectrum of substances that are able to increase the immunogenicity of antigens incorporated into or co-administered with an adjuvant in question. In the context of the present invention an adjuvant will preferably enhance the specific immunogenic effect of the active agents of the present invention. Typically, "adjuvant" or "adjuvant component" has the same meaning and can be used mutually. Adjuvants may be divided, e.g., into immunopotentiators, antigenic delivery systems or even combinations thereof.

The term "adjuvant" is typically understood not to comprise agents which confer immunity by themselves. An adjuvant assists the immune system unspecifically to enhance the antigen-specific immune response by e.g. promoting presentation of an antigen to the immune system or induction of an unspecific innate immune response. Furthermore, an adjuvant may preferably e.g. modulate the antigen-specific immune response by e.g. shifting the dominating Th2-based antigen specific response to a more Th1-based antigen specific response or vice versa. Accordingly, an adjuvant may favorably modulate cytokine expression/secretion, antigen presentation, type of immune response etc.

Immunostimulatory RNA: An immunostimulatory RNA (isRNA) in the context of the invention may typically be an RNA that is able to induce an innate immune response itself. It usually does not have an open reading frame and thus does not provide a peptide-antigen or immunogen but elicits an innate immune response e.g. by binding to a specific kind of Toll-like-receptor (TLR) or other suitable receptors. However, of course also mRNAs having an open reading frame and coding for a peptide/protein (e.g. an antigenic function) may induce an innate immune response.

The term "antibody" as used herein, includes both an intact antibody and an antibody fragment. Typically, an intact "antibody" is an immunoglobulin that specifically binds to a particular antigen. An antibody may be a member of any immunoglobulin class, including any of the human classes: IgG, IgM, IgE, IgA and IgD. Typically, an intact antibody is a tetramer. Each tetramer consists of two identical pairs of polypeptide chains, each pair having a "light" chain and a "heavy" chain. An "antibody fragment" includes a portion of an intact antibody, such as the antigen-binding or variable region of an antibody. Examples of antibody fragments include Fab, Fab', F(ab') 2 and Fv fragments; the tribes; Tetra; linear antibodies; single-chain antibody molecules; and multi specific antibodies formed from antibody fragments. E.g., the antibody fragments comprise isolated fragments, "Fv" fragments consisting of heavy and light chain variable regions, recombinant single chain polypeptide molecules in which the light and heavy chain variable regions are linked together by a peptide linker ("ScFv Proteins") and minimal recognition units consisting of amino acid residues that mimic the hypervariable region. Examples of antigen-binding fragments of an antibody include, but are not limited to, Fab fragment, Fab' fragment, F (ab') 2 fragment, scFv fragment, Fv fragment, dsFv diabody, dAb fragment, fragment Fd', Fd fragment and an isolated complementarity determining region (CDR). Suitable antibodies that may be encoded by the therapeutic RNA of the invention include monoclonal antibodies, polyclonal antibodies, antibody mixtures or cocktails, human or humanized antibodies, chimeric antibodies, Fab fragments, or bispecific antibodies. In the context of the invention, an antibody may be provided by the at least one therapeutic RNA of the inventive combination/composition.

The term "antigen" in the context of the present invention refers typically to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells. In the sense of the present invention an antigen may be the product of translation of a provided nucleic acid molecule, preferably an mRNA as defined herein. In this context, also fragments, variants and derivatives of peptides and proteins comprising at least one epitope are understood as antigen. Accordingly, the term "antigen" as used herein will be recognized and understood by the person of ordinary skill in the art, and is e.g. intended to refer to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells. Also fragments, variants and derivatives of peptides or proteins derived from e.g. cancer antigens comprising at least one epitope may be understood as antigens. In the context of the present invention, an antigen may be the product of translation of a provided therapeutic RNA (e.g. coding RNA, replicon RNA, mRNA). The term "antigenic peptide or protein" will be recognized and understood by the person of ordinary skill in the art, and is e.g. intended to refer to a peptide or protein derived from a (antigenic) protein which may stimulate the body's adaptive immune system to provide an adaptive immune response. Therefore an "antigenic peptide or protein" comprises at least one epitope or antigen of the protein it is derived from (e.g. a tumor antigen, a viral antigen, a bacterial antigen, a protozoan antigen). In the context of the invention, an antigen may be provided by the at least one therapeutic RNA of the inventive combination/composition.

The term "derived from" as used throughout the present specification in the context of a nucleic acid, i.e. for a nucleic acid "derived from" (another) nucleic acid, means that the nucleic acid, which is derived from (another) nucleic acid, shares e.g. at least about 50%, 60%, 70%, 80%, 90%, 91%, 92%, 93%, 94%, 95%, 96%, 97%, 98%, or about 99% sequence identity with the nucleic acid from which it is derived. The skilled person is aware that sequence identity is typically calculated for the same types of nucleic acids, i.e. for DNA sequences or for RNA sequences. Thus, it is understood, if a DNA is "derived from" an RNA or if an RNA is "derived from" a DNA, in a first step the RNA sequence is converted into the corresponding DNA sequence (in particular by replacing U by T throughout the sequence) or, vice versa, the DNA sequence is converted into the corresponding RNA sequence (in particular by replacing the T by U throughout the sequence). Thereafter, the sequence identity of the DNA sequences or the sequence identity of the RNA sequences is determined. Preferably, a nucleic acid "derived from" a nucleic acid also refers to nucleic acid, which is modified in comparison to the nucleic acid from which it is derived, e.g. in order to increase RNA stability even further and/or to prolong and/or increase protein production. In the context of amino acid sequences, the term "derived from" means that the amino acid sequence, which is derived from (another) amino acid sequence, shares e.g. at least about 70%, 80%, 90%, 91%, 92%, 93%, 94%, 95%, 96%, 97%, 98%, or about 99% sequence identity with the amino acid sequence from which it is derived.

Epitope (also called "antigen determinant"): T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence.

These fragments are typically recognized by T cells in form of a complex consisting of the peptide fragment and an MHC molecule.

B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

A "tolerogenic composition" is a composition that promotes immune tolerance in cells or cellular systems to an antigen, wherein the antigen may be a self-antigen or a non-self antigen. In other words, there is no immune response or a reduced immune response to the antigen. Contrary thereto, a vaccine composition according to the present invention induces an immune response to a specific antigen, namely the antigen encoded by the at least one nucleic acid. The antigen may also be a self-antigen or a non-self antigen, and the overall aim of a vaccine composition of the present invention is to create a (strong) immune response to this antigen, wherein the overall aim of a tolerogenic composition is to at least partly, at best completely, suppress an immune response to this antigen.

A "tolerogenic nucleic acid" is a nucleic acid that promotes immune tolerance in cells or cellular systems to an antigen, wherein the nucleic acid may be a chemically modified mRNA and/or encode a tolerogenic polypeptide. Contrary thereto, the at least one nucleic acid according to the present invention encodes at least one antigen or fragment thereof, against which a (strong) immune response is desired and induced upon administration.

A "tolerogenic polypeptide" is a polypeptide that promotes immune tolerance in cells or cellular systems, typically by decreasing the immune response via acting on underlying pathways, in particular by inhibiting underlying mediators in such pathways. Thus, a tolerogenic polypeptide may be an inhibitor of mTOR, IL-2, IL-10 or an antibody reactive to CD3 or CD40. Contrary thereto, the at least one antigen or fragment thereof according to the present invention does not promote immune tolerance in cells or cellular systems but induces a (strong) immune response against itself.

A tolerogenic composition may in particular comprise a tolerogenic nucleic acid, wherein the tolerogenic nucleic acid promotes immune tolerance as described above. The tolerogenic composition may in addition comprise a specific antigen, with the result that there is no immune response to this specific antigen or that the immune response to this specific antigen is reduced due to the presence of the tolerogenic nucleic acid. Contrary thereto, the vaccine composition according to the present invention in a preferred embodiment does not comprise an antigen but of course still comprises the at least one nucleic acid encoding at least one antigen or fragment thereof, since it is the overall aim of the vaccine composition of the present invention to elucidate a (strong) immune response towards the encoded at least one antigen or fragment thereof (and not, as is the aim of the tolerogenic composition, to block or reduce an immune response towards the co-administered antigen). In yet another preferred embodiment, the vaccine composition according to the present invention comprises the at least one nucleic acid encoding at least one antigen or fragment thereof as the only payload, and therefore cannot comprise an antigen (as does the tolerogenic composition discussed in this paragraph in addition to the tolerogenic nucleic acid).

The term "vaccine" is typically understood to be a prophylactic or therapeutic material providing at least one antigen or antigenic function. The antigen or antigenic function may stimulate the body's adaptive immune system to provide an adaptive immune response.

The term "antigen-providing mRNA" in the context of the invention may typically be an mRNA, having at least one open reading frame that can be translated by a cell or an organism provided with that mRNA. The product of this translation is a peptide or protein that may act as an antigen, preferably as an immunogen. The product may also be a fusion protein composed of more than one immunogen, e.g. a fusion protein that consist of two or more epitopes, peptides or proteins derived from the same or different virus-proteins, wherein the epitopes, peptides or proteins may be linked by linker sequences.

The term "artificial mRNA" (sequence) may typically be understood to be an mRNA molecule, that does not occur naturally. In other words, an artificial mRNA molecule may be understood as a non-natural mRNA molecule. Such mRNA molecule may be non-natural due to its individual sequence (which does not occur naturally) and/or due to other modifications, e.g. structural modifications of nucleotides which do not occur naturally. Typically, artificial mRNA molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot.

In a very preferred embodiment, the nucleic acid of the invention is an "isolated" mRNA.

"Isolated": As used herein, the term "isolated", in regard to a nucleic acid molecule, preferably an isolated mRNA, or a polypeptide, means that the nucleic acid molecule, preferably isolated mRNA, or polypeptide is in a condition other than its native environment, such as apart from blood and/or animal tissue. In some embodiments, an isolated nucleic acid molecule, preferably isolated mRNA, or polypeptide is substantially free of other nucleic acid molecules or other polypeptides, particularly other nucleic acid molecules or polypeptides of animal origin. In some embodiments, the nucleic acid molecule, preferably isolated mRNA, or polypeptide can be in a highly purified form, i.e., greater than 95% pure or greater than 99% pure. When used in this context, the term "isolated" does not exclude the presence of the same nucleic acid molecule or polypeptide in alternative physical forms, such as dimers or alternatively phosphorylated or derivatized forms. Isolated substances may also have varying levels of purity in reference to the substances from which they have been associated. Isolated substances and/or entities may also be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components. In the context of the present invention, description and claims, the term "mRNA" preferably means an "isolated mRNA" and vice versa.

The terms "heterologous" or "heterologous sequence" as used throughout the present specification in the context of a nucleic acid sequence or an amino acid sequence refers to a sequence (e.g. DNA, RNA, amino acid) will be recognized and understood by the person of ordinary skill in the art, and is intended to refer to a sequence that is derived from another gene, from another allele, from another species. Two sequences are typically understood to be "heterologous" if they are not derivable from the same gene or in the same allele. I.e., although heterologous sequences may be derivable from the same organism, they naturally (in nature) do not occur in the same nucleic acid molecule, such as e.g. in the same RNA or protein.

Bi-/multicistronic mRNA: mRNA, that typically may have two (bicistronic) or more (multicistronic) open reading frames (ORF) (coding regions or coding sequences). An open reading frame in this context is a sequence of several nucleotide triplets (codons) that can be translated into a peptide or protein. Translation of such an mRNA yields two (bicistronic) or more (multicistronic) distinct translation products (provided the ORFs are not identical). For expression in eukaryotes such mRNAs may for example comprise an internal ribosomal entry site (IRES) sequence.

Monocistronic mRNA: A monocistronic mRNA may typically be an mRNA, that comprises only one open reading frame (coding sequence or coding region). An open reading frame in this context is a sequence of several nucleotide triplets (codons) that can be translated into a peptide or protein.

3'-untranslated region (3'-UTR): A 3'-UTR is typically the part of an mRNA which is located between the protein coding region (i.e. the open reading frame) and the poly(A) sequence of the mRNA. A 3'-UTR of the mRNA is not translated into an amino acid sequence. The 3'-UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'-capping, splicing the pre-mature mRNA to excise optional introns and modifications of the 3'-end, such as polyadenylation of the 3'-end of the pre-mature mRNA and optional endo- or exonuclease cleavages etc. In the context of the present invention, a 3'-UTR corresponds to the sequence of a mature mRNA which is located 3' to the stop codon of the protein coding region, preferably immediately 3' to the stop codon of the protein coding region, and which extends to the 5'-side of the poly(A) sequence, preferably to the nucleotide immediately 5' to the poly(A) sequence. The term "corresponds to" means that the 3'-UTR sequence may be an RNA sequence, such as in the mRNA sequence used for defining the 3'-UTR sequence, or a DNA sequence which corresponds to such RNA sequence. In the context of the present invention, the term "a 3'-UTR of a gene", such as "a 3'-UTR of an albumin gene", is the sequence which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence of the 3'-UTR.

5'-untranslated region (5'-UTR): A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites or a 5'-Terminal Oligopyrimidine Tract. The 5'-UTR may be post-transcriptionally modified, for example by addition of a 5'-CAP. In the context of the present invention, a 5'-UTR corresponds to the sequence of a mature mRNA which is located between the 5'-CAP and the start codon. Preferably, the 5'-UTR corresponds to the sequence which extends from a nucleotide located 3' to the 5'-CAP, preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence which corresponds to such RNA sequence. In the context of the present invention, the term "a 5'-UTR of a gene", such as "a 5'-UTR of a TOP gene", is the sequence which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence of the 5'-UTR.

5'-Terminal Oligopyrimidine Tract (TOP): The 5'-terminal oligopyrimidine tract (TOP) is typically a stretch of pyrimidine nucleotides located at the 5'-terminal region of a nucleic acid molecule, such as the 5'-terminal region of certain mRNA molecules or the 5'-terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5'-TOP ends one nucleotide 5' to the first purine nucleotide located downstream of the TOP. Messenger RNA that contains a 5'-terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'-TOP as defined above. Thus, a TOP motif in the context of the present invention is preferably a stretch of pyrimidine nucleotides having a length of 3-30 nucleotides. Preferably, the TOP motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'-end with a cytosine nucleotide. In TOP genes and TOP mRNAs, the TOP motif preferably starts at its 5'-end with the transcriptional start site and ends one nucleotide 5' to the first purine residue in said gene or mRNA. A TOP motif in the sense of the present invention is preferably located at the 5'-end of a sequence which represents a 5'-UTR or at the 5'-end of a sequence which codes for a 5'-UTR. Thus, preferably, a stretch of 3 or more pyrimidine nucleotides is called "TOP motif" in the sense of the present invention if this stretch is located at the 5'-end of a respective sequence, such as the inventive mRNA, the 5'-UTR element of the inventive mRNA, or the nucleic acid sequence which is derived from the 5'-UTR of a TOP gene as described herein. In other words, a stretch of 3 or more pyrimidine nucleotides which is not located at the 5'-end of a 5'-UTR or a 5'-UTR element but anywhere within a 5'-UTR or a 5'-UTR element is preferably not referred to as "TOP motif".

TOP gene: TOP genes are typically characterized by the presence of a 5'-terminal oligopyrimidine tract. Furthermore, most TOP genes are characterized by a growth-associated translational regulation. However, also TOP genes with a tissue specific translational regulation are known. As defined above, the 5'-UTR of a TOP gene corresponds to the sequence of a 5'-UTR of a mature mRNA derived from a TOP gene, which preferably extends from the nucleotide located 3' to the 5'-CAP to the nucleotide located 5' to the start codon. A 5'-UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'-UTRs of TOP genes are generally rather short. The lengths of 5'-UTRs of TOP genes may vary between 20 nucleotides up to 500 nucleotides, and are typically less than about 200 nucleotides, preferably less than about 150 nucleotides, more preferably less than about 100 nucleotides. Exemplary 5'-UTRs of TOP genes in the sense of the present invention are the nucleic acid sequences extending from the nucleotide at position 5 to the nucleotide located immediately 5' to the start codon (e.g. the ATG) in the sequences according to SEQ ID NO:1-1363, SEQ ID NO:1395, SEQ ID NO:1421 and SEQ ID NO:1422 of the international patent application WO2013143700 or homologs or variants thereof, whose disclosure is incorporated herewith by reference. In this context a particularly preferred fragment of a 5'-UTR of a TOP gene is a 5'-UTR of a TOP gene lacking the 5'-TOP motif. The term "5'-UTR of a TOP gene" preferably refers to the 5'-UTR of a naturally occurring TOP gene.

Fragment of a nucleic acid sequence, particularly an mRNA: A fragment of a nucleic acid sequence consists of a continuous stretch of nucleotides corresponding to a continuous stretch of nucleotides in the full-length nucleic acid sequence which is the basis for the nucleic acid sequence of the fragment, which represents at least 20%, preferably at least 30%, more preferably at least 40%, more preferably at least 50%, even more preferably at least 60%, even more preferably at least 70%, even more preferably at least 80%, and most preferably at least 90% of the full-length nucleic acid sequence. Such a fragment, in the sense of the present invention, is preferably a functional fragment of the full-length nucleic acid sequence.

In the context of the present invention, a "fragment" or a "variant" of a protein or peptide may have at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% amino acid identity over a stretch of at least 10, at least 20, at least 30, at least 50, at least 75 or at least 100 amino acids of such protein or peptide. More preferably, a "fragment" or a "variant" of a protein or peptide as used herein is at least 40%, preferably at least 50%, more preferably at least 60%, more preferably at least 70%, even more preferably at least 80%, even more preferably at least 90%, most preferably at least 95% identical to the protein or peptide, from which the variant is derived.

Variant of a nucleic acid sequence, particularly an mRNA: A variant of a nucleic acid sequence refers to a variant of nucleic acid sequences which forms the basis of a nucleic acid sequence. For example, a variant nucleic acid sequence may exhibit one or more nucleotide deletions, insertions, additions and/or substitutions compared to the nucleic acid sequence from which the variant is derived. Preferably, a variant of a nucleic acid sequence is at least 40%, preferably at least 50%, more preferably at least 60%, more preferably at least 70%, even more preferably at least 80%, even more preferably at least 90%, most preferably at least 95% identical to the nucleic acid sequence the variant is derived from. Preferably, the variant is a functional variant. A "variant" of a nucleic acid sequence may have at least 70%, 75%, 80%, 85%, 90%, 95%, 98% or 99% nucleotide identity over a stretch of 10, 20, 30, 50, 75 or 100 nucleotide of such nucleic acid sequence.

Stabilized nucleic acid, preferably mRNA: A stabilized nucleic acid, preferably mRNA typically, exhibits a modification increasing resistance to in vivo degradation (e.g. degradation by an exo- or endo-nuclease) and/or ex vivo degradation (e.g. by the manufacturing process prior to vaccine administration, e.g. in the course of the preparation of the vaccine solution to be administered). Stabilization of RNA can, e.g., be achieved by providing a 5'-CAP-Structure, a polyA-Tail, or any other UTR-modification. It can also be achieved by chemical modification or modification of the G/C content of the nucleic acid. Various other methods are known in the art and conceivable in the context of the invention.

RNA In vitro transcription: The terms "RNA in vitro transcription" or "in vitro transcription" relate to a process wherein RNA is synthesized in a cell-free system (in vitro). DNA, particularly plasmid DNA, is used as template for the generation of RNA transcripts. RNA may be obtained by DNA-dependent in vitro transcription of an appropriate DNA template, which according to the present invention is preferably a linearized plasmid DNA template. The promoter for controlling in vitro transcription can be any promoter for any DNA-dependent RNA polymerase. Particular examples of DNA-dependent RNA polymerases are the T7, T3, and SP6 RNA polymerases. A DNA template for in vitro RNA transcription may be obtained by cloning of a nucleic acid, in particular cDNA corresponding to the respective RNA to be in vitro transcribed, and introducing it into an appropriate vector for in vitro transcription, for example into plasmid DNA. In a preferred embodiment of the present invention the DNA template is linearized with a suitable restriction enzyme, before it is transcribed in vitro. The cDNA may be obtained by reverse transcription of mRNA or chemical synthesis. Moreover, the DNA template for in vitro RNA synthesis may also be obtained by gene synthesis.

Methods for in vitro transcription are known in the art (see, e.g., Geall et al. (2013) Semin. Immunol. 25(2): 152-159; Brunelle et al. (2013) Methods Enzymol. 530:101-14). Reagents used in said method typically include:
1) a linearized DNA template with a promoter sequence that has a high binding affinity for its respective RNA polymerase such as bacteriophage-encoded RNA polymerases;
2) ribonucleoside triphosphates (NTPs) for the four bases (adenine, cytosine, guanine and uracil);
3) optionally, a CAP analogue as defined above (e.g. m7G(5')ppp(5')G (m7G));
4) a DNA-dependent RNA polymerase capable of binding to the promoter sequence within the linearized DNA template (e.g. T7, T3 or SP6 RNA polymerase);
5) optionally, a ribonuclease (RNase) inhibitor to inactivate any contaminating RNase;
6) optionally, a pyrophosphatase to degrade pyrophosphate, which may inhibit transcription;
7) MgCl₂, which supplies Mg²⁺ ions as a co-factor for the polymerase;
8) a buffer to maintain a suitable pH value, which can also contain antioxidants (e.g. DTT), and/or polyamines such as spermidine at optimal concentrations.

Full-length protein: The term "full-length protein" as used herein typically refers to a protein that substantially comprises the entire amino acid sequence of the naturally occurring protein. Nevertheless, substitutions of amino acids e.g. due to mutation in the protein are also encompassed in the term full-length protein.

Fragments of proteins: "Fragments" of proteins or peptides in the context of the present invention may, typically, comprise a sequence of a protein or peptide as defined herein, which is, with regard to its amino acid sequence (or its encoded nucleic acid molecule), N-terminally and/or C-terminally truncated compared to the amino acid sequence of the original (native) protein (or its encoded nucleic acid molecule). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid molecule of such a protein or peptide.

The term "variant" in the context of nucleic acid sequences of genes refers to nucleic acid sequence variants, i.e. nucleic acid sequences or genes comprising a nucleic acid sequence that differs in at least one nucleic acid from a reference (or "parent") nucleic acid sequence of a reference (or "parent") nucleic acid or gene. Variant nucleic acids or genes may thus preferably comprise, in their nucleic acid sequence, at least one mutation, substitution, insertion or deletion as compared to their respective reference sequence. Preferably, the term "variant" as used herein includes naturally occurring variants, and engineered variants of nucleic acid sequences or genes. Therefore, a "variant" as defined herein can be derived from, isolated from, related to, based on or homologous to the reference nucleic acid sequence. "Variants" may preferably have a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, to a nucleic acid sequence of the respective naturally occurring (wild-type) nucleic acid sequence or gene, or a homolog, fragment or derivative thereof.

Also, the term "variant" as used throughout the present specification in the context of proteins or peptides will be recognized and understood by the person of ordinary skill in the art, and is e.g. intended to refer to a proteins or peptide variant having an amino acid sequence which differs from the original sequence in one or more mutation(s), such as one or more substituted, inserted and/or deleted amino acid(s). Preferably, these fragments and/or variants have the same biological function or specific activity compared to the full-length native protein, e.g. its specific antigenic property. "Variants" of proteins or peptides as defined herein may comprise conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, e.g., an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra). A "variant" of a protein or peptide may have at least 70%, 75%, 80%, 85%, 90%, 95%, 98% or 99% amino acid identity over a stretch of at least 10, 20, 30, 50, 75 or 100 amino acids of such protein or peptide. Preferably, a variant of a protein comprises a functional variant of the protein, which means that the variant exerts the same effect or functionality or at least 40%, 50%, 60%, 70%, 80%, 90%, or 95% of the effect or functionality as the protein it is derived from.

Also, the term "fragment" in the context of nucleic acid sequences or genes refers to a continuous subsequence of the full-length reference (or "parent") nucleic acid sequence or gene. In other words, a "fragment" may typically be a shorter portion of a full-length nucleic acid sequence or gene. Accordingly, a fragment, typically, consists of a sequence that is identical to the corresponding stretch within the full-length nucleic acid sequence or gene. The term includes naturally occurring fragments as well as engineered fragments. A preferred fragment of a sequence in the context of the present invention, consists of a continuous stretch of nucleic acids corresponding to a continuous stretch of entities in the nucleic acid or gene the fragment is derived from, which represents at least 20%, preferably at least 30%, more preferably at least 40%, more preferably at least 50%, even more preferably at least 60%, even more preferably at least 70%, and most preferably at least 80% of the total (i.e. full-length) nucleic acid sequence or gene from which the fragment is derived. A sequence identity indicated with respect to such a fragment preferably refers to the entire nucleic acid sequence or gene. Preferably, a "fragment" may comprise a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, to a reference nucleic acid sequence or gene that it is derived from.

Also, in this context a fragment of a protein may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective naturally occurring full-length protein.

The term "identity" as used throughout the present specification in the context of a nucleic acid sequence or an amino acid sequence will be recognized and understood by the person of ordinary skill in the art, and is e.g. intended to refer to the percentage to which two sequences are identical. To determine the percentage to which two sequences are identical, e.g. nucleic acid sequences or amino acid (aa) sequences as defined herein, preferably the aa sequences encoded by the nucleic acid sequence as defined herein or the aa sequences themselves, the sequences can be aligned in order to be subsequently compared to one another. Therefore, e.g. a position of a first sequence may be compared with the corresponding position of the second sequence. If a position in the first sequence is occupied by the same residue as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using an algorithm, e.g. an algorithm integrated in the BLAST program.

Fragments of proteins or peptides in the context of the present invention may furthermore comprise a sequence of a protein or peptide as defined herein, which has a length of for example at least 5 amino acids, preferably a length of at least 6 amino acids, preferably at least 7 amino acids, more preferably at least 8 amino acids, even more preferably at least 9 amino acids; even more preferably at least 10 amino acids; even more preferably at least 11 amino acids; even more preferably at least 12 amino acids; even more preferably at least 13 amino acids; even more preferably at least 14 amino acids; even more preferably at least 15 amino acids; even more preferably at least 16 amino acids; even more preferably at least 17 amino acids; even more preferably at least 18 amino acids; even more preferably at least 19 amino acids; even more preferably at least 20 amino acids; even more preferably at least 25 amino acids; even more preferably at least 30 amino acids; even more preferably at least 35 amino acids; even more preferably at least 50 amino acids; or most preferably at least 100 amino acids. For example such fragment may have a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form. Fragments of proteins or peptides may comprise at least one epitope of those proteins or peptides. Furthermore also domains of a protein, like the extracellular domain, the intracellular domain or the transmembrane domain and shortened or truncated versions of a protein may be understood to comprise a fragment of a protein.

Variants of proteins: "Variants" of proteins or peptides as defined in the context of the present invention may be generated, having an amino acid sequence which differs from the original sequence in one or more mutation(s), such as one or more substituted, inserted and/or deleted amino acid(s). Preferably, these fragments and/or variants have the same biological function or specific activity compared to the full-length native protein, e.g. its specific antigenic property. "Variants" of proteins or peptides as defined in the context of the present invention may comprise conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

A "variant" of a protein or peptide may have at least 70%, 75%, 80%, 85%, 90%, 95%, 98% or 99% amino acid identity over a stretch of 10, 20, 30, 50, 75 or 100 amino acids of such protein or peptide.

Furthermore, variants of proteins or peptides as defined herein, which may be encoded by a nucleic acid molecule, may also comprise those sequences, wherein nucleotides of the encoding nucleic acid sequence are exchanged according to the degeneration of the genetic code, without leading to an alteration of the respective amino acid sequence of the protein or peptide, i.e. the amino acid sequence or at least part thereof may not differ from the original sequence in one or more mutation(s) within the above meaning.

Identity of a sequence: In order to determine the percentage to which two sequences are identical, e.g. nucleic acid sequences or amino acid sequences as defined herein, preferably the amino acid sequences encoded by a nucleic acid sequence of the polymeric carrier as defined herein or the amino acid sequences themselves, the sequences can be aligned in order to be subsequently compared to one another. Therefore, e.g. a position of a first sequence may be compared with the corresponding position of the second sequence. If a position in the first sequence is occupied by the same component (residue) as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program.

Derivative of a protein or peptide: A derivative of a peptide or protein is typically understood to be a molecule that is derived from another molecule, such as said peptide or protein. A "derivative" of a peptide or protein also encompasses fusions comprising a peptide or protein used in the present invention. For example, the fusion comprises a label, such as, for example, an epitope, e.g., a FLAG epitope or a V5 epitope. For example, the epitope is a FLAG epitope. Such a tag is useful for, for example, purifying the fusion protein.

Pharmaceutically effective amount: A pharmaceutically effective amount in the context of the invention is typically understood to be an amount that is sufficient to induce an immune response.

Carrier: A carrier in the context of the invention may typically be a compound that facilitates transport and/or complexation of another compound. Said carrier may form a complex with said other compound. A polymeric carrier is a carrier that is formed of a polymer.

Vehicle: An agent, e.g. a carrier that may typically be used within a pharmaceutical composition or vaccine for facilitating administering of the components of the pharmaceutical composition or vaccine to an individual.

### BRIEF DESCRIPTION OF THE DRAWINGS

The figures shown in the following are merely illustrative and shall describe the present invention in a further way. These figures shall not be construed to limit the present invention thereto.

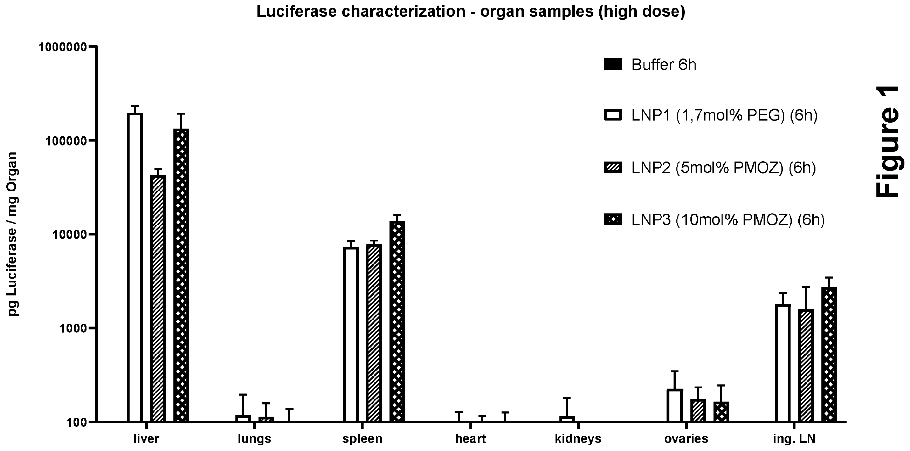

**Figure 1**: shows an organ distribution profile. The organ distribution profile can be regarded as generally similar when comparing GN01-based PEG-LNPs and PMOZ-LNPs (DMPE-PMOZ-v1). LNPs comprising a PMOZ-lipid as polymer conjugated lipid surprisingly did not have a negative effect on the efficacy of the LNPs, even a positive effect is apparent for spleen and lymph nodes luciferase values. As shown herein above, the inventors surprisingly found that PMOZ-LNPs performed very well when compared to standard PEG-LNPs. The full details can be found in **Example 4.**

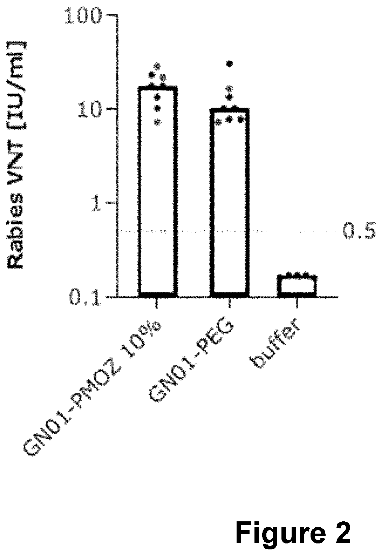

**Figure 2**: shows that already a single i.m. immunization with only 1 µg of mRNA formulated in PMOZ-LNPs (DMPE-PMOZ-v1) induced very robust VNTs well above the protective titer of 0.5 IU/ml in all animals. PMOZ-LNPs performed very well when compared to the standard controls, i.e. for all LNPs very high levels of Rabies VNTs were measured. The full details can be found in **Example 5.**

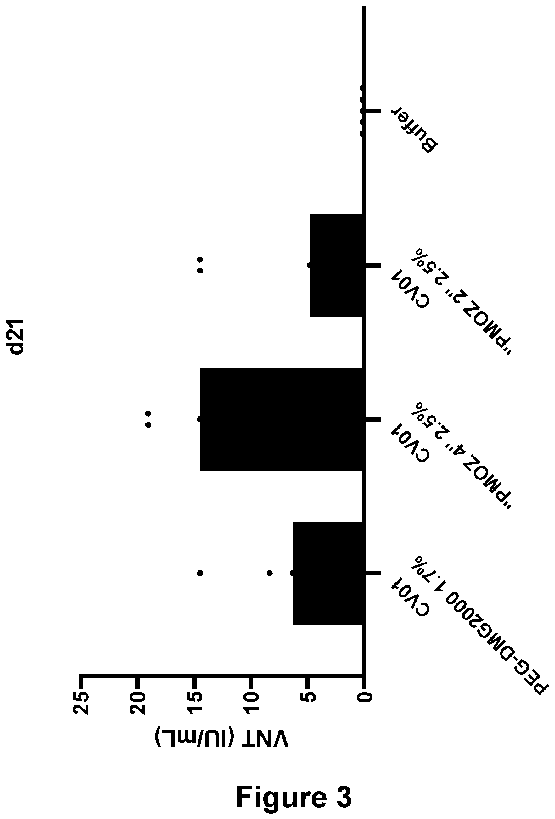

**Figure 3**: shows that already a single i.m. immunization with only 1 µg of mRNA formulated in PMOZ-LNPs induced very robust VNTs well above the protective titer of 0.5 IU/ml in all animals. PMOZ-LNPs performed very well when compared to the standard controls, i.e. for all LNPs very high levels of Rabies VNTs were measured. The full details can be found in **Example 7.**

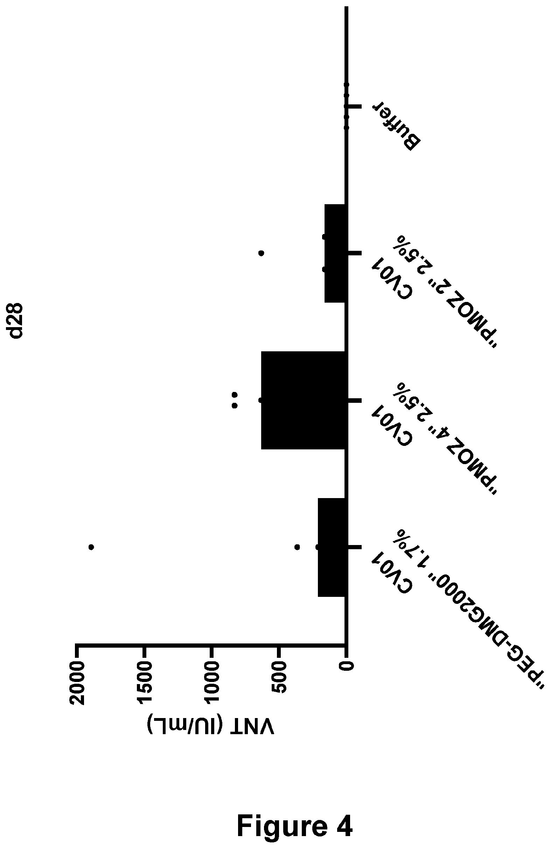

**Figure 4****:** shows that i.m. immunization with only 1 µg of mRNA formulated in PMOZ-LNPs induced very high VNTs well above the protective titer of 0.5 IU/ml in all animals after boost vaccination (day 28). PMOZ-LNPs performed very well when compared to the standard controls, i.e. for all LNPs very high levels of Rabies VNTs were measured. The full details can be found in **Example 7.**

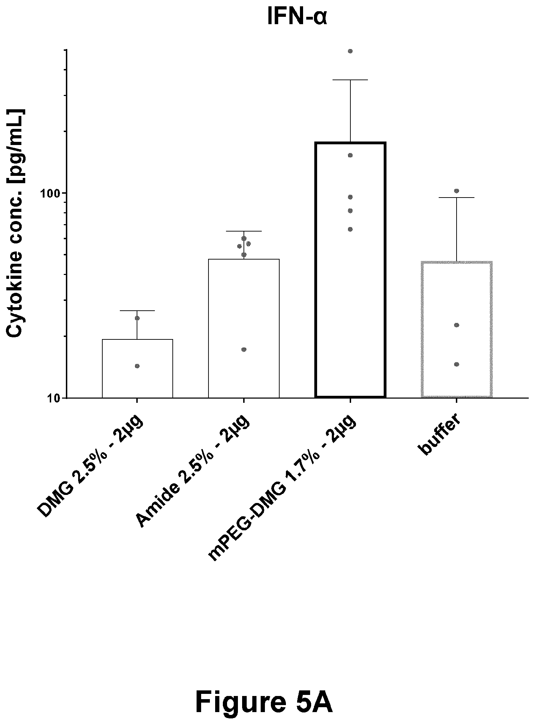

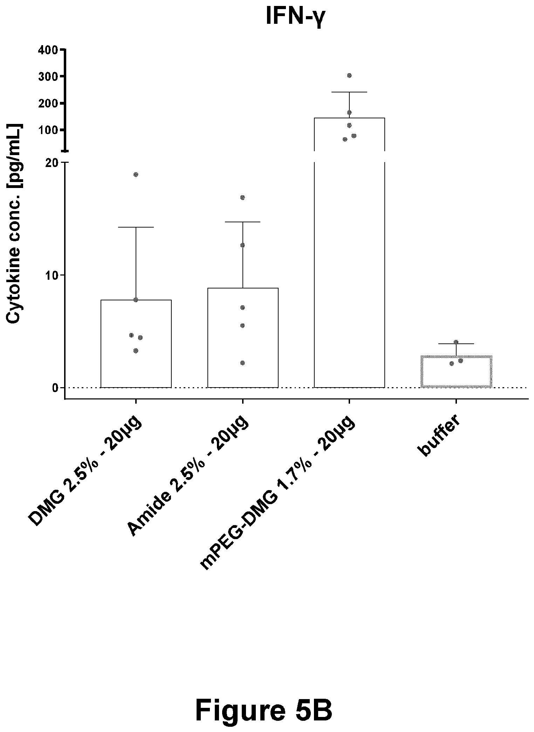

**Figures 5A** **and** **5B**: show that i.v. administration with 2 µg PpLuc mRNA (Figure 5A) or 20 µg PpLuc mRNA (Figure 5B) of mRNA formulated in PMOZ-LNPs reduced cytokine levels as when compared to PEG-LNPs. The full details can be found in **Example 8.**

### DETAILED DESCRIPTION OF THE INVENTION

Although the present disclosure is described in detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

### New polymer conjugated lipids

In a first aspect, the invention provides a polymer conjugated lipid that is defined as a compound according to formula (I):

[P]-[linker]-[L] formula (I)

wherein
- [P]: is a heteropolymer moiety or homopolymer moiety, preferably a homopolymer moiety, comprising at least one polyoxazoline (POZ) monomer unit 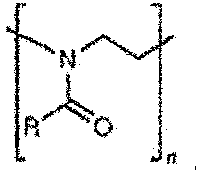 wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa
- [linker]: is an optional linker group, and
- [L]: is a lipid moiety.

R in [P] of formula (I) preferably is C1 (methyl), leading to a PMOZ unit.

The present invention is based on the inventors' surprising finding that the use of novel polymer conjugated lipids comprising polyoxazoline (POZ) according to formula (I), [P] preferably comprising
poly(2-methyl-2-oxazoline) (PMOZ),
poly(2-ethyl-2-oxazoline) (PEOZ),
poly(2-propyl-2-oxazoline) (PPOZ),
poly(2-butyl-2-oxazoline) (PBOZ),
poly(2-isopropyl-2-oxazoline) (PIPOZ),
poly(2-methoxymethyl-2-oxazoline) (PMeOMeOx), or
poly(2-dimethylamino-2-oxazoline) (PDMAOx)
and/or lipid nanoparticles (LNPs) comprising these new polymer conjugated lipids are highly effective in delivering nucleic acids such as mRNA to a living organism such as a human individual. This has enabled the inventors to create, for example, improved vaccines that deliver mRNA compounds encoding antigenic peptides or proteins and very efficiently induce antigen-specific immune responses at very low doses and to avoid the disadvantages accompanied by use of PEG. The present disclosure addresses these and other needs. Further advantages achieved by the present invention are that quite surprisingly, the inventors have discovered, according to aspects and embodiments of the invention a class of formulations for delivering mRNA vaccines in vivo that results in significantly enhanced, and in many respects synergistic, immune responses including enhanced antigen generation and functional antibody production with neutralization capability. These results can be achieved even when significantly lower doses of the mRNA are administered in comparison with mRNA doses used in other classes of lipid-based formulations. The formulations of the invention have demonstrated significant unexpected in vivo immune responses sufficient to establish the efficacy of functional mRNA vaccines as prophylactic and therapeutic agents. Summarized, it could surprisingly be shown by the inventors of the present invention, that several different polymer conjugated lipids according to formula (I), e.g. PMOZ-lipids, could be used for substituting standard PEG-lipids, yielding in LNPs with comparable or even enhanced performance. This unexpected finding could be validated by using several different LNP compositions, i.e. the inventors surprisingly found, that polymer conjugated lipid according to formula (I) were able to clearly enhance state of the art LNP-compositions.

Thusly, the invention is directed to a composition comprising a polymer conjugated lipid according to formula (I), preferably a POZ-lipid according to formula (I), more preferably a PMOZ-lipid, as described herein below. All options and preferences that are disclosed for polymer conjugated lipid according to formula (I), preferably a POZ-lipid, more preferably a PMOZ-lipid, as such are also applicable to the composition to this aspect of the invention. In other words, the specifically disclosed embodiments of polymer conjugated lipids, preferably POZ-lipids, more preferably PMOZ-lipids, and in particular the preferred PMOZ-lipid DMG-PMOZ, should be understood as also defining specific preferred embodiments of the composition according to the invention, i.e. compositions that are characterized in that they comprise a PMOZ lipid according to one of the specific selections described herein. In other words, the term "polymer conjugated lipid" refers to a molecule comprising both a lipid portion and a polymer portion. Preferably, the polymer conjugated lipid according to formula (I) is a POZ-lipid, more preferably a PMOZ-lipid. The terms "POZ-lipid" or "PMOZ-lipid" thusly refer to a molecule comprising both a lipid portion and a POZ or respectively a PMOZ portion. Thusly, a "PMOZ-lipid" is to be understood as a lipid comprising at least one homopolymer moiety comprising at least one polyoxazoline (POZ) unit, i.e. preferably a PMOZ-unit.

The composition may comprise further active and/or inactive excipients which are described further below. In one specific embodiment, in addition to the polymer conjugated lipid according to formula (I), preferably a PMOZ-lipid, the composition comprises one or more lipids selected from the group consisting of: (a) a steroid; (b) a neutral lipid; and (c) a cationic lipid.

In another embodiment, [P] is a heteropolymer moiety or homopolymer moiety comprising multiple monomer units selected from the group consisting of
poly(2-methyl-2-oxazoline) (PMOZ) 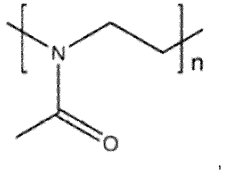
poly(2-ethyl-2-oxazoline) (PEOZ) 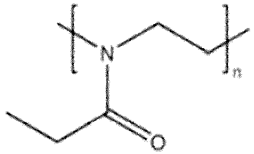
poly(2-propyl-2-oxazoline) (PPOZ) 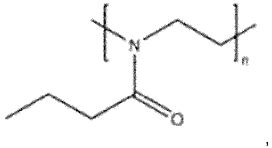
poly(2-butyl-2-oxazoline) (PBOZ) 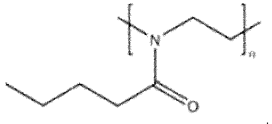
poly(2-isopropyl-2-oxazoline) (PIPOZ) 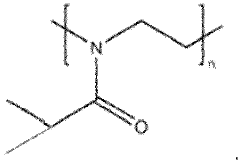
poly(2-methoxymethyl-2-oxazoline) (PMeOMeOx), and
poly(2-dimethylamino-2-oxazoline) (PDMAOx),

preferably wherein [P] is a homopolymer moiety comprising multiple PMOZ or PEOZ monomer units, more
preferably wherein [P] comprises or preferably consists of multiple PMOZ monomer units,
wherein
   (i) n has a mean value ranging from about 45 to about 55, preferably n is about 50 or wherein
   (ii) n is selected such that the [P] moiety has an average molecular weight of about 4.2 kDa to about 4.4 kDa, or most preferably about 4.3 kDa.

In another embodiment, [P] is a heteropolymer moiety or homopolymer moiety comprising multiple monomer units selected from the group consisting of

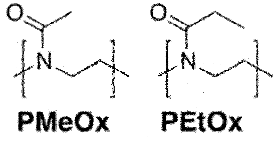

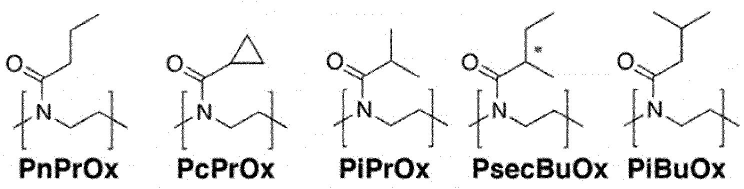

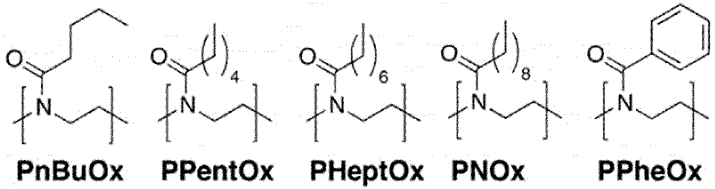

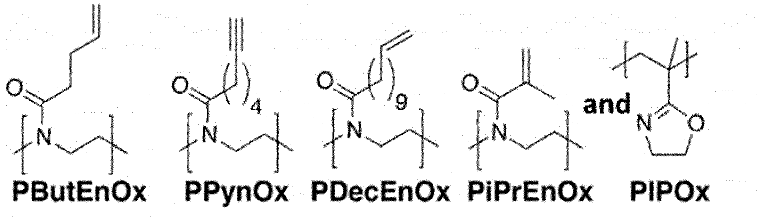

In yet another embodiment, the [P] from the polymer conjugated lipid according to formula (I) is selected from the group consisting of poly(2-methoxymethyl-2-oxazoline) (PMeOMeOx) and poly(2-dimethylamino-2-oxazoline) (PDMAOx).

In yet a further embodiment, the polymer conjugated lipid according to formula (I) is selected from the group consisting of a POZ-monoacylglycerol conjugate, POZ-diacylglycerol conjugate, a POZ-dialkyloxypropyl conjugate, a POZ-steroid or POZ-sterol conjugate, a POZ-phospholipid conjugate, a POZ-ceramide conjugate, and a mixture thereof.

In a preferred embodiment, the polymer conjugated lipid comprises a moiety based on 1,2-Dimyristoyl-rac-glycerol (DMG)

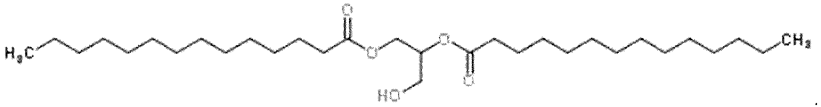

Preferably, the polymer conjugated lipid is "DMG-PMOZ", DMG-PMOZ being 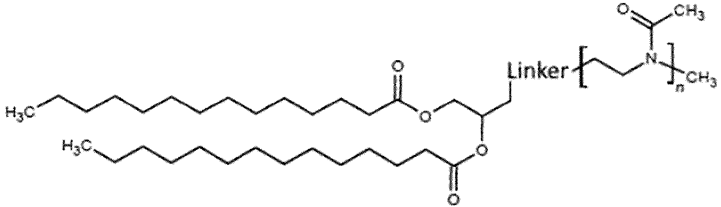 more preferably "PMOZ 2", with n having a mean value from 45 to 50, most preferably 50.

Further most preferred embodiments for PMOZ [P] moieties (polymethyloxazoline):
For PMOZ, the preferred average molecular mass of the [P] moiety is about 3.8 kDa to about 4.8 kDa, about 3.9 kDa to about 4.7 kDa, about 4 kDa to about 4.6 kDa, about 4.1 kDa to about 4.5 kDa, about 4.2 kDa to about 4.4 kDa, or most preferably about 4.3 kDa. Other preferred average molecular masses of the [P] moiety are (i) about 3.9 kDa to about 4.4 kDa, about 3.9 kDa to about 4.1 kDa, or about 4.2 kDa to about 4.4 kDa.
In further preferred embodiments, the average molecular mass of the [P] moiety is above 4.3 kDa. In other preferred embodiments, the preferred average molecular mass of the [P] moiety is about 4.25 kDa to about 4.675 kDa, about 4.675 kDa to about 5.1 kDa, about 5.1 kDa to about 5.525 kDa, about 5.525 kDa to about 5.95 kDa, about 5.95 kDa to about 6.375 kDa, about 6.375 kDa to about 6.8 kDa, or above 6.8 kDa.
In other preferred embodiments, for PMOZ according to 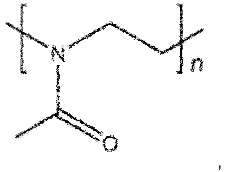
n has a mean value ranging from about 40 to about 80, preferably from about 45 to about 70, more preferably from about 50 to about 60, or most preferably n having a mean value of about 50.
In further preferred embodiments for PMOZ, n has a mean value of more than 50. In other preferred embodiments, n has a mean value of about 55, about 60, about 65, about 70, about 75 or about 80.
Thus the PMOZ moiety preferably is a PMOZ moiety having a molecular mass of about 4.3 kDa, although also shorter and longer moieties can also be used.

Further most preferred embodiments for PEOZ [P] moieties (polyethyloxazoline):
For PEOZ, the preferred average molecular mass of the [P] moiety is about 4.5 kDa to about 5.5 kDa, about 4.6 kDa to about 5.4 kDa, about 4.7 kDa to about 5.3 kDa, about 4.8 kDa to about 5.2 kDa, about 4.9 kDa to about 5.1 kDa, or most preferably about 5 kDa.
In further preferred embodiments, the average molecular mass of the [P] moiety is above 5 kDa. In other preferred embodiments, the preferred average molecular mass of the [P] moiety is about 4.95 kDa to about 5.445 kDa, about 5.445 kDa to about 5.94 kDa, about 5.94 kDa to about 6.435 kDa, about 6.435 kDa to about 6.93 kDa, about 6.93 kDa to about 7.425 kDa, about 7.425 kDa to about 7.92 kDa, or above 7.92 kDa.
In other preferred embodiments, for PEOZ according to 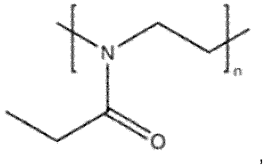
n has a mean value ranging from about 40 to about 80, preferably from about 45 to about 70, more preferably from about 50 to about 60, or most preferably n having a mean value of about 50.
In further preferred embodiments for PEOZ, n has a mean value of more than 50. In other preferred embodiments, n has a mean value of about 55, about 60, about 65, about 70, about 75 or about 80.
Thus the PEOZ moiety preferably is a PEOZ moiety having a molecular mass of about 5 kDa, although also shorter and longer moieties can also be used.

Further most preferred embodiments for PPOZ [P] moieties (polypropyloxazoline) or PIPOZ [P] moieties (poly-2-isopropyl-2-oxazoline)):
For PPOZ or equally PIPOZ, the preferred average molecular mass of the [P] moiety is about 5.2 kDa to about 6.2 kDa, about 5.3 kDa to about 6.1 kDa, about 5.4 kDa to about 6 kDa, about 5.5 kDa to about 5.9 kDa, about 5.6 kDa to about 5.8 kDa, or most preferably about 5.7 kDa.
In further preferred embodiments, the average molecular mass of the [P] moiety is above 5.7 kDa. In other preferred embodiments, the preferred average molecular mass of the [P] moiety is about 5.65 kDa to about 6.215 kDa, about 6.215 kDa to about 6.78 kDa, about 6.78 kDa to about 7.345 kDa, about 7.345 kDa to about 7.91 kDa, about 7.91 kDa to about 8.475 kDa, about 8.475 kDa to about 9.04 kDa, or above 9.04 kDa.
In other preferred embodiments, for PPOZ according to 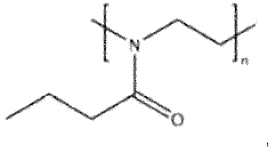
or equally PIPOZ (poly(2-isopropyl-2-oxazoline)), n has a mean value ranging from about 40 to about 80, preferably from about 45 to about 70, more preferably from about 50 to about 60, or most preferably n having a mean value of about 50.
In further preferred embodiments for PPOZ or equally PIPOZ, n has a mean value of more than 50. In other preferred embodiments, n has a mean value of about 55, about 60, about 65, about 70, about 75 or about 80.
Thus the PPOZ or equally PIPOZ moiety preferably is a PPOZ or equally PIPOZ moiety having a molecular mass of about 5,7 kDa, although also shorter and longer moieties can also be used.

In other preferred embodiments, "n" from the [P] moiety for the novel polymer conjugated lipids is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or 100 preferably 25, further preferably 50. In further preferred embodiments, "n" from the monomeric compound of [P] is selected such that the [P] moiety has an average molecular weight of 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, or 8 kDa, preferably 2,5 kDa, further preferably 5 kDa.

In further certain preferred embodiments, n is selected for the novel polymer conjugated lipids such that the [P] moiety has an average molecular weight of about 2 kDa; 2.1 kDa; 2.2 kDa; 2.3kDa; 2.4 kDa; 2.5 kDa; 2.6 kDa; 2.7 kDa; 2.8 kDa; 2.9 kDa; 3 kDa; 3.1 kDa; 3.2 kDa; 3.3 kDa; 3.4 kDa; 3.5 kDa; 3.6 kDa; 3.7 kDa; 3.8 kDa; 3.9 kDa; 4 kDa; 4.1 kDa; 4.2 kDa; 4.3 kDa; 4.4 kDa; 4.5 kDa; 4.6 kDa; 4.7 kDa; 4.8 kDa; 4.9 kDa; 5 kDa; 5.1 kDa; 5.2 kDa; 5.3 kDa; 5.4 kDa; 5.5 kDa; 5.6 kDa; 5.7 kDa; 5.8 kDa; 5.9 kDa; 6 kDa; 6.1 kDa; 6.2 kDa; 6.3 kDa; 6.4 kDa; 6.5 kDa; 6.6 kDa; 6.7 kDa; 6.8 kDa; 6.9 kDa; 7 kDa; 7.1 kDa; 7.2 kDa; 7.3 kDa; 7.4 kDa; 7.5 kDa; 7.6 kDa; 7.7 kDa; 7.8 kDa; 7.9 kDa; 8 kDa; 8.1 kDa; 8.2 kDa; 8.3 kDa; 8.4 kDa; 8.5 kDa; 8.6 kDa; 8.7 kDa; 8.8 kDa; 8.9 kDa; 9 kDa; 9.1 kDa; 9.2 kDa; 9.3 kDa; 9.4 kDa; 9.5 kDa; 9.6 kDa; 9.7 kDa; 9.8 kDa; 9.9 kDa; 10 kDa; 10.1 kDa; 10.2 kDa; 10.3 kDa; 10.4 kDa; 10.5 kDa; 10.6 kDa; 10.7 kDa; 10.8 kDa; 10.9 kDa; 11 kDa; 11.1 kDa; 11.2 kDa; 11.3 kDa; 11.4 kDa; 11.5 kDa; 11.6 kDa; 11.7 kDa; 11.8 kDa; 11.9 kDa; 12 kDa or above 12 kDa.

In even further preferred embodiments, the polymer conjugated lipid comprises as [P] a poly(2-methyl-2-oxazoline) (PMOZ) moiety, in which n is selected such that the [P] moiety has an average molecular weight of about 2 kDa; 2.1 kDa; 2.2 kDa; 2.3kDa; 2.4 kDa; 2.5 kDa; 2.6 kDa; 2.7 kDa; 2.8 kDa; 2.9 kDa; 3 kDa; 3.1 kDa; 3.2 kDa; 3.3 kDa; 3.4 kDa; 3.5 kDa; 3.6 kDa; 3.7 kDa; 3.8 kDa; 3.9 kDa; 4 kDa; 4.1 kDa; 4.2 kDa; 4.3 kDa; 4.4 kDa; 4.5 kDa; 4.6 kDa; 4.7 kDa; 4.8 kDa; 4.9 kDa; 5 kDa; 5.1 kDa; 5.2 kDa; 5.3 kDa; 5.4 kDa; 5.5 kDa; 5.6 kDa; 5.7 kDa; 5.8 kDa; 5.9 kDa; 6 kDa; 6.1 kDa; 6.2 kDa; 6.3 kDa; 6.4 kDa; 6.5 kDa; 6.6 kDa; 6.7 kDa; 6.8 kDa; 6.9 kDa; 7 kDa; 7.1 kDa; 7.2 kDa; 7.3 kDa; 7.4 kDa; 7.5 kDa; 7.6 kDa; 7.7 kDa; 7.8 kDa; 7.9 kDa; 8 kDa; 8.1 kDa; 8.2 kDa; 8.3 kDa; 8.4 kDa; 8.5 kDa; 8.6 kDa; 8.7 kDa; 8.8 kDa; 8.9 kDa; 9 kDa; 9.1 kDa; 9.2 kDa; 9.3 kDa; 9.4 kDa; 9.5 kDa; 9.6 kDa; 9.7 kDa; 9.8 kDa; 9.9 kDa; 10 kDa; 10.1 kDa; 10.2 kDa; 10.3 kDa; 10.4 kDa; 10.5 kDa; 10.6 kDa; 10.7 kDa; 10.8 kDa; 10.9 kDa; 11 kDa; 11.1 kDa; 11.2 kDa; 11.3 kDa; 11.4 kDa; 11.5 kDa; 11.6 kDa; 11.7 kDa; 11.8 kDa; 11.9 kDa; 12 kDa or above 12 kDa.

In further preferred embodiments, the polymer conjugated lipid comprises as [P] a polyethyloxazoline (PEOZ) moiety, in which n is selected in increasing order of preference from the group consisting of
- n having a mean value ranging from about 40 to about 60;
- n having a mean value ranging from about 45 to about 55;
- n having a mean value ranging from about 46 to about 54;
- n having a mean value ranging from about 47 to about 53;
- n having a mean value ranging from about 48 to about 52;
- n having a mean value ranging from about 49 to about 51;
- n having is about 50; and
- n = 50.

In even further most preferred embodiments, the polymer conjugated lipid comprises as [P] a poly(2-methyl-2-oxazoline) (PMOZ) moiety, in which n is selected in increasing order of preference from the group consisting of
- n having a mean value ranging from about 40 to about 60;
- n having a mean value ranging from about 45 to about 55;
- n having a mean value ranging from about 46 to about 54;
- n having a mean value ranging from about 47 to about 53;
- n having a mean value ranging from about 48 to about 52;
- n having a mean value ranging from about 49 to about 51;
- n having is about 50; and
- n = 50.

Further very preferred embodiments for PMOZ [P] moieties (polymethyloxazoline):
For PMOZ, the preferred average molecular mass of the [P] moiety is about 3.8 kDa to about 4.8 kDa, about 3.9 kDa to about 4.7 kDa, about 4 kDa to about 4.6 kDa, about 4.1 kDa to about 4.5 kDa, about 4.2 kDa to about 4.4 kDa, or most preferably about 4.3 kDa.
In further preferred embodiments, the average molecular mass of the [P] moiety is above 4.3 kDa. In other preferred embodiments, the preferred average molecular mass of the [P] moiety is about 4.25 kDa to about 4.675 kDa, about 4.675 kDa to about 5.1 kDa, about 5.1 kDa to about 5.525 kDa, about 5.525 kDa to about 5.95 kDa, about 5.95 kDa to about 6.375 kDa, about 6.375 kDa to about 6.8 kDa, or above 6.8 kDa.
In other preferred embodiments, for PMOZ according to 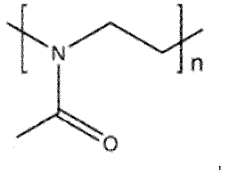
n has a mean value ranging from about 40 to about 80, preferably from about 45 to about 70, more preferably from about 50 to about 60, or most preferably n having a mean value of about 50.
In further preferred embodiments for PMOZ, n has a mean value of more than 50. In other preferred embodiments, n has a mean value of about 55, about 60, about 65, about 70, about 75 or about 80.
Thus the PMOZ moiety preferably is a PMOZ moiety having a molecular mass of about 4.3 kDa, although also shorter and longer moieties can also be used.

Further very preferred embodiments for PEOZ [P] moieties (polyethyloxazoline):
For PEOZ, the preferred average molecular mass of the [P] moiety is about 4.5 kDa to about 5.5 kDa, about 4.6 kDa to about 5.4 kDa, about 4.7 kDa to about 5.3 kDa, about 4.8 kDa to about 5.2 kDa, about 4.9 kDa to about 5.1 kDa, or most preferably about 5 kDa.
In further preferred embodiments, the average molecular mass of the [P] moiety is above 5 kDa. In other preferred embodiments, the preferred average molecular mass of the [P] moiety is about 4.95 kDa to about 5.445 kDa, about 5.445 kDa to about 5.94 kDa, about 5.94 kDa to about 6.435 kDa, about 6.435 kDa to about 6.93 kDa, about 6.93 kDa to about 7.425 kDa, about 7.425 kDa to about 7.92 kDa, or above 7.92 kDa.
In other preferred embodiments, for PEOZ according to 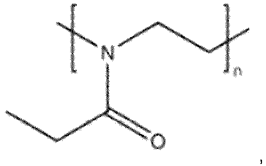
n has a mean value ranging from about 40 to about 80, preferably from about 45 to about 70, more preferably from about 50 to about 60, or most preferably n having a mean value of about 50.
In further preferred embodiments for PEOZ, n has a mean value of more than 50. In other preferred embodiments, n has a mean value of about 55, about 60, about 65, about 70, about 75 or about 80.
Thus the PEOZ moiety preferably is a PEOZ moiety having a molecular mass of about 5 kDa, although also shorter and longer moieties can also be used.

Further very preferred embodiments for PPOZ [P] moieties (polypropyloxazoline) or PIPOZ [P] moieties (poly-2-isopropyl-2-oxazoline)):
For PPOZ or equally PIPOZ, the preferred average molecular mass of the [P] moiety is about 5.2 kDa to about 6.2 kDa, about 5.3 kDa to about 6.1 kDa, about 5.4 kDa to about 6 kDa, about 5.5 kDa to about 5.9 kDa, about 5.6 kDa to about 5.8 kDa, or most preferably about 5.7 kDa.
In further preferred embodiments, the average molecular mass of the [P] moiety is above 5.7 kDa. In other preferred embodiments, the preferred average molecular mass of the [P] moiety is about 5.65 kDa to about 6.215 kDa, about 6.215 kDa to about 6.78 kDa, about 6.78 kDa to about 7.345 kDa, about 7.345 kDa to about 7.91 kDa, about 7.91 kDa to about 8.475 kDa, about 8.475 kDa to about 9.04 kDa, or above 9.04 kDa.
In other preferred embodiments, for PPOZ according to 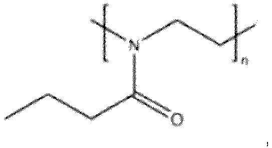 or equally PIPOZ (poly(2-isopropyl-2-oxazoline)), n has a mean value ranging from about 40 to about 80, preferably from about 45 to about 70, more preferably from about 50 to about 60, or most preferably n having a mean value of about 50.
In further preferred embodiments for PPOZ or equally PIPOZ, n has a mean value of more than 50. In other preferred embodiments, n has a mean value of about 55, about 60, about 65, about 70, about 75 or about 80.
Thus the PPOZ or equally PIPOZ moiety preferably is a PPOZ or equally PIPOZ moiety having a molecular mass of about 5.7 kDa, although also shorter and longer moieties can also be used.

In one embodiment, the lipid moiety [L] as shown in formula (I) ([P]-[linker]-[L]) comprises at least one straight or branched, saturated or unsaturated alkyl chain containing from 6 to 30 carbon atoms, preferably wherein the lipid moiety [L] comprises at least one straight or branched saturated alkyl chain, wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-,-C(O)S-, -SC(O)-, -C(S)O-, -OC(S)-, -OSi(R⁵)₂O-, -C(O)(CR³R⁴)C(O)O-, or-OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl).

In another embodiment, the the lipid moiety [L] comprises at least one straight or branched, saturated or unsaturated alkyl chain comprising 6, 7, 8, 9, 10, 11, 12, 13, 14, 15, 16, 17, 18, 19, 20, 21, 22, 23, 24, 25, 26, 27, 28, 29, or 30 carbon atoms, preferably in the range of 10 to 20 carbon atoms, more preferably in the range of 12 to 18 carbon atoms, even more preferably 14, 16 or 18 carbon atoms, even more preferably 16 or 18 carbon atoms, most preferably 14 carbon atoms, wherein all selections are independent of one another.

In one embodiment, the linker group [linker] as shown in formula (I) ([P]-[linker]-[L]) is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or-OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), -C(R5)=N-,-N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-,-C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), and sulfonate esters, as well as combinations thereof, wherein R³, R⁴ and R⁵ are, independently H or alkyl (e.g. C₁-C₄ alkyl).

In another embodiment, the linker group [linker] comprises an amide linker moiety, preferably an ester linker moiety, or wherein the linker group [linker] has the structure 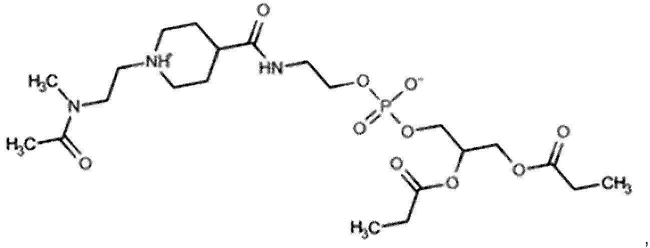 or

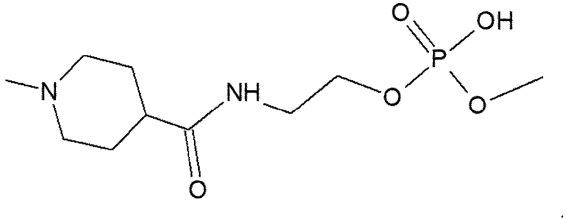

In a further embodiment, the polymer conjugated lipid has the structure of
(i) 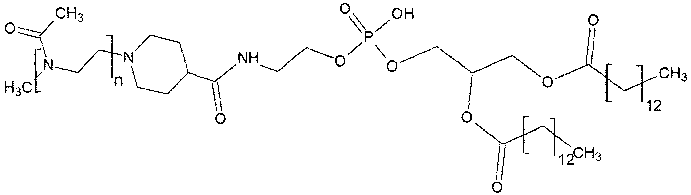 ("DMPE-PMOZ-v1") or preferably 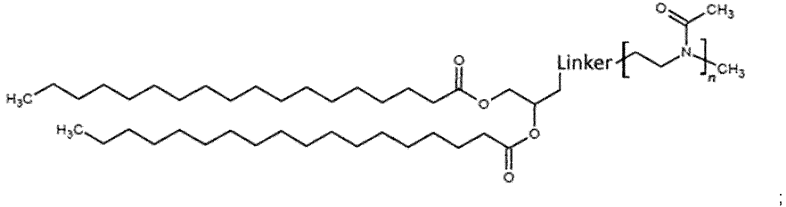 or 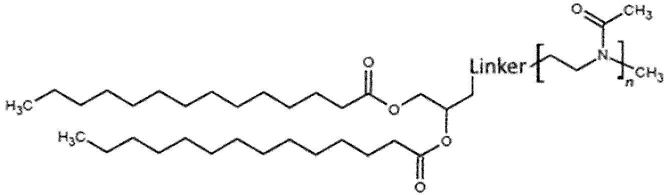 ; or 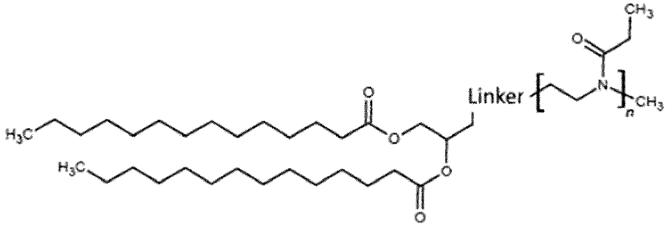 wherein the linker group [linker] is selected from any one of the linker groups as disclosed herein, preferably the linker group [linker] comprising an ester moiety;
   whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa;
   most preferably wherein the polymer conjugated lipid is DMG-PMOZ with n having a mean value from 45 to 50, most preferably 50.

In a very preferred embodiment, the polymer conjugated lipid has the structure of 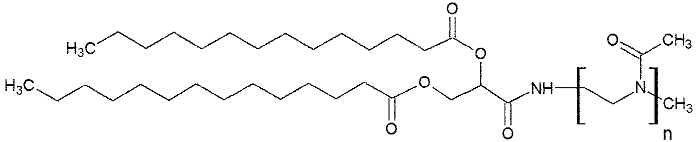 ["PMOZ 1"], more preferably with n = 50 i.e. having 50 monomer repeats.

In an even further preferred embodiment, the polymer conjugated lipid has the structure of 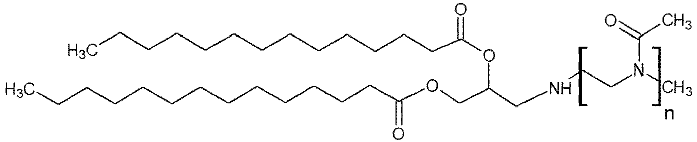 ["PMOZ 3"], more preferably with n = 50 i.e. having 50 monomer repeats.

In another preferred embodiment, the polymer conjugated lipid has the structure of 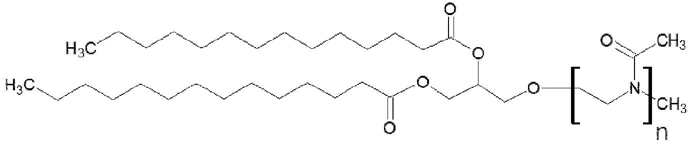 ["PMOZ 5"], more preferably with n = 50 i.e. having 50 monomer repeats.

In another very preferred embodiment, the polymer conjugated lipid has the structure of 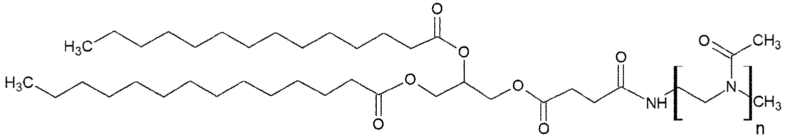 ["PMOZ 2"], more preferably with n = 50 i.e. having 50 monomer repeats.

In a most preferred embodiment, the polymer conjugated lipid has the structure of 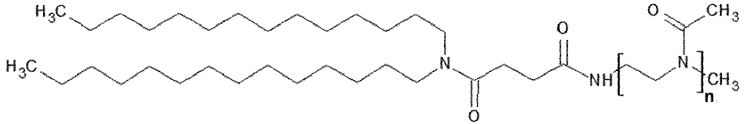 ["PMOZ 4"], more preferably with n = 50 i.e. having 50 monomer repeats, i.e. 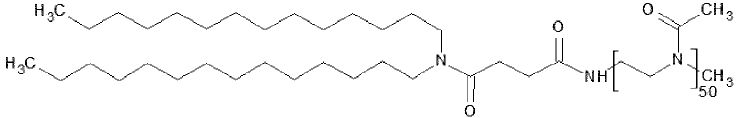 ["PMOZ 4" with n = 50 i.e. having 50 monomer repeats].

For "PMOZ 1" to "PMOZ 5", preferably n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa.

In another very preferred embodiment, the linker group [linker] comprises preferably an amide linker moiety.

In a further very preferred embodiment, the linker group [linker] comprises preferably an ester linker moiety.

In a further very preferred embodiment, the linker group [linker] comprises preferably a succinate linker moiety.

In another very preferred embodiment, the linker group [linker] comprises both an ester linker and an amid linker moiety. In another preferred embodiment, the linker group [linker] comprises both an ester linker, an amine linker and an amid linker moiety.

In another very preferred embodiment, the linker group [linker] preferably has the structure of 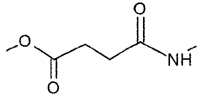 or 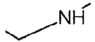 or the linker group [linker] preferably is an amine, preferably a secondary amine linker moiety.

The inventors surprisingly found, that advantageously, the above mentioned polymer conjugated lipids, preferably "PMOZ 1", "PMOZ 2", "PMOZ 3", "PMOZ 4" or "PMOZ 5", or respectively polymer conjugated lipids comprising the linker groups [linker] 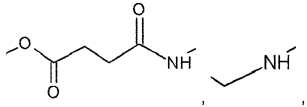 succinate, a peptide bond (-CO-NH-), an amine, or a secondary amine, more preferably wherein the linker group [linker] comprises succinamidyl (-NHC(O)CH₂CH₂C(O)-) or (-NHC(O)CH₂CH₂C(O)-)
have specific advantages when it comes to producibility or general synthesis, preferably GMP producibility. In other words, the production of these polymer conjugated lipids is easier to implement, more practicable, simpler and/or can be conducted in a more cost-effective way. In other words, the general synthesis of these compounds comprising the above mentioned preferred linkers is easier and more practicable. Lastly, polymer conjugated lipids with the aforementioned [linker] group(s) are more stable with regard to chemical stability. In other words, the polymer conjugated lipids and [linkers] as disclosed above have a highly advantageous and unexpected behaviour with regard to synthesis and production.

In a further embodiment, the lipid nanoparticle comprises the polymer conjugated lipid of the disclosure.

In a further preferred embodiment, the polymer conjugated lipid of the invention does not comprise a polyethylene glycol-(PEG)-moiety or residue; and/or does not comprise a sulphur group (-S-); and/or a terminating nucleophile.

In a further preferred embodiment, the polymer conjugated lipid of the invention does not comprise a polyethylene glycol-(PEG)-moiety or residue.

In a further preferred embodiment, the polymer conjugated lipid of the invention does not comprise a sulphur group (-S-).

In a further preferred embodiment, the polymer conjugated lipid of the invention does not comprise a terminating nucleophile.

In a further preferred embodiment, the polymer conjugated lipid of the invention does not comprise a sulphur group (-S-); and a terminating nucleophile.

In a further preferred embodiment, the polymer conjugated lipid is not covalently coupled to a biologically active ingredient being a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA.

In yet a further embodiment, the lipid nanoparticle does not comprise a polyethylene glycol-(PEG)-lipid conjugate or a conjugate of PEG and a lipid-like material, and preferably do not comprise PEG and/or (ii) the polymer conjugated lipid of the invention does not comprise a sulphur group (-S-), a terminating nucleophile, and/or is covalently coupled to a biologically active ingredient is a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA; or any combination thereof, preferably wherein the biologically active ingredient is chemically modified mRNA or chemically unmodified mRNA, more preferably wherein the biologically active ingredient is chemically unmodified mRNA.

In another very preferred embodiment, the polymer conjugated lipid of the invention does not comprise sulphur (S) or a sulphur group (-S-).

In a further embodiment, the lipid nanoparticle of the invention further comprises a sterol or steroid, preferably selected from the group consisting of cholesterol, cholesteryl hemisuccinate (CHEMS) and a derivate thereof, preferably wherein the lipid nanoparticle further comprises cholesterol.

In yet another embodiment, the lipid nanoparticle comprises
(i) an amount of 1, 2, 3, 4, 5, 6, 7, 8, 9, or 10 mol% of the inventive polymer conjugated lipid as disclosed herein;
(ii) preferably an amount of 5 mol% of the inventive polymer conjugated lipid as disclosed herein,
(iii) more preferably an amount of 2.5 mol% of the inventive polymer conjugated lipid as disclosed herein, or
(iv) also preferably an amount of 1.7 mol% of the inventive polymer conjugated lipid as disclosed herein based upon a mol-percentage of the composition of 100% of all lipid components or excipients.

In a further embodiment, the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate); formula III-3 being described further below in the specification in the section related to cationic lipids), 29.3 mol% cholesterol, 10 mol% neutral lipid and 1.7 mol% of the polymer conjugated lipid of the invention;
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of the invention;
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of the invention;
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of the invention;
(v) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of the invention;
(vi) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of the invention;
(vii) 47.4 mol% formula III-3 (((4-hydroxybutyl)azanediyl)bis(hexane-6,1-diyl)bis(2-hexyldecanoate)), 40.9 mol% cholesterol, 10 mol% DSPC, and 1.7 mol% of the polymer conjugated lipid of the invention;
(viii) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% 2-[(PMOZ)]ₙ-N,N-ditetradecylacetamide] (N,N-ditetradecylacetamide described further below in the specification); and
(ix) 47.4 mol% formula III-3 (((4-hydroxybutyl)azanediyl)bis(hexane-6,1-diyl)bis(2-hexyldecanoate)), 40.9 mol% cholesterol, 10 mol% DSPC and 1.7 mol% 2-[(PMOZ))ₙ-N,N-ditetradecylacetamide],
wherein n has a mean value ranging from about 45 to about 55, preferably n is about 50 or wherein n is selected such that the polymer moiety has an average molecular weight of about 4.2 kDa to about 4.4 kDa, or most preferably about 4.3 kDa.

In a further preferred embodiment, the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24, 29.3 mol% cholesterol, 10 mol% neutral lipid and 1.7 mol% of the polymer conjugated lipid of the invention;
(ii) 59 mol% cationic or ionizable lipid, preferably one of the ionizable lipid structures of C1 to C24, more preferably the ionizable lipid structure C24, 28.5 mol% cholesterol, 10 mol% neutral lipid and 2.5 mol% of the polymer conjugated lipid of the invention;
(iii) 59 mol% cationic or ionizable lipid, preferably one of the ionizable lipid structures of C1 to C24, more preferably the ionizable lipid structure C24, 28.3 mol% cholesterol, 10 mol% DSPC or DPhyPE, preferably DPhyPE, 1 mol% DHPC, and 2.5 mol% of the polymer conjugated lipid of the invention;
(iv) 49 mol% cationic or ionizable lipid, preferably one of the ionizable lipid structures of C1 to C24, more preferably the ionizable lipid structure C24, 29.3 mol% cholesterol, 10 mol% DSPC or DPhyPE, preferably DPhyPE, 10 mol% DHPC, and 2.5 mol% of the polymer conjugated lipid of the invention;
(v) 47.4 mol% cationic or ionizable lipid, preferably one of the ionizable lipid structures of **C1 to C27,** more preferably the ionizable lipid structure C24, 40.9 mol% cholesterol, 10 mol% DSPC or DPhyPE, preferably DPhyPE, and 1.7 mol% of the polymer conjugated lipid of the invention;
(vi) 47.4 mol% C24, 40.1 mol% cholesterol, 10 mol% DSPC and 2.5 mol% of the polymer conjugated lipid of the invention;
(vii) 47.4 mol% C24, 40.9 mol% cholesterol, 10 mol% DSPC, and 1.7 mol% of the polymer conjugated lipid of the invention;
(viii) 47.4 mol% C24, 40.1 mol% cholesterol, 10 mol% DSPC and 2.5 mol% 2-[(PMOZ)]ₙ-N,N-ditetradecylacetamide] (N,N-ditetradecylacetamide described further below in the specification); and
(ix) 47.4 mol% C24, 40.9 mol% cholesterol, 10 mol% DSPC and 1.7 mol% 2-[(PMOZ)]ₙ-N,N-ditetradecylacetamide],
wherein n has a mean value ranging from about 45 to about 55, preferably n is about 50 or wherein n is selected such that the polymer moiety has an average molecular weight of about 4.2 kDa to about 4.4 kDa, or most preferably about 4.3 kDa.

In a most preferred embodiment, the lipid nanoparticle comprises 59 mol% C24, 28.5 mol% cholesterol, 10 mol% DPhyPE and 2.5 mol% "PMOZ 4".

In a further embodiment, the biologically active ingredient comprised within the lipid nanoparticle, is a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA; or any combination thereof, preferably wherein the biologically active ingredient is chemically modified mRNA or chemically unmodified mRNA, more preferably wherein the biologically active ingredient is chemically unmodified mRNA.

In a very preferred embodiment, the nucleic acid compound is an artificial or isolated mRNA.

In yet another embodiment, the lipid nanoparticle comprises at least one coding sequence encoding a pathogenic antigen, wherein the pathogenic antigen
(i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
(ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
(iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof (e.g. receptor binding domain (RBD), critical neutralisation domain (CND)); and/or
(iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation. In even a further embodiment, the polymer conjugated lipid is selected from the group consisting of 2-[(PMOZ)]ₙ-N,N-ditetradecylacetamide], 2-[(PEOZ)]ₙ-N,N-ditetradecylacetamide], 2-[(PPOZ)]ₙ-N,N-ditetradecylacetamide], 2-[(PBOZ)]ₙ-N,N-ditetradecylacetamide], 2-[(PIPOZ)]ₙ-N,N-ditetradecylacetamide], preferably the polymer conjugated lipid is 2-[(PM(O)Z)]ₙ-N,N-ditetradecylacetamide], wherein n has a mean value ranging from about 45 to about 55, preferably n is about 50 or wherein n is selected such that the polymer moiety has an average molecular weight of about 4.2 kDa to about 4.4 kDa, or most preferably about 4.3 kDa.

In other preferred embodiments, new polymer conjugated lipids may be derived from the polymer conjugated lipids disclosed in WO2018078053 (i.e. a lipid as derived from a N,N-ditetradecylacetamide-based compound or claim 5 of WO2018078053), the disclosure of WO2018078053 hereby incorporated by reference in its entirety.

It is noted herein, that all chemical compounds mentioned throughout the whole specification may be produced via processes known to a skilled worker; starting materials and/or reagents used in the processes are obtainable through routine knowledge of a skilled worker on the basis of common general knowledge (e.g. from text books or from e.g. patent applications WO2022173667, WO2009043027, WO2013067199, WO2010006282, WO2009089542, WO2016019340, WO2008106186, WO2020264505, and WO2020023947, the complete disclosure of said patent applications is incorporated by reference herein).

### Lipid Compositions

In some aspects of the invention, the LNPs comprise a lipid-conjugate, preferably a polymer conjugated lipid as described above, a cationic lipid, a steroid and a neutral lipid.

### Cationic, ionizable or cationisable lipids

The cationic lipid is preferably ionizable or cationisable, i.e. it becomes protonated as the pH is lowered below the pKₐ of the ionizable group of the lipid, but is progressively more neutral at higher pH values. When positively charged, the lipid is then able to associate with negatively charged nucleic acids. In certain embodiments, the cationic lipid comprises a zwitterionic lipid that assumes a positive charge on pH decrease.

In some embodiments, the lipid nanoparticle comprises a molar ratio of 20-60% ionizable cationic lipid. For example, the lipid nanoparticle may comprise a molar ratio of 20-50%, 20-40%, 20-30%, 30-60%, 30-50%, 30-40%, 40-60%, 40-50%, or 50-60% ionizable cationic lipid. In some embodiments, the lipid nanoparticle comprises a molar ratio of 20%, 30%, 40%, 50, or 60% ionizable cationic lipid.

In some embodiments, the lipid nanoparticle comprises a molar ratio of 5-25% non-cationic lipid. For example, the lipid nanoparticle may comprise a molar ratio of 5-20%, 5-15%, 5-10%, 10-25%, 10-20%, 10-25%, 15-25%, 15-20%, or 20-25% non-cationic lipid. In some embodiments, the lipid nanoparticle comprises a molar ratio of 5%, 10%, 15%, 20%, or 25% non-cationic lipid.

In some embodiments, the lipid nanoparticle comprises a molar ratio of 25-55% sterol. For example, the lipid nanoparticle may comprise a molar ratio of 25-50%, 25-45%, 25-40%, 25-35%, 25-30%, 30-55%, 30-50%, 30-45%, 30-40%, 30-35%, 35-55%, 35-50%, 35-45%, 35-40%, 40-55%, 40-50%, 40-45%, 45-55%, 45-50%, or 50-55% sterol. In some embodiments, the lipid nanoparticle comprises a molar ratio of 25%, 30%, 35%, 40%, 45%, 50%, or 55% sterol.

In some embodiments, the lipid nanoparticle comprises a molar ratio of 0.5-15% polymer conjugated lipid of the disclosure. For example, the lipid nanoparticle may comprise a molar ratio of 0.5-10%, 0.5-5%, 1-15%, **1-10%, 1-**5%, 2-15%, 2-10%, 2-5%, 5-15%, 5-10%, or 10-15%. In some embodiments, the lipid nanoparticle comprises a molar ratio of 0.5%, 1%, 2%, 3%, 4%, 5%, 6%, 7%, 8%, 9%, 10%, 11%, 12%, 13%, 14%, or 15% polymer conjugated lipid of the disclosure.

In some embodiments, the lipid nanoparticle comprises a molar ratio of 20-60% ionizable cationic lipid, 5-25% non-cationic lipid, 25-55% sterol, and 0.5-15% polymer conjugated lipid of the disclosure.

### Ionizable Lipids

The cationic lipid of an LNP may be cationisable, i.e. it becomes protonated as the pH is lowered below the pK of the ionizable group of the lipid, but is progressively more neutral at higher pH values. At pH values below the pK, the lipid is then able to associate with negatively charged nucleic acids. In certain embodiments, the cationic lipid comprises a zwitterionic lipid that assumes a positive charge on pH decrease.

Preferred cationic lipids are defined as a compound according to formula (Cat-I):

R^{a}-A-R^{b} formula (Cat-I)

wherein is
R^{a} is selected from: 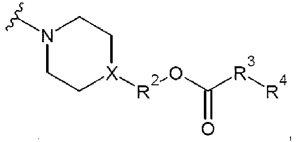 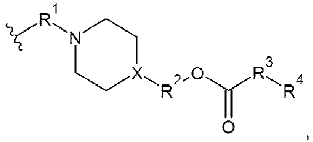 or

   -R¹-N(H)-C(O)-R³-R⁴;
R^{b} is selected from:

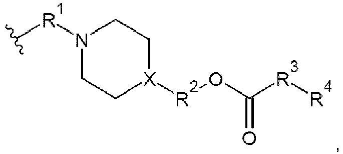

   -R¹-N(H)-C(O)-R³-R⁴, or

   -R¹-N(CH₃)₂;
A is -S-, -S-S-, -NH-C(O)-, -NH-C(O)O-, -NH-C(O)-NH-, -S-C(O)-N(H)-, -C(O)O-, or -O-P(O)(OH)-O-;
R¹ is an optionally substituted ethanediyl, propanediyl, butanediyl, or linear or unbranched alkanediyl having 2 to 8 carbon atoms;
R² is an alkanediyl having 2 to 8 carbon atoms;
R³ is optional, and if present, is -R⁵-C(O)-O-, -R⁵-O-C(O)-, -R⁵-C(O)-NH-, -R⁵-OC(O)-NH-, or R⁵-NH-C(O)O-;
R⁴ is a lipophilic substituent with 12 to 36 carbon atoms;
R⁵ is an alkanediyl having 1 to 6 carbon atoms;
X is a carbon or nitrogen atom;

wherein all selections are independent of one another,
optionally provided that if R¹, R² and R⁵ are all linear unsubstituted ethanediyl, A is -S-S-, and R^{a} and R^{b} are identical, then R⁴ is not

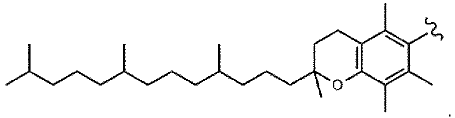

In other embodiments, R⁴ is

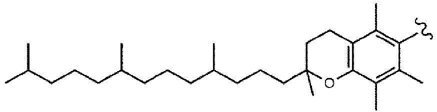

In one preferred embodiment, A is -S-, and R^{a} and R^{b} are identical, and R⁴ is

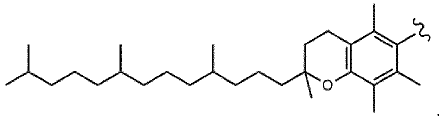

In another preferred embodiment, A is -S- and R⁴ is

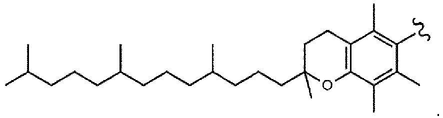

R⁴ from formula (Cat-I) is defined as a lipophilic substituent with 12 to 36 carbon atoms. This "tail" end of R^{a} and optionally also of R^{b} (unless R^{b} is -R¹-N(CH₃)₂) is believed to provide the degree of lipophilicity which is typically required for molecules to be able to cross biological membranes. Therefore, R⁴ may in principle be of any structure that is substantially lipophilic. For example, a hydrocarbon structure is lipophilic. In one embodiment, R⁴, in at least one of its occurrences, may consist of only carbon and hydrogen atoms. In one preferred embodiment, R⁴ represents a linear or branched alkyl or alkenyl, preferably having 12 to 25 carbon atoms. The branched alkyl or alkenyl may optionally have a plurality of side chains, such as 2, 3, 4 or more methyl side chains. In another embodiment, R⁴ may be an alkyl or alkenyl comprising a single alkyl or alkenyl side chain with e.g. 2 to 10 carbon atoms. For example, R⁴ may be 1-n-hexyl-n-nonyl (or 7-n-pentadecyl), or 2-n-hexyl-n-decyl. In other embodiments, the lipophilic substituent may optionally include one or more heteroatoms such as O, S, or N. In other embodiments, the lipophilic substituent may optionally include one or more saturated, unsaturated, or aromatic ring structures that may optionally include one or more heteroatoms such as O, S, or N.

R⁴ may also include a small number of hetero atoms such as oxygen atoms, as long as the predominantly lipophilic character is maintained. In one embodiment, R⁴ comprises one or more oxygen atoms and no other hetero atoms. R⁴ may also comprise a cyclic structure, such as an aromatic or aliphatic ring structure optionally including one or more oxygen atoms. If present, it is preferred that the hetero atoms and/or the cyclic structure are located towards the optional R³ structure rather than towards the end of the "tail". In one embodiment, R⁴ is a lipophilic group derived from tocopherol or tocotreinol. In one embodiment, R⁴ is a lipophilic group derived from alpha-tocopherol, in particular 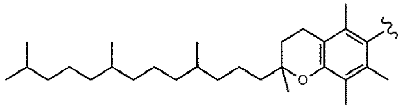 in particular if not all of R¹, R² and R⁵ are linear unsubstituted ethanediyl, A is -S-S-, and R^{a} and R^{b} are identical.

A "lipophilic group derived from tocopherol or tocotreinol" as referred to herein includes derivatives of tocopherol and tocotreinol, in particular the derivatives with the structures shown in **Scheme 1** below, i.e. the derivatives derived from alpha-tocopherol, beta-tocopherol, gamma-tocopherol, delta-tocopherol, alpha-tocotreinol, beta-tocotreinol, gamma-tocotreinol and delta-tocotreinol.

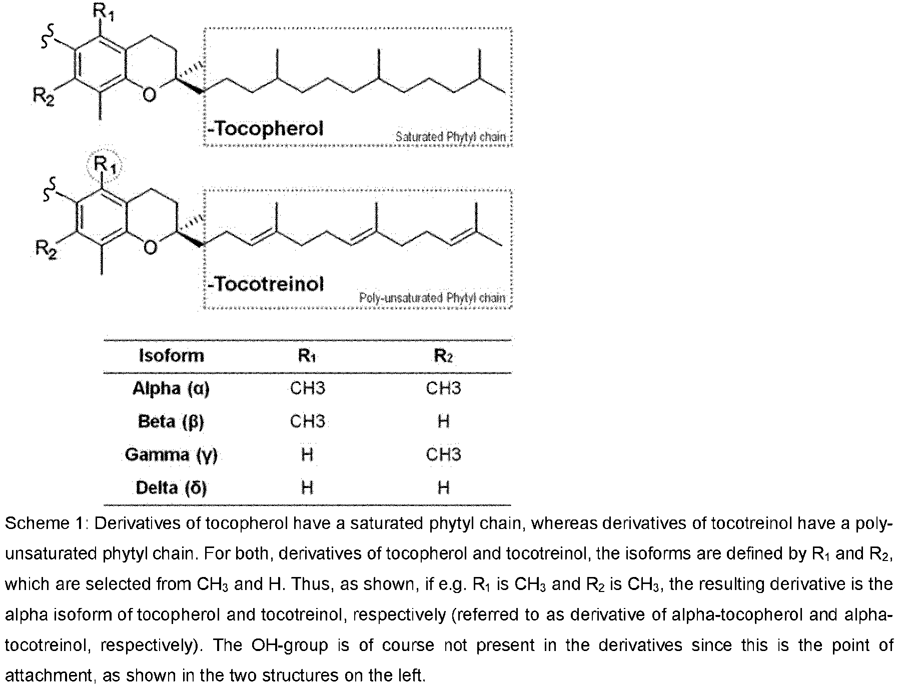

In a preferred embodiment, in particular of aspect A above, R⁴ is either a linear or branched alkyl or alkenyl having 12 to 25 carbon atoms or is a lipophilic group selected from the group consisting of the derivatives of alpha-tocopherol, beta-tocopherol, gamma-tocopherol, delta-tocopherol, alpha-tocotreinol, beta-tocotreinol, gamma-tocotreinol and delta-tocotreinol as shown herein in **Scheme 1.**

In yet another preferred embodiment, in particular of aspect A above, R⁴ is either a linear or branched alkyl or alkenyl having 12 to 25 carbon atoms or

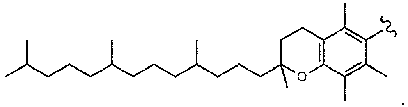

In other preferred embodiments, the at least one nucleic acid (e.g. DNA or RNA), preferably the at least one RNA of the composition is complexed with one or more lipids thereby forming LNPs, wherein the cationic lipid of the LNP is selected from structures C1 to C23, or respectively C1 to C27 of Table 1 or a lipid derived from formula (I) of PCT patent application PCT/EP2019/086825 or the subsequent patent application thereof claiming the priority of PCT/EP2019/086825 i.e. WO2021123332. In other embodiments, the at least one nucleic acid (e.g. DNA or RNA), preferably the at least one RNA of the composition is complexed with one or more lipids thereby forming LNPs, wherein the cationic lipid of the LNP is derived from structures C1 to C23, or respectively C1 to C27 of Table 1 of PCT patent application PCT/EP2019/086825 or the subsequent patent application thereof claiming the priority of PCT/EP2019/086825 i.e. WO2021123332, wherein the element "A" from formula (I) of PCT/EP2019/086825 is - S-. Accordingly, formulas C1 to C23, or respectively C1 to C27, of PCT patent application PCT/EP2019/086825 or the subsequent patent application thereof claiming the priority of PCT/EP2019/086825 i.e. WO2021123332, and the specific disclosure relating thereto, are herewith incorporated by reference.

In yet a further embodiment, the cationic lipid preferably is selected from the cationic lipids as listed herein in **Table 1.**

**Table 1: Preferred cationic lipids according to formula (I) - when it is referred to specific lipids from this table, e.g. lipid C1, reference is made e.g. to "Lipid C1", "Lipid Compound 1", "HEXA-C4DE-PipSS" or "C1"**

| **Cationic Lipid Compou nd No.** | **Structure** | **Name** |
|---|---|---|
| C1 | 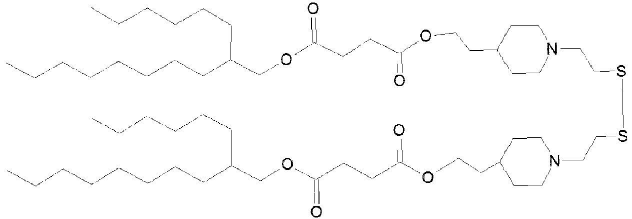 | HEXA-C4DE-PipSS |
| C2 | 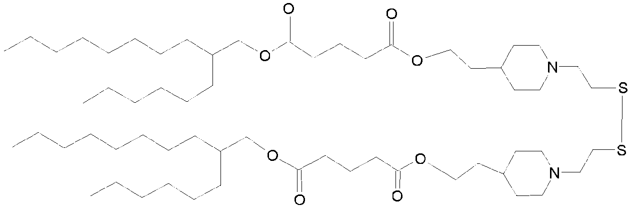 | HEXA-C5DE-PipSS (GN02-lipid) |
| C3 | 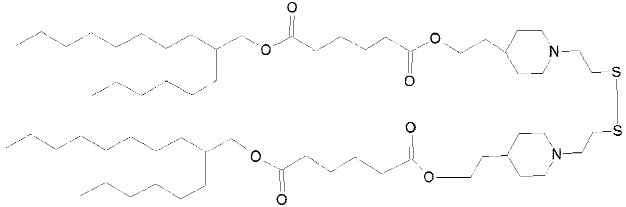 | HEXA-C6DE-PipSS |
| C4 | 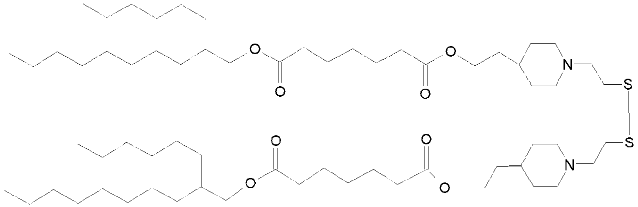 | HEXA-C7DE-PipSS |
| C5 | 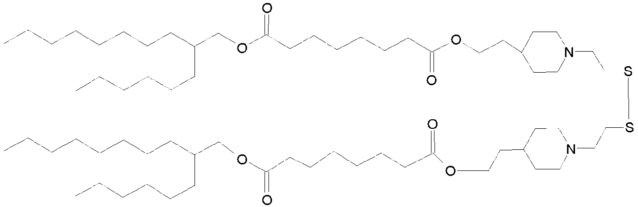 | HEXA-C8DE-PipSS |
| C6 | 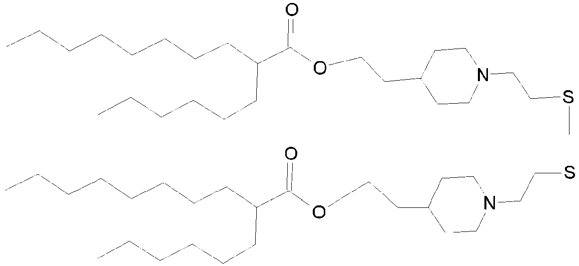 | HEXACA -C3ME-PipSS |
| C7 | 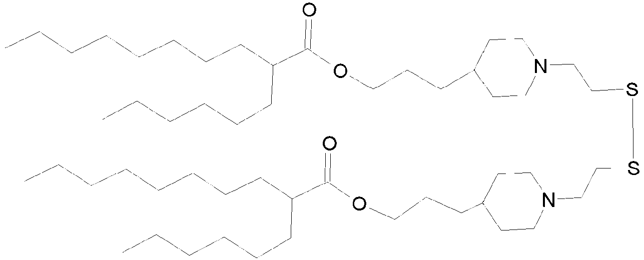 | HEXACA-C4ME-PipSS |
| C8 | 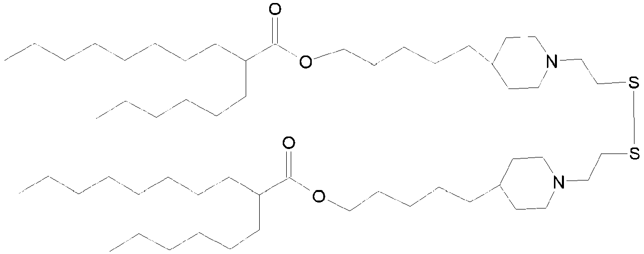 | HEXACA-C6ME-PipSS |
| C9 | 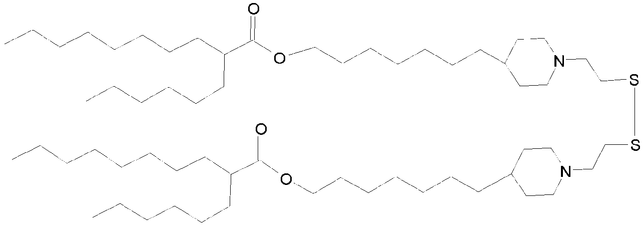 | HEXACA-C8ME-PipSS |
| C10 | 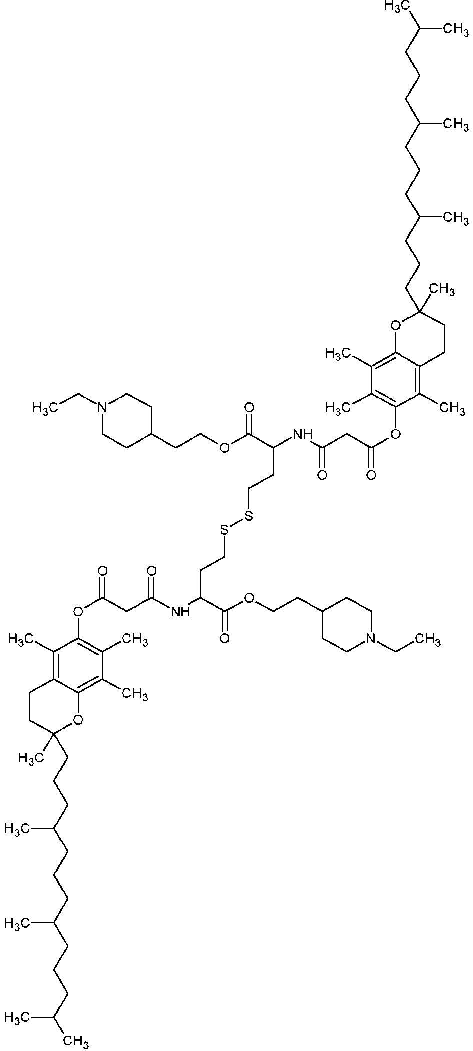 | C10 / Compound 10 |
| C11 | 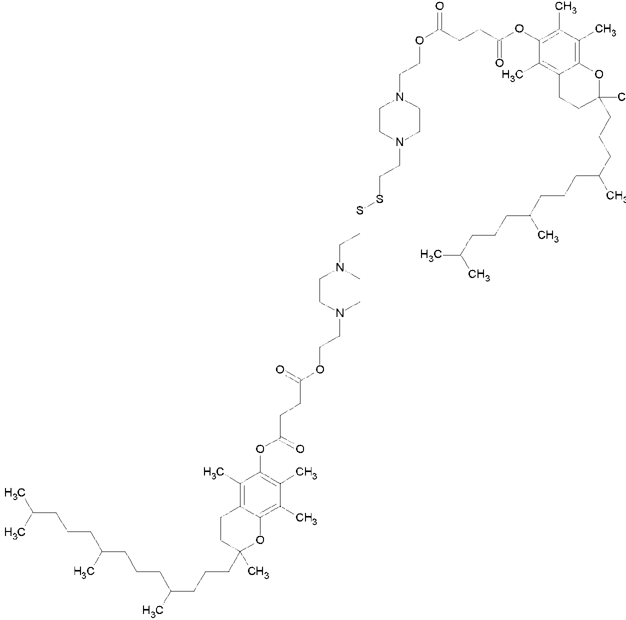 | C11/Compound 11 |
| C12 | 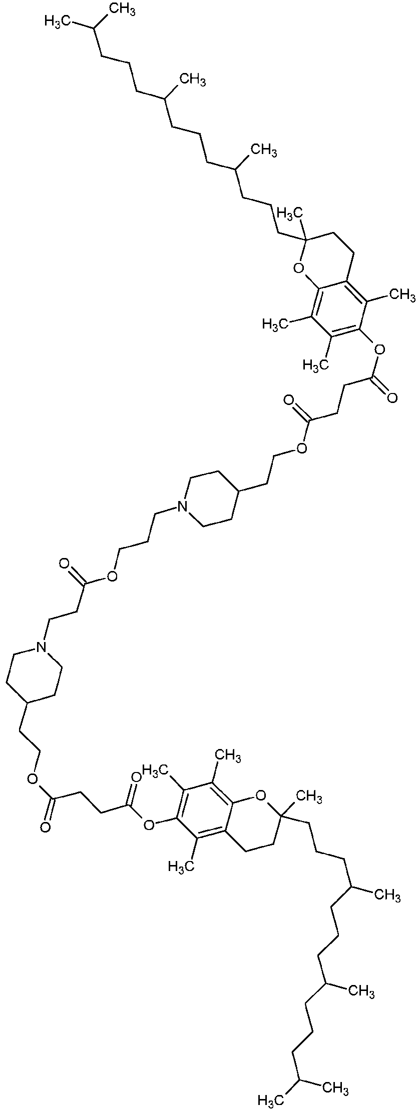 | C12/Compound 12 |
| C13 | 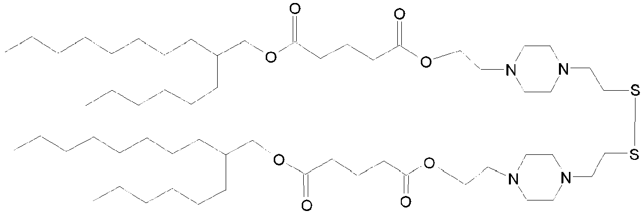 | HEXA-C5DE-PipAZSS |
| C14 | 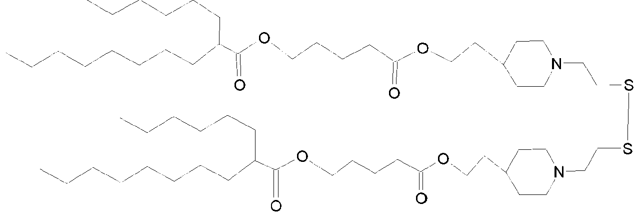 | HEXACA-C5DE-PipSS |
| C15 | 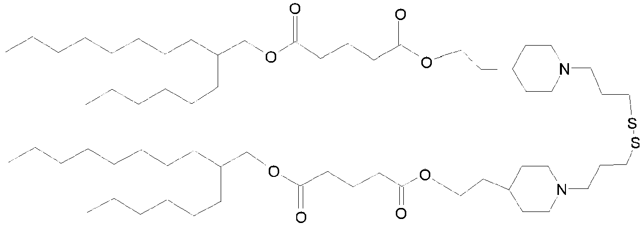 | HEXA-C5DE-PipC3SS |
| C16 | 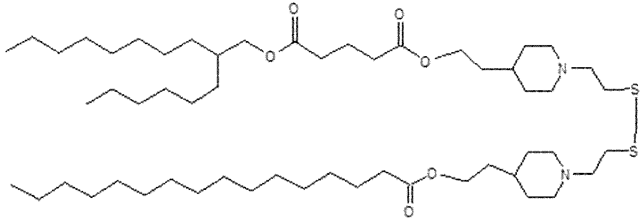 | C16-HEXA-C5DE-PipSS |
| C17 | 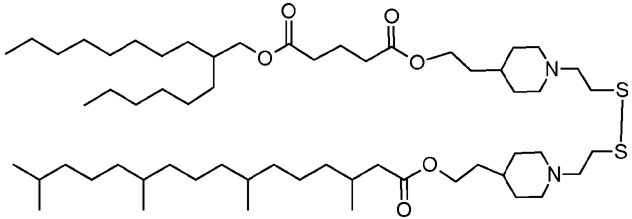 | DPhy-HEXA-C5DE-PipSS |
| C18 | 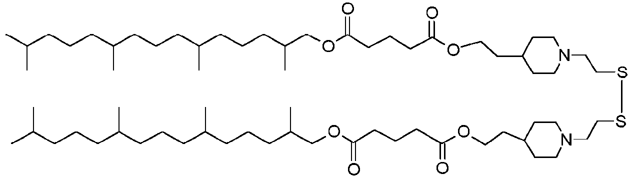 | 2DPhy-C5DE-PipSS |
| C19 | 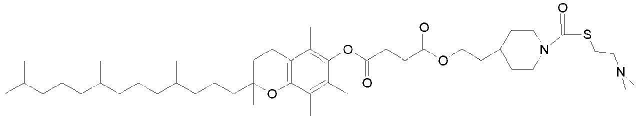 | Vit E-C5DE-Pip-TEN |
| C20 | 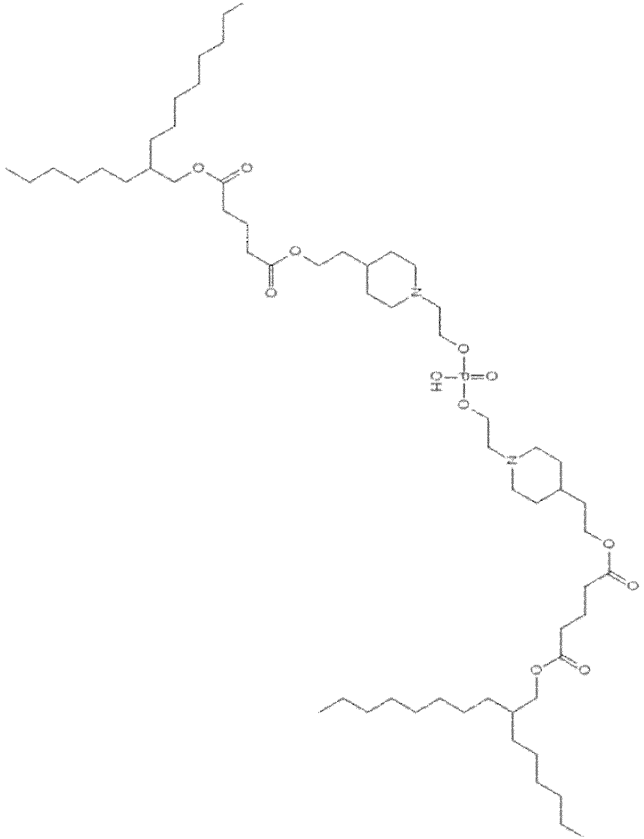 | HEXA-C5DE-Pip-Phosphate |
| C21 | 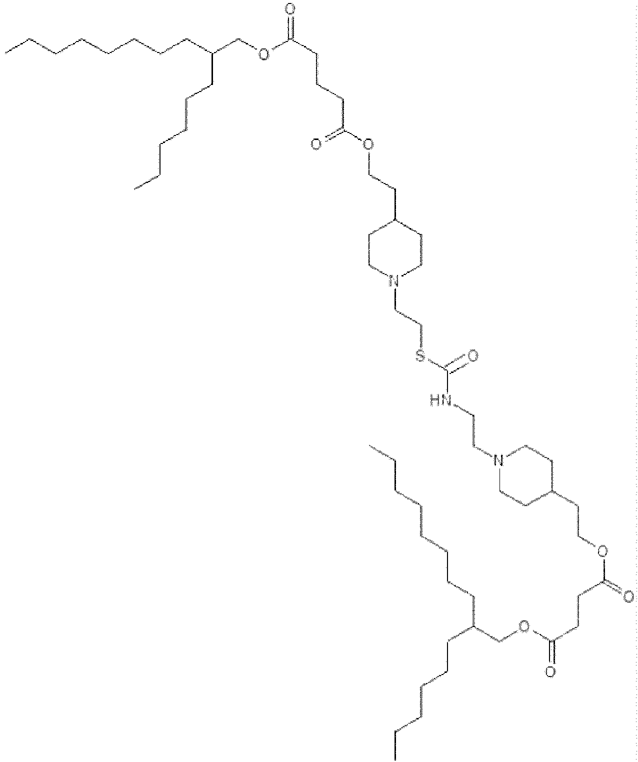 | HEXA-C5DE-Pip-Thiocarbamate |
| C22 | 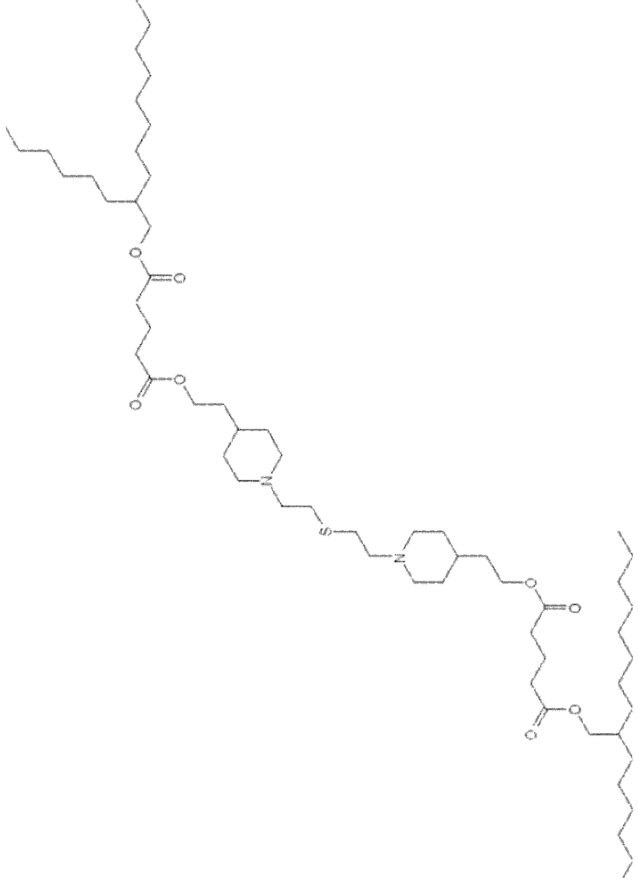 | HEXA-C5DE-Pip-Thioether |
| C23 | 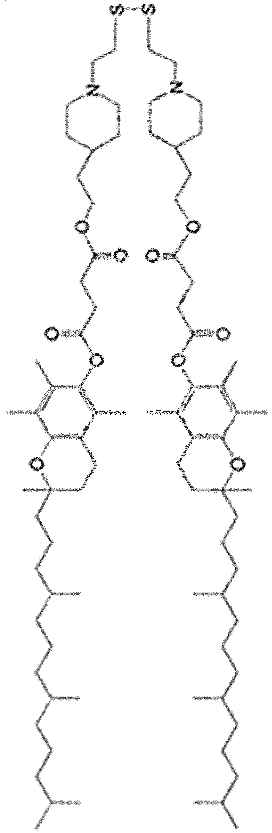 | COATSOME^{®} SS-EC |
| C24 | 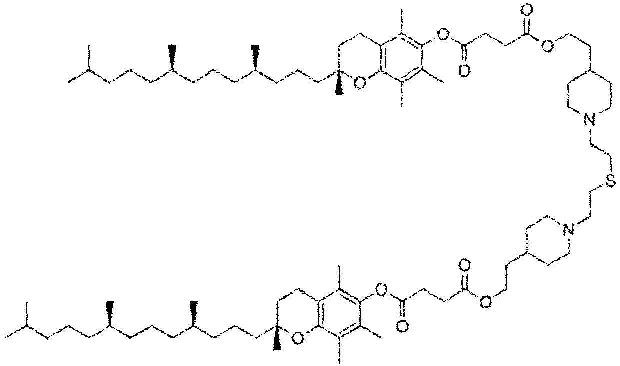 | THIOETHER or VitE-C4DE-Piperidine-Thioether |
| C25 | 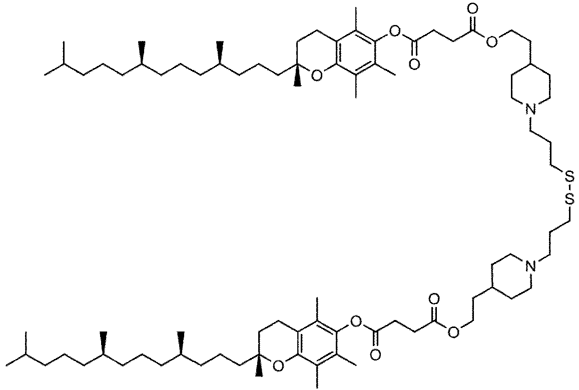 | "C3SS" or "VitE-C4DE-Piperidine-C3SS" |
| C26 | 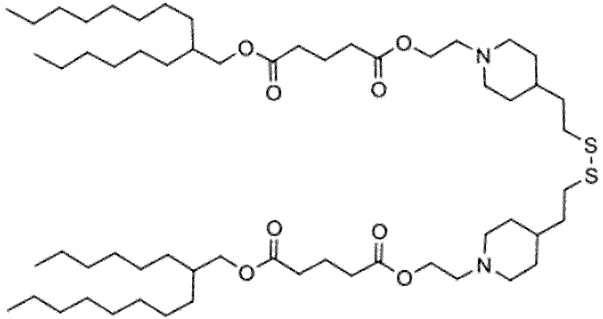 | HEXA-C5DE-inverted-PipSS |
| C27 | 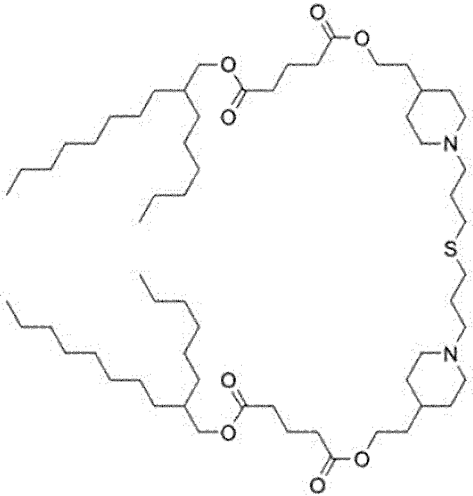 | HEXA-C5DE-Pip-C3 thioether or HEXA-C5DE-piperidine-C3 thioether |

Accordingly, the invention is directed to a composition comprising the cationic lipid as described above. For example, the composition may comprise a cationic lipid selected from compounds **C1** to **C27** of **Table 1.**

In other preferred embodiments, the at least one nucleic acid (e.g. DNA or RNA), preferably the at least one RNA of the composition is complexed with one or more lipids thereby forming LNPs, wherein the cationic lipid of the LNP has the structure "C24", which in turn is the most preferred structure for a cationic lipid comprised in a lipid nanoparticle composition of the invention:

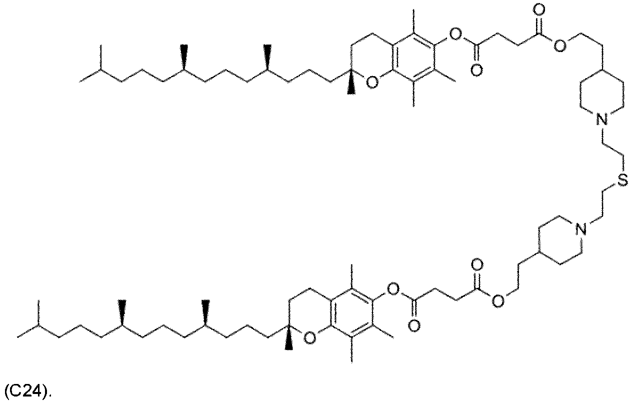

Cationic, ionizable or cationisable lipids also include, but are not limited to, DSDMA, N,N-dioleyl-N,N-dimethylammonium chloride (DODAC), N,N-distearyl-N,N-dimethylammonium bromide (DDAB), 1,2-dioleoyltrimethyl ammonium propane chloride (DOTAP) (also known as N-(2,3-dioleoyloxy)propyl)-N,N,N-trimethylammonium chloride and 1,2-Dioleyloxy-3-trimethylaminopropane chloride salt), N-(1-(2,3-dioleyloxy)propyl)-N,N,N-trimethylammonium chloride (DOTMA), N,N-dimethyl-2,3-dioleyloxy)propylamine (DODMA), ckk-E12, ckk, 1,2-DiLinoleyloxy-N,N-dimethylaminopropane (DLinDMA), 1,2-Dilinolenyloxy-N,N-dimethylaminopropane (DLenDMA), 1,2-di-y-linolenyloxy-N,N-dimethylaminopropane (γ-DLenDMA), 98N12-5, 1,2-Dilinoleylcarbamoyloxy-3-dimethylaminopropane (DLin-C-DAP), 1,2-Dilinoleyoxy-3-(dimethylamino)acetoxy-propane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3-dimethylamino-propane (DLin-2-DMAP), 1,2-Dilinoleyloxy-3-trimethylaminopropane chloride salt (DLin-TMA.Cl), ICE (Imidazol-based), HGT5000, HGT5001, DMDMA, CLinDMA, CpLinDMA, DMOBA, DOcarbDAP, DLincarbDAP, DLinCDAP, KLin-K-DMA, DLin-K-XTC2-DMA, XTC (2,2-Dilinoleyl-4-dimethylaminoethyl-[1,3]-dioxolane) HGT4003, 1,2-Dilinoleoyl-3-trimethylaminopropane chloride salt (DLin-TAP.Cl), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DM A), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine, (6Z,9Z,28Z,31Z)-heptatriaconta-6,9,28,31-tetraen-19-yl-4-(dimethylamino)butanoate (MC3), ALNY-100 ((3aR,5s,6aS)-N,N-dimethyl-2,2-di((9Z,12Z)-octadeca-9,12-dienyl)tetrahydro-3aH-cyclopenta[d] [1,3]dioxol-5-amine)), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminomethyl-[1,3]-dioxolane (DLin-K-DMA), NC98-5 (4,7, 13-tris(3-oxo-3-(undecylamino)propyl)-N1,N16-diundecyl-4,7,10,13-tetraazahexadecane-l,16-diamide), (6Z,9Z,28Z,31Z)-heptatriaconta-6,9,28,31-tetraen-19-yl 4-(dimethylamino) butanoate (DLin-M-C3-DMA), 3-((6Z,92,28Z,31Z)-heptatriaconta-6,9,28,31-tetraen-19-yloxy)-N,N-dimethylpropan-1-amine (MC3 Ether), 4-((6Z,9Z,28Z,31Z)-heptatriaconta-6,9,28,31-tetraen-19-yloxy)-N,N-dimethylbutan-1-amine (MC4 Ether), LIPOFECTIN^{®} (commercially available cationic liposomes comprising DOTMA and 1,2-dioleoyl-sn-3phospho-ethanolamine (DOPE), from GIBCO/BRL, Grand Island, N.Y.); LIPOFECTAMINE^{®} (commercially available cationic liposomes comprising N-(1-(2,3dioleyloxy)propyl)-N-(2-(sperminecarboxamido)ethyl)-N,N-dimethylammonium trifluoroacetate (DOSPA) and (DOPE), from GIBCO/BRL); and TRANSFECTAM^{®} (commercially available cationic lipids comprising dioctadecylamidoglycyl carboxyspermine (DOGS) in ethanol from Promega Corp., Madison, Wis.) or any combination of any of the foregoing. Further suitable cationic lipids for use in the compositions and methods of the invention include those described in international patent publications WO2010053572 (and particularly, CI 2-200 described at paragraph [00225]) and WO2012170930, both of which are incorporated herein by reference, HGT4003, HGT5000, HGTS001, HGT5001, HGT5002 (see US20150140070).

In some embodiments, the cationic lipid may be an amino lipid.

Representative amino lipids include, but are not limited to, 1,2-dilinoleyoxy-3-(dimethylamino)acetoxypropane (DLin-DAC), 1,2-dilinoleyoxy-3morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,Ndilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), and 2,2-dilinoleyl-4-dimethylaminomethyl-[1,3]-dioxolane (DLin-K-DMA), 2,2-dilinoleyl-4-(2-dimethylaminoethyl)-[1,3]-dioxolane (DLin-KC2-DMA); dilinoleyl-methyl-4-dimethylaminobutyrate (DLin-MC3-DMA); MC3 (US20100324120).

In embodiments, the cationic lipid may an aminoalcohol lipidoid.

Aminoalcohol lipidoids which may be used in the present invention may be prepared by the methods described in U.S. Patent No. 8,450,298, herein incorporated by reference in its entirety. Suitable (ionizable) lipids can also be the compounds as disclosed in Tables 1, 2 and 3 and as defined in claims **1-24** of WO2017075531, hereby incorporated by reference.

In another embodiment, suitable lipids can also be the compounds as disclosed in WO2015074085 (i.e. ATX-001 to ATX-032 or the compounds as specified in claims 1-26), U.S. Appl. Nos. 61/905,724 and 15/614,499 or U.S. Patent Nos. 9,593,077 and 9,567,296 hereby incorporated by reference in their entirety.

In other embodiments, suitable cationic lipids can also be the compounds as disclosed in WO2017117530 (i.e. lipids 13, 14, 15, 16, 17, 18, 19, 20, or the compounds as specified in the claims), hereby incorporated by reference in its entirety.

In preferred embodiments, ionizable or cationic lipids may also be selected from the lipids disclosed in WO2018078053 (i.e. lipids derived from formula I, II, and III of WO2018078053, or lipids as specified in Claims 1 to 12 of WO2018078053), the disclosure of WO2018078053 hereby incorporated by reference in its entirety. In that context, lipids disclosed in Table 7 of WO2018078053 (e.g. lipids derived from formula I-1 to I-41) and lipids disclosed in Table 8 of WO2018078053 (e.g. lipids derived from formula II-1 to II-36) may be suitably used in the context of the invention. Accordingly, formula I-1 to formula I-41 and formula II-1 to formula II-36 of WO2018078053, and the specific disclosure relating thereto, are herewith incorporated by reference.

In preferred embodiments, cationic lipids may be derived from formula III of published PCT patent application WO2018078053. Accordingly, formula III of WO2018078053, and the specific disclosure relating thereto, are herewith incorporated by reference.

In particularly preferred embodiments, the at least one nucleic acid (e.g. DNA or RNA), preferably the at least one RNA of the composition is complexed with one or more lipids thereby forming LNPs, wherein the cationic lipid of the LNP is selected from structures III-1 to III-36 of Table 9 of published PCT patent application WO2018078053. Accordingly, formula III-1 to III-36 of WO2018078053, and the specific disclosure relating thereto, are herewith incorporated by reference.

In particularly preferred embodiment of the second aspect, the at least one nucleic acid (e.g. DNA or RNA), preferably the at least one RNA is complexed with one or more lipids thereby forming LNPs, wherein the LNPs comprise a cationic lipid according to 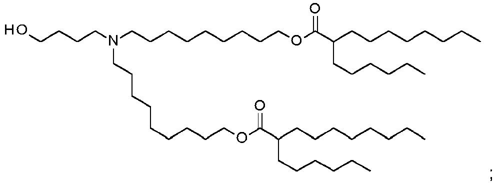 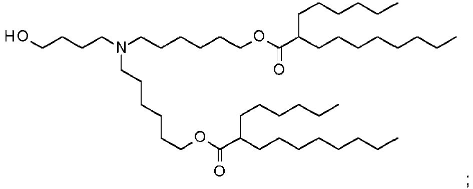 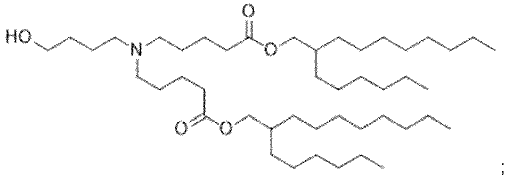 or 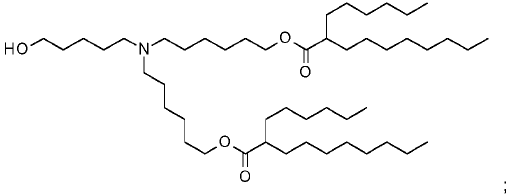 or most preferably formula III-3 of WO2018078053, i.e. (4-hydroxybutyl)azanediyl)bis(hexane-6,1-diyl)bis(2-hexyldecanoate):

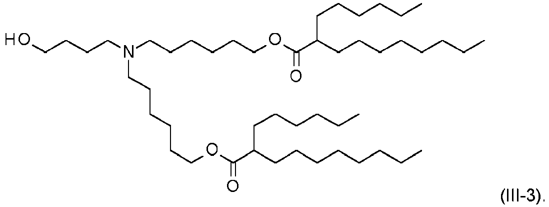

The lipid of formula III-3 as suitably used herein has the chemical term ((4-hydroxybutyl)azanediyl)bis(hexane-6,1-diyl)bis(2-hexyldecanoate), also referred to as ALC-0315 i.e. CAS Number 2036272-55-4.

In certain embodiments, the cationic lipid as defined herein, more preferably cationic lipid compound III-3 ((4-hydroxybutyl) azanediyl)bis(hexane-6,1-diyl)bis(2-hexyldecanoate)), is present in the LNP in an amount from about 30 mol% to about 80 mol%, preferably about 30 mol% to about 60 mol%, more preferably about 40 mol% to about 55 mol%, more preferably about 47.4 mol%, relative to the total lipid content of the LNP. If more than one cationic lipid is incorporated within the LNP, such percentages apply to the combined cationic lipids.

In embodiments, the cationic lipid is present in the LNP in an amount from about 30 mol% to about 70 mol%. In one embodiment, the cationic lipid is present in the LNP in an amount from about 40 mol% to about 60 mol%, such as about 40, 41, 42, 43, 44, 45, 46, 47, 48, 49, 50, 51, 52, 53, 54, 55, 56, 57, 58, 59 or 60 mol%, respectively. In embodiments, the cationic lipid is present in the LNP in an amount from about 47 mol% to about 48 mol%, such as about 47.0, 47.1, 47.2, 47.3, 47.4, 47.5, 47.6, 47.7, 47.8, 47.9, 50.0 mol%, respectively, wherein 47.4 mol% are particularly preferred.

In some embodiments, the cationic lipid is present in a ratio of from about 20 mol% to about 70 mol% or 75 mol% or from about 45 mol% to about 65 mol% or about 20, 25, 30, 35, 40, 45, 50, 55, 60, 65, or about 70 mol% of the total lipid present in the LNP. In further embodiments, the LNPs comprise from about 25% to about 75% on a molar basis of cationic lipid, e.g., from about 20 to about 70%, from about 35 to about 65%, from about 45 to about 65%, about 60%, about 57.5%, about 57.1%, about 50% or about 40% on a molar basis (based upon 100% total moles of lipid in the lipid nanoparticle). In some embodiments, the ratio of cationic lipid to nucleic acid (e.g. coding RNA or DNA) is from about 3 to about 15, such as from about 5 to about 13 or from about 7 to about 11.

Other suitable (cationic or ionizable) lipids are disclosed in WO2009086558, WO2009127060, WO2010048536, WO2010054406, WO2010088537, WO2010129709, WO2011153493, WO2013063468, US20110256175, US20120128760, US20120027803, US8158601, WO2016118724, WO2016118725, WO2017070613, WO2017070620, WO2017099823, WO2012040184, WO2011153120, WO2011149733, WO2011090965, WO2011043913, WO2011022460, WO2012061259, WO2012054365, WO2012044638, WO2010080724, WO201021865, WO2008103276, WO2013086373, WO2013086354, US Patent Nos. 7,893,302, 7,404,969, 8,283,333, 8,466,122 and 8,569,256 and US Patent Publication No. US20100036115, US20120202871, US20130064894, US20130129785, US20130150625, US20130178541, US20130225836, US20140039032 and WO2017112865. In that context, the disclosures of WO2009086558, WO2009127060, WO2010048536, WO2010054406, WO2010088537, WO2010129709, WO2011153493, WO2013063468, US20110256175, US20120128760, US20120027803, US8158601, WO2016118724, WO2016118725, WO2017070613, WO2017070620, WO2017099823, WO2012040184, WO2011153120, WO2011149733, WO2011090965, WO2011043913, WO2011022460, WO2012061259, WO2012054365, WO2012044638, WO2010080724, WO201021865, WO2008103276, WO2013086373, WO2013086354, US Patent Nos. 7,893,302, 7,404,969, 8,283,333, 8,466,122 and 8,569,256 and US Patent Publication No. US20100036115, US20120202871, US20130064894, US20130129785, US20130150625, US20130178541, US20130225836 and US20140039032 and WO2017112865 specifically relating to (cationic) lipids suitable for LNPs are incorporated herewith by reference.

In other embodiments, the cationic or ionizable lipid is 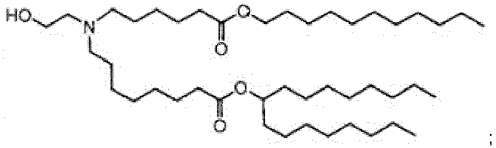 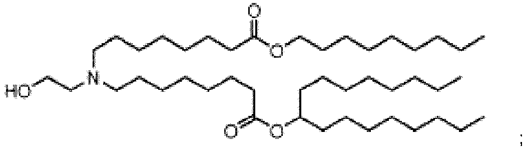 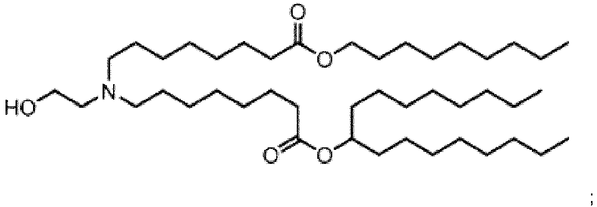 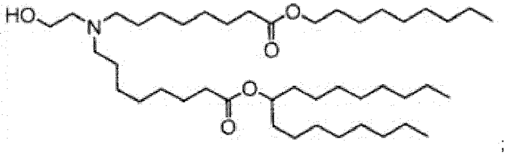 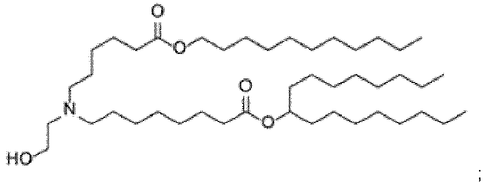 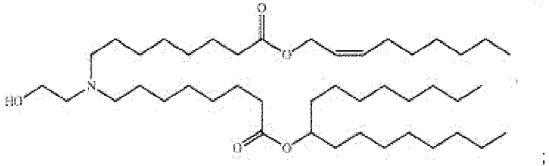 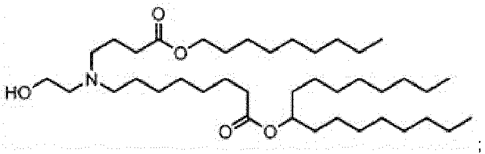 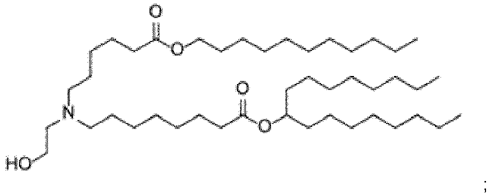 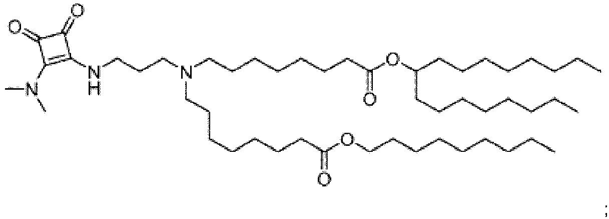 or

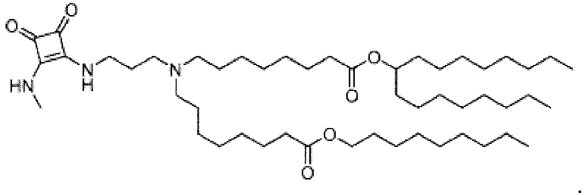

In embodiments, amino or cationic lipids as defined herein have at least one protonatable or deprotonatable group, such that the lipid is positively charged at a pH at or below physiological pH (e.g. pH 7.4), and neutral at a second pH, preferably at or above physiological pH. It will, of course, be understood that the addition or removal of protons as a function of pH is an equilibrium process, and that the reference to a charged or a neutral lipid refers to the nature of the predominant species and does not require that all of lipids have to be present in the charged or neutral form. Lipids having more than one protonatable or deprotonatable group, or which are zwitterionic, are not excluded and may likewise suitable in the context of the present invention. In some embodiments, the protonatable lipids have a pKa of the protonatable group in the range of about 4 to about 11, e.g., a pKa of about 5 to about 7. LNPs can comprise two or more (different) cationic lipids as defined herein. Cationic lipids may be selected to contribute to different advantageous properties. For example, cationic lipids that differ in properties such as amine pKa, chemical stability, half-life in circulation, half-life in tissue, net accumulation in tissue, or toxicity can be used in the LNP. In particular, the cationic lipids can be chosen so that the properties of the mixed-LNP are more desirable than the properties of a single-LNP of individual lipids.

The amount of the permanently cationic lipid, lipidoid or preferably ionizable cationic lipid may be selected taking the amount of the nucleic acid cargo into account. In one embodiment, these amounts are selected such as to result in an N/P ratio of the nanoparticle(s) or of the composition in the range from about 0.1 to about 20, or
(i) at an amount such as to achieve an N/P ratio in the range of about 1 to about 20, preferably about 2 to about 15, more preferably about 3 to about 10, even more preferably about 4 to about 9, most preferably about 6;
(ii) at an amount such as to achieve an N/P ratio in the range of about 5 to about 20, more preferably about 10 to about 18, even more preferably about 12 to about 16, most preferably about 14;
(iii) at an amount such as to achieve a lipid : mRNA weight ratio in the range of 20 to 60, preferably from about 3 to about 15, 5 to about 13, about 4 to about 8 or from about 7 to about 11; or
(iv) at an amount such as to achieve an N/P ratio in the range of about 6 for a lipid nanoparticle according to the invention, especially a lipid nanoparticle comprising the cationic lipid III-3.

In other preferred embodiments, the N/P ratio can be in the range of about 1 to about 50. In other embodiments, the range is about 1 to about 20, and preferably about 1 to about 15. For the inventive lipid nanoparticles, a preferred N/P (lipid to RNA mol ratio) is about 14 or about 17. A further preferred N/P i.e. lipid to RNA mol ratio is about 6. Another preferred N/P ratio is about 4.85 or 5 (lipid to RNA mol ratio).

In very preferred embodiments, the amount of the ionizable cationic lipid is selected taking the amount of the nucleic acid cargo into account, at an amount such as to achieve an N/P ratio in the range of about 12 to about 16, most preferably about 14.

In this context, the N/P ratio is defined as the mole ratio of the nitrogen atoms ("N") of the basic nitrogen-containing groups of the lipid or lipidoid to the phosphate groups ("P") of the nucleic acid which is used as cargo. The N/P ratio may be calculated on the basis that, for example, 1 µg RNA typically contains about 3 nmol phosphate residues, provided that the RNA exhibits a statistical distribution of bases. The "N"-value of the cationic lipid or lipidoid may be calculated on the basis of its molecular weight and the relative content of permanently cationic and - if present - cationisable groups. If more than one cationic lipid is present, the N-value should be calculated on the basis of all cationic lipids comprised in the lipid nanoparticles.

In other aspects, the ionizable lipids of the present disclosure may be one or more of compounds of Formula (Cat-II): 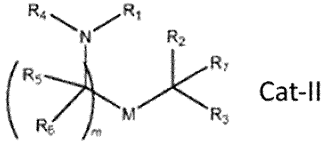 or their N-oxides, or salts or isomers thereof, wherein:
R₁ is selected from the group consisting of C5-30 alkyl, C5-20 alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and - R*OR", or R₂ and R₃, together with the atom to which they are attached, form a heterocycle or carbocycle;
R₄ is selected from the group consisting of hydrogen, a C₃₋₆ carbocycle, -(CH₂)ₙQ, -(CH₂)ₙCHQR, -CHQR, -CQ(R)₂, and unsubstituted C₁₋₆ alkyl, where Q is selected from a carbocycle, heterocycle, -OR, - O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, - N(R)C(O)N(R)₂, -N(R)C(S)N(R)_{2,} -N(R)R₈, -N(R)S(O)₂RS, -O(CH₂)ₙOR, -N(R)C(=NR₉)N(R)₂, -N(R)C(=CHR₉)N(R)₂, -OC(O)N(R)₂, -N(R)C(O)OR, -N(OR)C(O)R, -N(OR)S(O)₂R, -N(OR)C(O)OR, -N(OR)C(O)N(R)₂, -N(OR)C(S)N(R)₂, -N(OR)C(=NR₉)N(R)₂, -N(OR)C(=CHR₉)N(R)₂, -C(=NR₉)N(R)₂, -C(=NR₉)R, -C(O)N(R)OR, and - C(R)N(R)₂C(O)OR, and each n is independently selected from 1, 2, 3, 4, and 5;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -OC(O)-M"-C(O)O-, -C(O)N(R')-, -N(R')C(O)-, -C(O)-, -C(S)-, -C(S)S-, -SC(S)-, -CH(OH)-, -P(O)(OR')O-, -S(O)₂-, -S -S-, an aryl group, and a heteroaryl group, in which M" is a bond, C₁₋₁₃ alkyl or C₂₋₁₃ alkenyl;
R₇ is selected from the group consisting of C₁₋₃ alkyl, C₂₋₃ alkenyl, and H;
R₈ is selected from the group consisting of C₃₋₆ carbocycle and heterocycle;
R₉ is selected from the group consisting of H, CN, NO₂, C₁₋₆ alkyl, -OR, -S(O)₂R, -S(O)₂N(R)₂, C₂₋₆ alkenyl, C₃₋₆ carbocycle and heterocycle;
each R is independently selected from the group consisting of C₁₋₃ alkyl, C₂₋₃ alkenyl, and H;
each R' is independently selected from the group consisting of C₁₋₁₃ alkyl, C₂₋₁₈ alkenyl, -R*YR", -YR", and H;
each R" is independently selected from the group consisting of C₃-₁₅ alkyl and C₃-₁₅ alkenyl;
each R* is independently selected from the group consisting of C₁₋₁₂ alkyl and C₂-₁₂ alkenyl;
each Y is independently a C₃₋₆ carbocycle;
each X is independently selected from the group consisting of F, Cl, Br, and I; and m is selected from 5, 6, 7, 8, 9, 10, 11, 12, and 13; and wherein when R₄ is -(CH₂)ₙQ, -(CH₂)ₙCHQR, -CHQR, or -CQ(R)₂, then (i) Q is not - N(R)₂ when n is 1, 2, 3, 4 or 5, or (ii) Q is not 5, 6, or 7-membered heterocycloalkyl when n is 1 or 2.

As used herein, the term "ionizable lipid" has its ordinary meaning in the art and may refer to a lipid comprising one or more charged moieties. In some embodiments, an ionizable lipid may be positively charged or negatively charged. An ionizable lipid may be positively charged, in which case it can be referred to as "cationic lipid". In certain embodiments, an ionizable lipid molecule may comprise an amine group, and can be referred to as an ionizable amino lipid. As used herein, a "charged moiety" is a chemical moiety that carries a formal electronic charge, e.g., monovalent (+1, or -1), divalent (+2, or -2), trivalent (+3, or -3), etc. The charged moiety may be anionic (i.e., negatively charged) or cationic (i.e., positively charged). Examples of positively-charged moieties include amine groups (e.g., primary, secondary, and/or tertiary amines), ammonium groups, pyridinium group, guanidine groups, and imidazolium groups. In a particular embodiment, the charged moieties comprise amine groups. Examples of negatively- charged groups or precursors thereof, include carboxylate groups, sulfonate groups, sulfate groups, phosphonate groups, phosphate groups, hydroxyl groups, and the like. The charge of the charged moiety may vary, in some cases, with the environmental conditions, for example, changes in pH may alter the charge of the moiety, and/or cause the moiety to become charged or uncharged. In general, the charge density of the molecule may be selected as desired. It should be understood that the terms "charged" or "charged moiety" does not refer to a "partial negative charge" or "partial positive charge" on a molecule. The terms "partial negative charge" and "partial positive charge" are given its ordinary meaning in the art. A "partial negative charge" may result when a functional group comprises a bond that becomes polarized such that electron density is pulled toward one atom of the bond, creating a partial negative charge on the atom. Those of ordinary skill in the art will, in general, recognize bonds that can become polarized in this way. In some embodiments, the ionizable lipid is an ionizable amino lipid, sometimes referred to in the art as an "ionizable cationic lipid". In one embodiment, the ionizable amino lipid may have a positively charged hydrophilic head and a hydrophobic tail that are connected via a linker structure.

Interestingly, the inventors found that one of the advantageous features of the inventive compositions and lipid nanoparticles, e.g. the GN01 formulation comprising a polymer conjugated lipid according to formula (I), e.g. PMOZ, is capable of inducing strong CD8+ T cells responses. This is due to the fact, that e.g. for malaria, as CD8+ T cells are a major protective immune mechanism against intracellular infections caused by Malaria parasites, an effective Malaria vaccine should induce strong CD8+ T cells responses.

A particularly preferred embodiment for a lipid nanoparticle of the present invention is given when the following combination of excipients is used for formulating a lipid nanoparticle designated "GN01": 59 mol% cationic lipid C23 as disclosed in **Table 1,** i.e. COATSOME^{®} SS-EC (former name: SS-33/4PE-15; NOF Corporation, Tokyo, Japan), 29.3 mol% cholesterol as steroid, 10 mol% DPhyPE (4ME 16:0 PE) as neutral lipid / phospholipid and 1.7 mol% PMOZ-1,2-dimyristoyl-sn-glycero-3-phosphoethanolamine (DMPE; 14:0 PE) or preferably DMG-PMOZ (i.e. PMOZ-equivalent to 1,2-dimyristoyl-rac-glycero-3-methoxypolyethylene glycol-2000 i.e. DMG-PEG 2000) as polymer conjugated lipid. Such LNPs comprising the cationic lipid C23 are designated "GN01" herein. With respect to the molar ratios mentioned in this paragraph, any GN01-LNP composition comprising a polymer conjugated lipid according to formula (I) is called herein and in the working examples "GN01-PMOZ". SS-EC has a positive charge at pH 4 and a neutral charge at pH 7, which is advantageous for the LNPs and formulations / compositions of the present invention. For "GN01-PMOZ", N/P (lipid to mRNA mol ratio) preferably is 14 and total lipid/mRNA mass ratio preferably is 40 (m/m).

A further particularly preferred embodiment for a lipid nanoparticle of the present invention is given when the following combination of excipients is used for formulating a lipid nanoparticle, i.e. 59 mol% C2 or C24 lipid as disclosed in **Table 1** as cationic lipid (i.e. HEXA-C5DE-PipSS, cationic lipid compound C2 in **Table 1,** or respectively VitE-C4DE-Piperidine-Thioether, cationic lipid compound C24 in **Table 1),** 29.3 mol% cholesterol as steroid, 10 mol% DPhyPE as neutral lipid / phospholipid and 1.7 mol% PMOZ-DMPE or preferably DMG-PMOZ as polymer conjugated lipid. Such LNPs comprising the cationic lipid C2 are designated "GN02" herein. With respect to the molar ratios mentioned in this paragraph, any GN02-LNP composition comprising a polymer conjugated lipid according to formula (I) is called herein and in the working examples "GN02-PMOZ". For "GN02-PMOZ", N/P (lipid to mRNA mol ratio) is 17.5, more preferably 14, and total lipid/mRNA mass ratio preferably is 40 (m/m).

Furthermore, for a preferred composition, the
(i) cationic lipid may be selected from the compounds of **Table 1;** and/or the
(ii) neutral lipid or neutral phospholipid is a zwitterionic compound selected from the group consisting of 1,2-diphytanoyl-sn-glycero-3-phosphoethanolamine (DPhyPE; also referred to as 1,2-di-(3,7,11,15-tetramethylhexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; also referred to as dioleoylphosphatidylcholine), 1,2-Dipalmitoyl-sn-glycero-3-phosphocholine (DPPC, also referred to as dipalmitoylphosphatidylcholine), 1,2-dioleoyl-sn-glycero-3-phosphoethanolamine (DOPE), phosphatidylethanolamines, distearoylphosphatidylcholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyloleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Dimyristoyl-sn-glycero-3-phosphoethanolamine (DMPE), dipalmitoyl phosphatidyl ethanolamine (DPPE), dimyristoylphosphoethanolamine (DMPE), 1,2-Dilinoleoyl-sn-glycero-3-phosphoethanolamine (DLoPE), distearoyl-phosphatidylethanolamine (DSPE), 1-Palmitoyl-2-oleoyl-sn-glycero-3-phosphoethanolamine (POPE), 1,2-Dilauroyl-sn-glycero-3-phosphoethanolamine (DLPE), 16-O-monomethyl-phosphoethanolamine, 16-O-dimethyl phosphatidylethanolamine, 1,2-Dierucoyl-sn-glycero-3-phosphoethanol-amine (DEPE), 18-1-trans phosphatidylethanolamine, 1-stearoyl-2-oleoylphosphatidylethanolamine (SOPE), 1,2-Disqualeoyl-sn-glycero-3-phosphoethanolamine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodium salt), 1-O-hexadecanyl-2-O-(9Z-octadecenyl)-sn-glycero-3-phosphoethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesterylhemisuccinoyl-sn-glycero-3-phosphocholine (PChemsPC), 1,2-dicholesterylhemisuccinoyl-sn-glycero-3-phosphocholine (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine); and/or
(iii) the polymer conjugated lipid is a polymer conjugated lipid according to formula (I):

   [P]-[linker]-[L] formula (I),

   wherein
   [P] is a homopolymer moiety comprising at least one polyoxazoline (POZ) monomer unit 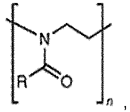
   wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa
   [linker] is an optional linker group, and
   [L] is a lipid moiety.

In other preferred embodiments, the lipid-based carriers comprise a cationic or ionizable lipid.

The cationic or ionizable lipid of the lipid-based carriers may be cationisable or ionizable, i.e. it becomes protonated as the pH is lowered below the pK of the ionizable group of the lipid, but is progressively more neutral at higher pH values. At pH values below the pK, the lipid is then able to associate with negatively charged nucleic acids. In certain embodiments, the cationic lipid comprises a zwitterionic lipid that assumes a positive charge on pH decrease.

In preferred embodiments, the lipid-based carriers comprise a cationic or ionizable lipid that preferably carries a net positive charge at physiological pH, more preferably the cationic or ionizable lipid comprises a tertiary nitrogen group or quaternary nitrogen group. Accordingly, in preferred embodiments, the lipid-based carriers comprise a cationic or ionizable lipid selected from an amino lipid.

In further embodiments, the lipid formulation comprises cationic or ionizable lipids as defined in Formula I of paragraph [00251] of WO2021222801 or a lipid selected from the disclosure of paragraphs [00260] or [00261] of WO2021222801. In other embodiments, the lipid formulation comprises cationic or ionizable lipids selected from the group consisting of ATX-001 to ATX-132 as disclosed in claim 90 of WO2021183563, preferably ATX-0126. The disclosure of WO2021222801 and WO2021183563, especially aforementioned lipids, are incorporated herewith by reference.

Further suitable cationic lipids may be selected or derived from cationic lipids according to each of PCT claims 1 to **14** of published patent application WO2021123332, or table 1 of WO2021123332, the disclosure relating to each of claims 1 to **14** or table 1 of WO2021123332 herewith incorporated by reference. Accordingly, suitable cationic lipids may be selected or derived from cationic lipids according Compound 1 to Compound 27 (C1-C27) of Table 1 of WO2021123332.

In other preferred embodiments, the lipid-based carriers (e.g. LNPs) of the pharmaceutical composition comprise a cationic lipid selected or derived from (COATSOME^{®} SS-EC) SS-33/4PE-15 (see C23 in Table 1 of WO2021123332).

In other preferred embodiments, the lipid-based carriers (e.g. LNPs) of the pharmaceutical composition comprise a cationic lipid selected or derived from HEXA-C5DE-PipSS (see C2 in Table 1 of WO2021123332). In most preferred embodiments, the lipid-based carriers (e.g. LNPs) of the pharmaceutical composition comprise a cationic lipid selected or derived from compound C26 as disclosed in Table 1 of WO2021123332:

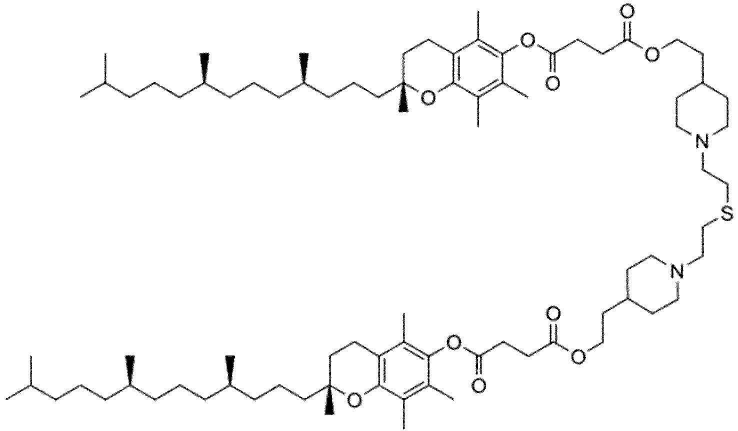

In other embodiments, the lipid-based carriers (e.g. LNPs) of the pharmaceutical composition comprise a cationic lipid selected orderived from 9-Heptadecanyl 8-{(2-hydroxyethyl)[6-oxo-6-(undecyloxy)hexyl]amino}octanoate, also referred to as SM-102. Other preferred lipid-based carriers (e.g. LNPs) of the pharmaceutical composition comprise a squaramide ionizable amino lipid, more preferably a cationic lipid selected from the group consisting of formulas (M1) and (M2): 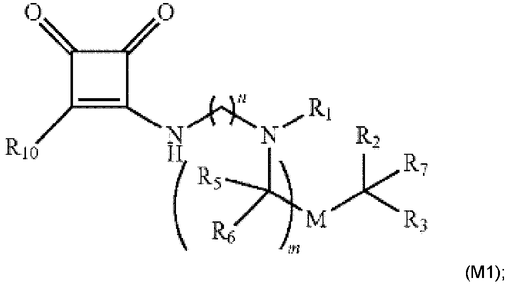 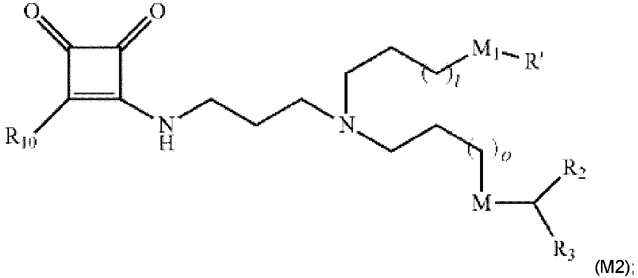 wherein the substituents (e.g. R₁, R₂, R₃, R₅, R₆, R₇, R₁₀, M, M₁, m, n, o, l) are defined in claims 1 to 13 of US10392341B2; US10392341B2 being incorporated herein in its entirety.

Accordingly, in preferred embodiments, the lipid-based carriers (e.g. LNPs) of the pharmaceutical composition comprise a cationic lipid selected or derived from above mentioned ALC-0315, SM-102, SS-33/4PE-15, HEXA-C5DE-PipSS, or compound C26 (see C26 in Table 1 of WO2021123332).

In particularly preferred embodiments, the lipid-based carriers, preferably the LNPs of the pharmaceutical composition comprise a cationic lipid selected or derived from ALC-0315.

In some embodiments, the lipid-based carriers of the invention comprise two or more (different) cationic lipids as defined herein.

In certain embodiments, the cationic lipid as defined herein, more preferably cationic lipid ALC-0315, is present in the lipid-based carriers in an amount from about 30mol% to about 95mol%, relative to the total lipid content of the lipid-based carriers. If more than one cationic lipid is incorporated within the lipid-based carriers, such percentages apply to the combined cationic lipids.

In embodiments, the cationic lipid is present in the lipid-based carriers in an amount from about 30mol% to about 70mol%. In one embodiment, the cationic lipid is present in the lipid-based carriers in an amount from about 40mol% to about 60mol%, such as about 40, 41, 42, 43, 44, 45, 46, 47, 48, 49, 50, 51, 52, 53, 54, 55, 56, 57, 58, 59 or 60mol%, respectively. In embodiments, the cationic lipid is present in the lipid-based carriers in an amount from about 47mol% to about 48mol%, such as about 47.0, 47.1, 47.2, 47.3, 47.4, 47.5, 47.6, 47.7, 47.8, 47.9, 50.0mol%, respectively, wherein 47.4mol% are particularly preferred. In other preferred embodiments, the cationic lipid is present in the lipid-based carriers in an amount from about 55mol% to about 65mol%, such as about 55, 56, 57, 58, 59, 60, 61, 62, 63, 64 or 65mol%, respectively, wherein 59mol% are particularly preferred.

In some embodiments, the cationic lipid is present in a ratio of from about 20mol% to about 70mol% or 75mol% or from about 45mol% to about 65mol% or about 20, 25, 30, 35, 40, 45, 50, 55, 60, 65, or about 70mol% of the total lipid present in the lipid-based carriers. In further embodiments, the LNPs comprise from about 25% to about 75% on a molar basis of cationic lipid, e.g., from about 20 to about 70%, from about 35 to about 65%, from about 45 to about 65%, about 60%, about 57.5%, about 57.1%, about 50% or about 40% on a molar basis (based upon 100% total moles of lipid in the lipid nanoparticle).

In some embodiments, the ratio of cationic lipid to RNA is from about 3 to about 15, such as from about 5 to about 13 or from about 7 to about 11.

### Steroid

A "steroid" is an organic compound with four rings arranged in a specific molecular configuration. It comprises the following carbon skeleton:

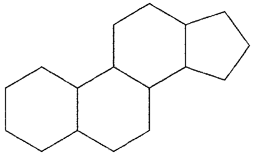

Steroids and neutral steroids include both naturally occurring steroids and analogues thereof (e.g. being amphipathic lipid cholesteryl hemisuccinate (CHEMS) which consists of succinic acid esterified to the beta-hydroxyl group of cholesterol as cholesterol derivate). Using the definition for "neutral" as provided herein, the neutral steroid may be a steroid either having no atoms or groups that are ionizable under physiological conditions, or it may be a zwitterionic steroid. In one of the preferred embodiments, the neutral steroid is free of atoms or groups that are ionizable under physiological conditions. In some preferred embodiments, the steroid or steroid analogue is cholesterol. The term "steroid" and "neutral steroid" is used herein interchangeably. In other embodiments, the sterol may be selected from the group consisting of a phytosterol, e.g. β-sitosterol, campesterol, stigmasterol, fucosterol, stigmastanol, dihydrocholesterol, ent-cholesterol, epi-cholesterol, desmosterol, cholestanol, cholestanone, cholestenone, cholesteryl-2'-hydroxyethyl ether, cholesteryl-4'-hydroxybutyl ether, 3β-[N-(N'N'-dimethylaminoethyl)carbamoyl cholesterol (DC-Chol), 24(S)-hydroxycholesterol, 25-hydroxy cholesterol, 25(R)-27-hydroxycholesterol, 22-oxacholesterol, 23-oxacholesterol, 24-oxacholesterol, cycloartenol, 22-ketosterol, 20-hydroxysterol, 7-hydroxycholesterol, 19-hydroxycholesterol, 22-hydroxycholesterol, 25-hydroxy cholesterol, 7-dehydrocholesterol, 5a-cholest-7-en-3β-ol, 3,6,9-trioxaoctan-1-ol-cholesteryl-3e-ol, dehydroergosterol, dehydroepiandrosterone, lanosterol, dihydrolanosterol, lanostenol, lumisterol, sitocalciferol, calcipotriol, coprostanol, cholecalciferol, lupeol, ergocalciferol, 22-dihydroegocalciferol, ergosterol, brassicasterol, tomatidine, tomatine, ursolic acid, cholic acid, chenodeoxycholic acid, zymosterol, diosgenin, fucosterol, fecosterol, or fecosterol, or a salt or ester thereof, cholesterol, cholesterol succinic acid, cholesterol sulfate, cholesterol hemisuccinate, cholesterol phthalate, cholesterol phosphate, cholesterol valerate, cholesterol acetate, cholesteryl oleate, cholesteryl linoleate, cholesteryl myristate, cholesteryl palmitate, cholesteryl arachidate, cholesteryl phosphorylcholine, and sodium cholate.

In a further embodiment, the steroid is an imidazole cholesterol ester or "ICE" as disclosed in paragraphs [0320] and [0339]-[0340] of WO2019226925; which is herein incorporated by reference in its entirety.

In other embodiments, the polymer conjugated lipid of the invention is a POZ-steroid or POZ-sterol conjugate. In further embodiments, the **lipid moiety [L] from formula (I)** (i.e. [P]-[linker]-[L]) comprises a steroid, cholesterol, or a cholesterol-derivate, selected from the group consisting of a phytosterol, e.g. β-sitosterol, campesterol, stigmasterol, fucosterol, stigmastanol, dihydrocholesterol, ent-cholesterol, epi-cholesterol, desmosterol, cholestanol, cholestanone, cholestenone, cholesteryl-2'-hydroxyethyl ether, cholesteryl-4'-hydroxybutyl ether, 3β-[N-(N'N'- dimethylaminoethyl)carbamoyl cholesterol (DC-Chol), 24(S)-hydroxycholesterol, 25-hydroxy cholesterol, 25(R)-27-hydroxycholesterol, 22-oxacholesterol, 23-oxacholesterol, 24-oxacholesterol, cycloartenol, 22-ketosterol, 20-hydroxysterol, 7-hydroxycholesterol, 19-hydroxycholesterol, 22-hydroxycholesterol, 25-hydroxy cholesterol, 7-dehydrocholesterol, 5a-cholest-7-en-3β-ol, 3,6,9-trioxaoctan-1-ol-cholesteryl-3e-ol, dehydroergosterol, dehydroepiandrosterone, lanosterol, dihydrolanosterol, lanostenol, lumisterol, sitocalciferol, calcipotriol, coprostanol, cholecalciferol, lupeol, ergocalciferol, 22-dihydroegocalciferol, ergosterol, brassicasterol, tomatidine, tomatine, ursolic acid, cholic acid, chenodeoxycholic acid, zymosterol, diosgenin, fucosterol, fecosterol, or fecosterol, or a salt or ester thereof, cholesterol, cholesterol succinic acid, cholesterol sulfate, cholesterol hemisuccinate, cholesterol phthalate, cholesterol phosphate, cholesterol valerate, cholesterol acetate, cholesteryl oleate, cholesteryl linoleate, cholesteryl myristate, cholesteryl palmitate, cholesteryl arachidate, cholesteryl phosphorylcholine, and sodium cholate, imidazole cholesterol ester or "ICE", or derivates thereof.

In other preferred embodiments, the lipid-based carriers of the pharmaceutical composition comprise a steroid, steroid analogue or sterol.

Suitably, the steroid, steroid analogue or sterol may be derived or selected from cholesterol, cholesteryl hemisuccinate (CHEMS) and a derivate thereof. In other embodiments, the lipid-based carriers of the pharmaceutical composition comprise a steroid, steroid analogue or sterol derived from a phytosterol (e.g., a sitosterol, such as beta-sitosterol), preferably from a compound having the structure of Formula I as disclosed in claim 1 of WO2020061332; the disclosure of WO2020061332, especially the disclosure of Formula I and phytosterols being incorporated by reference herewith. In a further embodiment, the steroid is an imidazole cholesterol ester or "ICE" as disclosed in paragraphs [0320] and [0339]-[0340] of WO2019226925; WO2019226925 being incorporated herein by reference in its entirety.

In particularly preferred embodiments, the lipid-based carriers of the pharmaceutical composition comprise cholesterol.

The molar ratio of the cationic lipid to cholesterol in the lipid-based carriers may be in the range from about 2:1 to about 1:1.

In some embodiments, the lipid-based carrier comprises about 10mol% to about 60mol% or about 25mol% to about 40mol% sterol (based on 100% total moles of lipids in the lipid-based carrier). In one embodiment, the sterol is about 10, 15, 20, 25, 30, 35, 40, 45, 50, 55, or about 60mol% of the total lipid present in the lipid-based carrier. In another embodiment, the lipid-based carriers include from about 5% to about 50% on a molar basis of the sterol, e.g., about 15% to about 45%, about 20% to about 40%, about 48%, about 40%, about 38.5%, about 35%, about 34.4%, about 31.5% or about 30% on a molar basis (based upon 100% total moles of lipid in the lipid-based carrier). In preferred embodiments, the lipid-based carrier comprises about 28%, about 29% or about 30% sterol (based on 100% total moles of lipids in the lipid-based carrier). In most preferred embodiments, the lipid-based carrier comprises about 40.9% sterol (based on 100% total moles of lipids in the lipid-based carrier).

References to other suitable cationic or ionizable, neutral, steroid/sterol or aggregation reducing lipids:
Other suitable cationic or ionizable, neutral, steroid/sterol or aggregation reducing lipids are disclosed in WO2010053572, WO2011068810, WO2012170889, WO2012170930, WO2013052523, WO2013090648, WO2013149140, WO2013149141, WO2013151663, WO2013151664, WO2013151665, WO2013151666, WO2013151667, WO2013151668, WO2013151669, WO2013151670, WO2013151671, WO2013151672, WO2013151736, WO2013185069, WO2014081507, WO2014089486, WO2014093924, WO2014144196, WO2014152211, WO2014152774, WO2014152940, WO2014159813, WO2014164253, WO2015061461, WO2015061467, WO2015061500, WO2015074085, WO2015105926, WO2015148247, WO2015164674, WO2015184256, WO2015199952, WO2015200465, WO2016004318, WO2016022914, WO2016036902, WO2016081029, WO2016118724, WO2016118725, WO2016176330, WO2017004143, WO2017019935, WO2017023817, WO2017031232, WO2017049074, WO2017049245, WO2017070601, WO2017070613, WO2017070616, WO2017070618, WO2017070620, WO2017070622, WO2017070623, WO2017070624, WO2017070626, WO2017075038, WO2017075531, WO2017099823, WO2017106799, WO2017112865, WO2017117528, WO2017117530, WO2017180917, WO2017201325, WO2017201340, WO2017201350, WO2017201352, WO2017218704, WO2017223135, WO2018013525, WO2018081480, WO2018081638, WO2018089540, WO2018089790, WO2018089801, WO2018089851, WO2018107026, WO2018118102, WO2018119163, WO2018157009, WO2018165257, WO2018170245, WO2018170306, WO2018170322, WO2018170336, WO2018183901, WO2018187590, WO2018191657, WO2018191719, WO2018200943, WO2018231709, WO2018231990, WO2018232120, WO2018232357, WO2019036000, WO2019036008, WO2019036028, WO2019036030, WO2019040590, WO2019089818, WO2019089828, WO2019140102, WO2019152557, WO2019152802, WO2019191780, WO2019222277, WO2019222424, WO2019226650, WO2019226925, WO2019232095, WO2019232097, WO2019232103, WO2019232208, WO2020061284, WO2020061295, WO2020061332, WO2020061367, WO2020081938, WO2020097376, WO2020097379, WO2020097384, WO2020102172, WO2020106903, WO2020146805, WO2020214946, WO2020219427, WO2020227085, WO2020232276, WO2020243540, WO2020257611, WO2020257716, WO2021007278, WO2021016430, WO2021022173, WO2021026358, WO2021030701, WO2021046260, WO2021050986, WO2021055833, WO2021055835, WO2021055849, WO2021127394, WO2021127641, WO2021202694, WO2021231697, WO2021231901, WO2008103276, WO2009086558, WO2009127060, WO2010048536, WO2010054406, WO2010080724, WO2010088537, WO2010129709, WO201021865, WO2011022460, WO2011043913, WO2011090965, WO2011149733, WO2011153120, WO2011153493, WO2012040184, WO2012044638, WO2012054365, WO2012061259, WO2013063468, WO2013086354, WO2013086373, US7893302B2, US7404969B2, US8158601B2, US8283333B2, US8466122B2, US8569256B2, US20100036115, US20110256175, US20120202871, US20120027803, US20120128760, US20130064894, US20130129785, US20130150625, US20130178541, US20130225836, and US20140039032; the disclosures specifically relating to cationic or ionizable, neutral, sterol or aggregation reducing lipids suitable for lipid-based carriers of the foregoing publications are incorporated herewith by reference.

For example, suitable cationic lipids or cationisable or ionizable lipids include, but are not limited to, DSDMA, N,N-dioleyl-N,N-dimethylammonium chloride (DODAC), N,N-distearyl-N,N-dimethylammonium bromide (DDAB), 1,2-dioleoyltrimethyl ammonium propane chloride (DOTAP) (also known as N-(2,3-dioleoyloxy)propyl)-N,N,N-trimethylammonium chloride and 1,2-Dioleyloxy-3-trimethylaminopropane chloride salt), N-(1-(2,3-dioleyloxy)propyl)-N,N,N-trimethylammonium chloride (DOTMA), N,N-dimethyl-2,3-dioleyloxy)propylamine (DODMA), ckk-E12 (WO2015200465), 1,2-DiLinoleyloxy-N,N-dimethylaminopropane (DLinDMA), 1,2-Dilinolenyloxy-N,N-dimethylaminopropane (DLenDMA), 1,2-di-y-linolenyloxy-N,N-dimethylaminopropane (γ-DLenDMA), 98N12-5, 1,2-Dilinoleylcarbamoyloxy-3-dimethylaminopropane (DLin-C-DAP), ICE (Imidazol-based), HGT5000, HGT5001, DMDMA, CLinDMA, CpLinDMA, DMOBA, DOcarbDAP, DLincarbDAP, DLinCDAP, KLin-K-DMA, DLin-K-XTC2-DMA, XTC (2,2-Dilinoleyl-4-dimethylaminoethyl-[1,3]-dioxolane) HGT4003, 1,2-Dilinoleoyl-3-trimethylaminopropane chloride salt (DLin-TAP.Cl), 1,2-Dilinoleyloxo-3-(2-N,N-dimethylamino)ethoxypropane (DLin-EG-DM A), 2,2-Dilinoleyl-4-dimethylaminomethyl-[1,3]-dioxolane (DLin-K-DMA), (6Z,9Z,28Z,31Z)-heptatriaconta-6,9,28,31-tetraen-19-yl-4-(dimethylamino)butanoate (MC3, US20100324120), ALNY-100 ((3aR,5s,6aS)-N,N-dimethyl-2,2-di((9Z,12Z)-octadeca-9,12-dienyl)tetrahydro-3aH-cyclopenta[d] [1,3]dioxol-5-amine)), NC98-5 (4,7, 13-tris(3-oxo-3-(undecylamino)propyl)-N,N 16-diundecyl-4,7, 10,13-tetraazahexadecane-l,16-diamide), (6Z,9Z,28Z,31Z)-heptatriaconta-6,9,28,31-tetraen-19-yl 4-(dimethylamino) butanoate (DLin-M-C3-DMA), 3-((6Z,9Z,28Z,31Z)-heptatriaconta-6,9,28,31-tetraen-19-yloxy)-N,N-dimethylpropan-1-amine (MC3 Ether), 4-((6Z,9Z,28Z,31Z)-heptatriaconta-6,9,28,31-tetraen-19-yloxy)-N,N-dimethylbutan-1-amine (MC4 Ether), LIPOFECTIN^{®} (commercially available cationic liposomes comprising DOTMA and 1,2-dioleoyl-sn-3phosphoethanolamine (DOPE), from GIBCO/BRL, Grand Island, N.Y.); LIPOFECTAMINE^{®} (commercially available cationic liposomes comprising N-(1-(2,3dioleyloxy)propyl)-N-(2-(sperminecarboxamido)ethyl)-N,N-dimethylammonium trifluoroacetate (DOSPA) and (DOPE), from GIBCO/BRL); and TRANSFECTAM^{®} (commercially available cationic lipids comprising dioctadecylamidoglycyl carboxyspermine (DOGS) in ethanol from Promega Corp., Madison, Wis.) or any combination of any of the foregoing. Further suitable cationic or ionizable lipids include those described in international patent publications WO2010053572 (and particularly, 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (C12-200) described at paragraph [00225] of WO2010053572) and WO2012170930, both of which are incorporated herein by reference, HGT4003, HGT5000, HGTS001, HGT5001, HGT5002 (see US2015140070), 1,2-dilinoleyoxy-3-(dimethylamino)acetoxypropane (DLin-DAC), 1,2-dilinoleyoxy-3morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3dimethylaminopropane (DLin-2-DMAP), 1,2-dilinoleyloxy-3-trimethylaminopropane chloride salt (DLin-TMA.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 2,2-dilinoleyl-4-(2-dimethylaminoethyl)-[1,3]-dioxolane (DLin-KC2-DMA, WO2010042877); dilinoleyl-methyl-4-dimethylaminobutyrate (DLin-MC3-DMA).

### Neutral lipid, neutral phospholipid

A "neutral lipid", also termed "helper lipid" according to the invention preferably is a phospholipid or neutral phospholipid. As used herein, a "neutral phospholipid" is an amphiphilic compound consisting of molecules that typically have two hydrophobic fatty acid "tails" and a hydrophilic "head" comprising a phosphate group. The phosphate group can be modified with simple organic molecules such as choline, ethanolamine or serine. Phospholipids occur abundantly in nature. For example, they represent a significant fraction of the excipients of biological membranes. As used herein, the expression "phospholipid" or "neutral phospholipid" covers both natural and synthetic phospholipids.

The terms "neutral lipid", "neutral phospholipid" or "zwitterionic compound", as used herein interchangeably, refer to any one of a number of lipid species that exist in either an uncharged or neutral zwitterionic form at physiological pH. Representative neutral lipids include diacylphosphatidylcholines, diacylphosphatidylethanolamines, ceramides, sphingomyelins, dihydro sphingomyelins, cephalins, and cerebrosides as further described herein below.

According to one of the preferred embodiments, the composition comprises a neutral lipid that is zwitterionic, such as a phosphatidylcholine or a phosphatidylethanolamine. Examples of suitable phosphatidylcholines include native or purified mixtures, sometimes referred to as "lecithin" or "phosphatidylcholine", often derived from egg yolk or soy beans; or highly purified or semisynthetic compounds such as phosphatidylcholines having two fatty acyl moieties selected from myristoyl, palmitoyl, stearoyl, oleoyl and the like.

In another preferred embodiment, the neutral lipid or neutral phospholipid is a zwitterionic compound selected from, but not limited to the group of 1,2-diphytanoyl-sn-glycero-3-phosphoethanolamine (DPhyPE; also referred to as 1,2-di-(3,7,11,15-tetramethylhexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; also referred to as dioleoylphosphatidylcholine), 1,2-Dipalmitoyl-sn-glycero-3-phosphocholine (DPPC, also referred to as dipalmitoylphosphatidylcholine), 1,2-dioleoyl-sn-glycero-3-phosphoethanolamine (DOPE), phosphatidylethanolamines, distearoylphosphatidylcholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyloleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Dimyristoyl-sn-glycero-3-phosphoethanolamine (DMPE), 1,2-Dilinoleoyl-sn-glycero-3-phosphoethanolamine (DLoPE), distearoyl-phosphatidylethanolamine (DSPE), 1-Palmitoyl-2-oleoyl-sn-glycero-3-phosphoethanolamine (POPE), 1,2-Di-lauroyl-sn-glycero-3-phosphoethanolamine (DLPE), 16-0-monomethylphosphoethanolamine, 16-O-dimethyl phosphatidylethanolamine, 1,2-Dierucoyl-sn-glycero-3-phosphoethanolamine (DEPE), 18-1-trans phosphatidylethanolamine, 1-stearoyl-2-oleoylphosphatidyethanolamine (SOPE), 1,2-Disqualeoyl-sn-glycero-3-phospho-ethanolamine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodium salt), 1-O-hexadecanyl-2-O-(9Z-octadecenyl)-sn-glycero-3-phosphoethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesterylhemisuccinoyl-sn-glycero-3-phosphocholine (PChemsPC), 1,2-dicholesterylhemisuccinoyl-sn-glycero-3-phosphocholine (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine).

In other preferred embodiments, the inventive lipid nanoparticle further comprises "DPhyPS" or "WT-PS" (i.e. 1-stearoyl-2-oleoyl-sn-glycero-3-phospho-L-serine or 18:0-18:1 PS, in accordance with the two different fatty acid / alkyl chains of WT-PS which is distributed widely among animals, plants and microorganisms), 16:0-PS, 14:0-PS, 10:0-PS, 6:0-PS and 18:1-PS DOPS, the serine is bound to a first carbon atom of the glycerine via a phosphodiester while the second and third carbon atoms of the glycerine are bound to a fatty acid, each via an ester. The structures of the phosphatidylserines mentioned above are as follows (it is noted that all of these lipids are commercially available, e.g. at Avanti Polar Lipids): 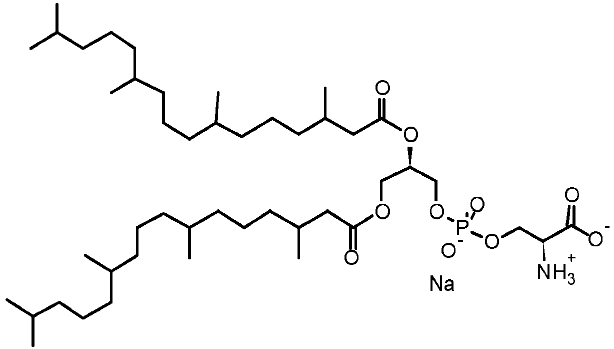 DPhyPS 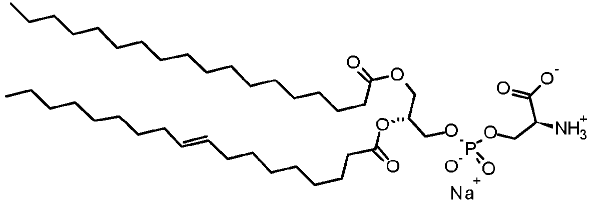 WT-PS (18:0-18:1 PS) 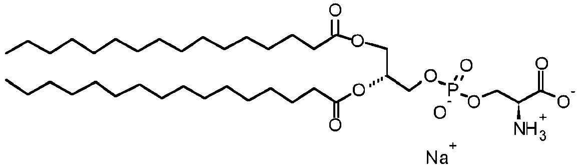 16:0 PS 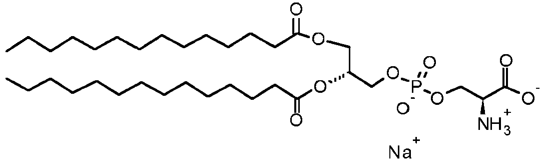 14:0 PS 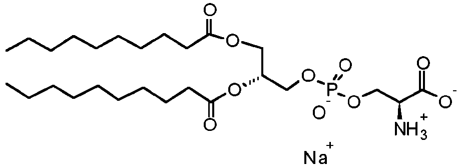 10:0 PS 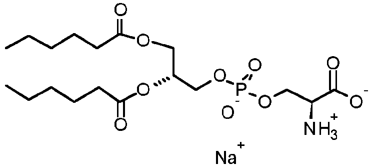 6:0 PS 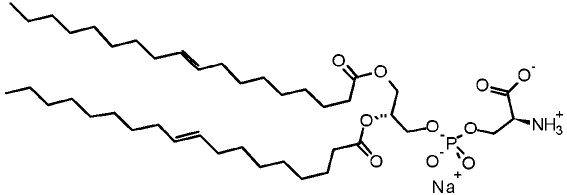 18:1 PS DOPS 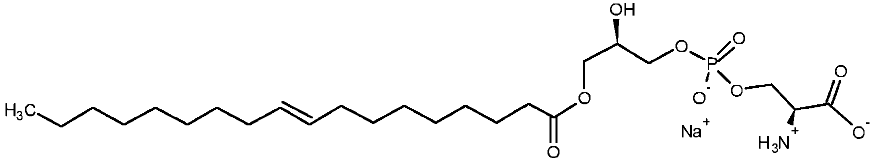 18:1 Lyso PS 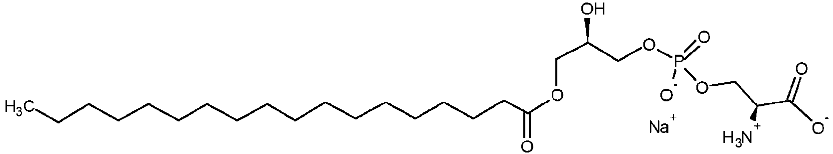 18:0 Lyso PS

In this constellation, the two fatty acids may be identical (see e.g. DPhyPS, 16:0 PS, 14:0-PS, 10:0-PS, 6:0-PS and 18:1-PS DOPS ) or may be different (see e.g. WT-PS or 18:0-18:1 PS). In other examples, e.g. in the case of 18:1-Lyso PS and 18:0-Lyso PS, the serine is again bound to a first carbon atom of the glycerine via a phosphodiester while only one further carbon atom of the glycerine is bound to a fatty acid via an ester, leaving a single OH-group at the remaining carbon atom of the glycerine. Such constellations are typically referred to as a "lysophosphatidylserine", which is included in view of the above definition in the term "phosphatidylserine" as used herein. Preferred embodiments, which are related to DPhyPS, are described herein under section "Fourth Set of Embodiments".

In other very preferred embodiments, the inventive lipid nanoparticle comprises a phosphatidylserine selected from the group consisting of DPhyPS, WT-PS, 16:0-PS, 14:0-PS, 10:0-PS, 6:0-PS, 18:1-PS DOPS, 18:1-Lyso PS and 18:0-Lyso PS. It is most preferred that the phosphatidylserine is either DPhyPS or WT-PS (18:0-18:1 PS).

In another preferred embodiment, the neutral lipid according to the invention is 1,2-Dipalmitoyl-sn-glycero-3-phosphocholine (DPPC), 1,2-dioleoyl-sn-glycero-3-phosphocholine (DOPC) or 1,2-dioleoyl-sn-glycero-3-phosphoethanolamine (DOPE). In a more preferred embodiment, the neutral lipid according to the invention is 1,2-diphytanoyl-sn-glycero-3-phosphocholine (DPhyPC). In an even more preferred particularly preferred embodiment, the neutral lipid according to the invention is 1,2-diphytanoyl-sn-glycero-3-phosphoethanolamine (DPhyPE). The inventive advantage connected with the use of DPhyPE is the high capacity for fusogenicity due to its bulky tails, whereby it is able to fuse at a high level with endosomal lipids. Therefore, in another embodiment, the invention is related to the use of a lipid with high fusogenicity in a lipid-based carrier or nucleic acid-lipid particle, preferably DPhyPE, as depicted here:

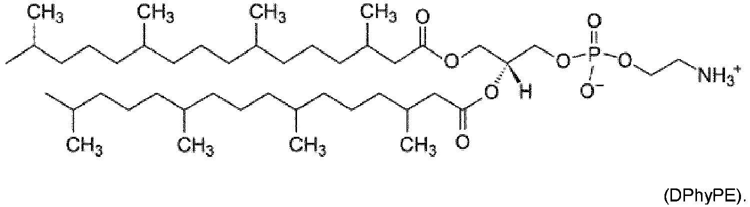

Specifically the advantageous use of 1,2-diphytanoyl-sn-glycero-3-phosphoethanolamine (DPhyPE) as disclosed herein, preferably in combination with the inventive lipids as disclosed herein, specifically for delivering mRNA vaccines in vivo resulting in significantly enhanced immune responses, is a surprising finding by the inventors resembling specific aspects and embodiments of the present invention. In other words, the inventors surprisingly found that the use of DPhyPE gave a clear advantage over DSPC which to date is used in the art as standard neutral lipid in nearly all state of the art LNP-compositions for mRNA and also siRNA, specifically, but not limited to, vaccination settings. In other words, the compositions of the invention have a highly advantageous and unexpected behaviour in vivo resulting in highly enhanced immune responses.

Further, the data presented in the Examples demonstrate significant enhanced immune responses using the compositions of the invention, i.e. all inventive RNA vaccines are useful according to the invention. Surprisingly, in contrast to prior art knowledge which shows that DSPC is the most common and unquestioned neutral lipid for lipid nanoparticles, it was found by the inventors that it is preferable to use DPhyPE for mRNA formulations in compositions for the production of vaccines.

DSPC, DOPC or DOPE, which are routinely used in the art as phospholipid in LNPs, each have two C₁₈ chains side arms as apparent from the structures shown herein below:

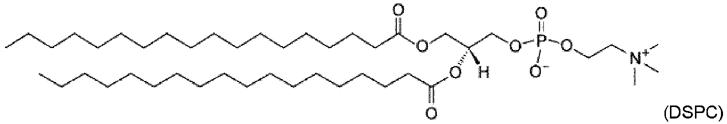

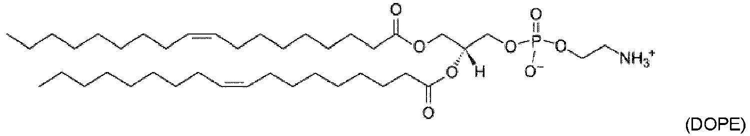

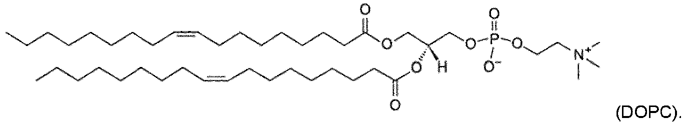

Surprisingly, in a further aspect of the invention, the inventors found that the addition of phospholipids with shorter alkyl chains than e.g. state of the art DSPC or DOPE, were highly beneficial for the efficacy of lipid nanoparticles of the invention, comprising polymer conjugated lipids according to formula (I) as when compared to lipid nanoparticles not comprising said phospholipids with shorter alkyl chains. Specifically the advantageous use of (07:0) PC (DHPC; 1,2-diheptanoyl-sn-glycero-3-phosphocholine) with shorter alkyl chains than e.g. state of the art DSPC as disclosed herein, preferably in combination with the inventive polymer conjugated lipids as disclosed herein, for delivering mRNA vaccines in vivo, resulting in significantly enhanced immune responses, is a further very surprising finding made by the inventors and resembles specific aspects and embodiments of the present invention.

The structure of (07:0) PC (DHPC; 1,2-diheptanoyl-sn-glycero-3-phosphocholine) from is shown herein below:

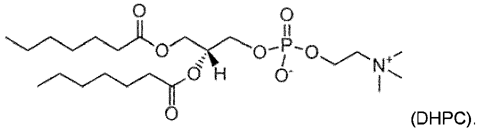

The inventors further surprisingly found that the addition of at least one further neutral lipid to the above neutral lipid, in particular a second neutral lipid, can also enhance the immune responses (see the corresponding examples). As noted above, it is preferred for the (first) neutral lipid of the invention that it has two fatty acyl moieties selected from myristoyl, palmitoyl, stearoyl, oleoyl and the like, which in particular means that the fatty acyl moieties are rather long moieties starting from moieties with 14 carbon atoms. The inventors found that the addition of a neutral lipid with shorter fatty acyl moieties provides for beneficial effects, in particular if the additional neutral lipid has two fatty acid moieties selected from pentanoyl, hexanoyl, heptanoyl, octanoyl, nonaoyl and decanoyl, i.e. moieties with at most 10 carbon atoms. A particularly preferred additional neutral lipid is 1,2-diheptanoyl-sn-glycero-3-phosphocholine (DHPC). Neutral lipids related to DHPC, such as e.g. 05:0 PC (1,2-dipentanoyl-sn-glycero-3-phosphocholine), 06:0 PC (1,2-dihexanoyl-sn-glycero-3-phosphocholine), 08:0 PC (1,2-dioctanoyl-sn-glycero-3-phosphocholine), 09:0 PC (1,2-dinonanoyl-sn-glycero-3-phosphocholine), and 10:0 PC (1,2-dihexanoyl-sn-glycero-3-phosphocholine) are comprised as alternative within this disclosure.

Therefore, in one aspect of the invention, the lipid nanoparticles of the invention comprise a neutral lipid or phospholipid having at least one alkyl chain with a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇. In another embodiment of the invention, the lipid nanoparticles of the invention comprise a neutral lipid or phospholipid having at least two alkyl chains, whereby each alkyl chain independently has a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇. In a preferred embodiment, the lipid nanoparticles of the invention comprise additionally DHPC. In a further embodiment, one or more alkyl chains may comprise carbon double-bonds.

In other embodiment, the lipid nanoparticles comprise an additional phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine).

In other preferred embodiments, the lipid-based carriers (e.g. LNPs) comprise a neutral lipid or phospholipid.

The term "neutral lipid" refers to any one of a number of lipid species that exist in either an uncharged or neutral zwitterionic form at physiological pH. Suitable neutral lipids include diacylphosphatidylcholines, diacylphosphatidylethanolamines, ceramides, sphingomyelins, dihydrosphingomyelins, cephalins, and cerebrosides. The selection of neutral lipids for use in the particles described herein is generally guided by consideration of, e.g., lipid particle size and stability of the lipid particle in the bloodstream. Preferably, the neutral lipid is a lipid having two acyl groups (e.g. diacylphosphatidylcholine and diacylphosphatidylethanolamine). In one embodiment, the neutral lipids contain saturated fatty acids with carbon chain lengths in the range of C10 to C20. In another embodiment, neutral lipids with mono or diunsaturated fatty acids with carbon chain lengths in the range of C10 to C20 are used. Additionally, neutral lipids having mixtures of saturated and unsaturated fatty acid chains can be used.

In some embodiments, the lipid-based carriers comprises one or more neutral lipids, wherein the neutral lipid is selected from the group comprising distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoylphosphatidyethanol amine (SOPE), and 1,2-dielaidoyl-sn-glycero-3-phophoethanolamine (transDOPE), 1,2-diphytanoyl-sn-glycero-3-phosphoethanolamine (DPhyPE), or mixtures thereof.

In preferred embodiments, the neutral lipid of the lipid-based carriers (e.g. LNPs) of the pharmaceutical composition is selected or derived from 1,2-diheptanoyl-sn-glycero-3-phosphocholine (DHPC).

In other preferred embodiments, the neutral lipid of the lipid-based carriers (e.g. LNPs) of the pharmaceutical composition is selected or derived from 1,2-diphytanoyl-sn-glycero-3-phosphoethanolamine (DPhyPE).

Accordingly, in preferred embodiments, the lipid-based carriers (e.g. LNPs) of the pharmaceutical composition comprise a neutral lipid selected or derived from DSPC, DHPC, or DPhyPE.

In particularly preferred embodiments, the lipid-based carriers, preferably the LNPs of the pharmaceutical composition comprise a neutral lipid selected or derived from 1,2-distearoyl-sn-glycero-3-phosphocholine (DSPC).

In various embodiments, the molar ratio of the cationic lipid to the neutral lipid in the lipid-based carriers ranges from about 2:1 to about 8:1.

The neutral lipid is preferably from about 5mol% to about 90mol%, about 5mol% to about 10mol%, about 5, 10, 15, 20, 25, 30, 35, 40, 45, 50, 55, 60, 65, 70, 75, 80, 85, or about 90mol% of the total lipid present in the lipid-based carrier. In one embodiment, the lipid-based carrier include from about 0% to about 15% or 45% on a molar basis of neutral lipid, e.g. from about 3% to about 12% or from about 5% to about 10%. For instance, the lipid-based carrier may include about 15%, about 10%, about 7.5%, or about 7.1% of neutral lipid on a molar basis (based upon 100% total moles of lipid in the lipid-based carrier).

### Lipid nanoparticle compositions

The terms "lipid nanoparticle composition" and "composition" are used herein interchangeably. In the context of the present invention, lipid nanoparticles are not restricted to any particular morphology, and should be interpreted as to include any morphology generated when a cationic lipid and optionally one or more further lipids are combined, e.g. in an aqueous environment and/or in the presence of a nucleic acid compound. For example, a liposome, a lipid complex, a lipoplex and the like are within the scope of a lipid nanoparticle.

In the context of the invention, a "composition" refers to any type of composition in which the specified ingredients may be incorporated, optionally along with any further excipients, usually with at least one pharmaceutically acceptable carrier or excipient. Thus, the composition may be a dry composition such as a powder or granules, or a solid unit such as a lyophilized form or a tablet. Alternatively, the composition may be in liquid form, and each excipient may be independently incorporated in dissolved or dispersed (e.g. suspended or emulsified) form. In one of the preferred embodiments, the composition is formulated as a sterile solid composition, such as a powder or lyophilized form for reconstitution with an aqueous liquid carrier. Such formulation is also preferred for those versions of the composition which comprise a nucleic acid cargo as described in further detail below.

In some embodiments, the lipid nanoparticles disclosed herein encapsulating a nucleic acid are lyophilized lipid nanoparticles. A lyophilized lipid nanoparticle is one from which liquid (e.g., water) has been removed by freeze drying, in which a liquid product is frozen and subsequently placed under a vacuum to remove solvent (e.g., water) by sublimation, leaving a composition substantially free of solvent (e.g., water). In some embodiments, a lyophilized lipid nanoparticle as disclosed herein comprises an inventive polymer conjugated lipid, preferably a lipid comprising polyoxazoline, more preferably a PMOZ-lipid. In some embodiments, a lyophilized lipid nanoparticle as disclosed herein comprises nucleic acid. In some embodiments, a lyophilized lipid nanoparticle as disclosed herein comprises nucleic acid encapsulated within lipid nanoparticles. In some embodiments, a lyophilized lipid nanoparticle as disclosed herein comprises a compound of Formula I. In some embodiments, a lyophilized lipid nanoparticle as disclosed herein comprises PMOZ. In some embodiments, a lyophilized lipid nanoparticle as disclosed herein comprises lipids, nucleic acids, a compound of Formula I, or any mixture thereof.

In the composition of the invention, the cationic lipid may be present within, or as part of, lipid nanoparticles (LNPs). In other words, such composition comprises lipid nanoparticles, and the cationic lipid is present in the lipid nanoparticles.

A "nanoparticle", as used herein, is a submicron particle having any structure or morphology. Submicron particles may also be referred to as colloids, or colloidal. With respect to the material on which the nanoparticle is based, and to the structure or morphology, a nanoparticle may be classified, for example, as a nanocapsule, a vesicle, a liposome, a lipid nanoparticle, a micelle, a cross-linked micelle, a lipoplex, a polyplex, a mixed or hybrid complex, to mention only a few of the possible designations of specific types of nanoparticles.

As defined above, lipid nanoparticles include any type of nanoparticles formed or co-formed by lipids. In particular, lipid nanoparticles may co-formed by combinations of lipids comprising at least one amphiphilic, vesicle-forming lipid. Liposomes and lipoplexes are examples of lipid nanoparticles.

In some embodiments, such lipid nanoparticles comprise a cationic lipid (e.g., a lipid of formula (I)) and one or more excipients selected from neutral lipids, charged lipids, steroids and polymer conjugated lipids (e.g., a polymer conjugated lipid such as a polymer conjugated lipid as described above having formula (I). It is currently believed by the inventors that a composition comprising the cationic lipid as defined herein, a steroid, a neutral lipid, and a polymer conjugated lipid according to formula (II) will, at least in an aqueous environment, typically exist as a composition comprising lipid nanoparticles that are formed by these excipients.

An LNP may comprise any lipid capable of forming a particle to which the one or more nucleic acid molecules are attached, or in which the one or more nucleic acid molecules are encapsulated. In some embodiments, the mRNA, or a portion thereof, is encapsulated in the lipid portion of the lipid nanoparticle or an aqueous space enveloped by some or all of the lipid portion of the lipid nanoparticle, thereby protecting it from enzymatic degradation or other undesirable effects induced by the mechanisms of the host organism or cells e.g. an adverse immune response. In some embodiments, the mRNA or a portion thereof is associated with the lipid nanoparticles.

As mentioned, a composition comprising the lipidic excipients as described herein will normally form lipid nanoparticles, at least in an aqueous environment. As defined herein, the nanoparticles have a predominantly submicron size. In certain embodiments, the mRNA, when present in the lipid nanoparticles, is resistant in aqueous solution to degradation with a nuclease. As used herein, the mean diameter may be represented by the z-average as determined by dynamic light scattering. In one embodiment, the composition is a sterile liquid composition comprising lipid nanoparticles having a mean hydrodynamic diameter (or mean size) as determined by dynamic laser scattering from about 30 nm to about 800 nm. In various embodiments, the lipid nanoparticles have a mean diameter of from about 30 nm to about 150 nm, from about 50 nm to about 200 nm, from about 60 nm to about 200 nm, from about 70 nm to about 200 nm, from about 80 nm to about 200 nm, from about 90 nm to about 200 nm, from about 90 nm to about 190 nm, from about 90 nm to about 180 nm, from about 90 nm to about 170 nm, from about 90 nm to about 160 nm, from about 90 nm to about 150 nm, from about 90 nm to about 140 nm, from about 90 nm to about 130 nm, from about 90 nm to about 120 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, 150 nm, 160 nm, 170 nm, 180 nm, 190 nm, or 200 nm, and are substantially non-toxic. In another preferred embodiment of the invention the lipid nanoparticles have a hydrodynamic diameter in the range from about 50 nm to about 300 nm, or from about 60 nm to about 250 nm, from about 60 nm to about 150 nm, or from about 60 nm to about 120 nm, or from about 80 nm to about 160, or from about 90 nm to about 140 nm, 50 nm to about 300 nm, or from about 60 nm to about 250 nm, or from about 60 nm to about 200 nm, or from about 70 to 200 nm, or from about 75 nm to about 160, or from about 100 nm to about 140 nm, or from about 90 nm to about 140 nm. Also preferred is a range of about 50 nm to about 60 nm or a range of about 60 nm to about 80 nm.

Compositions comprising the lipidic excipients as described herein yielding lipid nanoparticles of the invention may be relatively homogenous. A polydispersity index (PDI) may be used to indicate the homogeneity of a nanoparticle composition, e.g., the particle size distribution of the nanoparticle compositions. A small (e.g., less than 0.3) polydispersity index generally indicates a narrow particle size distribution. A nanoparticle composition of the invention may have a polydispersity index from about 0 to about 0.35, such as 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34 or 0.35. In some embodiments, the polydispersity index (PDI) of a nanoparticle composition may be from about 0.1 to about 0.2.

Various optional features, selections and preferences relating to the composition of the invention in general have been described herein: all of these also apply to the lipid nanoparticles, as will be clearly understood by a person skilled in the art. Similarly, the options and preferences apply to compositions comprising such lipid nanoparticles.

For example, the lipid nanoparticles according to one of the preferred embodiments comprise a cationic lipid as defined above, a neutral lipid which may be DPhyPE, a steroid which may be cholesterol, and a polymer conjugated lipid that may be a polymer conjugated lipid according to formula (I):

[P]-[linker]-[L] formula (I),

wherein
[P] is a homopolymer moiety comprising at least one polyoxazoline (POZ) monomer unit 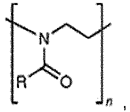
wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa
[linker] is an optional linker group, and
[L] is a lipid moiety;
wherein the cationic lipid may optionally be selected from the compounds **C1 to C27** listed in **Table 1,** or wherein preferably the cationic lipid preferably is the ionizable lipid structure C24 or the cationic lipid according to "formula III-3" (((4-hydroxybutyl) azanediyl)bis(hexane-6,1-diyl)bis(2-hexyldecanoate)).

In the context of the present invention, the mRNA is thus preferably comprised in a liquid or semi-liquid composition, wherein the mRNA is complexed with or associated with a lipid nanoparticle according to one of the preferred embodiments. I.e. in a preferred embodiment, said liquid or semi-liquid composition comprises a complex, wherein the complex comprises the mRNA, wherein the complex is preferably present as a lipid nanoparticle as defined herein.

With respect to the amounts of the respective excipients, it is preferred that the cationic lipid is incorporated in the lipid nanoparticles, or in the composition according to the invention, at a relatively high molar amount compared to the molar amount at which the polymer conjugated lipid according to formula (I) is present. Moreover, the molar amount of the cationic lipid is also preferably higher than the molar of amount of the neutral lipid in the composition or in the nanoparticles, respectively. Furthermore, the molar amount of the steroid is optionally higher than the molar amount of the polymer conjugated lipid according to formula (I).

In certain embodiments, the polymer conjugated lipid according to formula (I) is present in the LNP in an amount from about 1 mol% to about 10 mol%, relative to the total lipid content of the nanoparticle. In one embodiment, the polymer conjugated lipid according to formula (I) is present in the LNP in an amount from about 1 mol% to about 5 mol% percent. In one embodiment, the polymer conjugated lipid according to formula (I) is present in the LNP in about 1 mol% or about 1.5 mol%. In a preferred embodiment, the polymer conjugated lipid according to formula (I) is present in the LNP in an amount from about 1, 2, 3, 4, 5, 6, 7, 8, 9, or 10 mol%; preferably in an amount of 5 mol%, more preferably in an amount of 2.5 mol% or also preferably in an amount of 1.7 mol%, based upon a mol-percentage of the composition of 100% of all lipid components or excipients.

In various embodiments, the molar ratio of the cationic lipid (e.g., lipid of formula (I)) to the polymer conjugated lipid according to formula (I) ranges from about 100:1 to about 25:1, from about 50:1 to about 25:1, or from about 40:1 to about 25:1.

In certain embodiments, the LNP comprises one or more additional lipids which stabilize the formation of particles during their formation. Suitable stabilizing lipids include neutral lipids and anionic lipids. In various embodiments, the molar ratio of the cationic lipid (e.g., lipid of formula (I)) to the neutral lipid ranges from about 2:1 to about 8:1, from about 3:1 to about 7:1, or from about 4:1 to about 6:1.

As used herein, references to molar amounts of lipidic excipients in the composition of the invention should be understood as also describing the molar amounts of the respective excipients in the lipid nanoparticles comprised in the composition, as the lipid nanoparticles are typically formed by these excipients and reflect the same quantitative ratios of excipients as the overall composition containing the nanoparticles.

In general, the amount of the cationic lipid in the composition (and thus in the lipid nanoparticles) is typically at least about 20 mol%, relative to the total molar amount of all lipidic excipients in the composition (or nanoparticles). In another embodiment, the amount of the cationic lipid is at least about 25 mol%, or at least 30 mol%, respectively. In other preferred embodiments, the amount of the cationic lipid in the composition is from about 30 mol% to about 70 mol%, or from about 40 mol% to about 70 mol%, or from about 45 mol% to about 65 mol%, respectively; such as about 30, 35, 40, 45, 50, 55, 60, 65, or 70 mol%, or from about 40 mol% to about 60 mol%, respectively; such as about 40, **41,** 42, 43, 44, 45, 46, 47, 48, 49, 50, 51, 52, 53, 54, 55, 56, 57, 58, 59 or 60 mol%, respectively.

The amount of the steroid in the composition may optionally at least about 10 mol%, or it may be in the range from about 10 mol% to about 60 mol%, or from about 20 mol% to about 50 mol%, or from about 25 mol% to about 45 mol%, respectively; such as about 10, 15, 20, 25, 30, 35, 40, 45, 50, 55, or 60 mol%, respectively. Again, for the avoidance of doubt, the molar percentages are relative the total molar amount of all lipidic excipients in the composition.

The neutral lipid may optionally be present at an amount of at least about 5 mol%. In some embodiments, the amount of the neutral lipid in the composition is in the range from about 5 mol% to about 25 mol%, or from about 5 mol% to about 15 mol%, or from about 8 mol% to about 12 mol%, respectively; such as about 5 mol%, 6 mol%, 7 mol%, 8 mol%, 9 mol%, 10 mol%, 11 mol%, 12 mol%, 13 mol%, 14 mol%, 15 mol%, 16 mol%, 17 mol%, 18 mol%, 19 mol%, 20 mol%, 21 mol%, 22 mol%, 23 mol%, 24 mol% or 25 mol%, respectively, using the same basis for the molar percentages.

The amount of polymer conjugated lipid according to formula (I) in the composition or in the lipid nanoparticles may, for example, be selected to be about 0.1 mol% and higher. In certain embodiments, the amount of the polymer conjugated lipid according to formula (I) is in the range from about 1 mol% to about 15 mol%, or from about 2 mol% to about 12 mol%, respectively, using again the total molar amount of all lipidic excipients as basis for the molar percentages. In other certain embodiments, the composition or the lipid nanoparticles may comprise 0.1; 0.2; 0.3; 0.4; 0.5; 0.6; 0.7; 0.8; 0.9; 1.0; 1.1; 1.2; 1.3; 1.4; 1.5; 1.6; 1.7; 1.8; 1.9; 2.0; 2.1; 2.2; 2.3; 2.4; 2.5; 2.6; 2.7; 2.8; 2.9; 3.0; 3.1; 3.2; 3.3; 3.4; 3.5; 3.6; 3.7; 3.8; 3.9; 4.0; 4.1; 4.2; 4.3; 4.4; 4.5; 4.6; 4.7; 4.8; 4.9; 5.0; 5.1; 5.2; 5.3; 5.4; 5.5; 5.6; 5.7; 5.8; 5.9; 6; 6.1; 6.2; 6.3; 6.4; 6.5; 6.6; 6.7; 6.8; 6.9; 7; 7.1; 7.2; 7.3; 7.4; 7.5; 7.6; 7.7; 7.8; 7.9; 8; 8.1; 8.2; 8.3; 8.4; 8.5; 8.6; 8.7; 8.8; 8.9; 9; 9.1; 9.2; 9.3; 9.4; 9.5; 9.6; 9.7; 9.8; 9.9; 10; 10.1; 10.2; 10.3; 10.4; 10.5; 10.6; 10.7; 10.8; 10.9; 11; 11.1; 11.2; 11.3; 11.4; 11.5; 11.6; 11.7; 11.8; 11.9; or 12 mol% or more than 12 mol% polymer conjugated lipid. In a preferred embodiment, the content of the polymer conjugated lipid according to formula (I) of the invention is about 1 to 5 mol% of the overall lipid content of the formulation, preferably 1.7 mol% or 2.5 mol%. As a non-limiting preferred example, the lipid nanoparticle comprises 5% polymer conjugated lipid. As another non-limiting example preferred, the lipid nanoparticle comprises 10% polymer conjugated lipid. As another non-limiting example, the lipid nanoparticle comprises 7.5% polymer conjugated lipid.

In one embodiment, the composition comprises lipid nanoparticles which comprise:
(a) the cationic lipid at an amount of 30-70 mol%;
(b) the steroid at an amount of 20-50 mol%;
(c) the neutral lipid at an amount of 5-25 mol%; and
(d) polymer conjugated lipid according to formula (I) at an amount of 1-10 mol%;
each amount being relative to the total molar amount of all lipidic excipients of the lipid nanoparticles.

In another embodiment, the composition comprises lipid nanoparticles comprising:
(a) the cationic lipid at an amount of 40-70 mol%;
(b) the steroid at an amount of 20-50 mol%;
(c) the neutral lipid at an amount of 5-15 mol%; and
(d) polymer conjugated lipid according to formula (I) at an amount of 1-10 mol%;
each amount being relative to the total molar amount of all lipidic excipients of the lipid nanoparticles.

In one embodiment, the composition comprises lipid nanoparticles which comprise:
(a) the cationic lipid at an amount of 20-60 mol%;
(b) the steroid at an amount of 25-55 mol%;
(c) the neutral lipid at an amount of 5-25 mol%; and
(d) polymer conjugated lipid according to formula (I) at an amount of 1-15 mol%;
each amount being relative to the total molar amount of all lipidic excipients of the lipid nanoparticles.

In a further embodiment, the composition comprises lipid nanoparticles which comprise:
(a) the cationic lipid at an amount of 45-65 mol%;
(b) the steroid at an amount of 25-45 mol%;
(c) the neutral lipid at an amount of 8-12 mol%; and
(d) polymer conjugated lipid according to formula (I) at an amount of 1-10 mol%, preferably 1.7 mol%;
each amount being relative to the total molar amount of all lipidic excipients of the lipid nanoparticles.

In a further preferred embodiment, the composition comprises lipid nanoparticles which comprise:
(a) the cationic lipid at an amount of 45-65 mol%;
(b) the steroid at an amount of 25-45 mol%;
(c) the neutral lipid at an amount of 8-12 mol%; and
(d) polymer conjugated lipid according to formula (I) at an amount of 1-10 mol%, preferably 1.7 mol%;
each amount being relative to the total molar amount of all lipidic excipients of the lipid nanoparticles.

In a further preferred embodiment, the composition comprises lipid nanoparticles which comprise:
(a) a cationic lipid at an amount of 45-65 mol%;
(b) cholesterol at an amount of 25-45 mol%;
(c) the neutral lipid at an amount of 8-12 mol% and optionally DHPC at an amount of 1 to 10 mol%; and
(d) polymer conjugated lipid according to formula (I) at an amount of 1-3 mol%;
each amount being relative to the total molar amount of all lipidic excipients of the lipid nanoparticles.

In a further preferred embodiment, the composition comprises lipid nanoparticles that contain:
(a) a cationic lipid at an amount of 45-65 mol%;
(b) cholesterol at an amount of 25-45 mol%;
(c) DPhyPE at an amount of 8-12 mol% and optionally DHPC at an amount of 1 to 10 mol%; and
(d) polymer conjugated lipid according to formula (I) at an amount of 1-3 mol%;
each amount being relative to the total molar amount of all lipidic excipients of the lipid nanoparticles.

In a further preferred embodiment, the composition comprises lipid nanoparticles that contain:
(a) a cationic lipid at an amount of 45-65 mol%;
(b) cholesterol at an amount of 25-45 mol%;
(c) DPhyPE at an amount of 8-12 mol% and optionally DHPC at an amount of 1 to 10 mol%; and
(d) DMG-PMOZ or DMPE-PMOZ-v1 at an amount of 1-10 mol%;
each amount being relative to the total molar amount of all lipidic excipients of the lipid nanoparticles.

In a further preferred embodiment, the composition comprises lipid nanoparticles that contain:
(a) a cationic lipid at an amount of 45-65 mol%;
(b) cholesterol at an amount of 25-45 mol%;
(c) DPhyPE at an amount of 8-12 mol% and optionally DHPC at an amount of 1 to 10 mol%; and
(d) DMG-PMOZ or DMPE-PMOZ-v1 at an amount of 1-3 mol%;
each amount being relative to the total molar amount of all lipidic excipients of the lipid nanoparticles.

In these embodiments, the cationic lipid is preferably a compound selected according to any one of the preferences disclosed herein. For example, the cationic lipid may be selected from the compounds listed in **Table 1.** Moreover, these embodiments may also comprise a steroid, a neutral lipid, and/or a polymer conjugated lipid selected according to any one of the preferences disclosed herein. In all embodiments which recite compositions or lipid nanoparticles as described herein and where mol%-values are given for each excipient, each amount should be seen being relative to the total molar amount of all lipidic excipients of the lipid nanoparticles.

In a further preferred embodiment, the composition or the lipid nanoparticle as described herein comprises 59 mol% cationic lipid, 10 mol% neutral lipid, 29.3 mol% steroid and 1.7 mol% polymer conjugated lipid according to formula (I).

In one embodiment, the composition or the lipid nanoparticles described herein comprise 59 mol% cationic lipid, 10 mol% DPhyPE, 29.3 mol% cholesterol and 1.7 mol% polymer conjugated lipid according to formula (I). In one embodiment, composition or the the lipid nanoparticles described herein comprise 59 mol% cationic lipid, 10 mol% DPhyPE, 28.5 mol% cholesterol and 2.5 mol% polymer conjugated lipid according to formula (I). In one embodiment, the composition or the lipid nanoparticles described herein comprise 59 mol% cationic lipid, 10 mol% DPhyPE, 28.5 mol% cholesterol and 2.5 mol% "DMG-PMOZ"

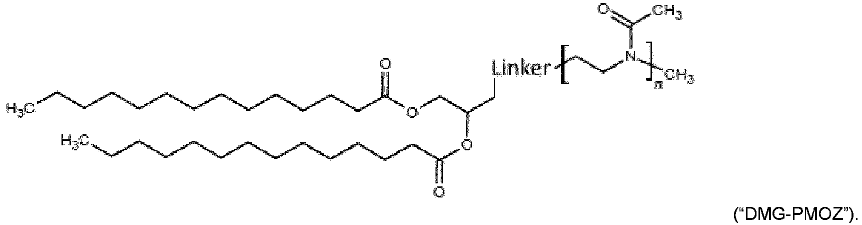

In another embodiment, the composition or the lipid nanoparticle as described herein comprises 47.4 mol% cationic lipid, 10 mol% neutral lipid, 40.9 mol% steroid and 1.7 mol% polymer conjugated lipid according to formula (I).

In a further embodiment, the composition or the lipid nanoparticles described herein comprise 47.4 mol% cationic lipid, 10 mol% DPhyPE, 40.9 mol% cholesterol and 1.7 mol% polymer conjugated lipid according to formula (I). In one embodiment, the composition or the lipid nanoparticles described herein comprise 47.4 mol% cationic lipid, 10 mol% DPhyPE, 40.1 mol% cholesterol and 2.5 mol% polymer conjugated lipid according to formula (I). In one embodiment, the composition or the lipid nanoparticles described herein comprise 47.4 mol% cationic lipid, 10 mol% DPhyPE, 40.1 mol% cholesterol and 2.5 mol% "DMG-PMOZ".

In most preferred embodiments, the composition or the lipid nanoparticles described herein comprise 59 mol% cationic lipid (preferably "THIOETHER"), 10 mol% neutral lipid, preferably DPhyPE, 28.5 mol% cholesterol and 2.5 mol% polymer conjugated lipid as described herein above or below, preferably according to formula (I), also preferably a lipid selected from the group consisting of PMOZ 1, PMOZ 2, PMOZ 3, PMOZ 4 and PMOZ 5. In another most preferred embodiment, the composition or the lipid nanoparticles described herein comprise 49 mol% cationic lipid (preferably "THIOETHER"), 10 mol% neutral lipid, preferably DPhyPE, 40.9 mol% cholesterol and 1.7 mol% polymer conjugated lipid as described herein above or below, preferably according to formula (I), also preferably a lipid selected from the group consisting of PMOZ 1, PMOZ 2, PMOZ 3, PMOZ 4 and PMOZ 5. In another most preferred embodiment, the composition or the lipid nanoparticles described herein comprise 59 mol% cationic lipid (preferably "THIOETHER"), 10 mol% neutral lipid, preferably DPhyPE, 28.5 mol% cholesterol and 2.5 mol% polymer conjugated lipid as described herein above or below, preferably according to formula (I), also preferably a lipid selected from the group consisting of PMOZ 1, PMOZ 2, PMOZ 3, PMOZ 4 and PMOZ 5.

In further most preferred embodiments, the composition or the lipid nanoparticles described herein comprise 59 mol% cationic lipid (preferably "THIOETHER"), 10 mol% neutral lipid, preferably DPhyPE, 28.5 mol% cholesterol and 2.5 mol% polymer conjugated lipid as described herein above or below, preferably according to formula (I), also preferably a lipid selected from the group consisting of PMOZ 2 and PMOZ 4. In another most preferred embodiment, the composition or the lipid nanoparticles described herein comprise 59 mol% C24, 10 mol% DPhyPE, 28.5 mol% cholesterol and 2.5 mol% PMOZ 4.

In any of the above embodiments in this section disclosing specific compositions or lipid nanoparticles having distinct %-values for excipients, if 1,2-diphytanoyl-sn-glycero-3-phosphoethanolamine (DPhyPE) is mentioned as neutral lipid, in further embodiments DPhyPE may be exchanged with another neutral lipid, preferably 1,2-diphytanoyl-sn-glycero-3-phosphocholine (DPhyPC). Furthermore, In any of the above embodiments in this section disclosing specific compositions or lipid nanoparticles having distinct %-values for excipients, if 1,2-diphytanoyl-sn-glycero-3-phosphoethanolamine (DPhyPE) is mentioned as neutral lipid, in even further embodiments DPhyPE may be exchanged with another neutral lipid, preferably 1,2-dioleoyl-sn-glycero-3-phosphocholine (DOPC; also referred to as dioleoylphosphatidylcholine) or alternatively 1,2-dioleoyl-sn-glycero-3-phosphoethanolamine (DOPE).

Further preferred lipid compositions according to further specific embodiments of the present invention comprise the at least four lipid excipients as disclosed herein in **Table E.** For example, a preferred lipid composition comprises the excipients as disclosed in line "E1" which are "C1" as cationic lipid (as disclosed herein in **Table 1),** DPhyPE as neutral lipid, cholesterol as sterol and DMG-PMOZ as polymer conjugated lipid excipient. As another example a preferred lipid composition comprises the excipients as disclosed in line "E35" which are "C12" as cationic lipid (as disclosed herein in **Table 1),** DPhyPE as neutral lipid, cholesterol as sterol and DMPE-PMOZ-v1 as polymer conjugated lipid excipient.

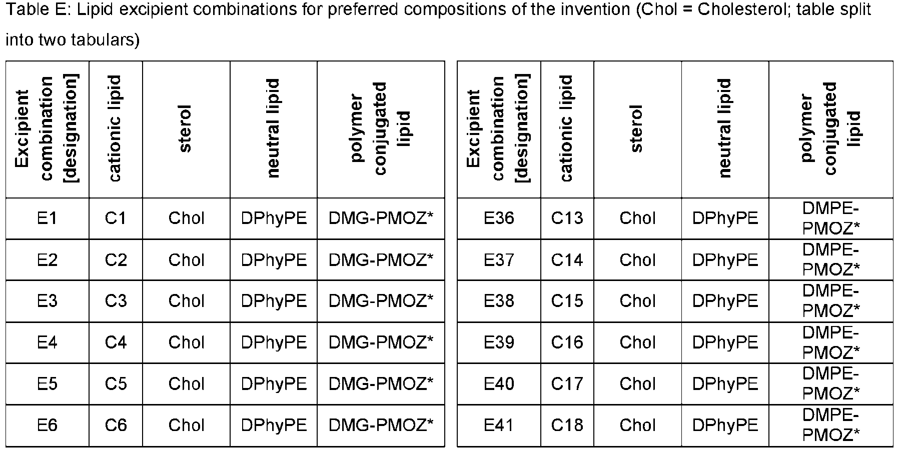

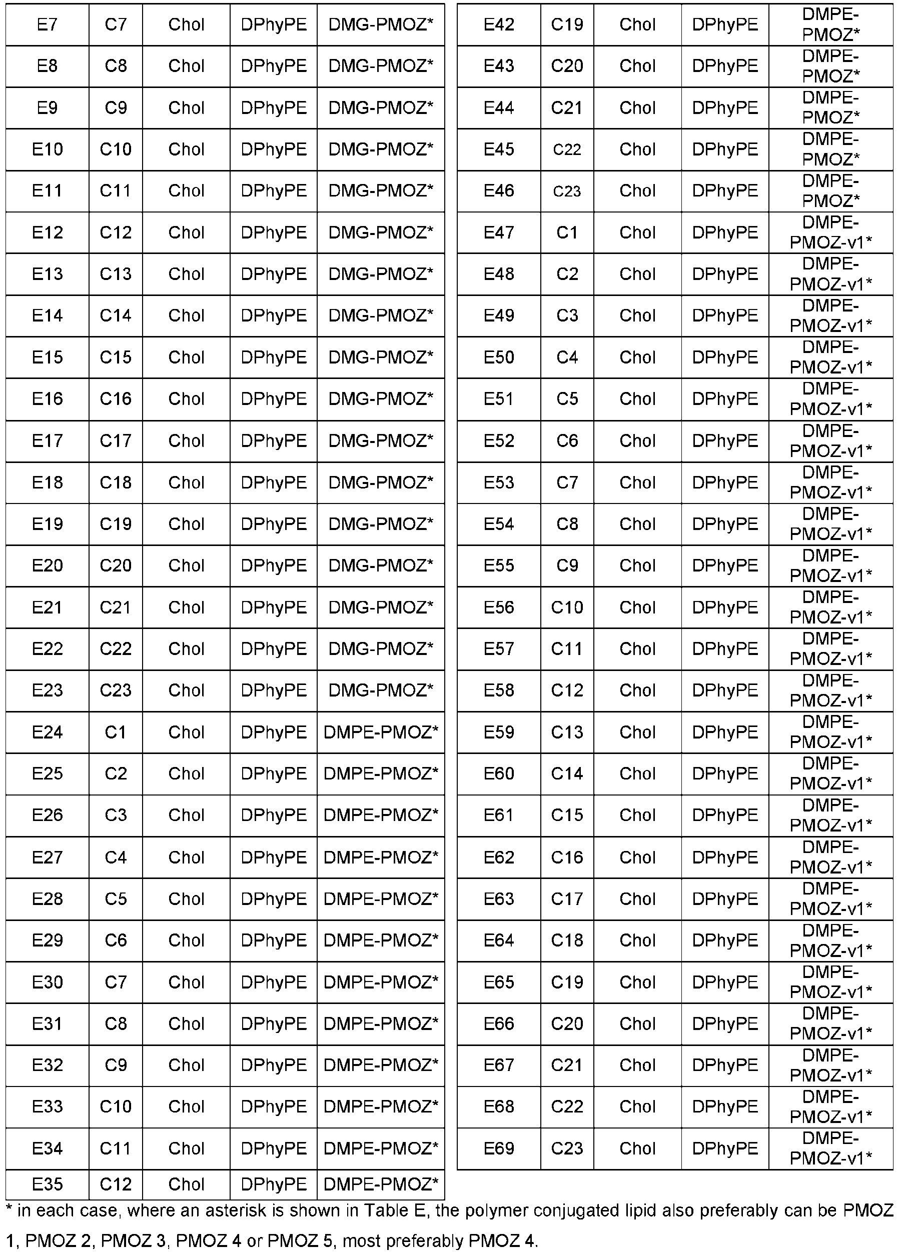

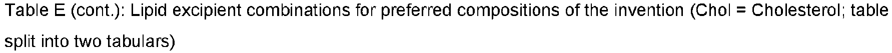

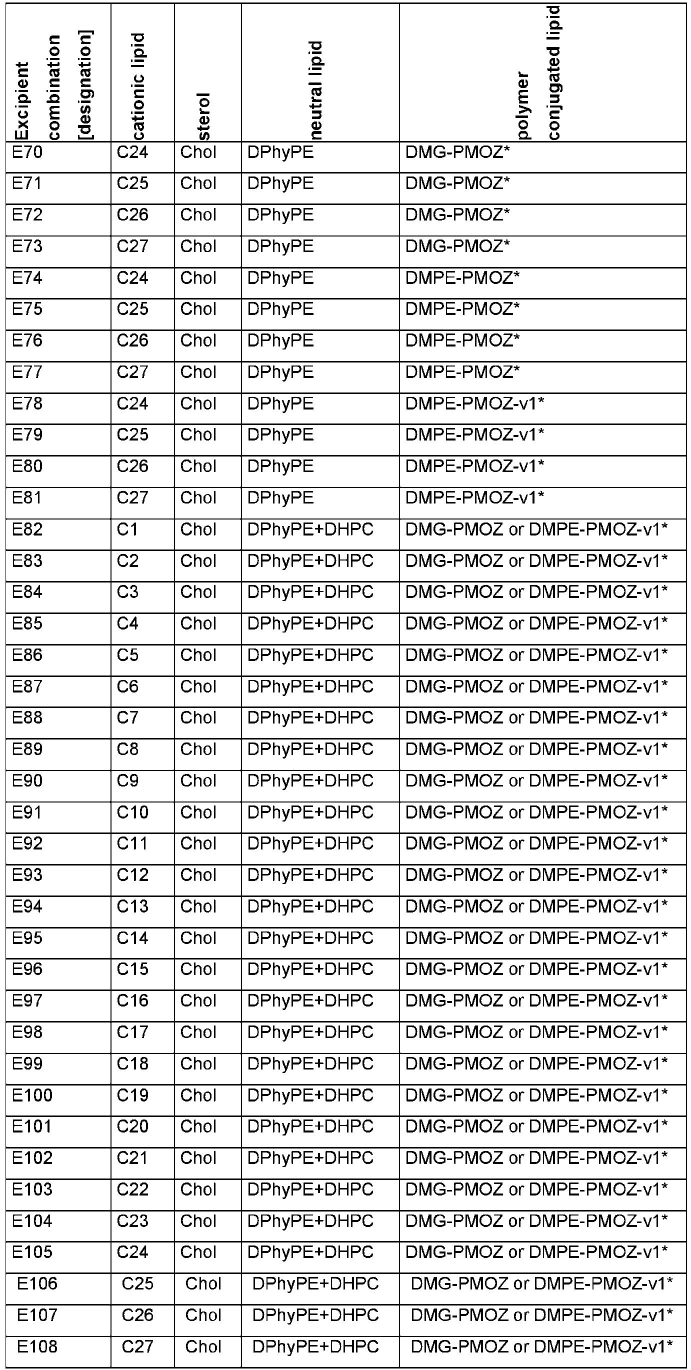

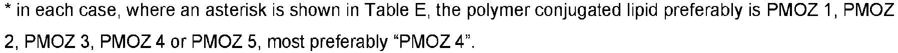

Furthermore, preferred lipid formulations of the invention showing distinct mol-percentages of the at least four lipid excipients of the inventive compositions are shown in **Table** F. For example, a preferred lipid composition comprises the mol-percentages of lipids as disclosed in line "F1", i.e. 59 mol% cationic lipid, 29.3 mol% sterol, 10 mol% neutral lipid, and 1.7 mol% polymer conjugated lipid. As another example, a preferred lipid composition comprises the mol-percentages of lipids as disclosed in line "F31", i.e. 45 mol% cationic lipid, 43,5 mol% sterol, 10 mol% neutral lipid and 1.5 mol% polymer conjugated lipid.

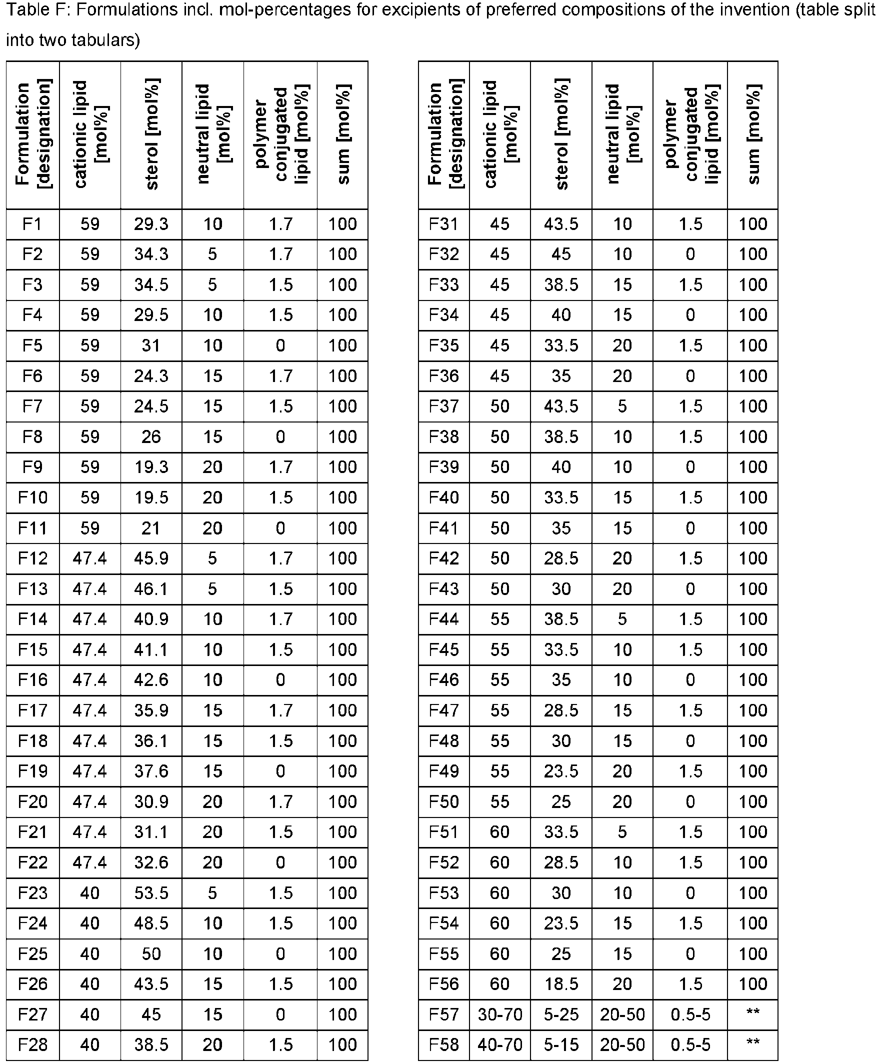

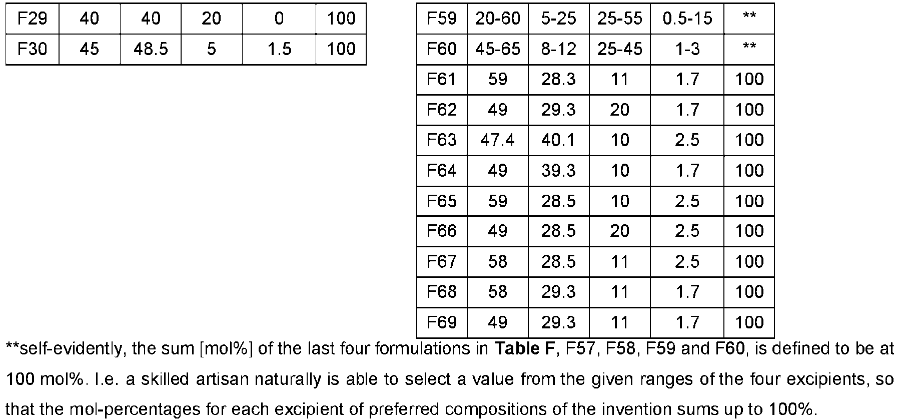

Accordingly, in a further preferred embodiment of the invention, a composition of the invention comprises excipients as disclosed in **Table E** selected from the group consisting of Excipient combination designation
E1, E2, E3, E4, E5, E6, E7, E8, E9, E10, E11, E12, E13, E14, E15, E16, E17, E18, E19, E20, E21, E22, E23, E24, E25, E26, E27, E28, E29, E30, E31, E32, E33, E34, E35, E36, E37, E38, E39, E40, E41, E42, E43, E44, E45, E46, E47, E48, E49, E50, E51, E52, E53, E54, E55, E56, E57, E58, E59, E60, E61, E62, E63, E64, E65, E66, E67, E68, E69, E70, E71, E72, E73, E74, E75, E76, E77, E78, E79, E80, E81, E82, E83, E84, E85, E86, E87, E88, E89, E90, E91, E92, E93, E94, E95, E96, E97, E98, E99, E100, E101, E102, E103, E104, E105, E106, E107 and E108;
in distinct mol-percentages as disclosed in **Table F** selected from the group consisting of formulation designation F1, F2, F3, F4, F5, F6, F7, F8, F9, F10, F11, F12, F13, F14, F15, F16, F17, F18, F19, F20, F21, F22, F23, F24, F25, F26, F27, F28, F29, F30, F31, F32, F33, F34, F35, F36, F37, F38, F39, F40, F41, F42, F43, F44, F45, F46, F47, F48, F49, F50, F51, F52, F53, F54, F55, F56, F57, F58, F59, F60, F61, F62, F63, F64, F65, F66, F67, F68 and F69.

A most preferred embodiment is Lipid excipient combination E1 with "PMOZ 4" as conjugated lipid and mol-percentages as indicated in Formulation F65.

Alternatively, the composition may be provided in solid form. In particular, it may be provided as a sterile solid composition for reconstitution with a sterile liquid carrier; the solid composition may in this case further comprise one or more inactive ingredients selected from pH-modifying agents, bulking agents, stabilizers, non-ionic surfactants and antioxidants. In this embodiment, the sterile liquid carrier is preferably an aqueous carrier.

The zeta potential of a nanoparticle composition may be used to indicate the electrokinetic potential of the composition. For example, the zeta potential may describe the surface charge of a nanoparticle composition. The lipid nanoparticles according to the invention may, due to the presence of both negatively and positively charged compounds, exhibit a relatively neutral zeta potential. The zeta potential (sometimes abbreviated as "charge") may be determined along with the particle size of the particles, for example, by dynamic light scattering and Laser Doppler Microelectrophoresis, for example using a Malvern Zetasizer Nano (Malvern Instruments Ltd.; Malvern, UK). Depending on the amount and nature of charged compounds in the lipid nanoparticles, the nanoparticles may be characterized by a zeta potential. In a preferred embodiment, the zeta potential is in the range from about -50 mV to about +50 mV. In other preferred embodiments, the zeta potential is in the range from about -25 mV to about +25 mV. In some embodiments, the zeta potential of a lipid nanoparticle of the invention may be from about -10 mV to about +20 mV, from about -10 mV to about +15 mV, from about -10 mV to about +10 mV, from about -10 mV to about +5 mV, from about -10 mV to about 0 mV, from about -10 mV to about -5 mV, from about -5 mV to about +20 mV, from about -5 mV to about +15 mV, from about -5 mV to about +10 mV, from about -5 mV to about +5 mV, from about -5 mV to about 0 mV, from about 0 mV to about +20 mV, from about 0 mV to about +15 mV, from about 0 mV to about +10 mV, from about 0 mV to about +5 mV, from about +5 mV to about +20 mV, from about +5 mV to about +15 mV, or from about +5 mV to about +10 mV. Preferably, the zeta potential of the inventive lipid nanoparticles exhibit a zeta potential in the range of -50 mV to +50 mV, preferably in the range of -25 mV to +25 mV, more preferably in the range of -10 mV to +10 mV, most preferably in the range of -5 mV to +5 mV.

In certain embodiments, the LNP comprises one or more targeting moieties which are capable of targeting the LNP to a cell or cell population. For example, in one embodiment, the targeting moiety is a ligand which directs the LNP to a receptor found on a cell surface.

In certain embodiments, the LNP comprises one or more internalization domains. For example, in one embodiment, the LNP comprises one or more domains which bind to a cell to induce the internalization of the LNP. For example, in one embodiment, the one or more internalization domains bind to a receptor found on a cell surface to induce receptor-mediated uptake of the LNP. In certain embodiments, the LNP is capable of binding a biomolecule in vivo, where the LNP-bound biomolecule can then be recognized by a cell-surface receptor to induce internalization. For example, in one embodiment, the LNP binds systemic ApoE, which leads to the uptake of the LNP and associated cargo. In certain embodiments of the invention, ApoE may be supplemented to the medium or pharmaceutical composition used.

Preferably, in one embodiment, the compositions of the invention further comprise a biologically active ingredient.

In preferred embodiments of the invention, a lipid nanoparticle comprises a polymer conjugated lipid comprising at least one polyoxazoline (POZ) monomer unit 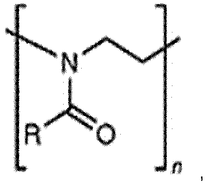
wherein R is C1-9 alkyl or C2-9 alkenyl, preferably C1 or C2 alkyl, and n has a mean value ranging from 2 to 200, preferably from 20 to 100, more preferably from 24 to 26 or 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa,
preferably, wherein the homopolymer moiety comprising multiple monomer units comprises poly(2-methyl-2-oxazoline) (PMOZ), poly(2-ethyl-2-oxazoline) (PEOZ), poly(2-propyl-2-oxazoline) (PPOZ), poly(2-butyl-2-oxazoline) (PBOZ), poly(2-isopropyl-2-oxazoline) (PIPOZ), poly(2-methoxymethyl-2-oxazoline) (PMeOMeOx), or poly(2-dimethylamino-2-oxazoline) (PDMAOx),
more preferably any of the polymer conjugated lipids as described herein above or below, most preferably "PMOZ 4".

### Encapsulation/Complexation in LNPs:

In preferred embodiments of the second aspect, the at least one nucleic acid (e.g. DNA or RNA), preferably the at least one RNA, and optionally the at least one further nucleic acid, is complexed, encapsulated, partially encapsulated, or associated with one or more lipids (e.g. cationic lipids and/or neutral lipids), thereby forming liposomes, lipid nanoparticles (LNPs), lipoplexes, and/or nanoliposomes.

The liposomes, lipid nanoparticles (LNPs), lipoplexes, and/or nanoliposomes - incorporated nucleic acid (e.g. DNA or RNA) may be completely or partially located in the interior space of the liposomes, lipid nanoparticles (LNPs), lipoplexes, and/or nanoliposomes, within the lipid layer/membrane, or associated with the exterior surface of the lipid layer/membrane. The incorporation of a nucleic acid into liposomes/LNPs is also referred to herein as "encapsulation" wherein the nucleic acid, e.g. the RNA is entirely contained within the interior space of the liposomes, lipid nanoparticles (LNPs), lipoplexes, and/or nanoliposomes. The purpose of incorporating nucleic acid into liposomes, lipid nanoparticles (LNPs), lipoplexes, and/or nanoliposomes is to protect the nucleic acid, preferably RNA from an environment which may contain enzymes or chemicals or conditions that degrade nucleic acid and/or systems or receptors that cause the rapid excretion of the nucleic acid. Moreover, incorporating nucleic acid, preferably RNA into liposomes, lipid nanoparticles (LNPs), lipoplexes, and/or nanoliposomes may promote the uptake of the nucleic acid, and hence, may enhance the therapeutic effect of the nucleic acid, e.g. the RNA encoding antigenic SARS-CoV-2 (nCoV-2019) proteins. Accordingly, incorporating a nucleic acid, e.g. RNA or DNA, into liposomes, lipid nanoparticles (LNPs), lipoplexes, and/or nanoliposomes may be particularly suitable for a coronavirus vaccine (e.g. a SARS-CoV-2 vaccine), e.g. for intramuscular and/or intradermal administration.

In this context, the terms "complexed" or "associated" refer to the essentially stable combination of nucleic acid with one or more lipids into larger complexes or assemblies without covalent binding.

The term "lipid nanoparticle", also referred to as "LNP", is not restricted to any particular morphology, and include any morphology generated when a cationic lipid and optionally one or more further lipids are combined, e.g. in an aqueous environment and/or in the presence of a nucleic acid, e.g. an RNA. For example, a liposome, a lipid complex, a SNALP, a lipoplex and the like all fall within the scope of a lipid nanoparticle (LNP). A "lipid nanoparticle" (LNP) therefore is a nanoparticle formed by lipids, typically including at least one amphiphilic, membrane-forming lipid, and optionally other lipids, further optionally including a cargo material such as a nucleic acid compound. As used herein, the expression "lipid nanoparticles" or "LNP" includes any sub-types and morphologies of nanoparticles formed or co-formed by lipids, such as aforementioned liposomes and lipoplexes.

Liposomes, lipid nanoparticles (LNPs), lipoplexes, and/or nanoliposomes can be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50 nm in diameter, and a large unilamellar vesicle (LUV) which may be between 50 nm and 500 nm in diameter.

LNPs of the invention are suitably characterized as microscopic vesicles having an interior aqua space sequestered from an outer medium by a membrane of one or more bilayers. Bilayer membranes of LNPs are typically formed by amphiphilic molecules, such as lipids of synthetic or natural origin that comprise spatially separated hydrophilic and hydrophobic domains. Bilayer membranes of the liposomes can also be formed by amphophilic polymers and surfactants (e.g., polymerosomes, niosomes, etc.). In the context of the present invention, an LNP typically serves to transport the at least one nucleic acid, preferably the at least one RNA to a target tissue.

Accordingly, in preferred embodiments of the second aspect, the at least one nucleic acid, preferably the at least one RNA is complexed with one or more lipids thereby forming lipid nanoparticles (LNP). Preferably, said LNP is particularly suitable for intramuscular and/or intradermal administration.

### Biologically Active Ingredients

As used herein, a biologically active ingredient means any compound or material having a biological activity due to which the compound or material is potentially useful for the prevention, management, improvement, treatment or therapy of a disease or condition in a subject, such as an animal, and in particular in a human subject.

In one of the preferred embodiments, the active ingredient is a nucleic acid compound. Examples of nucleic acid compounds that are potentially useful for carrying out the invention include nucleic acid compounds selected from the group consisting of chemically modified or unmodified messenger RNA (mRNA), chemically modified or unmodified RNA, single-stranded or double-stranded RNA, coding or non-coding RNA, viral RNA, replicon RNA, and self-replicating RNA, or any combination thereof; preferably wherein the biologically active ingredient is an mRNA.

In preferred embodiments, the nucleic acid compound is complexed or associated with one or more lipids (e.g. cationic lipids and/or neutral lipids), thereby forming liposomes, lipid nanoparticles (LNPs), lipoplexes, and/or nanoliposomes. In this context, the terms "complexed" or "associated" refer to the essentially stable combination of nucleic acid compound of the first aspect with one or more lipids into larger complexes or assemblies without covalent binding.

In specific embodiments, the active ingredient may include a CRISPR RNA (crRNA) plus a tracer RNA (tracrRNA), a guide RNA (gRNA) or a single guide RNA (sgRNA) and/or a donor DNA in conjunction with a CRISPR endonuclease. Suitably the CRISPR endonuclease may be provided as a protein or polypeptide or as an mRNA encoding said CRISPR endonuclease. A composition or formulation comprising this combination is suitable for delivering a CRISPR gene editing activity to a target cell. In one embodiment, compositions in accordance with the invention may provide the gRNA and mRNA encoding a CRISPR endonuclease, for separate, sequential or simultaneous administration. That is, the gRNA and mRNA may be provided within the same formulation or lipid nanoparticle in accordance with the invention or may be provided in separate lipid nanoparticles for separate, simultaneous or sequential administration. Suitably the ratio of gRNA to mRNA for administration is 1:1, 1:3, 1:9, 1:19, for example (i.e. 50%, 25%, 10% and 5% of guide RNA). In one embodiment, a gRNA and an mRNA encoding a CRISPR endonuclease such as cas9 are co-loaded into a formulation in accordance with the invention. Advantageously, co-loading enables a better encapsulation efficiency (EE) to be obtained. Suitably, a formulation or pharmaceutical composition in accordance with the invention into which gRNA and mRNA are co-loaded comprises LNPs with a mean diameter of between 80 and 160 nm. In one embodiment, the gRNA may be a modified gRNA sequence. Suitable modifications are described, for example in WO2016089433, WO2017068377 and PCT/GB2016/053312. Other suitable modifications will be familiar to those skilled in the art.

By "CRISPR endonuclease" is meant an endonuclease that can be used in a CRISPR gene editing composition. Suitable "CRISPR endonucleases" include cas9 and its mutants and modified forms. Accordingly, the mRNA for use in combination with a gRNA is one which encodes a CRISPR endonuclease, preferably cas9. Other "CRISPR endonucleases" include cpf1, for example. The skilled person will be aware that a gRNA pairs with a particular "CRISPR endonuclease". Accordingly, the invention contemplates a composition using a suitable gRNA/endonuclease pairing. Suitably, a gRNA is specific for a target gene, preferably wherein the target gene is a gene associated with liver disease.

In another embodiment, the peptide or protein expressed by the nucleic acid compound is a therapeutic protein, or a fragment or variant thereof, wherein the therapeutic protein is beneficial for the treatment or prophylaxis of any inherited or acquired disease or which improves the condition of an individual. Particularly, therapeutic proteins play a key role in the design of new therapeutic agents that could modify and repair genetic deficiencies, destroy cancer cells or pathogen infected cells, treat or prevent immune system disorders, or treat or prevent metabolic or endocrine disorders, among other functions.

In another embodiment, the peptide or protein expressed by the nucleic acid compound is an antigen. As defined in more detail herein above, an antigen is a compound or material which may be recognized by the immune system, preferably by the adaptive immune system, such as to trigger an antigen-specific immune response.

In some embodiments, the active ingredient is siRNA. siRNA are small interfering RNA as, for example, described in international patent application WO2004015107 and PCT/EP03/08666. These molecules typically consist of a double-stranded RNA structure which comprises between 15 and 25, preferably 18 to 23 nucleotide pairs which are capable of base-pairing to each other, i. e. are essentially complementary to each other, typically mediated by Watson-Crick base-pairing. One strand of this double-stranded RNA molecule is essentially complementary to a target nucleic acid, preferably an mRNA, whereas the second strand of said double-stranded RNA molecule is essentially identical to a stretch of said target nucleic acid. The siRNA molecule may be flanked on each side and each stretch, respectively, by a number of additional nucleotides which, however, do not necessarily have to base-pair to each other.

In some embodiments, the active ingredient is RNAi. RNAi has essentially the same design as siRNA, however, the molecules are significantly longer compared to siRNA. RNAi molecules typically comprise 50 or more nucleotides and base pairs, respectively.

In some embodiments, the active ingredient is an antisense nucleic acid Antisense nucleic acids, as preferably used herein, are oligonucleotides which hybridise based on base complementarity with a target RNA, preferably mRNA, thereby activating RNaseH. RNaseH is activated by both phosphodiester and phosphothioate-coupled DNA. Phosphodiester-coupled DNA, however, is rapidly degraded by cellular nucleases although phosphothioate-coupled DNA is not. Antisense polynucleotides are thus effective only as DNA-RNA hybrid complexes. Preferred lengths of antisense nucleic acids range from 16 to 23 nucleotides. Examples for this kind of antisense oligonucleotides are described, among others, in US patent 5,849,902 and US patent 5,989,912.

In some embodiments, the active ingredient is a ribozyme. Ribozymes are catalytically active nucleic acids preferably consisting of RNA which basically comprises two moieties. The first moiety shows a catalytic activity, whereas the second moiety is responsible for the specific interaction with the target nucleic acid. Upon interaction between the target nucleic acid and the said moiety of the ribozyme, typically by hybridisation and Watson-Crick base-pairing of essentially complementary stretches of bases on the two hybridising strands, the catalytically active moiety may become active which means that it cleaves, either intramolecularly or intermolecularly, the target nucleic acid in case the catalytic activity of the ribozyme is a phosphodiesterase activity. Ribozymes, the use and design principles are known to the ones skilled in the art and, for example, described in Doherty and Doudna (Annu. Ref. Biophys. Biomolstruct. 2000; 30: 457-75).

In some embodiments, the active ingredient is an aptamer. Aptamers are D-nucleic acids which are either single-stranded or double-stranded and which specifically interact with a target molecule. The manufacture or selection of aptamers is, e.g., described in European patent EP0533838. In contrastto RNAi, siRNA, antisense-nucleotides and ribozymes, aptamers do not degrade any target mRNA but interact specifically with the secondary and tertiary structure of a target compound such as a protein. Upon interaction with the target, the target typically shows a change in its biological activity. The length of aptamers typically ranges from as little as 15 to as much as 80 nucleotides, and preferably ranges from about 20 to about 50 nucleotides.

In some embodiments, the active ingredient is a spiegelmer. Spiegelmers are, for example, described in international patent application WO1998008856. Spiegelmers are molecules similar to aptamers. However, spiegelmers consist either completely or mostly of L-nucleotides rather than D-nucleotides in contrast to aptamers. Otherwise, particularly with regard to possible lengths of spiegelmers, the same applies to spiegelmers as outlined in connection with aptamers.

Formulation in lipid nanoparticles or lipid-based carriers:
In the context of the invention, a typical "lipid-based carrier" is selected from liposomes, lipid nanoparticles (LNPs), lipoplexes, and/or nanoliposomes. In the context of the invention, the formulation in lipid nanoparticles relates to the term "lipid-based carriers" which encompass lipid based delivery systems for RNA that comprise a lipid component. A lipid nanoparticle or lipid-based carrier may additionally comprise other components suitable for encapsulating/incorporating/complexing an RNA including a cationic or polycationic polymer, a cationic or polycationic polysaccharide, a cationic or polycationic protein, a cationic or polycationic peptide, or any combinations thereof.

The RNA of the pharmaceutical composition may completely or partially incorporated or encapsulated in a lipid-based carrier, wherein the RNA may be located in the interior space of the lipid-based carrier, within the lipid layer/membrane of the lipid-based carrier, or associated with the exterior surface of the lipid-based carrier. The incorporation of RNA into lipid-based carriers may be referred to as "encapsulation". A "lipid-based carrier" is not restricted to any particular morphology, and include any morphology generated when e.g. an aggregation reducing lipid and at least one further lipid are combined, e.g. in an aqueous environment in the presence of RNA. For example, an LNP, a liposome, a lipid complex, a lipoplex and the like are within the scope of the term "lipid-based carrier". Lipid-based carriers can be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50nm in diameter, and a large unilamellar vesicle (LUV) which may be between 50nm and 500nm in diameter. Liposomes, a specific type of lipid-based carrier, are characterized as microscopic vesicles having an interior aqua space sequestered from an outer medium by a membrane of one or more bilayers. In a liposome, the at least one RNA is typically located in the interior aqueous space enveloped by some or the entire lipid portion of the liposome. Bilayer membranes of liposomes are typically formed by amphiphilic molecules, such as lipids of synthetic or natural origin that comprise spatially separated hydrophilic and hydrophobic domains. Lipid nanoparticles (LNPs), a specific type of lipid-based carrier, are characterized as microscopic lipid particles having a solid core or partially solid core. Typically, an LNP does not comprise an interior aqua space sequestered from an outer medium by a bilayer. In an LNP, the at least one RNA may be encapsulated or incorporated in the lipid portion of the LNP enveloped by some or the entire lipid portion of the LNP. An LNP may comprise any lipid capable of forming a particle to which the RNA may be attached, or in which the RNA may be encapsulated. Preferably, said lipid-based carriers are particularly suitable for intramuscular and/or intradermal administration.

In preferred embodiments, the lipid-based carriers of the pharmaceutical composition are selected from liposomes, lipid nanoparticles, lipoplexes, and/or nanoliposomes.

In preferred embodiments, the lipid-based carriers of the pharmaceutical composition are lipid nanoparticles (LNPs). In particularly preferred embodiments, the lipid nanoparticles of the pharmaceutical composition encapsulate the at least one RNA of the invention.

The term "encapsulated", e.g. incorporated, complexed, encapsulated, partially encapsulated, associated, partially associated, refers to the essentially stable combination of RNA with one or more lipids into lipid-based carriers (e.g. larger complexes or assemblies) preferably without covalent binding of the RNA. The lipid-based carriers - encapsulated RNA may be completely or partially located in the interior of the lipid-based carrier (e.g. the lipid portion and/or an interior space) and/or within the lipid layer/membrane of the lipid-based carriers. The encapsulation of an RNA into lipid-based carriers is also referred to herein as "incorporation" as the RNA is preferably contained within the interior of the lipid-based carriers. Without wishing to be bound to theory, the purpose of incorporating or encapsulating RNA into lipid-based carriers may be to protect the RNA from an environment which may contain enzymes, chemicals, or conditions that degrade the RNA. Moreover, incorporating RNA into lipid-based carriers may promote the uptake of the RNA, and hence, may enhance the therapeutic effect of the RNA when administered to a cell or a subject.

The term "fusogenic" or "fusogenicity" is meant to refer to a lipid which aids the fusion of a lipid-based carrier or nucleic acid-lipid particle with a cell membrane to help the nucleic acid contained in the lipid-based carrier or nucleic acid-lipid particle to enter the cell.

In preferred embodiments, the lipid-based carriers of the pharmaceutical composition comprise at least one or more lipids selected from at least one aggregation-reducing lipid, at least one cationic lipid, at least one neutral lipid or phospholipid, or at least one steroid or steroid analogue.

In preferred embodiments, the lipid-based carriers of the pharmaceutical composition comprise an aggregation-reducing lipid, a cationic lipid or ionizable lipid, a neutral lipid or phospholipid, and a steroid or steroid analogue.

The term "PMOZ-LNPs" refers to lipid nanoparticles, comprising the inventive polyoxazoline lipids, preferably PMOZ-lipids, as polymer conjugated lipids. In preferred embodiments, PMOZ-LNPs do not comprise PEG-lipids. In other preferred embodiments, PMOZ-LNPs do not comprise polymer conjugated lipids, comprising a sulphur (-S-)-group. In other preferred embodiments, PMOZ-LNPs do not comprise lipids being covalently coupled to a biologically active ingredient, said biologically active ingregient being mRNA.

Aggregation reducing lipids or polymer conjugated lipids of the invention:
Under storage conditions or during formulation, the lipid-based carriers may undergo charge-induced aggregation, a condition which can be undesirable for the stability of the lipid-based carriers. Therefore, it can be desirable to include a lipid compound which can reduce aggregation, for example by sterically stabilizing the lipid-based carriers. Such a steric stabilization may occur when a compound having a sterically bulky but uncharged moiety that shields or screens the charged portions of a lipid-based carriers from close approach to other lipid-based carriers in the composition. In the context of the invention, stabilization of the lipid-based carriers is achieved by including lipids which may comprise a lipid bearing a sterically bulky group which, after formation of the lipid-based carrier, is preferably located on the exterior of the lipid-based carrier.

The terms "aggregation reducing lipid" or "polymer conjugated lipid" refer to a molecule comprising both a lipid portion and a moiety suitable of reducing or preventing aggregation of the lipid-based carriers comprising the cargo, preferably the mRNA. Thus, "aggregation reducing lipids", also referred herein to as "polymer conjugated lipid", are lipids comprising a polymer as aggregation reducing group. A polymer, as apparent from the context of the invention, has to be understood as a substance or material consisting of very large molecules, or macromolecules, composed of many repeating subunits. A suitable polymer in the context of the invention may be a hydrophilic polymer. In preferred embodiments, the lipid-based carriers of the pharmaceutical composition comprise an aggregation reducing lipid selected from a polymer conjugated lipid.

In some embodiments, lipid-based carriers include less than about 3mol%, 2mol%, or 1mol% of aggregation reducing lipid, based on the total moles of lipid in the lipid-based carrier. In further embodiments, lipid-based carriers comprise from about 0.1% to about 10% of the aggregation reducing lipid or polymer conjugated lipid on a molar basis, e.g. about 0.5% to about 10%, about 0.5% to about 5%, about 10%, about 5%, about 4%, about 3%, about 2%, about 1.5%, about 1%, about 0.5%, or about 0.3% on a molar basis (based on 100% total moles of lipids in the lipid-based carrier). In other preferred embodiments, lipid-based carriers comprise from about 1.0% to about 2.0% of the aggregation reducing lipid or polymer conjugated lipid on a molar basis, e.g. about 1.2% to about 1.9%, about 1.2% to about 1.8%, about 1.3% to about 1.8%, about 1.4% to about 1.8%, about 1.5% to about 1.8%, about 1.6% to about 1.8%, in particular about 1.4%, about 1.5%, about 1.6%, about 1.7%, about 1.8%, about 1.9%, most preferably 1.7% (based on 100% total moles of lipids in the lipid-based carrier). In other preferred embodiments, lipid-based carriers comprise about 2.5%, 3%, 3.5%, 4%, 4.5% or 5%, preferably 2.5% of the aggregation reducing lipid or polymer conjugated lipid on a molar basis (based on 100% total moles of lipids in the lipid-based carrier). In very preferred embodiments, lipid-based carriers comprise about 2.5% of the polymer conjugated lipid on a molar basis (based on 100% total moles of lipids in the lipid-based carrier).

In various embodiments, the molar ratio of the cationic lipid to the aggregation reducing lipid or polymer conjugated lipid ranges from about 100:1 to about 25:1.

### Lipid-based carrier compositions:

In preferred embodiments, the lipid-based carriers of the pharmaceutical composition, preferably the LNPs, comprise at least one RNA as defined in the first aspect, a cationic lipid as defined herein, an aggregation reducing lipid as defined herein, optionally, a neutral lipid as defined herein, and, optionally, a steroid or steroid analogue as defined herein.

In preferred embodiments, the lipid-based carriers comprising at least one RNA of the first aspect comprise
(i) at least one cationic lipid or ionizable lipid, preferably as defined herein;
(ii) at least one neutral lipid or phospholipid, preferably as defined herein;
(iii) at least one steroid or steroid analogue, preferably as defined herein; and
(iv) at least one aggregation reducing lipid, preferably as defined herein.

In preferred embodiments, the lipid-based carriers comprising at least one RNA of the first aspect comprise
(i) at least one cationic lipid selected or derived from ALC-0315, SM-102, SS-33/4PE-15, HEXA-C5DE-PipSS or compound C26 (see C26 in Table 1 of WO2021123332);
(ii) at least one neutral lipid selected or derived from DSPC, DHPC, or DPhyPE;
(iii) at least one steroid or steroid analogue selected or derived from cholesterol; and
(iv) at least one aggregation reducing lipid; and
wherein the lipid-based carriers encapsulate the RNA.

In preferred embodiments, the cationic lipids (as defined herein), neutral lipid (as defined herein), steroid or steroid analogue (as defined herein), and/or aggregation reducing lipid (as defined herein) may be combined at various relative ratios.

In preferred embodiments, the lipid-based carriers comprise (i) to (iv) in a molar ratio of about 20-60% cationic lipid or ionizable lipid, about 5-25% neutral lipid, about 25-55% steroid or steroid analogue, and about 0.5-15% aggregation reducing lipid e.g. polymer conjugated lipid, preferably wherein the lipid-based carriers encapsulate the RNA.

For example, the ratio of cationic lipid or ionizable lipid to neutral lipid to steroid or steroid analogue to aggregation reducing lipid may be between about 30-60:20-35:20-30:1-15, or at a ratio of about 40:30:25:5, 50:25:20:5, 50:20:25:5, 50:27:20:3, 40:30:20:10, 40:32:20:8, 40:32:25:3 or 40:33:25:2, respectively.

In preferred embodiments, the lipid-based carriers, preferably the LNPs comprising at least one RNA of the first aspect comprise
(i) at least one cationic lipid selected from SM-102;
(ii) at least one neutral lipid selected from DSPC;
(iii) at least one steroid or steroid analogue selected from cholesterol; and
(iv) at least one aggregation reducing lipid; and

wherein the lipid-based carriers encapsulate the RNA, preferably wherein i) to (iv) are in a weight ratio of about 50% cationic lipid, about 10% neutral lipid, about 38.5% steroid or steroid analogue, and about 1.5% aggregation reducing lipid,
preferably wherein the lipid-based carriers encapsulate the RNA.

In preferred embodiments, the lipid-based carriers, preferably the LNPs comprising at least one RNA of the first aspect comprise
(i) at least one cationic lipid selected from SM-102;
(ii) at least one neutral lipid selected from DSPC;
(iii) at least one steroid or steroid analogue selected from cholesterol; and
(iv) at least one aggregation reducing lipid; and

wherein the lipid-based carriers encapsulate the RNA, preferably wherein i) to (iv) are in a weight ratio of about 48.5% cationic lipid, about **11.1%** neutral lipid, about 38.9% steroid or steroid analogue, and about 1.5% aggregation reducing lipid,
preferably wherein the lipid-based carriers encapsulate the RNA. A preferred N/P ratio for this formulation is about 4.85 (lipid to RNA mol ratio).

In preferred embodiments, the lipid-based carriers, preferably the LNPs comprising at least one RNA of the first aspect comprise
(i) at least one cationic lipid selected from SS-33/4PE-15, HEXA-C5DE-PipSS or or compound C26 (see C26 in Table 1 of WO2021123332);
(ii) at least one neutral lipid selected from DPhyPE;
(iii) at least one steroid or steroid analogue selected from cholesterol; and
(iv) at least one aggregation reducing lipid; and
wherein the lipid-based carriers encapsulate the RNA. Such LNPs are herein referred to as GN-LNPs.

In preferred embodiments in that context, lipid-based carriers, preferably the GN-LNPs comprising the RNA comprise 59mol% HEXA-C5DE-PipSS lipid (see compound C2 in Table 1 of WO2021123332) as cationic lipid or preferably 59mol% compound C26 (see C26 in Table 1 of WO2021123332), 10mol% DPhyPE as neutral lipid, 29.3mol% cholesterol as steroid and 1.7mol% aggregation reducing lipid.

In another preferred embodiment in that context, lipid-based carriers, preferably the GN-LNPs comprising the RNA comprise 59mol% compound C26 (see C26 in Table 1 of WO2021123332) as cationic lipid, 10mol% DPhyPE as neutral lipid, 28.5mol% cholesterol as steroid and 2.5mol% aggregation reducing lipid.

In preferred embodiments, the wt/wt ratio of lipid to RNA in the lipid-based carrier is from about 10:1 to about 60:1, e.g. about 40:1. In particularly preferred embodiments, the wt/wt ratio of lipid to RNA is from about 20:1 to about 30:1, e.g. about 25:1. In other preferred embodiments, the wt/wt ratio of lipid to RNA is in the range of 20 to 60, preferably from about 3 to about 15, about 5 to about 13, about 4 to about 8 or from about 7 to about 11.

The amount of lipid comprised in the lipid-based carriers may be selected taking the amount of the RNA cargo into account. In one embodiment, these amounts are selected such as to result in an N/P ratio of the lipid-based carriers encapsulating the RNA in the range of about 0.1 to about 20. The N/P ratio is defined as the mole ratio of the nitrogen atoms ("N") of the basic nitrogen-containing groups of the lipid to the phosphate groups ("P") of the RNA which is used as cargo. The N/P ratio may be calculated on the basis that, for example, 1µg RNA typically contains about 3nmol phosphate residues, provided that the RNA exhibits a statistical distribution of bases. The "N"-value of the lipid or lipidoid may be calculated on the basis of its molecular weight and the relative content of permanently cationic and - if present - cationisable groups.

In embodiments, the N/P ratio can be in the range of about 1 to about 50. In other embodiments, the range is about 1 to about 20, and preferably about 1 to about 15. For "GN-LNPs", a suitable N/P (lipid to RNA mol ratio) is about 14 or about 17. A further preferred N/P i.e. lipid to RNA mol ratio is about 6. Another preferred N/P ratio is about 4.85 or 5 (lipid to RNA mol ratio).

In various embodiments, the pharmaceutical composition comprises lipid-based carriers (encapsulating RNA) that have a defined size (particle size, homogeneous size distribution).

The size of the lipid-based carriers of the pharmaceutical composition is typically described herein as Z-average size. The terms "average diameter", "mean diameter", "diameter" or "size" for particles (e.g. lipid-based carrier) are used synonymously with the value of the Z-average. The term "Z-average size" refers to the mean diameter of particles as measured by dynamic light scattering (DLS) with data analysis using the so-called cumulant algorithm, which provides as results the so-called Z-average with the dimension of a length, and the polydispersity index (PI), which is dimensionless (Koppel, D., J. Chem. Phys. 57, 1972, pp 4814-4820, ISO 13321).

The term "dynamic light scattering" or "DLS" refers to a method for analyzing particles in a liquid, wherein the liquid is typically illuminated with a monochromatic light source and wherein the light scattered by particles in the liquid is detected. DLS can thus be used to measure particle sizes in a liquid. Suitable DLS protocols are known in the art. DLS instruments are commercially available (such as the Zetasizer Nano Series, Malvern Instruments, Worcestershire, UK). DLS instruments employ either a detector at 90° (e.g. DynaPro^{®} NanoStar^{®} from Wyatt Technology or Zetasizer Nano S90^{®} from Malvern Instruments) or a backscatter detection system at 173° (e.g., Zetasizer Nano S^{®} from Malvern Instruments) and at 158° (DynaPro Plate Reader^{®} from Malvern Instruments) close to the incident light of 180°. Typically, DLS measurements are performed at a temperature of about 25°C. DLS is also used in the context of the present invention to determine the polydispersity index (PDI) and/or the main peak diameter of the lipid-based carriers incorporating RNA.

In various embodiments, the lipid-based carriers of the pharmaceutical composition encapsulating RNA have a Z-average size ranging from about 50nm to about 200nm, from about 50nm to about 190nm, from about 50nm to about 180nm, from about 50nm to about 170nm, from about 50nm to about 160nm, 50nm to about 150nm, 50nm to about 140nm, 50nm to about 130nm, 50nm to about 120nm, 50nm to about 110nm, 50nm to about 100nm, 50nm to about 90nm, 50nm to about 80nm, 50nm to about 70nm, 50nm to about 60nm, 60nm to about 200nm, from about 60nm to about 190nm, from about 60nm to about 180nm, from about 60nm to about 170nm, from about 60nm to about 160nm, 60nm to about 150nm, 60nm to about 140nm, 60nm to about 130nm, 60nm to about 120nm, 60nm to about 110nm, 60nm to about 100nm, 60nm to about 90nm, 60nm to about 80nm, or 60nm to about 70nm, for example about 50nm, 55nm, 60nm, 65nm, 70nm, 75nm, 80nm, 85nm, 90nm, 95nm, 100nm, 105nm, 110nm, 115nm, 120nm, 125nm, 130nm, 135nm, 140nm, 145nm, 150nm, 160nm, 170nm, 180nm, 190nm, or 200nm.

In preferred embodiments, the lipid-based carriers of the pharmaceutical composition encapsulating RNA have a Z-average size ranging from about 50nm to about 200nm, preferably in a range from about 50nm to about 150nm, more preferably from about 50nm to about 120nm, also more preferably about 65nm to about 90nm.

Preferably, the pharmaceutical composition comprises less than about 10%, 9%, 8%, 7%, 6%, 5%, 4%, 3%, 2%, 1% lipid-based carriers that have a particle size exceeding about 500nm.

Preferably, the pharmaceutical composition comprises less than about 10%, 9%, 8%, 7%, 6%, 5%, 4%, 3%, 2%, 1% LNPs that have a particle size smaller than about 20nm.

Preferably, at least about 80%, 85%, 90%, 95% of lipid-based carriers of the composition have a spherical morphology.

In preferred embodiments, the polydispersity index (PDI) of the lipid-based carriers is typically in the range of 0.1 to 0.5. In a particular embodiment, a PDI is below 0.2. Typically, the PDI is determined by dynamic light scattering.

In preferred embodiments, 80% of RNA comprised in the pharmaceutical composition is encapsulated in lipid based carriers, preferably 85% of the RNA comprised in the pharmaceutical composition is encapsulated in lipid based carriers, more preferably 90% of the RNA comprised in the pharmaceutical composition is encapsulated in lipid based carriers, most preferably 95% of the RNA comprised in the pharmaceutical composition is encapsulated in lipid based carriers. The percentage of encapsulation may be determined by a RiboGreen assay as known in the art.

According to a preferred embodiments the lipid-based carriers preferably encapsulating or comprising RNA are purified by at least one purification step, preferably by at least one step of TFF and/or at least one step of clarification and/or at least one step of filtration.

### mRNA

In one of the preferred embodiments, the nucleic acid compound is an mRNA or an mRNA compound. As has been found by the inventors, the lipids and the compositions according to the present invention are particularly suitable for the in vivo delivery of mRNA compounds expressing antigens, and thus enable highly effective, potent, versatile and safe vaccines that can be rapidly developed at moderate cost. Specific antigens of interest for carrying out the present invention are described in more detail below. The mRNA compound according to the invention in encapsulated in or associated with a lipid nanoparticle.

Advantages of the mRNA encoding at least one antigenic peptide or protein comprised in lipid nanoparticles (LNPs) are:
- Induction of a strong humoral immune response
- Induction of B-cell memory
- Faster onset of immune protection
- Longevity of the induced immune responses
- Induction of broad cellular T-cell responses
- Induction of a (local and transient) pro-inflammatory environment
- No induction of systemic cytokine or chemokine response
- Good tolerability, no side-effects, non-toxic
- Advantageous stability characteristics
- Formulation compatible with many different antigens: larger antigen cocktails feasible based on the same (production) technology
- No vector immunity, i.e. technology can be used to vaccinate the same subject multiple times against multiple (different) antigens
- Speed, adaptability, simplicity and scalability of production.

In certain embodiments, the lipid nanoparticles comprise at least:
(i) a cationic lipid and/or a polymer conjugated lipid according to formula (I); and
(ii) an mRNA compound comprising an mRNA sequence encoding an antigenic peptide or protein.

In other particular embodiments, the lipid nanoparticle composition comprises:
(a) a cationic lipid;
(b) a steroid;
(c) a neutral lipid;
(d) a polymer conjugated lipid according to formula (I); and
(e) an mRNA compound encoding a peptide or protein.

With respect to the cationic lipid, the steroid, the neutral lipid, the polymer conjugated lipid according to formula (I), and the mRNA compound encoding a peptide or protein, the same options, preferences and alternatives apply as have been described with respect to these features herein above. For example, in one of the preferred embodiments, the peptide or protein expressed by the mRNA compound is an antigen.

The amount of the cationic lipid relative to that of the mRNA compound in the lipid nanoparticle may also be expressed as a weight ratio (abbreviated e.g. "m/m"). For example, the lipid nanoparticles comprise the mRNA compound at an amount such as to achieve a lipid to mRNA weight ratio in the range of about 20 to about 60, or about 10 to about 50. In other embodiments, the ratio of cationic lipid to nucleic acid or mRNA is from about 3 to about 15, such as from about 5 to about 13, from about 4 to about 8 or from about 7 to about 11. In a very preferred embodiment of the present invention, the total lipid/mRNA mass ratio is about 40 or 40, i.e. about 40 or 40 times mass excess to ensure mRNA encapsulation. Another preferred RNA/lipid ratio is between about 1 and about 10, about 2 and about 5, about 2 and about 4, or preferably about 3.

Further, the amount of the cationic lipid may be selected taking the amount of the nucleic acid cargo such as the mRNA compound into account. In one embodiment, the N/P ratio can be in the range of about 1 to about 50. In another embodiment, the range is about 1 to about 20, about 1 to about 10, about 1 to about 5. In one preferred embodiment, these amounts are selected such as to result in an N/P ratio of the lipid nanoparticles or of the composition in the range from about 10 to about 20. In a further very preferred embodiment, the N/P is 14 (i.e. 14 times mol excess of positive charge to ensure mRNA encapsulation). In other very preferred embodiments, the N/P is 17.5 (i.e. 17.5 times mol excess of positive charge to ensure mRNA encapsulation) or
(i) at an amount such as to achieve an N/P ratio in the range of about 1 to about 20, preferably about 2 to about 15, more preferably about 3 to about 10, even more preferably about 4 to about 9, most preferably about 6;
(ii) at an amount such as to achieve an N/P ratio in the range of about 5 to about 20, more preferably about 10 to about 18, even more preferably about 12 to about 16, most preferably about 14; or
(iii) at an amount such as to achieve a lipid : mRNA weight ratio in the range of 20 to 60, preferably from about 3 to about 15, 5 to about 13, about 4 to about 8 or from about 7 to about 11

The total amount of mRNA in the lipid nanoparticles varies and may be defined depending on the mRNA to total lipid w/w ratio. In one embodiment of the invention the invention the mRNA to total lipid ratio is less than 0.06 w/w, preferably between 0.03 and 0.04 w/w.

Preferably, the mRNA compound or the coding sequence thereof has a length of about 50 to about 20000, or 100 to about 20000 nucleotides, preferably of about 250 to about 20000 nucleotides, more preferably of about 500 to about 10000, even more preferably of about 500 to about 5000.

As mentioned, the peptide or protein expressed by the mRNA compound may be an antigen. In other words, the composition comprises an mRNA compound which comprises an mRNA sequence encoding an antigenic peptide or protein, or a fragment, variant or derivative thereof. Such antigens, or antigenic peptides or proteins, may be derived from pathogenic antigens, tumor antigens, allergenic antigens or autoimmune self-antigens, or fragments or variants thereof, preferably as defined herein.

### Pathogenic antigens

Pathogenic antigens are derived from pathogenic organisms, in particular bacterial, viral or protozoological (multicellular) pathogenic organisms, which evoke an immunological reaction by subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Accordingly, in some preferred embodiments, the artificial nucleic acid (RNA) molecule may encode in its at least one coding region at least one pathogenic antigen selected from a bacterial, viral, fungal or protozoal antigen. The encoded (poly-)peptide or protein may consist or comprise of a pathogenic antigen or a fragment, variant or derivative thereof.

Pathogenic antigens are peptide or protein antigens preferably derived from a pathogen associated with an infectious disease which are preferably selected from, but not limited to, the group of antigens derived from the pathogens disclosed on pages 21-35 in WO2018078053; WO2018078053 being incorporated herein by reference in its entirety. Furthermore, pathogenic antigens are peptide or protein antigens preferably derived from a pathogen associated with an infectious disease which are preferably selected from, but not limited to, the group of antigens derived from the pathogens disclosed on page 57 paragraph 3 - page 63, paragraph 2 in WO2019077001; WO2019077001 being incorporated herein by reference in its entirety.

Even further pathogenic antigens are peptide or protein antigens preferably derived from a pathogen associated with infectious disease which are preferably selected from antigens derived from the pathogens selected from, but not limited to, the group of antigens derived from the pathogens disclosed on pages 32 line 26 - page 34 line 27 in WO2013120628. Furthermore in this regard, the pathogenic antigen (antigen derived from a pathogen associated with infectious disease) may be preferably selected from the antigens preferably selected from antigens selected from, but not limited to, the group of antigens as disclosed on pages 34 line 29 - page 59 line 5 (in brackets is the particular pathogen or the family of pathogens of which the antigen(s) is/are derived and the infectious disease with which the pathogen is associated) in WO2013120628; WO2013120628 being incorporated herein by reference in its entirety.

Among the preferred antigens expressed by the mRNA compound incorporated in the composition of the invention are pathogens selected from, but not limited to, the group consisting of a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), and Malaria parasites (e.g. Plasmodium falciparum, Plasmodium vivax, Plasmodium malariae, or Plasmodium ovale). In another one of the preferred embodiments, the pathogenic antigen is derived from a SARS coronavirus 2 (SARS-CoV-2), nCoV-2019 coronavirus, SARS coronavirus (SARS-CoV), Malaria parasite, an Influenza virus or a Rabies virus.

Further, pathogenic antigens may further preferably be selected from antigens derived from the pathogens selected from, but not limited to, the group consisting of Acinetobacter baumannii, Anaplasma genus, Anaplasma phagocytophi lum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia or other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, Coronaviruses, Coronaviridae family, Corynebacterium diphtheriae, Coxiella burnetii, Crimean-Congo haemorrhagic fever virus, Cryptococcus neoformans, Cryptosporidium genus, Cytomegalovirus (CMV), Dientamoeba fragilis, Ebola virus (EBOV - for example the envelope glycoprotein), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus or Enterovirus 71 (EV71), Epidermophyton spp, Epstein-Barr Virus (EBV), Escherichia coli O157:H7, O111 or 0104:H4, Fasciola hepatica or Fasciola gigantica, FFI prion, Feline immunodeficiency virus (FIV), Filarioidea superfami ly, Flaviviruses, Francisella tularensis, Fusobacterium genus, Geotrichum candidum, Giardia intestinalis, Gnathostoma spp, GSS prion, Guanarito virus, Haemophilus ducreyi, Haemophi lus influenzae, Helicobacter pylori, Henipavirus (Hendra virus, Nipah virus), Hepatitis A virus, Hepatitis B virus (HBV), Hepatitis C virus (HCV), Hepatitis D virus, Hepatitis E virus, Histoplasma capsulatum, Hortaea werneckii, Human bocavirus (HBoV), Human metapneumovirus (hMPV), Human parainfluenza viruses (HPIV), Japanese encephalitis virus, JC virus, Junin virus, Kingella kingae, Klebsiella granulomatis, Klebsiella pneumoniae, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae or Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rhinovirus, rhinoviruses, Rickettsia akari, Rickettsia genus, Rickettsia prowazekii, Rickettsia rickettsii, Rickettsia typhi, Rift Valley fever virus, Rotavirus (preferably e.g. VP8 antigen), Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus aureus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, vaccinia virus (preferably e.g. immune evasion proteins E3, K3, or B18), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yersinia enterocolitica, Yersinia pestis, or Yersinia pseudotuberculosis, Zika virus, Zika virus strains ZikaSPH2015-Brazil, Z1106033-Suriname, MR766-Uganda or Natal RGN, or an isoform, homolog, fragment, variant or derivative of any of these proteins, preferably from the Coronaviridae family.

In a further embodiment, pathogenic antigens useful for treating infections may be selected from the following antigens (the related infection and related pathogen are indicated in brackets after the respective antigens - naturally, also other antigens which may be derived from the following pathogens in brackets may be derived and used according to the invention):
- spike protein (S), an envelope protein (E), a membrane protein (M) or a nucleocapsid protein (N), or an immunogenic fragment or variant of any of these (infectious disease is "COVID-19 disease"; pathogen: SARS coronavirus 2 (SARS-CoV-2), nCoV-2019 coronavirus, SARS coronavirus (SARS-CoV));
- spike protein (S), a spike S1 fragment (S1), an envelope protein (E), a membrane protein (M) or a nucleocapsid protein (N) (infectious disease is MERS infection; pathogen: Middle East respiratory syndrome coronavirus (MERS coronavirus/MERS-CoV));
- replication protein E1, regulatory protein E2, protein E3, protein E4, protein E5, protein E6, protein E7, protein E8, major capsid protein L1, minor capsid protein L2 (infectious disease is Human papillomavirus (HPV) infection; pathogen: Human papillomavirus (HPV) or HPV16);
- fusion protein F, hemagglutinin-neuramidase HN, glycoprotein G, matrix protein M, phosphoprotein P, nucleoprotein N, polymerase L, hemagglutinin-neuraminidase, Fusion (F) glycoprotein F0, F1 or F2, Recombinant PIV3/PIV1 fusion glycoprotein (F) and hemagglutinin (HN), C protein, Phosphoprotein, D protein, matrix protein (M), nucleocapsid protein (N), viral replicase (L), non-structural V protein (infectious disease is Human parainfluenza virus infection; pathogen: Human parainfluenza viruses (HPIV/PIV) HPIV-1, HPIV-2, HPIV-3, or HPIV-4 serotype, preferably HPIV-3 serotype, preferably PIV3);
- fusion (F) glycoprotein, Glycoprotein G, Phosphoprotein P, Nucleoprotein N, Nucleocapsin protein (infectious disease: HMPV infection; pathogen: Human metapneumovirus (HMPV));
- hemagglutinin (HA), Neuraminidase (NA), Nucleoprotein (NP), M1 protein, M2 protein, NS1 protein, NS2 protein (NEP protein: nuclear export protein), PA protein, PB1 protein (polymerase basic 1 protein), PB1-F2 protein and PB2 protein, H10N8, H7N9, H10, H1N1, H3N2 (X31), H1, H2, H3, H4, H5, H6, H7, H8, H9, H10, H11, H12, H13, H14, H15, H16, H17, H18, antigenic subdomains of HA: HA1, HA2, neuraminidase (NA), nucleoprotein (NP), matrix protein 1 (M1), matrix protein 2 (M2), non-structural protein 1 (NS 1), nonstructural protein 2 (NS2), HA7 antigen, H7 or H10 and B, pathogen: Orthomyxoviridae family, Influenza virus (flu));
- nucleoprotein N, large structural protein L, phosphoprotein P, matrix protein M, glycoprotein G, G protein (infectious disease is Rabies; pathogen: Rabies virus);
- HIV p24 antigen, HIV envelope proteins (Gp120, Gp41, Gp160), polyprotein GAG, negative factor protein Nef, trans-activator of transcription Tat, Brec1 (infectious disease HIV; pathogen: Human immunodeficiency virus);
- major outer membrane protein MOMP, probable outer membrane protein PMPC, outer membrane complex protein B OmcB, heat shock proteins Hsp60 HSP10, protein IncA, proteins from the type III secretion system, ribonucleotide reductase small chain protein NrdB, plasmid protein Pgp3, chlamydial outer protein N CopN, antigen CT521, antigen CT425, antigen CT043, antigen TC0052, antigen TC0189, antigen TC0582, antigen TC0660, antigen TC0726, antigen TC0816, antigen TC0828 (infectious disease: infection with Chlamydia trachomatis; pathogen: Chlamydia trachomatis);
- pp65 antigen, membrane protein pp15, capsid-proximal tegument protein pp150, protein M45, DNA polymerase UL54, helicase UL105, glycoprotein gM, glycoprotein gN, glycoprotein H, glycoprotein B gB, protein UL83, protein UL94, protein UL99, HCMV glycoprotein selected from gH gL, gB, gO, gN, and gM, HCMV protein selected from UL83, UL123, UL128, UL130 and UL131A, Tegument protein pp150 (pp150), Tegument protein pp65/lower matrix phosphoprotein (pp65), Envelope glycoprotein M (UL100), Regulatory protein IE1 (UL123), Envelopeprotein (UL128), Envelope glycoprotein (130), Envelopeprotein (UL131A), Envelope glycoprotein B (UL55), Structural glycoprotein N gpUL73 (UL73), Structural glycoprotein O gpUL74 (UL74) (infectious disease is Cytomegalovirus infection; pathogen: Cytomegalovirus (CMV/HCMV));
- capsid protein C, premembrane protein prM, membrane protein M, envelope protein E (domain I, domain II, domain II), protein NS1, protein NS2A, protein NS2B, protein NS3, protein NS4A, protein 2K, protein NS4B, protein NS5 (infectious disease Dengue fever; pathogen: Dengue viruses (DENV-1, DENV-2, DENV-3 and DENV-4));
- glycoprotein (GP), surface GP, wild type pro-GP, mature GP, secreted wild type pro-GP, secreted mature GP, nucleoprotein (NP), RNA polymerase L, and matrix protein selected from VP35, VP40, VP24, and VP30 (infectious disease: Ebola; pathogen: Ebola virus (EBOV));
- hepatitis B surface antigen HBsAg, Hepatitis B core antigen HbcAg, polymerase, protein Hbx, preS2 middle surface protein, surface protein L, large S protein, virus protein VP1, virus protein VP2, virus protein VP3, virus protein VP4 (infectious disease is Hepatitis B; pathogen: Hepatitis B Virus (HBV));
- fusionprotein F, F protein, nucleoprotein N, matrix protein M, matrix protein M2-1, matrix protein M2-2, phophoprotein P, small hydrophobic protein SH, major surface glycoprotein G, polymerase L, non-structural protein 1 NS1, non-structural protein 2 NS2, RSV attachment protein (G) (glycoprotein G), Fusion (F) glycoprotein (glycoprotein F), nucleoprotein (N), phosphoprotein (P), large polymerase protein (L), matrix protein (M, M2), small hydrophobic protein (SH), nonstructural protein 1 (NS1), nonstructural protein 2 (NS2), membrane-bound RSV F protein, membrane-bound DS-Cavl (stabilized prefusion RSV F protein) (infectious disease is infection with Respiratory syncytial virus (RSV); pathogen: Respiratory syncytial virus (RSV));
- secretory antigen SssA (Staphylococcus genus, Staphylococcal food poisoning); secretory antigen SssA (Staphylococcus genus e.g. aureus, Staphylococcal infection);
- molecular chaperone DnaK, cell surface lipoprotein Mpt83, lipoprotein P23, phosphate transport system permease protein pstA, 14 kDa antigen, fibronectin-binding protein C FbpC1, Alanine dehydrogenase TB43, Glutamine synthetase 1, ESX-1 protein, protein CFP10, TB10.4 protein, protein MPT83, protein MTB12, protein MTB8, Rpf-like proteins, protein MTB32, protein MTB39, crystallin, heat-shock protein HSP65, protein PST-S (infectious disease is Tuberculosis; pathogen: Mycobacterium tuberculosis);
- genome polyprotein, protein E, protein M, capsid protein C, protease NS3, protein NS1, protein NS2A, protein AS2B, protein NS4A, protein NS4B, protein NS5 (infectious disease is Yellow fever; pathogen: Yellow fever virus (YFV));
- circumsporozoite protein (CSP) (infectious disease is Malaria; pathogen: P. falciparum and P. vivax); and
- Zika virus proteins in accordance with WO2017140905, i.e. capsid protein (C), premembrane protein (prM), pr protein (pr), membrane protein (M), envelope protein (E), non-structural protein, prME antigen, capsid protein, premembrane/membrane protein, non-structural protein 1, non-structural protein 2A, non- structural protein 2B, nonstructural protein 3, non-structural protein 4A, non-structural protein 4B, non-structural protein 5, or a Zika virus envelope protein (E) wherein the fusion loop in domain II is mutated in accordance with WO2017140905; WO2017140905 being incorporated herein by reference in its entirety (infectious disease is Zika virus infection; pathogen: Zika virus (ZIKV));

In some embodiments of the present invention, disclosure is provided for methods of inducing an antigen specific immune response in a subject, comprising administering to the subject any of the RNA (e.g. mRNA) vaccine as provided herein in an amount effective to produce an antigen-specific immune response.

In some embodiments, the RNA (e.g. mRNA) vaccine is a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) vaccine. In other embodiments, the RNA (e.g. mRNA) vaccine is a rabies, an influenza or a malaria vaccine.

In some embodiments, the RNA (e.g., mRNA) vaccine is a combination vaccine comprising a combination of influenza vaccines (a broad spectrum influenza vaccine). In some embodiments, an antigen- specific immune response comprises a T cell response or a B cell response.

In some embodiments, a method of producing an antigen-specific immune response comprises administering to a subject a single dose (i.e. no booster dose) of an SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) RNA (e.g., mRNA) vaccine of the present disclosure.

In some embodiments, a method further comprises administering to the subject a second (booster) dose of an SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) RNA (e.g. mRNA) vaccine may be administered.

In some embodiments, the subjects exhibit a seroconversion rate of at least 80% (e.g., at least 85%, at least 90%, or at least 95%) following the first dose or the second (booster) dose of the vaccine. Seroconversion is the time period during which a specific antibody develops and becomes detectable in the blood. After seroconversion has occurred, a virus can be detected in blood tests for the antibody. During an infection or immunization, antigens enter the blood, and the immune system begins to produce antibodies in response. Before seroconversion, the antigen itself may or may not be detectable, but antibodies are considered absent. During seroconversion, antibodies are present but not yet detectable. Any time after seroconversion, the antibodies can be detected in the blood, indicating a prior or current infection. In some embodiments, an RNA (e.g., mRNA) vaccine is administered to a subject by intradermal injection, intramuscular injection, or by intranasal administration. In some embodiments, an RNA (e.g. mRNA) vaccine is administered to a subject by intramuscular injection.

Some embodiments, of the present disclosure provide methods of inducing an antigen specific immune response in a subject, including administering to a subject an SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) RNA (e.g., mRNA) vaccine in an effective amount to produce an antigen specific immune response in a subject.

Antigen-specific immune responses in a subject may be determined, in some embodiments, by assaying for antibody titer (for titer of an antibody that binds to an SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus, Chlamydia trachomatis (i.e. bacterium chlamydia causing chlamydia), or Malaria parasite (e.g. Plasmodium falciparum, Plasmodium vivax, Plasmodium malariae, or Plasmodium ovale) antigenic polypeptide) following administration to the subject of any of the Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) RNA (e.g., mRNA) vaccines of the present disclosure. In some embodiments, the anti-antigenic polypeptide antibody titer produced in the subject is increased by at least 1 log relative to a control. In some embodiments, the anti-antigenic polypeptide antibody titer produced in the subject is increased by 1-3 log relative to a control.

### Tumor antigens

In a further preferred embodiment, the mRNA compound comprising an mRNA encodes a tumor antigen, preferably as defined herein, or a fragment or variant thereof, wherein the tumor antigen is preferably selected from, but not limited to, the group consisting of tumor antigens disclosed on pages 47-51 in WO2018078053; WO2018078053 being incorporated herein by reference in its entirety.

Furthermore, cytokines, chemokines, suicide enzymes and gene products, apoptosis inducers, endogenous angiogenesis inhibitors, heat shock proteins, tumor antigens, innate immune activators, antibodies directed against proteins associated with tumor or cancer development, useful for the present invention e.g. for cancer treatment, are selected from, but not limited to, the group of cytokines, chemokines, suicide enzymes and gene products, apoptosis inducers, endogenous angiogenesis inhibitors, heat shock proteins, tumor antigens, innate immune activators, antibodies directed against proteins associated with tumor or cancer development as disclosed in Table 1, Table 2, Table 3, Table 4, Table 5, Table 6, Table 7, Table 8, Table 9, Table 10, Table 11 and Table 12 of WO2016170176; WO2016170176 and especially Tables 1-12 being specifically incorporated herein by reference in its entirety.

### Therapeutic proteins and use for treatment or prophylaxis of any inherited or acquired disease

In a further embodiment, the active ingredient is a nucleic acid compound comprising at least one coding sequence, wherein the at least one coding sequence encodes a peptide or protein, wherein the protein is a therapeutic protein, or a fragment or variant of a therapeutic protein. In this context, a therapeutic peptide, protein or fragment thereof may be any peptidic compound useful the prevention, management, improvement, treatment or therapy of a disease or condition in a subject, such as an animal, and in particular in a human subject.

Thusly, in one embodiment, the mRNA comprising at least one coding sequence may encode
(a) a peptide or protein, or a fragment or variant thereof, wherein the peptide or protein is an antigen, wherein the antigen preferably is derived from pathogenic antigens, tumor antigens, allergenic antigens or autoimmune self-antigens, or a fragment or variant thereof; or
(b) a therapeutic protein or a fragment or variant thereof. The therapeutic protein may, for example, be selected from the group consisting of
   (i) therapeutic proteins for use in enzyme replacement therapy for the treatment of metabolic, endocrine or amino acid disorders or for use in replacing an absent, deficient or mutated protein;
   (ii) therapeutic proteins for use in the treatment of blood disorders, diseases of the circulatory system, diseases of the respiratory system, infectious diseases or immune deficiencies;
   (iii) therapeutic proteins for use in the treatment of cancer or tumor diseases;
   (iv) therapeutic proteins for use in hormone replacement therapy;
   (v) therapeutic proteins for use in reprogramming somatic cells into pluri- or omnipotent stem cells;
   (vi) therapeutic proteins for use as adjuvant or immunostimulation;
   (vii) therapeutic proteins being a therapeutic antibody;
   (viii) therapeutic proteins being a gene editing agent; and
   (ix) therapeutic proteins for use in treating or preventing a liver disease selected from the group consisting of liver fibrosis, liver cirrhosis and liver cancer.

In a specific embodiment, the therapeutic protein, or fragment or variant thereof, is selected from:
(i) therapeutic proteins for use in enzyme replacement therapy for the treatment of metabolic, endocrine or amino acid disorders or for use in replacing an absent, deficient or mutated protein, including Acid sphingomyelinase, Adipotide, Agalsidase-beta, Alglucosidase, alpha-galactosidase A, alpha-glucosidase, alpha-L-iduronidase, alpha-N-acetylglucosaminidase, Amphiregulin, Angiopoietins (Ang1, Ang2, Ang3, Ang4, ANGPTL2, ANGPTL3, ANGPTL4, ANGPTL5, ANGPTL6, ANGPTL7), ATPase, Cu(2+)-transporting beta polypeptide (ATP7B), argininosuccinate synthetase (ASS1), Betacellulin, Beta-glucuronidase, Bone morphogenetic proteins BMPs (BMP1, BMP2, BMP3, BMP4, BMP5, BMP6, BMP7, BMP8a, BMP8b, BMP10, BMP15), CLN6 protein, Epidermal growth factor (EGF), Epigen, Epiregulin, Fibroblast Growth Factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23), Fumarylacetoacetate Hydrolase (FAH), Galsulphase, Ghrelin, Glucocerebrosidase, GM-CSF, Heparin-binding EGF-like growth factor (HB-EGF), Hepatocyte growth factor HGF, Hepcidin, Human albumin, increased loss of albumin, Idursulphase (lduronate-2-sulphatase), Integrins αVβ3, αVβ5 and α5β1, luduronate sulfatase, Laronidase, N-acetylgalactosamine-4-sulfatase (rhASB; galsulfase, Arylsulfatase A (ARSA), Arylsulfatase B (ARSB)), N-acetylglucosamine-6-sulfatase, Nerve growth factor (NGF, Brain-Derived Neurotrophic Factor (BDNF), Neurotrophin-3 (NT-3), and Neurotrophin 4/5 (NT-4/5), Neuregulin (NRG1, NRG2, NRG3, NRG4), Neuropilin (NRP-1, NRP-2), Obestatin, phenylalanine hydroxylase (PAH), Phenylalanine ammonia lyase (PAL), Platelet Derived Growth factor (PDGF (PDFF-A, PDGF-B, PDGF-C, PDGF-D), TGF beta receptors (endoglin, TGF-beta 1 receptor, TGF-beta 2 receptor, TGF-beta 3 receptor), Thrombopoietin (THPO) (Megakaryocyte growth and development factor (MGDF)), Transforming Growth factor (TGF (TGF-a, TGF-beta (TGFbeta1, TGFbeta2, and TGFbeta3))), VEGF (VEGF-A, VEGF-B, VEGF-C, VEGF-D, VEGF-E, VEGF-F und PIGF), Nesiritide, Trypsin, adrenocorticotrophic hormone (ACTH), Atrial-natriuretic peptide (ANP), Cholecystokinin, Gastrin, Leptin, Oxytocin, Somatostatin, Vasopressin (antidiuretic hormone), Calcitonin, Exenatide, Growth hormone (GH), somatotropin, Insulin, Insulin-like growth factor 1 IGF-1, Mecasermin rinfabate, IGF-1 analog, Mecasermin, IGF-1 analog, Pegvisomant, Pramlintide, Teriparatide (human parathyroid hormone residues 1-34), Becaplermin, Dibotermin-alpha (Bone morphogenetic protein 2), Histrelin acetate (gonadotropin releasing hormone; GnRH), Octreotide, hepatocyte nuclear factor 4 alpha (HNF4A), CCAAT/enhancer-binding protein alpha (CEBPA), fibroblast growth factor 21 (FGF21), extracellular matrix protease or human collagenase MMP1, Hepatocyte Growth Factor (HGF), TNF-related apoptosis-inducing ligand (TRAIL), opioid growth factor receptor-like 1 (OGFRL1), clostridial type II collagenase, Relaxin 1 (RLN1), Relaxin 2 (RLN2), Relaxin 3 (RLN3) and Palifermin (keratinocyte growth factor; KGF);
(ii) therapeutic proteins for use in the treatment of blood disorders, diseases of the circulatory system, diseases of the respiratory system, cancer or tumor diseases, infectious diseases or immune deficiencies, including Alteplase (tissue plasminogen activator; tPA), Anistreplase, Antithrombin III (AT-III), Bivalirudin, Darbepoetin-alpha, Drotrecogin-alpha (activated protein C, Erythropoietin, Epoetin-alpha, erythropoietin, erthropoyetin, Factor IX, Factor VIIa, Factor VIII, Lepirudin, Protein C concentrate, Reteplase (deletion mutein of tPA), Streptokinase, Tenecteplase, Urokinase, Angiostatin, Anti-CD22 immunotoxin, Denileukin diftitox, Immunocyanin, MPS (Metallopanstimulin), Aflibercept, Endostatin, Collagenase, Human deoxy-ribonuclease I, dornase, Hyaluronidase, Papain, L-Asparaginase, Peg-asparaginase, Rasburicase, Human chorionic gonadotropin (HCG), Human follicle-stimulating hormone (FSH), Lutropin-alpha, Prolactin, alpha-1-Proteinase inhibitor, Lactase, Pancreatic enzymes (lipase, amylase, protease), Adenosine deaminase (pegademase bovine, PEG-ADA), Abatacept, Alefacept, Anakinra, Etanercept, Interleukin-1 (IL-1) receptor antagonist, Anakinra, Thymulin, TNF-alpha antagonist, Enfuvirtide, and Thymosin α1;
(iii) therapeutic proteins for use in the treatment of cancer or tumor diseases, including cytokines, chemokines, suicide gene products, immunogenic proteins or peptides, apoptosis inducers, angiogenesis inhibitors, heat shock proteins, tumor antigens, beta-catenin inhibitors, activators of the STING pathway, checkpoint modulators, innate immune activators, antibodies, dominant negative receptors and decoy receptors, inhibitors of myeloid derived suppressor cells (MDSCs), IDO pathway inhibitors, and proteins or peptides that bind inhibitors of apoptosis;
(iv) therapeutic proteins selected from adjuvant or immunostimulating proteins, including human adjuvant proteins, particularly pattern recognition receptors TLR1, TLR2, TLR3, TLR4, TLR5, TLR6, TLR7, TLR8, TLR9, TLR10, TLR11; NOD1, NOD2, NOD3, NOD4, NOD5, NALP1, NALP2, NALP3, NALP4, NALP5, NALP6, NALP6, NALP7, NALP7, NALP8, NALP9, NALP10, NALP11, NALP12, NALP13, NALP14,I IPAF, NAIP, CIITA, RIG-I, MDA5 and LGP2, the signal transducers of TLR signaling including adaptor proteins including e.g. Trif and Cardif; components of the Small-GTPases signalling (RhoA, Ras, Rac1, Cdc42, Rab etc.), components of the PIP signalling (PI3K, Src-Kinases, etc.), components of the MyD88-dependent signalling (MyD88, IRAK1, IRAK2, IRAK4, TIRAP, TRAF6 etc.), components of the MyD88-independent signalling (TICAM1, TICAM2, TRAF6, TBK1, IRF3, TAK1, IRAK1 etc.); the activated kinases including e.g. Akt, MEKK1, MKK1, MKK3, MKK4, MKK6, MKK7, ERK1, ERK2, GSK3, PKC kinases, PKD kinases, GSK3 kinases, JNK, p38MAPK, TAK1, IKK, and TAK1; the activated transcription factors including e.g. NF-kB, c-Fos, c-Jun, c-Myc, CREB, AP-1, Elk-1, ATF2, IRF-3, IRF-7, heat shock proteins, such as HSP10, HSP60, HSP65, HSP70, HSP75 and HSP90, gp96, Fibrinogen, Typlll repeat extra domain A of fibronectin; or components of the complement system including C1q, MBL, C1r, C1s, C2b, Bb, D, MASP-1, MASP-2, C4b, C3b, C5a, C3a, C4a, C5b, C6, C7, C8, C9, CR1, CR2, CR3, CR4, C1qR, C1INH, C4bp, MCP, DAF, H, I, P and CD59, or induced target genes including e.g. Beta-Defensin, cell surface proteins; or human adjuvant proteins including trif, flt-3 ligand, Gp96 or fibronectin, cytokines which induce or enhance an innate immune response, including IL-1 alpha, IL1 beta, IL-2, IL-6, IL-7, IL-8, IL-9, IL-12, IL-13, IL-15, IL-16, IL-17, IL-18, IL-21, IL-23, TNFalpha, IFNalpha, IFNbeta, IFNgamma, GM-CSF, G-CSF, M-CSF; chemokines including IL-8, IP-10, MCP-1, MIP-1alpha, RANTES, Eotaxin, CCL21; cytokines which are released from macrophages, including IL-1, IL-6, IL-8, IL-12 and TNF-alpha; as well as IL-1R1 and IL-1 alpha;
(v) bacterial (adjuvant) proteins, including bacterial heat shock proteins or chaperons, including Hsp60, Hsp70, Hsp90, Hsp100; OmpA (Outer membrane protein) from gram-negative bacteria; OspA; bacterial porins, including OmpF; bacterial toxins, including pertussis toxin (PT) from Bordetella pertussis, pertussis adenylate cyclase toxin CyaA and CyaC from Bordetella pertussis, PT-9K/129G mutant from pertussis toxin, pertussis adenylate cyclase toxin CyaA and CyaC from Bordetella pertussis, tetanus toxin, cholera toxin (CT), cholera toxin B-subunit, CTK63 mutant from cholera toxin, CTE112K mutant from CT, Escherichia coli heat-labile enterotoxin (LT), B subunit from heat-labile enterotoxin (LTB) Escherichia coli heat-labile enterotoxin mutants with reduced toxicity, including LTK63, LTR72; phenol-soluble modulin; neutrophil-activating protein (HP-NAP) from Helicobacter pylori; Surfactant protein D; Outer surface protein A lipoprotein from Borrelia burgdorferi, Ag38 (38 kDa antigen) from Mycobacterium tuberculosis; proteins from bacterial fimbriae; Enterotoxin CT of Vibrio cholerae, Pilin from pili from gram negative bacteria, and Surfactant protein A and bacterial flagellins;
(vi) protozoan (adjuvant) proteins, including Tc52 from Trypanosoma cruzi, PFTG from Trypanosoma gondii, Protozoan heat shock proteins, LelF from Leishmania spp., profiling-like protein from Toxoplasma gondii;
(vii) viral (adjuvant) proteins, including Respiratory Syncytial virus fusion glycoprotein (F-protein), envelope protein from MMT virus, mouse leukemia virus protein, Hemagglutinin protein of wild-type measles virus;
   fungal (adjuvant) proteins, including fungal immunomodulatory protein (FIP; LZ-8);
   animal-derived proteins, including Keyhole limpet hemocyanin (KLH);
   therapeutic proteins used for hormone replacement therapy, wherein the hormones include oestrogens, progesterone or progestins, and testosterone; and
(viii) therapeutic proteins used for reprogramming somatic cells into pluri- or omnipotent stem cells, including Oct-3/4, Sox gene family (Sox1, Sox2, Sox3, and Sox15), Klf family (Klf1, Klf2, Klf4, and Klf5), Myc family (c-myc, L-myc, and N-myc), Nanog, and LIN28.

In further, specific preferred embodiments, the mRNA encapsulated in the lipid nanoparticle as described herein comprising the polymer conjugated lipid of the invention according to formula (I), encodes a protein selected from the group consisting of
- hepatocyte nuclear factor 4 alpha (HNF4a) for the treatment of liver diseases, e.g. preferably liver cirrhosis or liver fibrosis
- phenylalanine hydroxylase (PAH) for the treatment of phenylketonuria ;
- alpha-galactosidase A (aGAL) for the treatment of Fabry disease;
- ATPase, Cu(2+)-transporting, beta polypeptide (ATP7B) for the treatment of Wilsons disease;
- fumarylacetoacetate Hydrolase (FAH) for the treatment of Tyrosinemia;
- argininosuccinate synthetase (ASS1) for the treatment of Argininosuccinate Synthetase Deficiency (ASD, Citrullinemia);
- argininosuccinate Lyase (ASL) for the treatment of Argininosuccinate Lyase Deficiency (ASLD);
- cystic fibrosis transmembrane conductance regulator (CFTR) for the treatment of cystic fibrosis;
- Relaxin for the treatment of heart failure;
- PD-L1 for the treatment of autoimmune hepatitis;
- IL-2 for the treatment of autoimmune disorders;
- KRAS for the treatment of CRC, NSCLS, and/or pancreatic cancer;
- vascular endothelial growth factor (VEGF-A) for the treatment of myocardial ischemia;
- propionyl-CoA carboxylase subunit A/B (PCCA/PCCB) for the treatment of propionic acidemia (PA);
- methylmalonyl-CoA mutase (MUT) for the treatment of methylmalonic acidemia (MMA);
- glucose 6-phosphatase (G6Pase) for the treatment of Glycogen Storage Disorder Type 1a (GSD1a); and
- ornithine transcarbamylase for the treatment of OTC deficiency.

In an even further, specifically preferred embodiment, the mRNA encapsulated in the lipid nanoparticle as described herein comprising the polymer conjugated lipid of the invention, encodes at least one CRISPR-associated protein, preferably wherein said CRISPR-associated protein is selected from Cas9, Cpf1 (Cas12), C2c1, C2c3, Cas13, CasX or CasY, also preferably wherein said CRISPR-associated protein comprises or consists of an amino acid sequence according to any one of SEQ ID NO:428-441, 10999-11001, 442-1345 as disclosed in WO2018172556, or an amino acid sequence having, in increasing order of preference, at least 50%, 60%, 70%, 80%, 90%, 95%, 96%, 97%, 98%, or 99% sequence identity to the amino acid sequence according to any one of SEQ ID NO:428-441, 10999-11001, 442-1345 as disclosed in WO2018172556, or a variant or fragment of any of these sequences. WO2018172556 is incorporated herein by reference in its entirety.

This invention includes methods for preventing, ameliorating or treating a disease or condition in a subject in need comprising administering to the subject a composition as described herein. The compositions of this invention may be used in the treatment of the human or animal body.

In this context, particularly preferred therapeutic proteins which can be used inter alia in the treatment of metabolic or endocrine disorders are selected from those which are disclosed in Table A (in combination with Table C) of WO2017191274. Furthermore, diseases which preferably can be treated with the composition of the invention, preferably selected from infectious diseases, neoplasms (e.g. cancer or tumor diseases), diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, are disclosed in WO2017191274 on pages 95 line 4 - page 103 line 24. Further particularly preferred therapeutic proteins which can be used inter alia in the treatment of metabolic or endocrine disorders are disclosed in Table 1 of WO2017191274, which also refers to specific target / disease combinations, incorporated herein by reference, and also sequences. WO2017191274 incl. Tables A/C and Table 1 is incorporated herein by reference in its entirety.

In preferred embodiments, artificial nucleic acid (RNA) molecules, (pharmaceutical) compositions or vaccines or kits are used for treatment or prophylaxis of infectious diseases. The term "infection" or "infectious disease" relates to the invasion and multiplication of microorganisms such as bacteria, viruses, and parasites that are not normally present within the body. An infection may cause no symptoms and be subclinical, or it may cause symptoms and be clinically apparent. An infection may remain localized, or it may spread through the blood or lymphatic system to become systemic. Infectious diseases in this context, preferably include viral, bacterial, fungal or protozoological infectious diseases. In particular, infectious diseases are selected from the group as disclosed starting on page 157, section "Infectious diseases" (ending on page 160) of WO2019077001; WO2019077001 being incorporated herein by reference in its entirety.

In this context, further particularly preferred examples for diseases and/or conditions for which the compositions of the invention or respectively the translatable molecules of the invention can be used for treatment are disclosed in Table 2 of US20190002906; US20190002906 incl. Table 2 being incorporated herein by reference in its entirety.

Liver disease or liver-related diseases in animals, more particularly humans, may include but would not be limited to congenital diseases or acquired diseases for example viral and parasite infectious diseases, oncologic pathologies such as primary tumors and metastases, metabolic, amino acid and/or endocrine disorders as well as inflammatory and immune and auto-immune conditions. Liver diseases which may preferably be treated with the inventive composition are selected from, but not limited to the group consisting of Hepatitis C, Hepatitis B, Hepatitis, Hepatitis A, Cirrhosis, Liver Cancer, Hepatocellular Carcinoma, Hepatic Encephalopathy, Autoimmune Hepatitis, Alpha-1 Antitrypsin Deficiency (AAT-deficiency), Hepatitis D, Phenylketonuria (PKU), Wilson's disease (hepatolenticular degeneration), Tyrosinemia Type I (FAH deficiency), Alagille Syndrome, Portal Hypertension, Steatohepatitis, Chronic Hepatitis and Hepatitis E.

In a further preferred embodiment, the compositions of the present invention may be used in method of treating or preventing a disorder, wherein the disorder is a liver disease, preferably selected from the group consisting of liver fibrosis, liver cirrhosis and liver cancer. Accordingly, the mRNA comprising at least one coding sequence may encode a therapeutic protein or a fragment or variant thereof for use in treating or preventing a liver disease selected from the group consisting of liver fibrosis, liver cirrhosis and liver cancer. Furthermore, preferably, the mRNA for treating or preventing liver diseases or a liver disease selected from the group consisting of liver fibrosis, liver cirrhosis and liver cancer encodes a peptide or protein selected from the group consisting of hepatocyte nuclear factor 4 alpha (HNF4A), CCAAT/enhancer-binding protein alpha (CEBPA), fibroblast growth factor 21 (FGF21), extracellular matrix protease or human collagenase MMP1, Hepatocyte Growth Factor (HGF), TNF-related apoptosis-inducing ligand (TRAIL), opioid growth factor receptor-like 1 (OGFRL1), clostridial type II collagenase, Relaxin 1 (RLN1), Relaxin 2 (RLN2) and Relaxin 3 (RLN3). In this regard, the liver disease specific disclosure of WO2018104538 as well as the sequences which are disclosed in WO2018104538 is incorporated herein by reference.

### Other antigens

Further antigens useful for the present invention are listed in WO2018078053 on pages 48-51; WO2018078053 being incorporated herein by reference in its entirety.

### Allergenic antigens and autoimmune self-antigens

As mentioned, the mRNA compound comprised in the composition of the invention may, according to some embodiments, encode an antigen that represents an allergen, or an allergenic antigen or a self-antigen, also referred to as autoantigen or autoimmune antigen.

Such antigens and self-antigens associated with allergy or allergic disease (allergens or allergenic antigens) are derived from or preferably selected from, but not limited to, the group of antigens disclosed on pages 59-73 in WO2018078053; WO2018078053 being incorporated herein by reference in its entirety.

### Checkpoint modulators / Checkpoint inhibitors

In the context of the present invention, an immune checkpoint protein, checkpoint modulator or checkpoint inhibitor is typically a molecule, such as a protein (e.g. an antibody), a dominant negative receptor, a decoy receptor, or a ligand or a fragment or variant thereof, which modulates the function of an immune checkpoint protein, e.g. it inhibits or reduces the activity of checkpoint inhibitors (or inhibitory checkpoint molecules) or it stimulates or enhances the activity of checkpoint stimulators (or stimulatory checkpoint molecules). Therefore, checkpoint modulators as defined herein, influence the activity of checkpoint molecules. In this context, inhibitory checkpoint molecules are defined as checkpoint inhibitors and can be used synonymously. In addition, stimulatory checkpoint molecules are defined as checkpoint stimulators and can be used synonymously.

In a further preferred embodiment, the mRNA compound comprising an mRNA encodes an immune checkpoint protein, checkpoint modulators or checkpoint inhibitor, preferably as defined herein, or a fragment or variant thereof, wherein the immune checkpoint protein, checkpoint modulators or checkpoint inhibitor is preferably selected from, but not limited to, the group consisting of immune checkpoint proteins, checkpoint modulators or checkpoint inhibitors disclosed on pages 51-56 in WO2018078053; WO2018078053 being incorporated herein by reference in its entirety.

### RNA elements, mRNA elements

According to certain embodiments of the present invention, the mRNA sequence is mono-, bi-, or multicistronic, preferably as defined herein. The coding sequences in a bi- or multicistronic mRNA preferably encode distinct peptides or proteins as defined herein or a fragment or variant thereof. Preferably, the coding sequences encoding two or more peptides or proteins may be separated in the bi- or multicistronic mRNA by at least one IRES (internal ribosomal entry site) sequence, as defined below. Thus, the term "encoding two or more peptides or proteins" may mean, without being limited thereto, that the bi- or even multicistronic mRNA, may encode e.g. at least two, three, four, five, six or more (preferably different) peptides or proteins or their fragments or variants within the definitions provided herein. More preferably, without being limited thereto, the bi- or even multicistronic mRNA, may encode, for example, at least two, three, four, five, six or more (preferably different) peptides or proteins as defined herein or their fragments or variants as defined herein. In this context, a so-called IRES (internal ribosomal entry site) sequence as defined above can function as a sole ribosome binding site, but it can also serve to provide a bi- or even multicistronic mRNA as defined above, which encodes several peptides or proteins which are to be translated by the ribosomes independently of one another. Examples of IRES sequences, which can be used according to the invention, are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukemia virus (MLV), simian immunodeficiency viruses (SIV) or cricket paralysis viruses (CrPV).

According to a further embodiment the at least one coding region of the mRNA sequence according to the invention may encode at least two, three, four, five, six, seven, eight and more peptides or proteins (or fragments and derivatives thereof) as defined herein linked with or without an amino acid linker sequence, wherein said linker sequence can comprise rigid linkers, flexible linkers, cleavable linkers (e.g., self-cleaving peptides) or a combination thereof. Therein, the peptides or proteins may be identical or different or a combination thereof. Particular peptide or protein combinations can be encoded by said mRNA encoding at least two peptides or proteins as explained herein (also referred to herein as "multi-antigen-constructs/mRNA").

In another preferred embodiment, the mRNA compound comprised in the composition encodes a pathogenic antigen whose amino acid sequence is not modified with respect to the respective wild type amino acid sequence. In this case, the mRNA compound may also comprise a coding region with a nucleic acid sequence which is not modified with respect to the respective wild type mRNA sequence. For example, the mRNA compound may be a natural and non-modified mRNA. As used herein, natural and non-modified mRNA encompasses mRNA generated in vitro, without chemical modifications or changes in the sequence.

### Self-replicating RNA

In one embodiment, the cargo of the lipid nanoparticle, or respectively the mRNA or RNA sequence of the invention is capable of self-replication. Thusly, a polynucleotide may be capable of self-replication when introduced into a host cell. Examples of polynucleotides thus include self-replicating RNAs and DNAs and, for instance, selected from replicons, plasmids, cosmids, phagemids, transposons, viral vectors, artifical chromosomes (e.g., bacterial, yeast, etc.) as well as other self-replicating species. Polynucleotides include those that express antigenic polypeptides in a host cell (e.g., polynucleotide-containing antigens). Polynucleotides include self-replicating polynucleotides within which natural or synthetic sequences derived from eucaryotic or prokaryotic organisms (e.g., genomic DNA sequences, genomic RNA sequences, cDNA sequences, etc.) have been inserted. Specific examples of self-replicating polynucleotides include RNA vector constructs and DNA vector constructs, among others. Sequences that may be expressed include native sequences and modifications, such as deletions, additions and substitutions (generally conservative in nature), to native sequences, among others. These modifications may be deliberate, as through site-directed mutagenesis, or may be accidental, such as through mutations of hosts that produce antigens. In one aspect, the self-replicating RNA molecule is derived from or based on an alphavirus. In other aspects, the self-replicating RNA molecule is derived from or based on a virus other than an alphavirus, preferably, a positive-stranded RNA virus, and more preferably a picornavirus, flavivirus, rubivirus, pestivirus, hepacivirus, calicivirus, or coronavirus. Suitable wild-type alphavirus sequences are well-known and are available from sequence depositories, such as the American Type Culture Collection, Rockville, Md. Representative examples of suitable alphaviruses include Aura (ATCC VR-368), Bebaru virus (ATCC VR-600, ATCC VR-1240), Cabassou (ATCC VR-922), Chikungunya virus (ATCC VR-64, ATCC VR-1241), Eastern equine encephalomyelitis virus (ATCC VR-65, ATCC VR-1242), Fort Morgan (ATCC VR-924), Getah virus (ATCC VR-369, ATCC VR-1243), Kyzylagach (ATCC VR-927), Mayaro (ATCC VR-66), Mayaro virus (ATCC VR-1277), Middleburg (ATCC VR-370), Mucambo virus (ATCC VR-580, ATCC VR-1244), Ndumu (ATCC VR-371), Pixuna virus (ATCC VR-372, ATCC VR-1245), Ross River virus (ATCC VR-373, ATCC VR-1246), Semliki Forest (ATCC VR-67, ATCC VR-1247), Sindbis virus (ATCC VR-68, ATCC VR-1248), Tonate (ATCC VR-925), Triniti (ATCC VR-469), Una (ATCC VR-374), Venezuelan equine encephalomyelitis (ATCC VR-69, ATCC VR-923, ATCC VR-1250 ATCC VR-1249, ATCC VR-532), Western equine encephalomyelitis (ATCC VR-70, ATCC VR-1251, ATCC VR-622, ATCC VR-1252), Whataroa (ATCC VR-926), and Y-62-33 (ATCC VR-375).

Various methods are known in the art and conceivable in the context of the invention.

### mRNA Modifications and Sequences

In another embodiment of the invention, the mRNA compound comprises an artificial mRNA. In this context, artificial mRNA encompasses mRNA with chemical modifications, sequence modifications or non-natural sequences.

### Chemical Modifications

According to another embodiment of the invention, the mRNA compound comprised in the composition comprises at least one chemical modification. In one embodiment, the chemical modification may be selected from the group consisting of base modifications, sugar modifications, backbone modifications and lipid modifications. A backbone modification in connection with the present invention is a modification in which phosphates of the backbone of the nucleotides contained in an mRNA compound comprising an mRNA sequence as defined herein are chemically modified. A sugar modification in connection with the present invention is a chemical modification of the sugar of the nucleotides of the mRNA compound comprising an mRNA sequence as defined herein. Furthermore, a base modification in connection with the present invention is a chemical modification of the base moiety of the nucleotides of the mRNA compound comprising an mRNA sequence. In this context, nucleotide analogues or modifications are preferably selected from nucleotide analogues, which are applicable for transcription and/or translation.

### Sugar Modifications

The modified nucleosides and nucleotides, which may be incorporated into a modified mRNA compound comprising an mRNA sequence as described herein, can be modified in the sugar moiety. For example, the 2' hydroxyl group (OH) can be modified or replaced with a number of different "oxy" or "deoxy" substituents. Examples of "oxy" -2' hydroxyl group modifications include, but are not limited to, alkoxy or aryloxy (-OR, e.g., R=H, alkyl, cycloalkyl, aryl, aralkyl, heteroaryl or sugar); polyethylene glycols (PEG), -O(CH2CH2O)nCH2CH2OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified mRNA can include nucleotides containing, for instance, arabinose as the sugar.

### Backbone Modifications

The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phospho-rodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

### Lipid Modifications

A lipid-modified mRNA typically comprises an mRNA as defined herein. Such a lipid-modified mRNA as defined herein typically further comprises at least one linker covalently linked with that mRNA, and at least one lipid covalently linked with the respective linker. Alternatively, the lipid-modified mRNA comprises at least one mRNA as defined herein and at least one (bifunctional) lipid covalently linked (without a linker) with that mRNA. According to a third alternative, the lipid-modified mRNA comprises an mRNA molecule as defined herein, at least one linker covalently linked with that mRNA, and at least one lipid covalently linked with the respective linker, and also at least one (bifunctional) lipid covalently linked (without a linker) with that mRNA. In this context, it is particularly preferred that the lipid modification is present at the terminal ends of a linear mRNA sequence.

### Covalently

The term "covalently" refers to a linkage or conjugation by a chemical bond, i.e. through direct covalent chemical bonding, i.e. not through electrostatic association or interactions or the like.

### Base Modifications

In an alternative embodiment, the mRNA compound comprises at least one base modification.

Modified nucleosides and nucleotides, which may be incorporated into a modified mRNA compound comprising an mRNA sequence as described herein can further be modified in the nucleobase moiety. Examples of nucleobases found in mRNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methylinosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-Iodo-2'-deoxycytidine-5'-triphosphate, 5-iodouridine-5'-triphosphate, 5-Iodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, 06-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate. In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine. In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine. In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyl-adenosine, 2-methylthio-N6-threonyl carbamoyladenosine, N6,N6-dimethyladenosine, 7-methyladenine, 2-methyl-thio-adenine, and 2-methoxy-adenine. In other embodiments, modified nucleosides include inosine, 1-methylinosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. In some embodiments, the nucleotide can be modified on the major groove face and can include replacing hydrogen on C-5 of uracil with a methyl group or a halo group. In specific embodiments, a modified nucleoside is 5'-O-(1-thiophosphate)-adenosine, 5'-O-(1-thiophosphate)-cytidine, 5'-O-(1-thiophosphate)-guanosine, 5'-O-(1-thiophosphate)-uridine or 5'-O-(1-thiophosphate)-pseudouridine.

In further specific embodiments, a modified mRNA may comprise nucleoside modifications selected from 6-aza-cytidine, 2-thio-cytidine, α-thio-cytidine, Pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, Pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, α-thio-adenosine, 8-azido-adenosine, 7-deaza-adenosine.

In further embodiments, the chemical modification is selected from pseudouridine, N1-methylpseudouridine, N1-ethylpseudouridine, 2-thiouridine, 4'-thiouridine, 5-methylcytosine, 5-methyluridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine and 2'-O-methyl uridine.

In a specific embodiment, the chemical modification is selected from the group consisting of pseudouracil (psi or ψ), N1-methylpseudouridine (N1MPU, N1Mpsi or N1Mψ), 1-ethylpseudouracil, 2-thiouracil (s2U), 4-thiouracil, 5-methylcytosine, 5-methyluracil, 5-methoxyuracil, and any combination thereof, most preferably the chemical modification is N1-methylpseudouridine (N1MPU, N1Mpsi or N1Mψ).

In another preferred embodiment, the mRNA compound does not comprise nucleoside modifications, in particular no base modifications. In a further embodiment, the mRNA compound does not comprise 1-methylpseudouridine, pseudouridine or 5-methoxy-uridine modifications. In one preferred embodiment, the mRNA comprises only naturally existing nucleosides. In a further preferred embodiment, the mRNA compound does not comprise any chemical modification and optionally comprises sequence modifications. In a further preferred embodiment of the invention the mRNA compound only comprises the naturally existing nucleosides adenine, uracil, guanine and cytosine.

### Sequence Modifications

According to a further embodiment, the mRNA compound comprises a modified mRNA sequence. For example, a modification of the mRNA sequence may lead to the stabilization of the mRNA sequence. In one embodiment, the mRNA compound comprises a stabilized mRNA sequence comprising at least one coding region as defined herein. In particular, the composition of the invention as described herein may comprise an mRNA compound comprising a coding region encoding a peptide or a protein, such as defined in any of the embodiments described herein, wherein said coding region exhibits a sequence modification.

According to one embodiment, the mRNA compound comprises a "stabilized mRNA sequence", that is to say as an mRNA that is essentially resistant to in vivo degradation (e.g. by an exo- or endo-nuclease). Such stabilization can be effected, for example, by a modified phosphate backbone of the mRNA of the present invention. A backbone modification in connection with the present invention is a modification in which phosphates of the backbone of the nucleotides contained in the mRNA are chemically modified. Nucleotides that may be preferably used in this connection contain e.g. a phosphorothioate-modified phosphate backbone, preferably at least one of the phosphate oxygens contained in the phosphate backbone being replaced by a sulfur atom. Stabilized mRNAs may further include, for example: non-ionic phosphate analogues, such as, for example, alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include, without implying any limitation, modifications from the group consisting of methylphosphonates, phosphoramidates and phosphorothioates (e.g. cytidine-5'-O-(1-thiophosphate)).

In the following, specific modifications are described which are preferably capable of "stabilizing" the mRNA as defined herein.

### G/C content Modifications

According to one embodiment, the mRNA compound comprises an mRNA sequence which is modified, and thus stabilized, by a modification of its guanosine/cytosine (G/C) content. Such modification, or at least one of these modifications, is located in a coding region of the mRNA compound.

In one preferred embodiment, the G/C content of the coding region of the mRNA compound is increased compared to the G/C content of the coding region of the respective wild type mRNA, i.e. the unmodified mRNA. At the same time, the amino acid sequence encoded by the mRNA is preferably not modified as compared to the amino acid sequence encoded by the respective wild type mRNA. For example, the composition as described above may comprise an mRNA compound encoding a pathogenic antigen whose amino acid sequence is not modified with respect to the encoded amino acid sequence of the respective wild type nucleic acid.

This modification of the mRNA sequence of the present invention is based on the fact that the sequence of any mRNA region to be translated is important for efficient translation of that mRNA. Thus, the composition of the mRNA and the sequence of various nucleotides are important. In particular, sequences having an increased G (guanosine)/C (cytosine) content are more stable than sequences having an increased A (adenosine)/U (uracil) content. According to the invention, the codons of the mRNA are therefore varied compared to the respective wild type mRNA, while retaining the translated amino acid sequence, such that they include an increased amount of G/C nucleotides. In respect to the fact that several codons code for one and the same amino acid (so-called degeneration of the genetic code), the most favorable codons for the stability can be determined (so-called alternative codon usage). Depending on the amino acid to be encoded by the mRNA, there are various possibilities for modification of the mRNA sequence, compared to its wild type sequence. In the case of amino acids, which are encoded by codons, which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U is present. In contrast, codons which contain A and/or U nucleotides can be modified by substitution of other codons, which code for the same amino acids but contain no A and/or U. Examples of these are: the codons for Pro can be modified from CCU or CCA to CCC or CCG; the codons for Arg can be modified from CGU or CGA or AGA or AGG to CGC or CGG; the codons for Ala can be modified from GCU or GCA to GCC or GCG; the codons for Gly can be modified from GGU or GGA to GGC or GGG. In other cases, although A or U nucleotides cannot be eliminated from the codons, it is however possible to decrease the A and U content by using codons which contain a lower content of A and/or U nucleotides. Examples of these are: the codons for Phe can be modified from UUU to UUC; the codons for Leu can be modified from UUA, UUG, CUU or CUA to CUC or CUG; the codons for Ser can be modified from UCU or UCA or AGU to UCC, UCG or AGC; the codon for Tyr can be modified from UAU to UAC; the codon for Cys can be modified from UGU to UGC; the codon for His can be modified from CAU to CAC; the codon for Gln can be modified from CAA to CAG; the codons for Ile can be modified from AUU or AUA to AUC; the codons for Thr can be modified from ACU or ACA to ACC or ACG; the codon for Asn can be modified from AAU to AAC; the codon for Lys can be modified from AAA to AAG; the codons for Val can be modified from GUU or GUA to GUC or GUG; the codon for Asp can be modified from GAU to GAC; the codon for Glu can be modified from GAA to GAG; the stop codon UAA can be modified to UAG or UGA. In the case of the codons for Met (AUG) and Trp (UGG), on the other hand, there is no possibility of sequence modification. The substitutions listed above can be used either individually or in all possible combinations to increase the G/C content of the mRNA sequence of the present invention compared to its particular wild type mRNA (i.e. the original sequence). Thus, for example, all codons for Thr occurring in the wild type sequence can be modified to ACC (or ACG). Preferably, however, for example, combinations of the above substitution possibilities are used:
- substitution of all codons coding for Thr in the original sequence (wild type mRNA) to ACC (or ACG) and
- substitution of all codons originally coding for Ser to UCC (or UCG or AGC);
- substitution of all codons coding for Ile in the original sequence to AUC and
- substitution of all codons originally coding for Lys to AAG and
- substitution of all codons originally coding for Tyr to UAC;
- substitution of all codons coding for Val in the original sequence to GUC (or GUG) and
- substitution of all codons originally coding for Glu to GAG and
- substitution of all codons originally coding for Ala to GCC (or GCG) and
- substitution of all codons originally coding for Arg to CGC (or CGG);
- substitution of all codons coding for Val in the original sequence to GUC (or GUG) and
- substitution of all codons originally coding for Glu to GAG and
- substitution of all codons originally coding for Ala to GCC (or GCG) and
- substitution of all codons originally coding for Gly to GGC (or GGG) and
- substitution of all codons originally coding for Asn to AAC;
- substitution of all codons coding for Val in the original sequence to GUC (or GUG) and
- substitution of all codons originally coding for Phe to UUC and
- substitution of all codons originally coding for Cys to UGC and
- substitution of all codons originally coding for Leu to CUG (or CUC) and
- substitution of all codons originally coding for Gln to CAG and
- substitution of all codons originally coding for Pro to CCC (or CCG); etc.

Preferably, the G/C content of the coding region of the mRNA compound comprising an mRNA sequence of the present invention is increased by at least 7%, more preferably by at least 15%, particularly preferably by at least 20%, compared to the G/C content of the coding region of the wild type RNA. According to a specific embodiment at least 5%, 10%, 20%, 30%, 40%, 50%, 60%, more preferably at least 70%, even more preferably at least 80% and most preferably at least 90%, 95% or even 100% of the substitutable codons in the region coding for a peptide or protein as defined herein or a fragment or variant thereof or the whole sequence of the wild type mRNA sequence are substituted, thereby increasing the G/C content of said sequence. In this context, it is particularly preferable to increase the G/C content of the mRNA sequence of the present invention, preferably of the at least one coding region of the mRNA sequence according to the invention, to the maximum (i.e. 100% of the substitutable codons) as compared to the wild type sequence. According to the invention, a further preferred modification of the mRNA sequence of the present invention is based on the finding that the translation efficiency is also determined by a different frequency in the occurrence of tRNAs in cells. Thus, if so-called "rare codons" are present in the mRNA sequence of the present invention to an increased extent, the corresponding modified mRNA sequence is translated to a significantly poorer degree than in the case where codons coding for relatively "frequent" tRNAs are present. According to the invention, in the modified mRNA sequence of the present invention, the region which codes for a peptide or protein as defined herein or a fragment or variant thereof is modified compared to the corresponding region of the wild type mRNA sequence such that at least one codon of the wild type sequence, which codes for a tRNA which is relatively rare in the cell, is exchanged for a codon, which codes for a tRNA which is relatively frequent in the cell and carries the same amino acid as the relatively rare tRNA. By this modification, the sequence of the mRNA of the present invention is modified such that codons for which frequently occurring tRNAs are available are inserted. In other words, according to the invention, by this modification all codons of the wild type sequence, which code for a tRNA which is relatively rare in the cell, can in each case be exchanged for a codon, which codes for a tRNA which is relatively frequent in the cell and which, in each case, carries the same amino acid as the relatively rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons, which use for the particular amino acid the tRNA which occurs the most frequently, e.g. the Gly codon, which uses the tRNA, which occurs the most frequently in the (human) cell, are particularly preferred. According to the invention, it is particularly preferable to link the sequential G/C content which is increased, in particular maximized, in the modified mRNA sequence of the present invention, with the "frequent" codons without modifying the amino acid sequence of the protein encoded by the coding region of the mRNA sequence. This preferred embodiment allows provision of a particularly efficiently translated and stabilized (modified) mRNA sequence of the present invention. The determination of a modified mRNA sequence of the present invention as described above (increased G/C content; exchange of tRNAs) can be carried out using the computer program explained in WO2002098443 - the disclosure content of which is included in its full scope in the present invention. Using this computer program, the nucleotide sequence of any desired mRNA sequence can be modified with the aid of the genetic code or the degenerative nature thereof such that a maximum G/C content results, in combination with the use of codons which code for tRNAs occurring as frequently as possible in the cell, the amino acid sequence coded by the modified mRNA sequence preferably not being modified compared to the non-modified sequence. Alternatively, it is also possible to modify only the G/C content or only the codon usage compared to the original sequence. The source code in Visual Basic 6.0 (development environment used: Microsoft Visual Studio Enterprise 6.0 with Service Pack 3) is also described in WO2002098443. In a further preferred embodiment of the present invention, the A/U content in the environment of the ribosome binding site of the mRNA sequence of the present invention is increased compared to the A/U content in the environment of the ribosome binding site of its respective wild type mRNA. This modification (an increased A/U content around the ribosome binding site) increases the efficiency of ribosome binding to the mRNA. An effective binding of the ribosomes to the ribosome binding site (Kozak sequence: SEQ ID NO:1 or SEQ ID NO:2, the AUG forms the start codon, or a minimal Kozak binding site ACC) in turn has the effect of an efficient translation of the mRNA. According to a further embodiment of the present invention, the mRNA sequence of the present invention may be modified with respect to potentially destabilizing sequence elements. Particularly, the coding region and/or the 5' and/or 3' untranslated region of this mRNA sequence may be modified compared to the respective wild type mRNA such that it contains no destabilizing sequence elements, the encoded amino acid sequence of the modified mRNA sequence preferably not being modified compared to its respective wild type mRNA. It is known that, for example in sequences of eukaryotic mRNAs, destabilizing sequence elements (DSE) occur, to which signal proteins bind and regulate enzymatic degradation of mRNA in vivo. For further stabilization of the modified mRNA sequence, optionally in the region which encodes at least one peptide or protein as defined herein or a fragment or variant thereof, one or more such modifications compared to the corresponding region of the wild type mRNA can therefore be carried out, so that no or substantially no destabilizing sequence elements are contained there. According to the invention, DSE present in the untranslated regions (3'- and/or 5'-UTR) can also be eliminated from the mRNA sequence of the present invention by such modifications. Such destabilizing sequences are e.g. AU-rich sequences (AURES), which occur in 3'-UTR sections of numerous unstable mRNAs (Caput et al., Proc. Natl. Acad. Sci. USA 1986, 83: 1670-1674). The mRNA sequence of the present invention is therefore preferably modified compared to the respective wild type mRNA such that the mRNA sequence of the present invention contains no such destabilizing sequences. This also applies to those sequence motifs which are recognized by possible endonucleases, e.g. the sequence GAACAAG, which is contained in the 3'-UTR segment of the gene encoding the transferrin receptor (Binder et al., EMBO J. 1994, 13: 1969-1980). These sequence motifs are also preferably removed in the mRNA sequence of the present invention.

Further preferably, the G/C content of the coding region of the mRNA compound comprising an mRNA sequence of the present invention is increased by at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, or 40%, compared to the G/C content of the coding region of the wild type RNA.

According to a further embodiment, the mRNA compound comprises an mRNA sequence comprising a coding region that comprises or consists of any one of the RNA sequences as disclosed in Tabs. 1-5, Figs. 20-24 or in the sequence listing of WO2018078053; Tabs. 1-5 or Figs. 20-24 of WO2018078053; WO2018078053 incorporated by reference in its entirety.

### Sequences Adapted to Human Codon Usage

A further preferred modification of the mRNA compound is based on the finding that codons encoding the same amino acid typically occur at different frequencies. According to this embodiment, the frequency of the codons encoding the same amino acid in the coding region of the mRNA compound differs from the naturally occurring frequency of that codon according to the human codon usage as e.g. shown in **Table 2** (Human codon usage table). For example, in the case of the amino acid alanine (Ala), the wild type coding region is preferably adapted in a way that the codon "GCC" is used with a frequency of 0.40, the codon "GCT" is used with a frequency of 0.28, the codon "GCA" is used with a frequency of 0.22 and the codon "GCG" is used with a frequency of 0.10 etc. (see **Table 2).**

**Table 2: Human codon usage table, most frequent codons are marked with asterisks**

| **Amino acid** | **codon** | **fraction** | **/1000** | | **Amino acid** | **codon** | **fraction** | **/1000** |
|---|---|---|---|---|---|---|---|---|
| Ala | GCG | 0.10 | 7.4 | | Pro | CCG | 0.11 | 6.9 |
| Ala | GCA | 0.22 | 15.8 | | Pro | CCA | 0.27 | 16.9 |
| Ala | GCT | 0.28 | 18.5 | | Pro | CCT | 0.29 | 17.5 |
| Ala | GCC* | 0.40 | 27.7 | | Pro | CCC* | 0.33 | 19.8 |
| Cys | TGT | 0.42 | 10.6 | | Gln | CAG* | 0.73 | 34.2 |
| Cys | TGC* | 0.58 | 12.6 | | Gln | CAA | 0.27 | 12.3 |
| Asp | GAT | 0.44 | 21.8 | | Arg | AGG | 0.22 | 12.0 |
| Asp | GAC* | 0.56 | 25.1 | | Arg | AGA* | 0.21 | 12.1 |
| Glu | GAG* | 0.59 | 39.6 | | Arg | CGG | 0.19 | 11.4 |
| Glu | GAA | 0.41 | 29.0 | | Arg | CGA | 0.10 | 6.2 |
| Phe | TTT | 0.43 | 17.6 | | Arg | CGT | 0.09 | 4.5 |
| Phe | TTC* | 0.57 | 20.3 | | Arg | CGC | 0.19 | 10.4 |
| Gly | GGG | 0.23 | 16.5 | | Ser | AGT | 0.14 | 12.1 |
| Gly | GGA | 0.26 | 16.5 | | Ser | AGC* | 0.25 | 19.5 |
| Gly | GGT | 0.18 | 10.8 | | Ser | TCG | 0.06 | 4.4 |
| Gly | GGC* | 0.33 | 22.2 | | Ser | TCA | 0.15 | 12.2 |
| His | CAT | 0.41 | 10.9 | | Ser | TCT | 0.18 | 15.2 |
| His | CAC* | 0.59 | 15.1 | | Ser | TCC | 0.23 | 17.7 |
| Ile | ATA | 0.14 | 7.5 | | Thr | ACG | 0.12 | 6.1 |
| Ile | ATT | 0.35 | 16.0 | | Thr | ACA | 0.27 | 15.1 |
| Ile | ATC* | 0.52 | 20.8 | | Thr | ACT | 0.23 | 13.1 |
| Lys | AAG* | 0.60 | 31.9 | | Thr | ACC* | 0.38 | 18.9 |
| Lys | AAA | 0.40 | 24.4 | | Val | GTG* | 0.48 | 28.1 |
| Leu | TTG | 0.12 | 12.9 | | Val | GTA | 0.10 | 7.1 |
| Leu | TTA | 0.06 | 7.7 | | Val | GTT | 0.17 | 11.0 |
| Leu | CTG* | 0.43 | 39.6 | | Val | GTC | 0.25 | 14.5 |
| Leu | CTA | 0.07 | 7.2 | | Trp | TGG* | 1 | 13.2 |
| Leu | CTT | 0.12 | 13.2 | | Tyr | TAT | 0.42 | 12.2 |
| Leu | CTC | 0.20 | 19.6 | | Tyr | TAC* | 0.58 | 15.3 |
| Met | ATG* | 1 | 22.0 | | Stop | TGA* | 0.61 | 1.6 |
| Asn | AAT | 0.44 | 17.0 | | Stop | TAG | 0.17 | 0.8 |
| Asn | AAC* | 0.56 | 19.1 | | Stop | TAA | 0.22 | 1.0 |

| | | | | | | | | |
|---|---|---|---|---|---|---|---|---|
| *most frequent codon | | | | | | | | |

### Codon-optimized Sequences

In one embodiment, all codons of the wild type sequence which code for a tRNA, which is relatively rare in the cell, are exchanged for a codon which codes for a tRNA, which is relatively frequent in the cell and which, in each case, carries the same amino acid as the relatively rare tRNA. Therefore it is particularly preferred that the most frequent codons are used for each encoded amino acid (see **Table 2).** Such an optimization procedure increases the codon adaptation index (CAI) and ultimately maximizes the CAI. In the context of the invention, sequences with increased or maximized CAI are typically referred to as "codon-optimized" sequences and/or CAI increased and/or maximized sequences. According to a preferred embodiment, the mRNA compound comprising an mRNA sequence of the present invention comprises at least one coding region, wherein the coding region/sequence is codon-optimized as described herein. More preferably, the codon adaptation index (CAI) of the at least one coding sequence is at least 0.5, at least 0.8, at least 0.9 or at least 0.95. Most preferably, the codon adaptation index (CAI) of the at least one coding sequence is 1.

For example, in the case of the amino acid alanine (Ala) present in the amino acid sequence encoded by the at least one coding sequence of the RNA according to the invention, the wild type coding sequence is adapted in a way that the most frequent human codon "GCC" is always used for said amino acid, or for the amino acid Cysteine (Cys), the wild type sequence is adapted in a way that the most frequent human codon "TGC" is always used for said amino acid etc.

### C-optimized Sequences

According to another embodiment, the mRNA compound comprising an mRNA sequence having a modified - in particular increased - cytosine (C) content, preferably of the coding region of the mRNA sequence, compared to the C content of the coding region of the respective wild type mRNA, i.e. the unmodified mRNA. At the same time, the amino acid sequence encoded by the at least one coding region of the mRNA sequence of the present invention is preferably not modified as compared to the amino acid sequence encoded by the respective wild type mRNA.

In a preferred embodiment of the present invention, the modified mRNA sequence is modified such that at least 10%, 20%, 30%, 40%, 50%, 60%, 70% or 80%, or at least 90% of the theoretically possible maximum cytosine-content or even a maximum cytosine-content is achieved.

In further preferred embodiments, at least 10%, 20%, 30%, 40%, 50%, 60%, 70%, 80%, 90% or even 100% of the codons of the target mRNA wild type sequence, which are "cytosine content optimizable" are replaced by codons having a higher cytosine-content than the ones present in the wild type sequence.

In a further preferred embodiment, some of the codons of the wild type coding sequence may additionally be modified such that a codon for a relatively rare tRNA in the cell is exchanged by a codon for a relatively frequent tRNA in the cell, provided that the substituted codon for a relatively frequent tRNA carries the same amino acid as the relatively rare tRNA of the original wild type codon. Preferably, all of the codons for a relatively rare tRNA are replaced by a codon for a relatively frequent tRNA in the cell, except codons encoding amino acids, which are exclusively encoded by codons not containing any cytosine, or except for glutamine (Gln), which is encoded by two codons each containing the same number of cytosines.

In a further preferred embodiment of the present invention, the modified target mRNA is modified such that at least 80%, or at least 90% of the theoretically possible maximum cytosine-content or even a maximum cytosine-content is achieved by means of codons, which code for relatively frequent tRNAs in the cell, wherein the amino acid sequence remains unchanged.

Due to the naturally occurring degeneracy of the genetic code, more than one codon may encode a particular amino acid. Accordingly, 18 out of 20 naturally occurring amino acids are encoded by more than one codon (with Tryp and Met being an exception), e.g. by 2 codons (e.g. Cys, Asp, Glu), by three codons (e.g. Ile), by 4 codons (e.g. Al, Gly, Pro) or by 6 codons (e.g. Leu, Arg, Ser). However, not all codons encoding the same amino acid are utilized with the same frequency under in vivo conditions. Depending on each single organism, a typical codon usage profile is established.

The term "cytosine content-optimizable codon" as used within the context of the present invention refers to codons, which exhibit a lower content of cytosines than other codons encoding the same amino acid. Accordingly, any wild type codon, which may be replaced by another codon encoding the same amino acid and exhibiting a higher number of cytosines within that codon, is considered to be cytosine-optimizable (C-optimizable). Any such substitution of a C-optimizable wild type codon by the specific C-optimized codon within a wild type coding region increases its overall C-content and reflects a C-enriched modified mRNA sequence. According to a preferred embodiment, the mRNA sequence of the present invention, preferably the at least one coding region of the mRNA sequence of the present invention comprises or consists of a C-maximized mRNA sequence containing C-optimized codons for all potentially C-optimizable codons. Accordingly, 100% or all of the theoretically replaceable C-optimizable codons are preferably replaced by C-optimized codons over the entire length of the coding region.

In this context, cytosine-content optimizable codons are codons, which contain a lower number of cytosines than other codons coding for the same amino acid.

Any of the codons GCG, GCA, GCU codes for the amino acid Ala, which may be exchanged by the codon GCC encoding the same amino acid, and/or
the codon UGU that codes for Cys may be exchanged by the codon UGC encoding the same amino acid, and/or
the codon GAU which codes for Asp may be exchanged by the codon GAC encoding the same amino acid, and/or
the codon that UUU that codes for Phe may be exchanged for the codon UUC encoding the same amino acid, and/or
any of the codons GGG, GGA, GGU that code Gly may be exchanged by the codon GGC encoding the same amino acid, and/or
the codon CAU that codes for His may be exchanged by the codon CAC encoding the same amino acid, and/or any of the codons AUA, AUU that code for Ile may be exchanged by the codon AUC, and/or
any of the codons UUG, UUA, CUG, CUA, CUU coding for Leu may be exchanged by the codon CUC encoding the same amino acid, and/or
the codon AAU that codes for Asn may be exchanged by the codon AAC encoding the same amino acid, and/or
any of the codons CCG, CCA, CCU coding for Pro may be exchanged by the codon CCC encoding the same amino acid, and/or
any of the codons AGG, AGA, CGG, CGA, CGU coding for Arg may be exchanged by the codon CGC encoding the same amino acid, and/or
any of the codons AGU, AGC, UCG, UCA, UCU coding for Ser may be exchanged by the codon UCC encoding the same amino acid, and/or
any of the codons ACG, ACA, ACU coding for Thr may be exchanged by the codon ACC encoding the same amino acid, and/or
any of the codons GUG, GUA, GUU coding for Val may be exchanged by the codon GUC encoding the same amino acid, and/or
the codon UAU coding for Tyr may be exchanged by the codon UAC encoding the same amino acid.

In any of the above instances, the number of cytosines is increased by 1 per exchanged codon. Exchange of all non C-optimized codons (corresponding to C-optimizable codons) of the coding region results in a C-maximized coding sequence. In the context of the invention, at least 70%, preferably at least 80%, more preferably at least 90%, of the non C-optimized codons within the at least one coding region of the mRNA sequence according to the invention are replaced by C-optimized codons.

It may be preferred that for some amino acids the percentage of C-optimizable codons replaced by C-optimized codons is less than 70%, while for other amino acids the percentage of replaced codons is higher than 70% to meet the overall percentage of C-optimization of at least 70% of all C-optimizable wild type codons of the coding region.

Preferably, in a C-optimized mRNA sequence, at least 50% of the C-optimizable wild type codons for any given amino acid are replaced by C-optimized codons, e.g. any modified C-enriched mRNA sequence preferably contains at least 50% C-optimized codons at C-optimizable wild type codon positions encoding any one of the above mentioned amino acids Ala, Cys, Asp, Phe, Gly, His, Ile, Leu, Asn, Pro, Arg, Ser, Thr, Val and Tyr, preferably at least 60%.

In this context, codons encoding amino acids which are not cytosine content-optimizable and which are, however, encoded by at least two codons, may be used without any further selection process. However, the codon of the wild type sequence that codes for a relatively rare tRNA in the cell, e.g. a human cell, may be exchanged for a codon that codes for a relatively frequent tRNA in the cell, wherein both code for the same amino acid. Accordingly, the relatively rare codon GAA coding for Glu may be exchanged by the relative frequent codon GAG coding for the same amino acid, and/or
the relatively rare codon AAA coding for Lys may be exchanged by the relative frequent codon AAG coding for the same amino acid, and/or
the relatively rare codon CAA coding for Gln may be exchanged for the relative frequent codon CAG encoding the same amino acid.

In this context, the amino acids Met (AUG) and Trp (UGG), which are encoded by only one codon each, remain unchanged. Stop codons are not cytosine-content optimized, however, the relatively rare stop codons amber, ochre (UAA, UAG) may be exchanged by the relatively frequent stop codon opal (UGA).

The single substitutions listed above may be used individually as well as in all possible combinations in order to optimize the cytosine-content of the modified mRNA sequence compared to the wild type mRNA sequence.

Accordingly, the at least one coding sequence as defined herein may be changed compared to the coding region of the respective wild type mRNA in such a way that an amino acid encoded by at least two or more codons, of which one comprises one additional cytosine, such a codon may be exchanged by the C-optimized codon comprising one additional cytosine, wherein the amino acid is preferably unaltered compared to the wild type sequence.

According to a further preferred embodiment, the composition of the invention comprises an mRNA compound whose coding region has an increased G/C content compared to the G/C content of the corresponding coding region of the corresponding wild type mRNA, and/or an increased C content compared to the C content of the corresponding coding region of the corresponding wild type mRNA, and/or wherein the codons in the coding region are adapted to human codon usage, wherein the codon adaptation index (CAI) is preferably increased or maximized, and wherein the amino acid sequence encoded by the mRNA sequence is preferably not being modified compared to the amino acid sequence encoded by the corresponding wild type mRNA.

In one preferred embodiment of the invention, the composition comprises an mRNA compound comprising a coding region encoding a peptide or a protein, wherein the coding region exhibits a sequence modification selected from a G/C content modification, a codon modification, a codon optimization or a C-optimization of the sequence.

In another preferred embodiment, the composition or lipid nanoparticle as defined herein comprises an mRNA comprising a coding region encoding a peptide or protein as defined herein, wherein, compared with the coding region of the corresponding wild-type mRNA,
- the G/C content of the coding region is increased;
- the C content of the coding region is increased;
- the codon usage in the coding region is adapted to the human codon usage; and/or the codon adaptation index (CAI) is increased or maximized in the coding region.

### 5'-CAP Structure

According to another preferred embodiment of the invention, the mRNA compound may have a sequence modified by the addition of a so-called "5'-CAP structure", which preferably stabilizes the mRNA as described herein. A 5'-CAP is an entity, typically a modified nucleotide entity, which generally "caps" the 5'-end of a mature mRNA. A 5'-CAP may typically be formed by a modified nucleotide, particularly by a derivative of a guanine nucleotide. Preferably, the 5'-CAP is linked to the 5'-terminus via a 5'-5'-triphosphate linkage. A 5'-CAP may be methylated, e.g. m7GpppN, wherein N is the terminal 5' nucleotide of the nucleic acid carrying the 5'-CAP, typically the 5'-end of an mRNA. m7GpppN is the 5'-CAP structure, which naturally occurs in mRNA transcribed by polymerase II and is therefore preferably not considered as modification comprised in a modified mRNA in this context. Accordingly, a modified mRNA sequence of the present invention may comprise a m7GpppN as 5'-cap, but additionally the modified mRNA sequence typically comprises at least one further modification as defined herein. In one preferred embodiment, the mRNA compound of the invention comprises a 5'-CAP structure wherein said 5'-CAP structure is m7GpppN. In a most preferred embodiment, the 5'-cap structure is selected from the groups consisting of m7G(5'), m7G(5')ppp(5')(2'OMeA) and m7G(5')ppp(5')(2'OMeG) or respectively m7G(5')ppp(5')(2'OMeA)pG and m7G(5')ppp(5')(2'OMeG)pG.

In one embodiment, the 5'-end of an mRNA is "GGGAGA", preferably for an mRNA in which an mCap analog is used. In another embodiment, the 5'-end of an mRNA is "AGGAGA", preferably for an mRNA in which a CleanCap^{®} AG CAP analog is used. In a further embodiment, the 5'-end of an mRNA is "GGGAGA", preferably for an mRNA in which a CleanCap^{®} GG CAP analog is used.

In the context of the present invention, a 5'-CAP structure may also be formed in chemical RNA synthesis or RNA in vitro transcription (co-transcriptional capping) using CAP analogues, or a CAP structure may be formed in vitro using capping enzymes. Kits comprising capping enzymes are commercially available (e.g. ScriptCapTM Capping Enzyme and ScriptCap^{™} 2'-O-Methyltransferase (both from CellScript)). Therefore, the RNA transcript is preferably treated according to the manufacturer's instructions.

Thusly, a CAP analogue refers to a non-polymerizable di-nucleotide that has CAP functionality in that it facilitates translation or localization, and/or prevents degradation of the RNA molecule when incorporated at the 5'-end of the RNA molecule. Non-polymerizable means that the CAP analogue will be incorporated only at the 5'-terminus because it does not have a 5' triphosphate and therefore cannot be extended in the 3'-direction by a template-dependent RNA polymerase.

CAP analogues include, but are not limited to, a chemical structure selected from the group consisting of m7GpppG, m7GpppA, m7GpppC; unmethylated CAP analogues (e.g., GpppG); dimethylated CAP analogue (e.g., m2,7GpppG), trimethylated CAP analogue (e.g., m2,2,7GpppG), dimethylated symmetrical CAP analogues (e.g., m7Gpppm7G), or anti reverse CAP analogues (e.g., ARCA; m7,2'OmeGpppG, m7,2'dGpppG, m7,3'OmeGpppG, m7,3'dGpppG and their tetraphosphate derivatives) (Stepinski et al., 2001. RNA 7(10):1486-95).

Further examples of 5'-CAP structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-CAP structures are regarded as at least one modification in this context and may be used in the context of the present invention to modify the mRNA sequence of the inventive composition.

Particularly preferred modified 5'-CAP structures are CAP1 (methylation of the ribose of the adjacent nucleotide of m7G), CAP2 (additional methylation of the ribose of the 2nd nucleotide downstream of the m7G), CAP3 (additional methylation of the ribose of the 3rd nucleotide downstream of the m7G), CAP4 (methylation of the ribose of the 4th nucleotide downstream of the m7G), ARCA (anti-reverse CAP analogue, modified ARCA (e.g. phosphothioate modified ARCA), CleanCap or respectively m7G(5')ppp(5')(2'OMeA)pG or m7G(5')ppp(5')(2'OMeG)pG (TriLink) and or a CAP-structure as disclosed in WO2017053297 (herewith incorporated by reference), inosine, N1-methyl-guanosine, 2'-fluoro-guanosine, 7-deaza-guanosine, 8-oxo-guanosine, 2-amino-guanosine, LNA-guanosine, and 2-azido-guanosine. In particular, any CAP structures derivable from the structure disclosed in claim 1-5 of WO2017053297 may be suitably used to co-transcriptionally generate a modified CAP1 structure. Further, any CAP structures derivable from the structure defined in claim 1 or claim 21 of WO2018075827 may be suitably used to co-transcriptionally generate a modified CAP1 structure.

Furthermore, CAP analogues have been described previously (US7074596, WO2008016473, WO2008157688, WO2009149253, WO2011015347, and WO2013059475). The synthesis of N7-(4-chlorophenoxyethyl) substituted dinucleotide CAP analogues has been described recently (Kore et al. (2013) Bioorg. Med. Chem. 21(15): 4570-4). Further suitable CAP analogues in that context are described in WO2017066793, WO2017066781, WO2017066791, WO2017066789, WO2017066782, WO2018075827 and WO2017066797 wherein the specific disclosures referring to CAP analogues are incorporated herein by reference.

### Poly(A) sequence / polyA-tail

A polyA-tail also called "3'-poly(A) tail", "polyA sequence" or "poly(A) sequence" is typically a long sequence of adenosine nucleotides of up to about 400 adenosine nucleotides, e.g. from 10 to 200, 10 to 100, 40 to 80, 50 to 70, about 25 to about 400, preferably from about 50 to about 400, more preferably from about 50 to about 300, even more preferably from about 50 to about 250, most preferably from about 60 to about 250 adenosine nucleotides, or about 40 to about 150 adenosine nucleotides, added to the 3'-end of a RNA. In a particularly preferred embodiment, the poly(A) sequence comprises about 64 adenosine nucleotides. In another particularly preferred embodiment, the poly(A) sequence comprises about 100 adenosine nucleotides. Moreover, poly(A) sequences, or poly(A) tails may be generated in vitro by enzymatic polyadenylation of the RNA, e.g. using Poly(A)polymerases derived from E.coli or yeast. Suitably, the poly(A) sequence of the coding RNA may be long enough to bind at least 2, 3, 4, 5 or more monomers of PolyA Binding Proteins.

Polyadenylation is typically understood to be the addition of a poly(A) sequence to a nucleic acid molecule, such as an RNA molecule, e.g. to a premature mRNA. Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Thusly, according to a further preferred embodiment, the composition comprises an mRNA compound comprising an mRNA sequence containing a polyA tail on the 3'-terminus of typically about 10 to 200 adenosine nucleotides, preferably about 10 to 100 adenosine nucleotides, more preferably about 40 to 80 adenosine nucleotides or even more preferably about 50 to 70 adenosine nucleotides. Preferably, the poly(A) sequence is derived from a DNA template by RNA in vitro transcription. Alternatively, the poly(A) sequence may also be obtained in vitro by common methods of chemical-synthesis without being necessarily transcribed from a DNA-progenitor. Moreover, poly(A) sequences, or poly(A) tails may be generated by enzymatic polyadenylation of the RNA according to the present invention using commercially available polyadenylation kits and corresponding protocols known in the art.

Alternatively, the mRNA as described herein optionally comprises a polyadenylation signal, which is defined herein as a signal, which conveys polyadenylation to a (transcribed) RNA by specific protein factors (e.g. cleavage and polyadenylation specificity factor (CPSF), cleavage stimulation factor (CstF), cleavage factors I and II (CF I and CF II), poly(A) polymerase (PAP)). In this context, a consensus polyadenylation signal is preferred comprising the NN(U/T)ANA consensus sequence. In a particularly preferred aspect, the polyadenylation signal comprises one of the following sequences: AA(U/T)AAA or A(U/T)(U/T)AAA (wherein uridine is usually present in RNA and thymidine is usually present in DNA).

### Poly(C) Sequence

A poly-(C)-sequence is typically a long sequence of cytosine nucleotides, typically about 10 to about 200 cytosine nucleotides, preferably about 10 to about 100 cytosine nucleotides, more preferably about 10 to about 70 cytosine nucleotides or even more preferably about 20 to about 50 or even about 20 to about 30 cytosine nucleotides. A poly(C) sequence may preferably be located 3' of the coding region comprised by a nucleic acid.

Thusly, according to a further preferred embodiment, the composition of the invention comprises an mRNA compound comprising a poly(C) tail on the 3'-terminus of typically about 10 to 200 cytosine nucleotides, preferably about 10 to 100 cytosine nucleotides, more preferably about 20 to 70 cytosine nucleotides or even more preferably about 20 to 60 or even 10 to 40 cytosine nucleotides.

In one preferred embodiment, the mRNA compound comprises, preferably in 5'- to 3'-direction:
a) a 5'-CAP structure, preferably m7GpppN, more preferably CAP1 or m7G(5')ppp(5')(2'OMeA)pG;
b) optionally, a 5'-UTR element,
c) at least one coding region encoding at least one antigenic peptide or protein,
d) optionally, a poly(A) sequence, preferably comprising 64 or 100 adenosines;
e) optionally, a poly(C) sequence, preferably comprising 30 cytosines.

### UTRs

In a preferred embodiment, the composition comprises an mRNA compound comprising at least one 5'- or 3'-UTR element. In this context, an UTR element comprises or consists of a nucleic acid sequence, which is derived from the 5'- or 3'-UTR of any naturally occurring gene or which is derived from a fragment, a homolog or a variant of the 5'- or 3'-UTR of a gene. Preferably, the 5'- or 3'-UTR element used according to the present invention is heterologous to the at least one coding region of the mRNA sequence of the invention. Even if 5'- or 3'-UTR elements derived from naturally occurring genes are preferred, also synthetically engineered UTR elements may be used in the context of the present invention.

The term "3'-UTR element" typically refers to a nucleic acid sequence, which comprises or consists of a nucleic acid sequence that is derived from a 3'-UTR or from a variant of a 3'-UTR. A 3'-UTR element in the sense of the present invention may represent the 3'-UTR of an RNA, preferably an mRNA. Thus, in the sense of the present invention, preferably, a 3'-UTR element may be the 3'-UTR of an RNA, preferably of an mRNA, or it may be the transcription template for a 3'-UTR of an RNA. Thus, a 3'-UTR element preferably is a nucleic acid sequence which corresponds to the 3'-UTR of an RNA, preferably to the 3'-UTR of an mRNA, such as an mRNA obtained by transcription of a genetically engineered vector construct. Preferably, the 3'-UTR element fulfils the function of a 3'-UTR or encodes a sequence which fulfils the function of a 3'-UTR.

Preferably, the at least one 3'-UTR element comprises or consists of a nucleic acid sequence derived from the 3'-UTR of a chordate gene, preferably a vertebrate gene, more preferably a mammalian gene, most preferably a human gene, or from a variant of the 3'-UTR of a chordate gene, preferably a vertebrate gene, more preferably a mammalian gene, most preferably a human gene.

Preferably, the composition comprises an mRNA compound that comprises a 3'-UTR element, which may be derivable from a gene that relates to an mRNA with an enhanced half-life (that provides a stable mRNA), for example a 3'-UTR element as defined and described below. Preferably, the 3'-UTR element comprises or consists of a nucleic acid sequence derived from a 3'-UTR of a gene, which preferably encodes a stable mRNA, or from a homolog, a fragment or a variant of said gene.

In one preferred embodiment, the UTR-combinations which are disclosed in Table 1, claims 1 and claim 4, claims 6-8 and claim 9 of WO2019077001 are preferred UTR-combinations for mRNA compounds of the present invention. Further, preferably, the UTR-combinations as disclosed on page 24, second full paragraph after Table 1 and page 24, last paragraph to page 29, second paragraph of WO2019077001 are preferred UTR-combinations for mRNA compounds of the present invention. WO2019077001 is incorporated herein by reference in its entirety.

In a further preferred embodiment, that 3'-UTR element comprises or consists of a nucleic acid sequence which is derived from a 3'-UTR of a gene selected from the group consisting of a 3'-UTR of a gene selected from PSMB3 (SEQ ID NO:19, SEQ ID NO:20), ALB/albumin (SEQ ID NO:13-SEQ ID NO:18), alpha-globin (referred to as "muag" i.e. a mutated alpha-globin 3'-UTR; SEQ ID NO:11, SEQ ID NO:12), CASP1 (preferably SEQ ID NO:81 (DNA) or SEQ ID NO:82 (RNA)), COX6B1 (preferably SEQ ID NO:83 (DNA) or SEQ ID NO:84 (RNA)), GNAS (preferably SEQ ID NO:85 (DNA) or SEQ ID NO:86 (RNA)), NDUFA1 (preferably SEQ ID NO:87 (DNA) or SEQ ID NO:88 (RNA)) and RPS9 (preferably SEQ ID NO:79 (DNA) or SEQ ID NO:80 (RNA)), or from a homolog, a fragment or a variant of any one of these genes (for example, human albumin/alb 3'-UTR as disclosed in SEQ ID NO:1369 of WO2013143700, which is incorporated herein by reference), or from a homolog, a fragment or a variant thereof. In a further preferred embodiment, the 3'-UTR element comprises the nucleic acid sequence derived from a fragment of the human albumin gene according to SEQ ID NO:1376 of WO2013143700

(albumin/alb 3'-UTR). In a further preferred embodiment, the 3'-UTR element comprises or consists of a nucleic acid sequence which is derived from a 3'-UTR of an albumin gene, preferably a vertebrate albumin gene, more preferably a mammalian albumin gene, most preferably a human albumin gene such as from the 3'-UTR of the human albumin gene according to GenBank Accession number NM_000477.5, or a fragment or variant thereof. In another preferred embodiment, the 3'-UTR element comprises or consists of the center, α-complex-binding portion of the 3'-UTR of an α-globin gene, such as of a human α-globin gene, or a homolog, a fragment, or a variant of an α-globin gene, preferably (according to SEQ ID NO:5 or SEQ ID NO:6 (both HBA1) or SEQ ID NO:7 or SEQ ID NO:8 (both HBA2)), or an α-complex-binding portion of the 3'-UTR of an α-globin gene (also named herein as "muag", herein SEQ ID NO:11, SEQ ID NO:12; corresponding to SEQ ID NO:1393 of patent application WO2013143700).

In another preferred embodiment, the 3'-UTR element comprises or consists of a nucleic acid sequence which is derived from a 3'-UTR of an α- or β-globin gene, preferably a vertebrate α- or β -globin gene, and preferably a mammalian α- or β -globin gene, preferably a human α- or β globin gene according to SEQ ID NO:5, 7, 9, 11, 13, 15, 17, 19 or the corresponding RNA sequences SEQ ID NO:6, 8, 10, 12, 14, 16, 18, 20.

In this context it is also preferred that the 3'-UTR element of the mRNA sequence according to the invention comprises or consists of a corresponding RNA sequence of the nucleic acid sequence according to SEQ ID NO:11 as shown in SEQ ID NO:12, or a homolog, a fragment or variant thereof.

UTR-combination Slc7a3/PSMB3: In another preferred embodiment, the mRNA compound comprises a 5'-UTR element, which comprises or consists of a nucleic acid sequence which is derived from a cationic amino acid transporter 3 (solute carrier family 7 member 3, SLC7A3) gene, wherein said 5'-UTR element comprises or consists of a DNA sequence according to SEQ ID NO:15 as disclosed in WO2019077001 or respectively a RNA sequence according to SEQ ID NO:16 as disclosed in WO2019077001. In another preferred embodiment, the mRNA compound comprises a 3'-UTR element, which comprises or consists of a nucleic acid sequence which is derived from a proteasome subunit beta type-3 (PSMB3) gene, wherein said 3'-UTR element comprises or consists of a DNA sequence according to SEQ ID NO:23 as disclosed in WO2019077001 or respectively a RNA sequence according to SEQ ID NO:24 as disclosed in WO2019077001. In further preferred embodiments, the mRNA compound comprises an UTR-combination as disclosed in WO2019077001, i.e. both a 5'-UTR element, which comprises or consists of a nucleic acid sequence which is derived from a Slc7a3 gene and a 3'-UTR element, which comprises or consists of a nucleic acid sequence which is derived from a PSMB3 gene.

UTR-combination HSD17B4/PSMB3: In another preferred embodiment, the mRNA compound comprises a 5'-UTR element, which comprises or consists of a nucleic acid sequence which is derived from a 17-beta-hydroxysteroid dehydrogenase 4 gene, wherein said 5'-UTR element comprises or consists of a DNA sequence according to SEQ ID NO:1 as disclosed in WO2019077001 or respectively a RNA sequence according to SEQ ID NO:2 as disclosed in WO2019077001. In another preferred embodiment, the mRNA compound comprises a 3'-UTR element, which comprises or consists of a nucleic acid sequence which is derived from a proteasome subunit beta type-3 (PSMB3) gene, wherein said 3'-UTR element comprises or consists of a DNA sequence according to SEQ ID NO:23 as disclosed in WO2019077001 or respectively a RNA sequence according to SEQ ID NO:24 as disclosed in WO2019077001. In further preferred embodiments, the mRNA compound comprises an UTR-combination as disclosed in WO2019077001, i.e. both a 5'-UTR element, which comprises or consists of a nucleic acid sequence which is derived from a HSD17B4 gene and a 3'-UTR element, which comprises or consists of a nucleic acid sequence which is derived from a PSMB3 gene.

UTR-combination Rpl31/RPS9: In another preferred embodiment, the mRNA compound comprises a 5'-UTR element, which comprises or consists of a nucleic acid sequence which is derived from a 60S ribosomal protein L31 (RPL31) gene, wherein said 5'-UTR element comprises or consists of a DNA sequence according to SEQ ID NO:13 as disclosed in WO2019077001 or respectively a RNA sequence according to SEQ ID NO:14 as disclosed in WO2019077001. In another preferred embodiment, the mRNA compound comprises a 3'-UTR element, which comprises or consists of a nucleic acid sequence which is derived from a 40S ribosomal protein S9 (RPS9) gene, wherein said 3'-UTR element comprises or consists of a DNA sequence according to SEQ ID NO:33 as disclosed in WO2019077001 or respectively a RNA sequence according to SEQ ID NO:34 as disclosed in WO2019077001. In further preferred embodiments, the mRNA compound comprises an UTR-combination as disclosed in WO2019077001, i.e. both a 5'-UTR element, which comprises or consists of a nucleic acid sequence which is derived from a RPL31 gene and a 3'-UTR element, which comprises or consists of a nucleic acid sequence which is derived from a RPS9 gene.

In a very preferred embodiment, the 5'-UTR element of the mRNA sequence according to the invention comprises or consists of a corresponding RNA sequence of the nucleic acid sequence according to SEQ ID NO:21 or SEQ ID NO:22), i.e. HSD17B4. Also, in a very preferred embodiment, the 3'-UTR element of the mRNA sequence according to the invention comprises or consists of a corresponding RNA sequence of the nucleic acid sequence according to SEQ ID NO:19 or SEQ ID NO:20), i.e. PSMB3. In also a very preferred embodiment, the 5'-UTR element of the mRNA sequence and the 3'-UTR-element according to the invention comprises or consists of a combination of aforementioned HSD17B4 and PSMB3-UTRs.

The term "a nucleic acid sequence which is derived from the 3'-UTR of a [...] gene" preferably refers to a nucleic acid sequence which is based on the 3'-UTR sequence of a [...] gene or on a part thereof, such as on the 3'-UTR of an albumin gene, an α-globin gene, a β-globin gene, a tyrosine hydroxylase gene, a lipoxygenase gene, or a collagen alpha gene, such as a collagen alpha 1(I) gene, preferably of an albumin gene or on a part thereof. This term includes sequences corresponding to the entire 3'-UTR sequence, i.e. the full length 3'-UTR sequence of a gene, and sequences corresponding to a fragment of the 3'-UTR sequence of a gene, such as an albumin gene, α-globin gene, β-globin gene, tyrosine hydroxylase gene, lipoxygenase gene, or collagen alpha gene, such as a collagen alpha 1(I) gene, preferably of an albumin gene.

The term "a nucleic acid sequence which is derived from a variant of the 3'-UTR of a [...] gene" preferably refers to a nucleic acid sequence, which is based on a variant of the 3'-UTR sequence of a gene, such as on a variant of the 3'-UTR of an albumin gene, an α-globin gene, a β-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'-UTR of a gene, i.e. the full length variant 3'-UTR sequence of a gene, and sequences corresponding to a fragment of the variant 3'-UTR sequence of a gene. A fragment in this context preferably consists of a continuous stretch of nucleotides corresponding to a continuous stretch of nucleotides in the full-length variant 3'-UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 3'-UTR. Such a fragment of a variant, in the sense of the present invention, is preferably a functional fragment of a variant as described herein.

According to a preferred embodiment, the mRNA compound comprising an mRNA sequence according to the invention comprises a 5'-CAP structure and/or at least one 3'-untranslated region element (3'-UTR element), preferably as defined herein. More preferably, the RNA further comprises a 5'-UTR element as defined herein.

In one preferred embodiment, the mRNA compound comprises, preferably in 5'- to 3'-direction:
a) a 5'-CAP structure, preferably m7GpppN, more preferably CAP1 or m7G(5')ppp(5')(2'OMeA)pG;
b) optionally, a 5'-UTR element,
c) at least one coding region encoding at least one antigenic peptide or protein,
d) optionally, a 3'-UTR element, preferably comprising or consisting of a nucleic acid sequence which is derived from an alpha globin gene, preferably comprising the corresponding RNA sequence of the nucleic acid sequence according to SEQ ID NO:11 as shown in SEQ ID NO:12, a homolog, a fragment or a variant thereof;
e) optionally a histone stem-loop;
f) optionally, a poly(A) sequence, preferably comprising 64 or 100 adenosines;
g) optionally, a poly(C) sequence, preferably comprising 30 cytosines.

In another preferred embodiment, the mRNA compound comprises, preferably in 5'- to 3'-direction:
a) a 5'-CAP structure, preferably m7GpppN, more preferably CAP1 or m7G(5')ppp(5')(2'OMeA)pG;
b) a HSD17B4-derived 5'-UTR element as described herein above or below;
c) at least one coding region encoding at least one antigenic peptide or protein;
d) a PSMB3-derived 3'-UTR element as described herein above or below;
e) optionally a histone stem-loop;
f) optionally, a poly(A) sequence, preferably comprising 64 or 100 adenosines;
g) optionally, a poly(C) sequence, preferably comprising 30 cytosines.

In a further preferred embodiment, the mRNA compound comprises, preferably in 5'- to 3'-direction:
a) a 5'-CAP structure, preferably m7GpppN, more preferably CAP1 or m7G(5')ppp(5')(2'OMeA)pG;
b) optionally, a 5'-UTR element;
c) at least one coding region encoding at least one antigenic peptide or protein, preferably derived from a protein of SARS coronavirus 2 (SARS-CoV-2), nCoV-2019 coronavirus, SARS coronavirus (SARS-CoV), an Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) or a fragment or variant thereof,
d) optionally, a 3'-UTR element, preferably comprising or consisting of a nucleic acid sequence which is derived from an alpha globin gene, preferably comprising the corresponding RNA sequence of the nucleic acid sequence according to SEQ ID NO:11 as shown in SEQ ID NO:12, a homolog, a fragment or a variant thereof;
e) optionally, a poly(A) sequence, preferably comprising 64 or 100 adenosines;
f) optionally, a poly(C) sequence, preferably comprising 30 cytosines.

In a further preferred embodiment, the composition comprises an mRNA compound comprising at least one 5'-untranslated region element (5'-UTR element). Preferably, the at least one 5'-UTR element comprises or consists of a nucleic acid sequence, which is derived from the 5'-UTR of a TOP gene or which is derived from a fragment, homolog or variant of the 5'-UTR of a TOP gene. It is preferred that the 5'-UTR element does not comprise a TOP motif or a 5'-TOP, as defined above.

In some embodiments, the nucleic acid sequence of the 5'-UTR element, which is derived from a 5'-UTR of a TOP gene, terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'-UTR element does not comprise any part of the protein coding region. Thus, preferably, the only protein coding part of the at least one mRNA sequence is provided by the coding region.

The nucleic acid sequence derived from the 5'-UTR of a TOP gene is preferably derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'-UTR element may be selected from 5'-UTR elements comprising or consisting of a nucleic acid sequence, which is derived from a nucleic acid sequence selected from the group consisting of SEQ ID NO:1-1363, SEQ ID NO:1395, SEQ ID NO:1421 and SEQ ID NO:1422 of the patent application WO2013143700, whose disclosure is incorporated herein by reference, from the homologs of SEQ ID NO:1-1363, SEQ ID NO:1395, SEQ ID NO:1421 and SEQ ID NO:1422 of the patent application WO2013143700, from a variant thereof, or preferably from a corresponding RNA sequence. The term "homologs of SEQ ID NO:1-1363, SEQ ID NO:1395, SEQ ID NO:1421 and SEQ ID NO:1422 of the patent application WO2013143700" refers to sequences of other species than homo sapiens, which are homologous to the sequences according to SEQ ID NO:1-1363, SEQ ID NO:1395, SEQ ID NO:1421 and SEQ ID NO:1422 of the patent application WO2013143700.

In a preferred embodiment, the 5'-UTR element of the mRNA compound comprises or consists of a nucleic acid sequence, which is derived from a nucleic acid sequence extending from nucleotide position 5 (i.e. the nucleotide that is located at position 5 in the sequence) to the nucleotide position immediately 5' to the start codon (located at the 3'-end of the sequences), e.g. the nucleotide position immediately 5' to the ATG sequence, of a nucleic acid sequence selected from SEQ ID NO:1-1363, SEQ ID NO:1395, SEQ ID NO:1421 and SEQ ID NO:1422 of the patent application WO2013143700, from the homologs of SEQ ID NO:1-1363, SEQ ID NO:1395, SEQ ID NO:1421 and SEQ ID NO:1422 of the patent application WO2013143700 from a variant thereof, or a corresponding RNA sequence. It is particularly preferred that the 5'-UTR element is derived from a nucleic acid sequence extending from the nucleotide position immediately 3' to the 5'-TOP to the nucleotide position immediately 5' to the start codon (located at the 3'-end of the sequences), e.g. the nucleotide position immediately 5' to the ATG sequence, of a nucleic acid sequence selected from SEQ ID NO:1-1363, SEQ ID NO:1395, SEQ ID NO:1421 and SEQ ID NO:1422 of the patent application WO2013143700, from the homologs of SEQ ID NO:1-1363, SEQ ID NO:1395, SEQ ID NO:1421 and SEQ ID NO:1422 of the patent application WO2013143700, from a variant thereof, or a corresponding RNA sequence.

In a further preferred embodiment, the 5'-UTR element comprises or consists of a nucleic acid sequence, which is derived from a 5'-UTR of a TOP gene encoding a ribosomal protein or from a variant of a 5'-UTR of a TOP gene encoding a ribosomal protein. For example, the 5'-UTR element comprises or consists of a nucleic acid sequence, which is derived from a 5'-UTR of a nucleic acid sequence according to any of SEQ ID NO:67, 170, 193, 244, 259, 554, 650, 675, 700, 721, 913, 1016, 1063, 1120, 1138, and 1284-1360 of the patent application WO2013143700, a corresponding RNA sequence, a homolog thereof, or a variant thereof as described herein, preferably lacking the 5'-TOP motif. As described above, the sequence extending from position 5 to the nucleotide immediately 5' to the ATG (which is located at the 3'-end of the sequences) corresponds to the 5'-UTR of said sequences.

Accordingly, in a preferred embodiment, the 5'-UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO:23 or SEQ ID NO:24 (5'-UTR of human ribosomal protein Large 32 lacking the 5'-terminal oligopyrimidine tract; corresponding to SEQ ID NO: 1368 of the patent application WO2013143700) or preferably to a corresponding RNA sequence, or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO:23 or more preferably to a corresponding RNA sequence (SEQ ID NO:24), wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the mRNA compound comprises a 5'-UTR element, which comprises or consists of a nucleic acid sequence which is derived from the 5'-UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB, or from a homolog or variant thereof, wherein preferably the 5'-UTR element does not comprise a TOP motif or the 5'-TOP of said genes, and wherein optionally the 5'-UTR element starts at its 5'-end with a nucleotide located at position 1, 2, 3, 4, 5, 6, 7, 8, 9 or 10 downstream of the 5'-terminal oligopyrimidine tract (TOP) and wherein further optionally the 5'-UTR element which is derived from a 5'-UTR of a TOP gene terminates at its 3'-end with a nucleotide located at position 1, 2, 3, 4, 5, 6, 7, 8, 9 or 10 upstream of the start codon (A(U/T)G) of the gene it is derived from.

In further preferred embodiments, the 5'-UTR element comprises or consists of a nucleic acid sequence, which is derived from the 5'-UTR of a ribosomal protein Large 32 gene (RPL32), a cationic amino acid transporter 3 (solute carrier family 7 member 3, SLC7A3) protein, a ribosomal protein Large 35 gene (RPL35), a ribosomal protein Large 21 gene (RPL21), an ATP synthase, H+ transporting, mitochondrial F1 complex, alpha subunit 1, cardiac muscle gene (ATP5A1), an hydroxysteroid (17-beta) dehydrogenase 4 gene (HSD17B4; SEQ ID NO:21, SEQ ID NO:22), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4; SEQ ID NO:21, SEQ ID NO:22), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle gene (ATP5A1), a mammalian hydroxysteroid (17-beta) dehydrogenase 4 gene (HSD17B4; SEQ ID NO:21, SEQ ID NO:22), a mammalian androgen-induced 1 gene (AIG1), a mammalian cyto-chrome c oxidase subunit VIc gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle gene (ATP5A1), a human hydroxysteroid (17-beta) dehydrogenase 4 gene (HSD17B4; SEQ ID NO:21, SEQ ID NO:22), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'-UTR element does not comprise the 5'-TOP of said gene.

Accordingly, in a preferred embodiment, the 5'-UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO:1368, or SEQ ID NO:1412-1420 of the patent application WO2013143700, or a corresponding RNA sequence, or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO:1368, or SEQ ID NO:1412-1420 of the patent application WO2013143700, wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the at least one 5'-UTR element and the at least one 3'-UTR element act synergistically to increase protein production from the at least one mRNA sequence as described above.

According to a preferred embodiment, the composition of the invention comprises an mRNA compound that comprises, preferably in 5'- to 3'-direction:
a) a 5'-CAP structure, preferably m7GpppN, more preferably CAP1 or m7G(5')ppp(5')(2'OMeA)pG;
b) optionally, a 5'-UTR element which preferably comprises or consists of a nucleic acid sequence which is derived from the 5'-UTR of a TOP gene, more preferably comprising or consisting of the corresponding RNA sequence of a nucleic acid sequence according to SEQ ID NO:21, 23, 25 or respectively SEQ ID NO:22, 24 or 26, a homolog, a fragment or a variant thereof, most preferably according to SEQ ID NO:22 (HSD17B4);
c) at least one coding region encoding at least one antigenic peptide or protein preferably derived from a protein of a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale), or a fragment or variant thereof, preferably comprising or consisting of any one of the nucleic acid sequences as disclosed in the sequence listing having a numeric identifier <223> which starts with "derived and/or modified CDS sequence (wt) " or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Tabs. 1-5 or Figs. 20-24 or respective the sequence listing of PCT/EP2016/075843 or WO2018078053, incorporated by reference in their entirety; or an ORF comprised in SEQ ID NO:27-40 or SEQ ID NO:71-74 or of a fragment or variant of any one of these sequences; or at least one coding region encoding at least one antigenic peptide or protein preferably derived from a protein of an SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale);
d) optionally, a 3'-UTR element which preferably comprises or consists of a nucleic acid sequence which is derived from a gene providing a stable mRNA, preferably comprising or consisting of the corresponding RNA sequence of a nucleic acid sequence according to SEQ ID NO:6, 8, 10, 12, 14, 16, 18, 20, preferably according to SEQ ID NO:12 or SEQ ID NO:18 or a homolog, a fragment or a variant thereof, most preferably according to SEQ ID NO:20 (PSMB3);
e) optionally a histone stem-loop;
f) optionally, a poly(A) sequence preferably comprising 64 or 100 adenosines; and
g) optionally, a poly(C) sequence, preferably comprising 30 cytosines.

According to one embodiment, the mRNA compound comprises an miRNA sequence. A miRNA (microRNA) is typically a small, non-coding single stranded RNA molecules of about 20 to 25 nucleotides in length which may function in gene regulation, for example, but not limited to, by mRNA degradation or translation inhibition or repression. miRNAs are typically produced from hairpin precursor RNAs (pre-miRNAs), and they may form functional complexes with proteins. Furthermore, miRNAs may bind to 3'-UTR regions of target mRNAs. Preferably, the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof.

In one embodiment, the miRNA sequence is a naturally occurring miRNA sequence. In another embodiment, the miRNA sequence may be a mimetic, or a modification of a naturally-occurring miRNA sequence.

In some embodiments, a 3'-UTR comprises one or more of a polyadenylation signal, a binding site for proteins that affect a nucleic acid stability of location in a cell, or one or more miRNA or binding sites for miRNAs.

MicroRNAs (or miRNA) are 19-25 nucleotide long noncoding RNAs that bind to the 3'-UTR of nucleic acid molecules and down-regulate gene expression either by reducing nucleic acid molecule stability or by inhibiting translation. E.g., microRNAs are known to regulate RNA, and thereby protein expression, e.g. in liver (miR-122), heart (miR-ld, miR-149), endothelial cells (miR-17-92, miR-126), adipose tissue (let-7, miR-30c), kidney (miR-192, miR-194, miR-204), myeloid cells (miR-142-3p, miR-142-5p, miR-16, miR-21, miR-223, miR-24, miR-27), muscle (miR-133, miR-206, miR-208), and lung epithelial cells (let-7, miR-133, miR-126). The RNA may comprise one or more microRNA target sequences, microRNA sequences, or microRNA seeds. Such sequences may e.g. correspond to any known microRNA such as those taught in US20050261218 and US20050059005.

According to one preferred embodiment, the mRNA compound comprising an mRNA sequence according to the invention may further comprise, as defined herein:
a) a 5'-CAP structure, preferably m7GpppN, more preferably CAP1 or m7G(5')ppp(5')(2'OMeA)pG;
b) optionally at least one miRNA sequence, preferably wherein the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof;
c) at least one 5'-UTR element;
d) a coding sequence
e) at least one 3'-UTR element;
f) at least one poly(A) sequence;
g) at least one poly(C) sequence;
or any combinations of these.

In one preferred embodiment, the mRNA compound comprises, preferably in 5'- to 3'-direction:
h) a 5'-CAP structure, preferably m7GpppN, more preferably CAP1 or m7G(5')ppp(5')(2'OMeA)pG;
i) optionally, a 5'-UTR element,
j) at least one coding region encoding at least one antigenic peptide or protein,
k) optionally, a 3'-UTR element, preferably comprising or consisting of a nucleic acid sequence which is derived from an alpha globin gene, preferably comprising the corresponding RNA sequence of the nucleic acid sequence according to SEQ ID NO:11 as shown in SEQ ID NO:12, a homolog, a fragment or a variant thereof;
l) optionally, a poly(A) sequence, preferably comprising 64 adenosines or 100 adenosines;
m) optionally, a poly(C) sequence, preferably comprising 30 cytosines;
n) optionally, a histone stem-loop selected from SEQ ID NO:3 or 4; and/or
o) optionally, a 3'-terminal sequence element selected from SEQ ID NO:41-70.

In a further preferred embodiment, the mRNA compound comprises, preferably in 5'- to 3'-direction:
g) a 5'-CAP structure, preferably m7GpppN, more preferably CAP1 or m7G(5')ppp(5')(2'OMeA)pG;
h) optionally, a 5'-UTR element;
i) at least one coding region encoding at least one antigenic peptide or protein, preferably derived from a protein of SARS coronavirus 2 (SARS-CoV-2), nCoV-2019 coronavirus, SARS coronavirus (SARS-CoV), an Bunyavirales virus, Cytomegalovirus (CMV), Dengue viruses (DENV-1, DENV-2, DENV-3 and DENV-4), Ebola virus (EBOV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus (RABV), Respiratory Syncytial virus (RSV), Rhinovirus, Rotavirus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) or a fragment or variant thereof,
j) optionally, a 3'-UTR element, preferably comprising or consisting of a nucleic acid sequence which is derived from an alpha globin gene, preferably comprising the corresponding RNA sequence of the nucleic acid sequence according to SEQ ID NO:11 as shown in SEQ ID NO:12, a homolog, a fragment or a variant thereof;
k) optionally, a poly(A) sequence, preferably comprising 64 adenosines or 100 adenosines;
l) optionally, a poly(C) sequence, preferably comprising 30 cytosines;
m) optionally, a histone stem-loop selected from SEQ ID NO:3 or 4; and/or
n) optionally, a 3'-terminal sequence element selected from SEQ ID NO:41-70.

### Histone Stem-loop (hSL) / Histone 3'-UTR stem-loop

In a further preferred embodiment, the composition comprises an mRNA compound comprising a histone stem-loop sequence/structure (hSL). In said embodiment, the mRNA sequence may comprise at least one (or more) histone stem loop sequence or structure. Such histone stem-loop sequences are preferably selected from histone stem-loop sequences as disclosed in WO2012019780, the disclosure of which is incorporated herewith by reference. A histone stem-loop sequence that may be used within the present invention may preferably be derived from formulae (I) or (II) of WO2012019780. According to a further preferred embodiment the coding RNA may comprise at least one histone stem-loop sequence derived from at least one of the specific formulae (la) or (IIa) of the patent application WO2012019780. According to a further preferred embodiment the coding RNA may comprise at least one histone stem-loop sequence derived from a Histone stem-loop as disclosed in patent application WO2018104538 under formula (I), formula (II), formula (la) or on pages 49-52 under section "histone stem-loop" and WO2018104538- SEQ ID NO:1451-1452 as disclosed in WO2018104538; WO2018104538 which is herein incorporated by reference in its entirety, also especially SEQ ID NO:1451-1452.

In particularly preferred embodiment, the RNA of the invention comprises at least one histone stem-loop sequence, wherein said histone stem-loop sequence comprises a nucleic acid sequence being identical or at least 70%, 80%, 90%, 91%, 92%, 93%, 94%, 95%, 96%, 97%, 98%, or 99% identical to SEQ ID NO:3 or 4, or fragments or variants thereof.

In other embodiments, the nucleic acid comprises a 5'-UTR which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a sequence selected from SEQ ID NO:22848-22875 as disclosed in WO2021156267 or a fragment or a variant thereof.

In other embodiments, the nucleic acid comprises a 3'-UTR which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a sequence selected from SEQ ID NO:22876-22891 as disclosed in WO2021156267 or a fragment or a variant thereof.

In other embodiments, the nucleic acid comprises a 5'-end which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a single sequence selected from the group consisting of SEQ ID NO:176-177 and 22840-22844 as disclosed in WO2022137133 or a fragment or a variant thereof.

In other embodiments, the nucleic acid comprises a Kozak sequence which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a single sequence selected from the group consisting of SEQ ID NO:180-181, and 22845-22847 as disclosed in WO2022137133 or a fragment or a variant thereof.

In other embodiments, the nucleic acid comprises a 5'-UTR which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a single sequence selected from the group consisting of SEQ ID NO:231-252, 22848-22875, and 28522-28525 as disclosed in WO2021156267 or a fragment or a variant thereof.

In other embodiments, the nucleic acid comprises a 3'-UTR which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a single sequence selected from the group consisting of SEQ ID NO:253-268, 22876-22911, 26996-27003, and 28526-28539 as disclosed in WO2021156267 or a fragment or a variant thereof.

In other embodiments, the nucleic acid comprises a 3'-end which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a single sequence selected from the group consisting of SEQ ID NO:182-230, and 27004-27006 as disclosed in WO2021156267 or a fragment or a variant thereof.

In other embodiments, the nucleic acid comprises a histone stem-loop which comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to a sequence selected from the group consisting of SEQ ID NO:178 and 179 as disclosed in WO2022137133 or a fragment or a variant thereof.

### Signal Peptides

According to another embodiment, the composition of the invention comprises an mRNA compound which may, additionally or alternatively, encode a secretory signal peptide. Such signal peptides are sequences, which typically exhibit a length of about 15 to 30 amino acids and are preferably located at the N-terminus of the encoded peptide, without being limited thereto. Signal peptides as defined herein preferably allow the transport of the antigen, antigenic protein or antigenic peptide as encoded by the at least one mRNA sequence into a defined cellular compartment, preferably the cell surface, the endoplasmic reticulum (ER) or the endosomal-lysosomal compartment. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulins as defined herein, signal sequences of the invariant chain of immunoglobulins or antibodies as defined herein, signal sequences of Lamp1, Tapasin, Erp57, Calreticulin, Calnexin, and further membrane associated proteins or of proteins associated with the endoplasmic reticulum (ER) or the endosomal-lysosomal compartment. Most preferably, signal sequences of MHC class I molecule HLA-A*0201 may be used according to the present invention. For example, a signal peptide derived from HLA-A is preferably used in order to promote secretion of the encoded antigen as defined herein or a fragment or variant thereof. More preferably, an HLA-A signal peptide is fused to an encoded antigen as defined herein or to a fragment or variant thereof.

The mRNA compound to be incorporated in the composition according to the present invention may be prepared using any method known in the art, including synthetic methods such as e.g. solid phase RNA synthesis, as well as in vitro methods, such as RNA in vitro transcription reactions, particularly as described in the examples.

### Methods of Preparing Lipid Nanoparticle Compositions

The invention further relates to a method of preparing said lipid nanoparticles comprising the steps of:
(i) providing:
   a) cationic lipid of formula (I) as defined herein or a pharmaceutically acceptable salt, tautomer, prodrug or stereoisomer thereof;
   b) a polymer conjugated lipid as defined herein;
   c) at least one mRNA compound comprising an mRNA sequence encoding at least one antigenic peptide or protein;
   d) optionally, a steroid; and
   e) optionally, a neutral lipid;
(ii) solubilizing the cationic lipid and/or the polymer conjugated lipid according to formula (I) and optionally the neutral lipid and/or the steroid or a steroid derivative in an alcohol such as ethanol;
(iii) mixing the alcoholic lipid solution with an aqueous solution comprising the mRNA polynucleotide
(iv) removing the alcohol to form lipid nanoparticles encapsulating or associating with the mRNA polynucleotide; and optionally
(v) separating or purifying the lipid nanoparticles.

The alcohol may be removed by any suitable method which does not negatively affect the lipids or the forming lipid nanoparticles. In one embodiment of the invention the alcohol is removed by dialysis. In an alternative embodiment the alcohol is removed by diafiltration.

Separation and optional purification of the lipid nanoparticles might also be performed by any suitable method. Preferably the lipid nanoparticles are filtrated, more preferably the lipid nanoparticles are separated or purified by filtration through a sterile filter.

In some embodiments, the solutions are mixed in a microfluidic mixer to obtain the composition. Suitably, the microfluidic mixing conditions are chosen so as to obtain encapsulation of the pharmaceutically active compound at an encapsulation efficiency (EE) of above 80%, preferably above 90%, more preferably above 94%.

### Routes of administration

The choice of a pharmaceutically acceptable carrier is determined, in principle, by the manner, in which the pharmaceutical composition or vaccine according to the invention is administered. The composition or vaccine of the invention comprising the inventive POZ or PMOZ-lipids can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, intratumoral and sublingual injections. Administration to the respiratory system can be performed by spray administration or inhalation may in particular be performed by aerosol administration to the lungs, bronchi, bronchioli, alveoli, or paranasal sinuses.

In further preferred embodiments, the route of administration is selected from the group consisting of extravascular administration to a subject, such as by extravascular injection, infusion or implantation; topical administration to the skin or a mucosa; inhalation such as to deliver the composition to the respiratory system; or by transdermal or percutaneous administration. In even further preferred embodiments, the composition or vaccine of the invention comprising the inventive POZ or PMOZ-lipids can be administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, intraocular, periocular, periodontal, retrobulbar, subarachnoid, subconjunctival or suprachoroidal injection, infusion or implantation. Moreover, topical administration to the skin or a mucosa may be performed by dermal or cutaneous, nasal, buccal, sublingual, otic or auricular, ophthalmic, conjunctival, vaginal, rectal, intracervical, endosinusial, laryngeal, oropharyngeal, ureteral, urethral administration. Even more preferred routes of administration for a vaccine are intramuscular, intradermal, intranasal and oral administration (e.g. via a tablet comprising a polynucleotide, RNA or mRNA as disclosed herein).

Preferably, compositions or vaccines according to the present invention comprising the inventive POZ or PMOZ-lipids may be administered by an intradermal, subcutaneous, or intramuscular route, preferably by injection, which may be needle-free and/or needle injection. Compositions or vaccines according to the present invention comprising the inventive POZ or PMOZ-lipids are therefore preferably formulated in liquid or solid form. The suitable amount of the vaccine or composition according to the invention to be administered can be determined by routine experiments, e.g. by using animal models. Such models include, without implying any limitation, rabbit, sheep, mouse, rat, dog and non-human primate models.

Preferred unit dose forms for injection include sterile solutions of water, physiological saline or mixtures thereof. The pH of such solutions should be adjusted to a physiologically tolerable pH, such as about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive composition or vaccine is to be administered perorally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

### Pharmaceutical Compositions and Kits

The term "treatment" or "treating" of a disease includes preventing or protecting against the disease (that is, causing the clinical symptoms not to develop); inhibiting the disease (i.e., arresting or suppressing the development of clinical symptoms; and/or relieving the disease (i.e., causing the regression of clinical symptoms). In a preferred embodiment, the term "subject" refers to a human.

The invention further relates to a pharmaceutical composition comprising at least one lipid nanoparticle according to the present invention. The lipid nanoparticle might comprise an mRNA compound comprising a sequence encoding at least one antigenic peptide or protein as defined herein.

In one embodiment of the invention the mRNA sequence encodes one antigenic peptide or protein. In an alternative embodiment of the invention the mRNA sequence encodes more than one antigenic peptide or protein.

In one embodiment of the invention, the pharmaceutical composition comprises a lipid nanoparticle according to the invention, wherein the lipid nanoparticle comprises more than one mRNA compounds, which each comprise a different mRNA sequence encoding an antigenic peptide or protein.

In an alternative embodiment of the invention the pharmaceutical composition comprises a second lipid nanoparticle, wherein the mRNA compound comprised by the second lipid nanoparticle is different from the mRNA compound comprised by the first lipid nanoparticle.

In a further aspect, the present invention concerns a composition comprising mRNA comprising lipid nanoparticles wherein the mRNA comprises an mRNA sequence comprising at least one coding region as defined herein and a pharmaceutically acceptable carrier. The composition according to the invention is preferably provided as a pharmaceutical composition or as a vaccine.

The composition according to the invention might also comprise suitable pharmaceutically acceptable adjuvants. In preferred embodiments the adjuvant is preferably added in order to enhance the immunostimulatory properties of the composition. In this context, an adjuvant may be understood as any compound, which is suitable to support administration and delivery of the composition according to the invention. Furthermore, such an adjuvant may, without being bound thereto, initiate or increase an immune response of the innate immune system, i.e. a nonspecific immune response. In other words, when administered, the composition according to the invention typically initiates an adaptive immune response due to an antigen as defined herein or a fragment or variant thereof, which is encoded by the at least one coding sequence of the inventive mRNA contained in the composition of the present invention. Additionally, the composition according to the invention may generate an (supportive) innate immune response due to addition of an adjuvant as defined herein to the composition according to the invention.

In some embodiments, the invention provides a method of inducing an immune response in a subject, the method comprising administering to the subject the vaccine of the invention in an amount effective to produce an antigen-specific immune response in the subject. In other embodiments, the invention provides a pharmaceutical composition comprising a composition or a kit or kit of parts as described herein for use in vaccination of a subject comprising an effective dose of mRNA encoding a virus antigen.

Such an adjuvant may be selected from any adjuvant known to a skilled person and suitable for the present case, i.e. supporting the induction of an immune response in a mammal. Preferably, the adjuvant may be selected from the group consisting of adjuvants, without being limited thereto, as disclosed on page 160 line 3 **-161** line 8 in WO2018078053; WO2018078053 being incorporated herein by reference in its entirety.

Particularly preferred, an adjuvant may be selected from adjuvants, which support induction of a Th1-immune response or maturation of naïve T-cells, such as GM-CSF, IL-12, IFN-gamma, any immunostimulatory nucleic acid as defined above, preferably an immunostimulatory RNA, CpG DNA, et cetera.

In a further preferred embodiment it is also possible that the inventive composition contains besides the antigen-providing mRNA further components which are selected from the group comprising: further antigens (e.g. in the form of a peptide or protein) or further antigen-encoding nucleic acids; a further immunotherapeutic agent; one or more auxiliary substances; or any further compound, which is known to be immunostimulating due to its binding affinity (as ligands) to human Toll-like receptors; and/or an adjuvant nucleic acid, preferably an immunostimulatory RNA (isRNA).

The composition of the present invention can additionally contain one or more auxiliary substances in order to increase its immunogenicity or immunostimulatory capacity, if desired. A synergistic action of the mRNA as defined herein and of an auxiliary substance, which may be optionally contained in the inventive composition, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

Suitable adjuvants may furthermore be selected from nucleic acids having the formula GₗXmGₙ, wherein: G is guanosine, uracil or an analogue of guanosine or uracil; X is guanosine, uracil, adenosine, thymidine, cytosine or an analogue of the above-mentioned nucleotides; l is an integer from 1 to 40, wherein when l = 1 G is guanosine or an analogue thereof, when l > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof, or formula: (NᵤGₗXₘGₙNᵥ)ₐ, wherein: G is guanosine (guanine), uridine (uracil) or an analogue of guanosine (guanine) or uridine (uracil), preferably guanosine (guanine) or an analogue thereof; X is guanosine (guanine), uridine (uracil), adenosine (adenine), thymidine (thymine), cytidine (cytosine), or an analogue of these nucleotides (nucleosides), preferably uridine (uracil) or an analogue thereof; N is a nucleic acid sequence having a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides); a is an integer from 1 to 20, preferably from 1 to 15, most preferably from 1 to 10; l is an integer from 1 to 40, wherein when l = 1, G is guanosine (guanine) or an analogue thereof, when l > 1, at least 50% of these nucleotides (nucleosides) are guanosine (guanine) or an analogue thereof; m is an integer and is at least 3; wherein when m = 3, X is uridine (uracil) or an analogue thereof, and when m > 3, at least 3 successive uridines (uracils) or analogues of uridine (uracil) occur; n is an integer from 1 to 40, wherein when n = 1, G is guanosine (guanine) or an analogue thereof, when n > 1, at least 50% of these nucleotides (nucleosides) are guanosine (guanine) or an analogue thereof; u, v may be independently from each other an integer from 0 to 50, preferably wherein when u = 0, v ≥ 1, or when v = 0, u ≥ 1; wherein the nucleic acid molecule of formula (NᵤGₗXₘGₙNᵥ)ₐ has a length of at least 50 nucleotides, preferably of at least 100 nucleotides, more preferably of at least 150 nucleotides, even more preferably of at least 200 nucleotides and most preferably of at least 250 nucleotides. Other suitable adjuvants may furthermore be selected from nucleic acids having the formula: CₗXₘCₙ, wherein: C is cytosine, uracil or an analogue of cytosine or uracil; X is guanosine, uracil, adenosine, thymidine, cytosine or an analogue of the above-mentioned nucleotides; I is an integer from 1 to 40, wherein when I = 1 C is cytosine or an analogue thereof, when I > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.

In this context the disclosure of WO2008014979 (whole disclosure, especially the subject-matter of claim 1, claim 2, claim 3, claim 4 and claim 5) and WO2009095226 are also incorporated herein by reference in their entirety. In a further aspect, the present invention provides a vaccine, which is based on the mRNA comprising lipid nanoparticles according to the invention comprising at least one mRNA compound comprising a mRNA sequence comprising coding region as defined herein. The vaccine according to the invention is preferably a (pharmaceutical) composition as defined herein.

Accordingly, the vaccine according to the invention is based on the same components as the (pharmaceutical) composition described herein. Insofar, it may be referred to the description of the (pharmaceutical) composition as provided herein. Preferably, the vaccine according to the invention comprises at least one mRNA comprising lipid nanoparticles comprising at least one mRNA sequence as defined herein and a pharmaceutically acceptable carrier. In embodiments, where the vaccine comprises more than one mRNA sequence (such as a plurality of RNA sequences according to the invention, wherein each preferably encodes a distinct antigenic peptide or protein) encapsulated in mRNA comprising lipid nanoparticles, the vaccine may be provided in physically separate form and may be administered by separate administration steps. The vaccine according to the invention may correspond to the (pharmaceutical) composition as described herein, especially where the mRNA sequences are provided by one single composition. However, the inventive vaccine may also be provided physically separated. For instance, in embodiments, wherein the vaccine comprises more than one mRNA sequences/species encapsulated in mRNA comprising lipid nanoparticles as defined herein, these RNA species may be provided such that, for example, two, three, four, five or six separate compositions, which may contain at least one mRNA species/sequence each (e.g. three distinct mRNA species/sequences), each encoding distinct antigenic peptides or proteins, are provided, which may or may not be combined. Also, the inventive vaccine may be a combination of at least two distinct compositions, each composition comprising at least one mRNA encoding at least one of the antigenic peptides or proteins defined herein. Alternatively, the vaccine may be provided as a combination of at least one mRNA, preferably at least two, three, four, five, six or more mRNAs, each encoding one of the antigenic peptides or proteins defined herein. The vaccine may be combined to provide one single composition prior to its use or it may be used such that more than one administration is required to administer the distinct mRNA sequences/species encoding any of the antigenic peptides or proteins encapsulated in mRNA comprising lipid nanoparticles as defined herein. If the vaccine contains at least one mRNA comprising lipid nanoparticles, typically comprising at least two mRNA sequences, encoding the antigen combinations defined herein, it may e.g. be administered by one single administration (combining all mRNA species/sequences), by at least two separate administrations. Accordingly; any combination of mono-, bi- or multicistronic mRNAs encoding the at least one antigenic peptide or protein or any combination of antigens as defined herein (and optionally further antigens), provided as separate entities (containing one mRNA species) or as combined entity (containing more than one mRNA species), is understood as a vaccine according to the present invention. According to a particularly preferred embodiment of the inventive vaccine, the at least one antigen, preferably a combination as defined herein of at least two, three, four, five, six or more antigens encoded by the inventive composition as a whole, is provided as an individual (monocistronic) mRNA, which is administered separately.

As with the (pharmaceutical) composition according to the present invention, the entities of the vaccine may be provided in liquid and or in dry (e.g. lyophilized) form. They may contain further components, in particular further components allowing for its pharmaceutical use. The vaccine or the (pharmaceutical) composition may, e.g., additionally contain a pharmaceutically acceptable carrier and/or further auxiliary substances and additives and/or adjuvants.

The vaccine or (pharmaceutical) composition typically comprises a safe and effective amount of the mRNA compound according to the invention as defined herein, encoding an antigenic peptide or protein as defined herein or a fragment or variant thereof or a combination of antigens, encapsulate within and/or associated with the lipid nanoparticles. As used herein, "safe and effective amount" means an amount of the mRNA that is sufficient to significantly induce a positive modification of cancer or a disease or disorder related to cancer. At the same time, however, a "safe and effective amount" is small enough to avoid serious side-effects, that is to say to permit a sensible relationship between advantage and risk. The determination of these limits typically lies within the scope of sensible medical judgment. In relation to the vaccine or (pharmaceutical) composition of the present invention, the expression "safe and effective amount" preferably means an amount of the mRNA (and thus of the encoded antigen) that is suitable for stimulating the adaptive immune system in such a manner that no excessive or damaging immune reactions are achieved but, preferably, also no such immune reactions below a measurable level. Such a "safe and effective amount" of the mRNA of the (pharmaceutical) composition or vaccine as defined herein may furthermore be selected in dependence of the type of mRNA, e.g. monocistronic, bi- or even multicistronic mRNA, since a bi- or even multicistronic mRNA may lead to a significantly higher expression of the encoded antigen(s) than the use of an equal amount of a monocistronic mRNA. A "safe and effective amount" of the mRNA of the (pharmaceutical) composition or vaccine as defined above will furthermore vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the accompanying doctor. The vaccine or composition according to the invention can be used according to the invention for human and also for veterinary medical purposes, as a pharmaceutical composition or as a vaccine.

In a preferred embodiment, the mRNA comprising lipid nanoparticle of the (pharmaceutical) composition, vaccine or kit of parts according to the invention is provided in lyophilized form. Preferably, the lyophilized mRNA comprising lipid nanoparticles are reconstituted in a suitable buffer, advantageously based on an aqueous carrier, prior to administration, e.g. Ringer-Lactate solution, Ringer solution, a phosphate buffer solution. In a preferred embodiment, the (pharmaceutical) composition, the vaccine or the kit of parts according to the invention contains at least one, two, three, four, five, six or more mRNA compounds, which may be provided as a single species of lipid nanoparticles, or separately for each LNP species, optionally in lyophilized form (optionally together with at least one further additive) and which are preferably reconstituted separately in a suitable buffer (such as Ringer-Lactate solution) prior to their use so as to allow individual administration of each of the (monocistronic) mRNAs.

The vaccine or (pharmaceutical) composition according to the invention may typically contain a pharmaceutically acceptable carrier or excipient. Examples of suitable carriers and excipients are known to those skilled in the art and include but are not limited to preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The term "pharmaceutical composition" in the context of this invention means a composition comprising an active agent and comprising additionally one or more pharmaceutically acceptable carriers. The composition may further contain ingredients selected from, for example, diluents, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms.

The expression "pharmaceutically acceptable carrier" as used herein preferably includes the liquid or non-liquid basis of the inventive vaccine. If the inventive vaccine is provided in liquid form, the carrier will be water, typically pyrogen-free water; isotonic saline or buffered (aqueous) solutions, e.g phosphate, citrate etc. buffered solutions. Particularly for injection of the inventive vaccine, water or preferably a buffer, more preferably an aqueous buffer, may be used, containing a sodium salt, preferably at least 50 mM of a sodium salt, a calcium salt, preferably at least 0.01 mM of a calcium salt, and optionally a potassium salt, preferably at least 3 mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, Kl, KBr, K₂CO₃, KHCO₃, K₂SO₄, and examples of calcium salts include e.g. CaCl₂, Cal₂, CaBr₂, CaCO₃, CaSO₄, Ca(OH)₂. Furthermore, organic anions of the aforementioned cations may be contained in the buffer. According to a more preferred embodiment, the buffer suitable for injection purposes as defined above, may contain salts selected from sodium chloride (NaCl), calcium chloride (CaCl₂) and optionally potassium chloride (KCI), wherein further anions may be present additional to the chlorides. CaCl₂ can also be replaced by another salt like KCI. Typically, the salts in the injection buffer are present in a concentration of at least 50 mM sodium chloride (NaCl), at least 3 mM potassium chloride (KCI) and at least 0.01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. in "in vivo" methods occurring liquids such as blood, lymph, cytosolic liquids, or other body liquids, or e.g. liquids, which may be used as reference media in "in vitro" methods, such as common buffers or liquids. Such common buffers or liquids are known to a skilled person.

However, one or more compatible solid or liquid fillers or diluents or encapsulating compounds may be used as well, which are suitable for administration to a person. The term "compatible" as used herein means that the excipients of the inventive vaccine are capable of being mixed with the mRNA according to the invention as defined herein, in such a manner that no interaction occurs, which would substantially reduce the pharmaceutical effectiveness of the inventive vaccine under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or excipients thereof are sugars, such as, for example, lactose, glucose, trehalose and sucrose; starches, such as, for example, corn starch or potato starch; dextrose; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The choice of a pharmaceutically acceptable carrier is determined, in principle, by the manner, in which the pharmaceutical composition or vaccine according to the invention is administered. The composition or vaccine can be administered, for example, systemically or locally.

Routes for systemic administration in general include, for example, transdermal, oral, parenteral routes, including subcutaneous, intravenous, intramuscular, intraarterial, intradermal and intraperitoneal injections and/or intranasal administration routes. Preferred administration routes according to the invention for the administration of vaccines are intramuscular injection, intradermal injection, or any of the herein mentioned routes of administration.

Routes for local administration in general include, for example, topical administration routes but also intradermal, transdermal, subcutaneous, or intramuscular injections or intralesional, intracranial, intrapulmonal, intracardial, and sublingual injections. More preferably, composition or vaccines according to the present invention may be administered by an intradermal, subcutaneous, or intramuscular route, preferably by injection, which may be needle-free and/or needle injection, or any of the herein mentioned routes of administration.

According to preferred embodiments, the artificial nucleic acid (RNA) molecule, (pharmaceutical) composition or vaccine or kit is administered by a parenteral route, preferably via intradermal, subcutaneous, or intramuscular routes. Preferably, said artificial nucleic acid (RNA) molecule, (pharmaceutical) composition or vaccine or kit may be administered by injection, e.g. subcutaneous, intramuscular or intradermal injection, which may be needle-free and/or needle injection. Accordingly, in preferred embodiments, the medical use and/or method of treatment according to the present invention involves administration of said artificial nucleic acid (RNA) molecule, (pharmaceutical) composition or vaccine or kit by subcutaneous, intramuscular or intradermal injection, preferably by intramuscular or intradermal injection, more preferably by intradermal injection. Such injection may be carried out by using conventional needle injection or (needle-free) jet injection, preferably by using (needle-free) jet injection.

The term "jet injection", as used herein, refers to a needle-free injection method, wherein a fluid containing at least one inventive mRNA sequence and, optionally, further suitable excipients is forced through an orifice, thus generating an ultra-fine liquid stream of high pressure that is capable of penetrating mammalian skin and, depending on the injection settings, subcutaneous tissue or muscle tissue. In principle, the liquid stream forms a hole in the skin, through which the liquid stream is pushed into the target tissue. Preferably, jet injection is used for intradermal, subcutaneous or intramuscular injection of the mRNA sequence according to the invention. In a preferred embodiment, jet injection is used for intramuscular injection of the mRNA sequence according to the invention. In a further preferred embodiment, jet injection is used for intradermal injection of the mRNA sequence according to the invention.

Compositions/vaccines are therefore preferably formulated in liquid or solid form. The suitable amount of the vaccine or composition according to the invention to be administered can be determined by routine experiments, e.g. by using animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to a physiologically tolerable pH, such as about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive composition or vaccine is to be administered perorally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

The inventive vaccine or composition can additionally contain one or more auxiliary substances in order to further increase the immunogenicity. A synergistic action of the mRNA contained in the inventive composition and of an auxiliary substance, which may be optionally be co-formulated (or separately formulated) with the inventive vaccine or composition as described above, is preferably achieved thereby. Depending on the various types of auxiliary substances, various mechanisms may play a role in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response produced by the immune-stimulating adjuvant according to the invention to be enhanced and/or influenced in a targeted manner. Particularly preferred auxiliary substances are cytokines, such as monokines, lymphokines, interleukins or chemokines, that - additional to induction of the adaptive immune response by the encoded at least one antigen - promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, INF-alpha, IFN-beta, INF-gamma, GM-CSF, G-CSF, M-CSF, LT-beta or TNF-alpha, growth factors, such as hGH. Preferably, such immunogenicity increasing agents or compounds are provided separately (not co-formulated with the inventive vaccine or composition) and administered individually.

Further additives which may be included in the inventive vaccine or composition are emulsifiers, such as, for example, Tween; wetting agents, such as, for example, sodium lauryl sulfate; colouring agents; taste-imparting agents, pharmaceutical carriers; tablet-forming agents; stabilizers; antioxidants; preservatives.

The inventive vaccine or composition can also additionally contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an inventive vaccine or composition in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

According to another aspect of the present invention, the present invention also provides a kit, in particular a kit of parts, comprising the mRNA compound comprising mRNA sequence as defined herein and at least one lipid according to formula (I) or formula (II) as defined herein. According to another aspect of the present invention, the present invention also provides a kit, in particular a kit of parts, comprising the mRNA compound comprising mRNA sequence as defined herein and at least DPhyPE as neutral lipid / phospholipid. In a further embodiment the kit comprises a lipid nanoparticle as defined above or the (pharmaceutical) composition comprising a lipid nanoparticle as defined above, and/or the vaccine according to the invention, optionally a liquid vehicle for solubilizing and optionally technical instructions with information on the administration and dosage of the mRNA comprising lipid nanoparticles, the composition and/or the vaccine. The technical instructions may contain information about administration and dosage of the mRNA comprising lipid nanoparticles, the composition and/or the vaccine. Such kits, preferably kits of parts, may be applied e.g. for any of the above mentioned applications or uses, preferably for the use of the lipid nanoparticle according to the invention (for the preparation of an inventive medicament, preferably a vaccine) for the treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections or diseases or disorders related thereto.

The kits may also be applied for the use of the lipid nanoparticle, the composition or the vaccine as defined herein (for the preparation of an inventive vaccine) for the treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections or diseases or disorders related thereto, wherein the lipid nanoparticle, the composition and/or the vaccine may be capable of inducing or enhancing an immune response in a mammal as defined above.

Such kits may further be applied for the use of the lipid nanoparticle, the composition or the vaccine as defined herein (for the preparation of an inventive vaccine) for modulating, preferably for eliciting, e.g. to induce or enhance, an immune response in a mammal as defined above, and preferably for supporting treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections or diseases or disorders related thereto.

Kits of parts, as a special form of kits, may contain one or more identical or different compositions and/or one or more identical or different vaccines as described herein in different parts of the kit. Kits of parts may also contain an (e.g. one) composition, an (e.g. one) vaccine and/or the mRNA comprising lipid nanoparticles according to the invention in different parts of the kit, e.g. each part of the kit containing an mRNA comprising lipid nanoparticles as defined herein, preferably encoding a distinct antigen. Preferably, the kit or the kit of parts contains as a part a vehicle for solubilizing the mRNA according to the invention, the vehicle optionally being Ringer-lactate solution. Any of the above kits may be used in a treatment or prophylaxis as defined above.

In another embodiment of this aspect, the kit according to the present invention may additionally contain at least one adjuvant. In a further embodiment, the kit according to the present invention may additionally contain at least one further pharmaceutically active component, preferably a therapeutic compound suitable for treatment and/or prophylaxis of cancer or a related disorder. Moreover, in another embodiment, the kit may additionally contain parts and/or devices necessary or suitable for the administration of the composition or the vaccine according to the invention, including needles, applicators, patches, injection-devices.

### Antagonists of RNA sensing pattern recognition receptors:

In preferred embodiments, in particular in embodiments where the nucleic acid of the composition is an RNA, the pharmaceutical composition may comprise at least one antagonist of at least one RNA sensing pattern recognition receptor.

In preferred embodiments in that context, the pharmaceutical composition comprises at least one antagonist of at least one RNA sensing pattern recognition receptor selected from a Toll-like receptor, preferably a TLR7 antagonist and/or a TLR8 antagonist.

Suitable antagonist of at least one RNA sensing pattern recognition receptor are disclosed in published PCT patent application WO2021028439, the full disclosure herewith incorporated by reference. In particular, the disclosure relating to suitable antagonist of at least one RNA sensing pattern recognition receptors as defined in any one of the claims 1 to 94 of WO2021028439 are incorporated by reference.

In preferred embodiments, the at least one antagonist of at least one RNA sensing pattern recognition receptor is a single stranded oligonucleotide that comprises or consists of a nucleic acid sequence being identical or at least 90%, 91%, 92%, 93%, 94%, 95%, 96%, 97%, 98%, 99%, or 100% identical to a nucleic acid sequence selected from the group consisting of SEQ ID NOs: 85-212 of WO2021028439, or fragments of any of these sequences. A particularly preferred antagonist in that context is 5'-GAG CGmG CCA-3' (SEQ ID NO: 85 of WO2021028439), or a fragment or variant thereof.

In preferred embodiments, the molar ratio of the at least one antagonist of at least one RNA sensing pattern recognition receptor to the at least one RNA suitably ranges from about 20:1 to about 80:1.

In preferred embodiments, the weight to weight ratio of the at least one antagonist of at least one RNA sensing pattern recognition receptor to the at least one RNA suitably ranges from about 1:2 to about 1:10.

In embodiments, the at least one antagonist of at least one RNA sensing pattern recognition receptor and the at least one RNA encoding are separately formulated in the lipid-based carriers as defined herein or co-formulated in the lipid-based carriers as defined herein.

### Uses of Compositions

The composition according to the invention is particularly useful as a medicament, as will be clear from the description of the active ingredient that may be incorporated within the composition and delivered to a subject, such as a human subject, by means of the composition and/or of the lipid nanoparticles contained therein. As such, a further aspect of the invention is the use of the composition as described above as a medicament. Such use may also be expressed as the use of the composition for the manufacture of a medicament. According to a related aspect, the invention provides a method of treatment, the method comprising a step of administering the composition to a subject, such as a human subject in need thereof, the composition. According to a related aspect, the invention provides a method of treating, the method comprising administration of the composition to a subject, such as a human subject in need thereof, the composition.

In a preferred embodiment, the composition of the invention is used as a medicament, wherein the medicament is a vaccine.

In another preferred embodiment, the composition of the invention is used as a medicament, wherein the medicament is for or suitable for the prevention, prophylaxis, treatment and/or amelioration of a disease selected from infectious diseases including viral, bacterial or protozoological infectious diseases, cancer or tumor diseases, liver diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and diseases associated with a peptide or protein deficiency.

In another preferred embodiment, the composition of the invention is used as a medicament, wherein the medicament is for or suitable for the prevention, prophylaxis, treatment and/or amelioration of an infectious diseases including viral, bacterial or protozoological infectious diseases, wherein the medicament is a vaccine.

In another embodiment, the vaccine of the invention comprises a composition or a kit or kit of parts as described herein for prevention, prophylaxis, treatment and/or amelioration of a disease selected from infectious diseases including viral, bacterial or protozoological infectious diseases, cancer or tumor diseases.

In yet another aspect of the invention, a method of treating, a method of treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition is provided comprising the steps:
a) providing the mRNA, the composition, the vaccine, the kit or kit of parts as described herein; and
b) applying or administering the mRNA, the composition, the vaccine or the kit or kit of parts to a tissue or an organism.

In another embodiment, a method is provided, wherein the mRNA, the composition, the vaccine or the kit or kit of parts is administered to the tissue or to the organism by intravenous, intramuscular, subcutaneous or intradermal injection. In a preferred embodiment, a method is provided, wherein the mRNA, the composition, the vaccine or the kit or kit of parts is administered via intravenous injection.

In yet a further embodiment, a method of inducing an immune response in a subject, the method comprising administering to the subject the vaccine of the invention in an amount effective to produce an antigen-specific immune response in the subject is provided.

In a further embodiment, a pharmaceutical composition comprising a composition or a kit or kit of parts as described herein for use or suitable for use in vaccination of a subject comprising an effective dose of mRNA encoding a virus antigen is provided.

For all embodiments or aspects of the invention, in which mRNA encoding an "antigen" is recited, in further preferred embodiments or aspects of the invention, said mRNA may also encode a protein suitiable for enzyme-replacement therapy, an antibody, a therapeutic protein, or a fragment or variant thereof, wherein the therapeutic protein is selected from the group consisting of
(i) therapeutic proteins for use in enzyme replacement therapy for the treatment of metabolic, endocrine or amino acid disorders or for use in replacing an absent, deficient or mutated protein;
(ii) therapeutic proteins for use in the treatment of blood disorders, diseases of the circulatory system, diseases of the respiratory system, infectious diseases or immune deficiencies;
(iii) therapeutic proteins for use in the treatment of cancer or tumor diseases;
(iv) therapeutic proteins for use in hormone replacement therapy;
(v) therapeutic proteins for use in reprogramming somatic cells into pluri- or omnipotent stem cells;
(vi) therapeutic proteins for use as adjuvant or immunostimulation;
(vii) therapeutic proteins being a therapeutic antibody;
(viii) therapeutic proteins being a gene editing agent; and
(ix) therapeutic proteins for use in treating or preventing a liver disease selected from the group consisting of liver fibrosis, liver cirrhosis and liver cancer.

In another preferred embodiment, use of a pharmaceutical composition comprising a composition or a kit or kit of parts as described herein for (i) inducing an immune response or for (ii) inducing CD8+ T cells responses is provided.

In a specific embodiment, a method for preventing, ameliorating or treating a disease or condition in a subject in need comprising administering to the subject a composition or a kit or kit of parts as described herein is provided.

Further, in a specific embodiment, a method is provided in which administration of the composition results in expression of the antigen encoded by mRNA in the lymphocytes of the subject. Further, a method is provided, wherein administration of the composition results in an antigen specific antibody response, preferably wherein the antigen specific antibody response is measured by the presence of antigen-specific antibodies in serum.

In a specific embodiment. the medicament is for the prevention, prophylaxis, treatment and/or amelioration of a disease selected from cancer or tumor diseases, infectious diseases including viral, bacterial or protozoological infectious diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, amino acid disorders, endocrine, nutritional and metabolic diseases, eye diseases, ear diseases and diseases associated with a peptide or protein deficiency. In one of the preferred embodiments, the medicament is a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) vaccine.

In an alternative embodiment the present invention relates to the use of the pharmaceutical composition or the mRNA comprising lipid in the manufacture of a medicament. In particular said medicament is for therapeutically or prophylactically raising an immune response of a subject in need thereof.

In a preferred embodiment the medicament is for prevention or treatment of cancer or tumor diseases, infectious diseases, allergies, or autoimmune diseases or disorders related thereto.

In particular the medicament is for the treatment of a subject, preferably a vertebrate. In a preferred embodiment the subject is a mammal, preferably selected from the group comprising goat, cattle, swine, dog, cat, donkey, monkey, ape, a rodent such as a mouse, hamster, rabbit and, particularly, human.

Accordingly, in one preferred embodiment, the compositions as described herein are suitable for use as a medicament. In a in further preferred embodiment, said medicament is for the prevention, prophylaxis, treatment and/or amelioration of a disease selected from infectious diseases including viral, bacterial or protozoological infectious diseases, cancer or tumor diseases, liver diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and diseases associated with a peptide or protein deficiency. In a further preferred embodiment, the composition for use as a medicament preferably is a vaccine.

With respect to the administration of the composition to a subject, in particular to a human subject, any suitable route may be used. In one embodiment, the composition is adapted for administration by injection or infusion. As used herein, the expression "adapted for" means that the composition is formulated and processed such as to be suitable for the respective route of administration.

According to one aspect of the present invention, the mRNA comprising lipid nanoparticles, the (pharmaceutical) composition or the vaccine may be used according to the invention (for the preparation of a medicament) for use
(i) in the treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition; and/or
(ii) in enzyme replacement therapy for the treatment of metabolic, amino acid or endocrine disorders or for use in replacing an absent, deficient or mutated protein.

In this context particularly preferred is the treatment or prophylaxis of Malaria, Influenza virus or Rabies virus infections, or of a disorder related to such an infection.

Further particularly preferred is the treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections, or of a disorder related to such an infection.

Furthermore, also included in the present inventions are methods of treating or preventing cancer or tumor diseases, infectious diseases, allergies, or autoimmune diseases or disorders related thereto, preferably as defined herein, by administering to a subject in need thereof a pharmaceutically effective amount of the mRNA comprising lipid nanoparticles, the (pharmaceutical) composition or the vaccine according to the invention. Such a method typically comprises an optional first step of preparing the mRNA comprising lipid nanoparticles, the composition or the vaccine of the present invention, and a second step, comprising administering (a pharmaceutically effective amount of) said composition or vaccine to a patient/subject in need thereof. A subject in need thereof will typically be a mammal. In the context of the present invention, the mammal is preferably selected from the group comprising, without being limited thereto, e.g. goat, cattle, swine, dog, cat, donkey, monkey, ape, a rodent such as a mouse, hamster, rabbit and, particularly, human. In some embodiments of the invention, the subject is a bird, preferably a chicken.

In one embodiment, the composition, formulation or pharmaceutical composition in accordance with the invention preferentially targets cells in the liver but not in other organs (e.g. lung, kidney, heart). Liver cells include hepatocytes and hepatocyte precursors, stellate cells/pericytes, endothelial cells, Kupffer cells, macrophages and neutrophils, for example. In other preferred embodiments, the composition, formulation or pharmaceutical composition in accordance with the invention preferentially targets immune cells. In other preferred embodiments, the composition, formulation or pharmaceutical composition in accordance with the invention preferentially targets the spleen.

In one preferred embodiment, where the composition, formulation or pharmaceutical composition comprises a gRNA in combination with an mRNA encoding a CRISPR endonuclease such as cas9, the composition preferentially targets hepatocytes, pericentral hepatocytes (which act as stem cells in healthy livers) and, or, suitably hepatocyte stem cells. The preferential targeting of cells in the liver is due to the size and neutral charge of the lipid nanoparticles. In certain instances, targeting of the liver cells may have a secondary effect on, or influence other organs in the body. It will therefore be appreciated that the composition, formulations or pharmaceutical compositions of the present invention also have utility in the treatment of diseases other than those associated with the liver.

Suitably said pharmaceutical composition is for use, but not limited to in the treatment of liver disease or diseases where protein expression in the liver has an impact on vertebrate pathologies. As mentioned above, the pharmaceutical compositions described herein may also find use in the treatment of diseases not associated with the liver.

Suitably any transcript, transcript family or series of different transcripts or genomic chromosomal or mitochondrial sequences including but not limited to exons and introns of genes and regulatory elements involved in any liver disease or liver-related disorder may be targeted using a composition or formulation in accordance with the invention. Such a any transcript, transcript family or series of different transcripts may be targeted by any biologically active compound as described herein. In one embodiment, the biologically active compound is a nucleic acid molecule which recognises a pathology-related transcript e.g. an mRNA, gRNA, siRNA, saRNA etc. as described herein.

Suitably any gene involved in any liver disease may be targeted using a composition or formulation in accordance with the invention. Such a gene may be targeted by any biologically active compound as described herein. In one embodiment, the biologically active compound is a nucleic acid molecule which recognises a liver disease gene e.g. an mRNA, gRNA, siRNA etc. as described herein.

The present invention furthermore comprises the use of the mRNA comprising lipid nanoparticles, the (pharmaceutical) composition or the vaccine according to the invention as defined herein for modulating, preferably for inducing or enhancing, an immune response in a mammal as defined herein, more preferably for preventing and/or treating SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections, or of diseases or disorders related thereto.

In this context, support of the treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections may be any combination of a conventional SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DEN-V4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) therapy method such as therapy with antivirals such as neuraminidase inhibitors (e.g. oseltamivir and zanamivir) and M2 protein inhibitors (e.g. adamantane derivatives), and a therapy using the RNA or the pharmaceutical composition as defined herein.

Support of the treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections may be also envisaged in any of the other embodiments defined herein. Accordingly, any use of the mRNA comprising lipid nanoparticles, the (pharmaceutical) composition or the vaccine according to the invention in co-therapy with any other approach, preferably one or more of the above therapeutic approaches, in particular in combination with antivirals is within the scope of the present invention.

For administration, preferably any of the administration routes may be used as defined herein. In particular, an administration route is used, which is suitable for treating or preventing an SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infection as defined herein or diseases or disorders related thereto, by inducing or enhancing an adaptive immune response on the basis of an antigen encoded by the mRNA comprising lipid nanoparticles according to the invention.

Administration of the composition and/or the vaccine according to the invention may then occur prior, concurrent and/or subsequent to administering another composition and/or vaccine as defined herein, which may - in addition - contain another mRNA comprising lipid nanoparticle or combination of mRNA comprising lipid nanoparticles encoding a different antigen or combination of antigens, wherein each antigen encoded by the mRNA sequence according to the invention is preferably suitable for the treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections and diseases or disorders related thereto.

In this context, a treatment as defined herein may also comprise the modulation of a disease associated to SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infection and of diseases or disorders related thereto.

According to a preferred embodiment of this aspect of the invention, the (pharmaceutical) composition or the vaccine according to the invention is administered by injection. Any suitable injection technique known in the art may be employed. Preferably, the inventive composition is administered by injection, preferably by needle-less injection, for example by jet-injection.

In one embodiment, the inventive composition comprises at least one, two, three, four, five, six, seven, eight, nine, ten, eleven, twelve or more mRNAs as defined herein, each of which is preferably injected separately, preferably by needle-less injection. Alternatively, the inventive composition comprises at least one, two, three, four, five, six, seven, eight, nine, ten, eleven, twelve or more mRNAs, wherein the at least one, two, three, four, five, six, seven, eight, nine, ten, eleven, twelve or more mRNAs are administered, preferably by injection as defined herein, as a mixture.

In a further aspect the invention relates to a method of immunization of a subject against an antigen or a combination of antigens.

The immunization protocol for the immunization of a subject against an antigen or a combination of at least two, three, four, five, six, seven, eight, nine, ten, eleven, twelve or more antigens as defined herein typically comprises a series of single doses or dosages of the (pharmaceutical) composition or the vaccine according to the invention. A single dosage, as used herein, refers to the initial/first dose, a second dose or any further doses, respectively, which are preferably administered in order to "boost" the immune reaction. In this context, each single dosage preferably comprises the administration of the same antigen or the same combination of antigens as defined herein, wherein the interval between the administration of two single dosages can vary from at least one day, preferably 2, 3, 4, 5, 6 or 7 days, to at least one week, preferably 2, 3, 4, 5, 6, 7 or 8 weeks. The intervals between single dosages may be constant or vary over the course of the immunization protocol, e.g. the intervals may be shorter in the beginning and longer towards the end of the protocol. Depending on the total number of single dosages and the interval between single dosages, the immunization protocol may extend over a period of time, which preferably lasts at least one week, more preferably several weeks (e.g. 2, 3, 4, 5, 6, 7, 8, 9, 10, 11 or 12 weeks), even more preferably several months (e.g. 3, 4, 5, 6, 7, 8, 9, 10, 11, 12, 18 or 24 months). Each single dosage preferably encompasses the administration of an antigen, preferably of a combination of at least two, three, four, five, six, seven, eight, nine, ten, eleven, twelve or more antigens as defined herein and may therefore involve at least one, preferably 1, 2, 3, 4, 5, 6, 7, 8, 9, 10, 11 or 12 injections. In some cases, the composition or the vaccine according to the invention is administered as a single dosage typically in one injection. In the case, where the vaccine according to the invention comprises separate mRNA formulations encoding distinct antigens as defined herein, the minimum number of injections carried out during the administration of a single dosage corresponds to the number of separate components of the vaccine. In certain embodiments, the administration of a single dosage may encompass more than one injection for each component of the vaccine (e.g. a specific mRNA formulation comprising an mRNA encoding, for instance, one antigenic peptide or protein as defined herein). For example, parts of the total volume of an individual component of the vaccine may be injected into different body parts, thus involving more than one injection. In a more specific example, a single dosage of a vaccine comprising four separate mRNA formulations, each of which is administered in two different body parts, comprises eight injections. Typically, a single dosage comprises all injections required to administer all components of the vaccine, wherein a single component may be involve more than one injection as outlined above. In the case, where the administration of a single dosage of the vaccine according to the invention encompasses more than one injection, the injection are carried out essentially simultaneously or concurrently, i.e. typically in a time-staggered fashion within the time-frame that is required for the practitioner to carry out the single injection steps, one after the other. The administration of a single dosage therefore preferably extends over a time period of several minutes, e.g. 2, 3, 4, 5, 10, 15, 30 or 60 minutes.

Administration of the mRNA comprising lipid nanoparticles as defined herein, the (pharmaceutical) composition or the vaccine according to the invention may be carried out in a time staggered treatment. A time staggered treatment may be e.g. administration of the mRNA comprising lipid nanoparticles, the composition or the vaccine prior, concurrent and/or subsequent to a conventional therapy of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections or diseases or disorders related thereto, e.g. by administration of the mRNA comprising lipid nanoparticles, the composition or the vaccine prior, concurrent and/or subsequent to a therapy or an administration of a therapeutic suitable for the treatment or prophylaxis of SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale) infections or diseases or disorders related thereto. Such time staggered treatment may be carried out using e.g. a kit, preferably a kit of parts as defined herein.

Time staggered treatment may additionally or alternatively also comprise an administration of the mRNA comprising lipid nanoparticles as defined herein, the (pharmaceutical) composition or the vaccine according to the invention in a form, wherein the mRNA encoding an antigenic peptide or protein as defined herein or a fragment or variant thereof, preferably forming part of the composition or the vaccine, is administered parallel, prior or subsequent to another mRNA comprising lipid nanoparticles as defined above, preferably forming part of the same inventive composition or vaccine. Preferably, the administration (of all mRNA comprising lipid nanoparticles) occurs within an hour, more preferably within 30 minutes, even more preferably within 15, 10, 5, 4, 3, or 2 minutes or even within 1 minute. Such time staggered treatment may be carried out using e.g. a kit, preferably a kit of parts as defined herein.

In a preferred embodiment, the pharmaceutical composition or the vaccine of the present invention is administered repeatedly, wherein each administration preferably comprises individual administration of the at least one mRNA comprising lipid nanoparticles of the inventive composition or vaccine. At each time point of administration, the at least one mRNA may be administered more than once (e.g. 2 or 3 times). In a particularly preferred embodiment of the invention, at least two, three, four, five, six or more mRNA sequences (each encoding a distinct one of the antigens as defined herein) encapsulated or associated with mRNA comprising lipid nanoparticles as defined above, wherein the mRNA sequences are part of mRNA compounds of the same or different lipid nanoparticles, are administered at each time point, wherein each mRNA is administered twice by injection, distributed over the four limbs.

In another preferred embodiment, the use of a pharmaceutical composition comprising a composition of the invention or a kit or kit of parts of the invention for (i) inducing an immune response, for (ii) inducing an antigen specific T-cell response or preferably for (iii) inducing CD8+ T cells responses is provided. Said method for (i) inducing an immune response, for (ii) inducing an antigen specific T-cell response or preferably for (iii) inducing CD8+ T cells responses in a subject; comprises administering to a subject in need thereof at least once an effective amount of a composition as described herein comprises an mRNA encoding at least one immunogenic peptide or polypeptide as also described herein. In another embodiment, the use of a pharmaceutical composition comprising a composition of the invention or a kit or kit of parts of the invention for (i) inducing an immune response, for (ii) inducing an antigen specific T-cell response or preferably for (iii) inducing CD8+ T cells responses is provided. Said reference (lipid nanoparticle) formulation or composition in a preferred embodiment does not comprise DPhyPE and/or a polymer conjugated lipid according to formula (I).

### First and second/further medical use:

A further aspect relates to the first medical use of the provided nucleic acid, composition, polypeptide, vaccine, or kit, wherein the composition of the invention, comprising the inventive lipid excipient(s), is used for delivering said nucleic acid. Notably, embodiments relating to the nucleic acid, the composition, the polypeptide, the vaccine, or the kit or kit of parts may likewise be read on and be understood as suitable embodiments of medical uses of the invention.

Accordingly, the invention provides at least one nucleic acid (e.g. DNA or RNA), preferably RNA as defined in the first aspect for use as a medicament, a composition for use as a medicament, a polypeptide as defined for use as a medicament, a vaccine as defined for use as a medicament, and a kit or kit of parts for use as a medicament, wherein the composition of the invention, comprising the inventive lipid excipient(s), is used for delivering said nucleic acid.

The present invention furthermore provides several applications and uses of the nucleic acid, composition, polypeptide, vaccine, or kit, i.e. in particular, nucleic acid (preferably RNA), composition, polypeptide, vaccine, or kit may be used for human medical purposes and also for veterinary medical purposes, preferably for human medical purposes, wherein the composition of the invention, comprising the inventive lipid excipient(s), is used for delivering said nucleic acid.

In particular, nucleic acid (preferably RNA), composition, polypeptide, vaccine, or kit or kit of parts is for use as a medicament for human medical purposes, wherein said nucleic acid (preferably RNA), composition, polypeptide, vaccine, or kit or kit of parts may be suitable for young infants, newborns, immunocompromised recipients, as well as pregnant and breast-feeding women and elderly people. In particular, nucleic acid (preferably RNA, most preferably mRNA), composition, polypeptide, vaccine, or kit or kit of parts is for use as a medicament for human medical purposes, wherein said nucleic acid (preferably RNA, most preferably mRNA), composition, polypeptide, vaccine, or kit or kit of parts is particularly suitable for elderly human subjects.

Said nucleic acid (preferably RNA), composition, polypeptide, vaccine, or kit is for use as a medicament for human medical purposes, wherein said RNA, composition, vaccine, or the kit or kit of parts may be particularly suitable for intramuscular injection or intradermal injection.

In yet another aspect, the invention relates to the second medical use of the provided nucleic acid, composition, polypeptide, vaccine, or kit.

Accordingly, the invention provides at least one nucleic acid, wherein the nucleic acid is comprised in a composition of the invention, comprising the inventive lipid excipient(s) used for delivering said nucleic acid, preferably RNA, most preferably mRNA, for treatment or prophylaxis of an infection with a coronavirus, preferably a betacoronavirus, more preferably a severe acute respiratory syndrome coronavirus 2 (SARS-CoV-2), or a disorder or a disease related to such an infection, such as Coronavirus disease 2019 (COVID-19); a composition for treatment or prophylaxis of an infection with a coronavirus, preferably SARS-CoV-2 coronavirus, or a disorder or a disease related to such an infection, such as COVID-19; a polypeptide for treatment or prophylaxis of an infection with a coronavirus, preferably SARS-CoV-2 coronavirus, or a disorder or a disease related to such an infection, such as COVID-19; a vaccine for treatment or prophylaxis of an infection with a coronavirus, preferably SARS-CoV-2 coronavirus, or a disorder or a disease related to such an infection, such as COVID-19; a kit or kit of parts for treatment or prophylaxis of an infection with a coronavirus, preferably SARS-CoV-2 coronavirus, or a disorder or a disease related to such an infection, such as COVID-19.

In other embodiments, the nucleic acid, preferably RNA, most preferably mRNA, the composition, the polypeptide, the vaccine, or the kit or kit of parts is for use in the treatment or prophylaxis of an infection with a coronavirus, preferably with SARS-CoV-2 coronavirus, wherein the composition of the invention, comprising the inventive lipid excipient(s), is used for delivering said nucleic acid.

Particularly, the nucleic acid, preferably RNA, most preferably mRNA, the composition, the polypeptide, the vaccine, or the kit or kit of parts may be used in a method of prophylactic (pre-exposure prophylaxis or post-exposure prophylaxis) and/or therapeutic treatment of COVID-19 disease caused by a SARS-CoV-2 coronavirus infection, wherein the composition of the invention, comprising the inventive lipid excipient(s), is used for delivering said nucleic acid.

The nucleic acid, the composition, the polypeptide, or the vaccine may preferably be administered locally. In particular, composition or vaccines may be administered by an intradermal, subcutaneous, intranasal, or intramuscular route, wherein the composition of the invention, comprising the inventive lipid excipient(s), is used for delivering said nucleic acid. In other embodiments, said nucleic acid, composition, polypeptide, vaccine may be administered by conventional needle injection or needle-free jet injection. Preferred in that context is intramuscular injection.

In other embodiments, the nucleic acid as comprised in a composition of the invention, comprising the inventive lipid excipient(s), is used for delivering said nucleic acid as defined herein is provided in an amount of about 100 ng to about 500 µg, in an amount of about 1 µg to about 200 µg, in an amount of about 1 µg to about 100 µg, in an amount of about 5 µg to about 100 µg, preferably in an amount of about 10 µg to about 50 µg, specifically, in an amount of about 1 µg, 2 µg, 3 µg, 4 µg, 5 µg, 10 µg, 15 µg, 20 µg, 25 µg, 30 µg, 35 µg, 40 µg, 45 µg, 50 µg, 55 µg, 60 µg, 65 µg, 70 µg, 75 µg, 80 µg, 85 µg, 90 µg, 95 µg or 100 µg.

In one embodiment, the immunization protocol for the treatment or prophylaxis of a subject against coronavirus, preferably SARS-CoV-2 coronavirus comprises one single doses of the composition or the vaccine, wherein the composition of the invention, comprising the inventive lipid excipient(s), is used for delivering said nucleic acid.

In some embodiments, the effective amount is a dose of 1 µg, 2 µg, 3 µg, 4 µg, 5 µg, 6 µg, 7 µg, 8 µg, 9 µg, 10 µg, 11 µg, 12 µg, 13 µg, 14 µg, 15 µg, 16 µg, 20 µg, 30 µg, 40 µg, 50 µg, 75 µg, 100 µg or 200 µg administered to the subject in one vaccination, wherein the composition of the invention, comprising the inventive lipid excipient(s), is used for delivering said nucleic acid. In preferred embodiments, the immunization protocol for the treatment or prophylaxis of a coronavirus, preferably a SARS-CoV-2 coronavirus infection comprises a series of single doses or dosages, preferably a total of two doses, of the composition or the vaccine, wherein the composition of the invention, comprising the inventive lipid excipient(s), is used for delivering said nucleic acid. A single dosage, as used herein, refers to the initial/first dose, a second dose or any further doses, respectively, which are preferably administered in order to "boost" the immune reaction, wherein the composition of the invention, comprising the inventive lipid excipient(s), is used for delivering said nucleic acid.

In preferred embodiments, the vaccine/composition immunizes the subject against a coronavirus, preferably against a SARS-CoV-2 coronavirus infection (upon administration as defined herein) for at least 1 year, preferably at least 2 years, wherein for immunization the composition of the invention, comprising the inventive lipid excipient(s), is used for delivering said nucleic acid.

### Standard therapy

More preferably, the subject receiving the pharmaceutical composition or vaccine comprising RNAs of the invention, the combination thereof or the pharmaceutical composition or vaccine comprising said RNA(s) is a patient suffering from a tumor or cancer disease as described herein and who received or receives chemotherapy (e.g. first-line or second-line chemotherapy), radiotherapy, chemoradiotherapy / chemoradiation (combination of chemotherapy and radiotherapy), kinase inhibitors, antibody therapy and/or checkpoint modulators (e.g. CTLA4 inhibitors, PD1 pathway inhibitors), or a patient, who has achieved partial response or stable disease after having received one or more of the treatments specified above. More preferably, the subject is a patient suffering from a tumor or cancer disease as described herein and who received or receives a compound conventionally used in any of these diseases as described herein, more preferably a patient who receives or received a checkpoint modulator.

Compounds which preferably are used in standard therapies and which can be applied in combination with the pharmaceutical compositions or vaccines comprising RNAs of the invention include but are not limited to those disclosed on pages 56-58 in WO2018078053; WO2018078053 being incorporated herein by reference in its entirety.

### Tumor indications

As used herein, the terms "tumor", "cancer" or "cancer disease" refer to a malignant disease, which is preferably selected from, but not limited to, the group of malignant diseases disclosed on pages 58-59 in WO2018078053; WO2018078053 being incorporated herein by reference in its entirety.

### EXEMPLARY EMBODIMENTS

In the following, different embodiments of the invention are disclosed. It is intended herein, that each and every embodiment can be combined with each other, i.e. embodiment 1 may be combined with e.g. embodiment 4 or 14 or 24, or sub-embodiments like e.g. "embodiment 63.1". Also, several sets of different embodiments of the invention are disclosed within. It is also intended herein, that each and every embodiment stemming from a different set of embodiments can be combined with each other, i.e. embodiment 1 from the First Set of Embodiments may be combined with e.g. embodiment 5 or embodiment 25 from the Second Set of Embodiments. Also back references to, e.g. "embodiment 1" are intended to comprise also a back-reference to, e.g. sub-embodiment 1.1, 1.2, et cetera.

### First Set of Embodiments

Embodiment 1. A polymer conjugated lipid according to formula (I):

[P]-[linker]-[L] formula (I)

or a pharmaceutically acceptable salt, prodrug, tautomer or stereoisomer thereof, wherein
- [P]: is a homopolymer moiety comprising at least one polyoxazoline (POZ) monomer unit 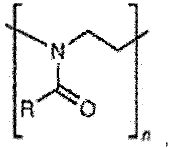 wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa
- [linker]: is an optional linker group, and
- [L]: is a lipid moiety.

Embodiment 2. The polymer conjugated lipid of **embodiment 1,** wherein [P] is a heteropolymer moiety or homopolymer moiety comprising multiple monomer units selected from the group consisting of
poly(2-methyl-2-oxazoline) (PMOZ) 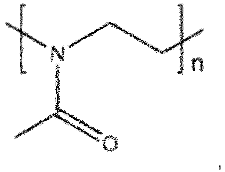
poly(2-ethyl-2-oxazoline) (PEOZ) 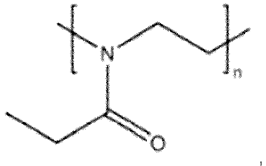
poly(2-propyl-2-oxazoline) (PPOZ) 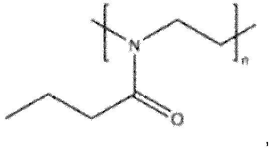
poly(2-butyl-2-oxazoline) (PBOZ) 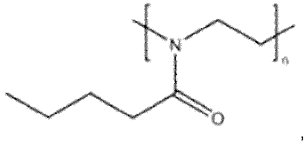
poly(2-isopropyl-2-oxazoline) (PIPOZ) 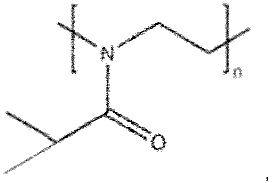
poly(2-methoxymethyl-2-oxazoline) (PMeOMeOx), and
poly(2-dimethylamino-2-oxazoline) (PDMAOx),

preferably wherein [P] is a homopolymer moiety comprising multiple PMOZ or PEOZ monomer units, more preferably wherein [P] comprises or preferably consists of multiple PMOZ monomer units,
wherein
   (i) n has a mean value ranging from about 45 to about 55, preferably n is about 50 or wherein
   (ii) n is selected such that the [P] moiety has an average molecular weight of about 4.2 kDa to about 4.4 kDa, or most preferably about 4.3 kDa.

Embodiment 3. The polymer conjugated lipid of any one of **embodiment 1 to embodiment 2,** wherein the polymer conjugated lipid is selected from the group consisting of a POZ-monoacylglycerol conjugate, POZ-diacylglycerol conjugate, a POZ-dialkyloxypropyl conjugate, a POZ-steroid or POZ-sterol conjugate, a POZ-phospholipid conjugate, a POZ-ceramide conjugate, and a mixture thereof.

Embodiment 4. The polymer conjugated lipid of any one of **embodiment 1 to embodiment 3,** wherein the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain,
wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH- ), succinyl (-(O)CCH₂CH₂C(O)-), succinamidyl (-NHC(O)CH₂CH₂C(O)NH-), (-NHC(O)CH₂CH₂C(O)-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl).

Embodiment 4.1 The polymer conjugated lipid of any one of **embodiment 1 to embodiment 3,** wherein
(i) the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain,
   wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH- ), succinyl (-(O)CCH₂CH₂C(O)-), succinamidyl (-NHC(O)CH₂CH₂C(O)NH-), (-NHC(O)CH₂CH₂C(O)-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl), or
(ii) the lipid moiety [L] comprises ditetradecylamin, preferably wherein the linker group [linker] is (-NHC(O)CH₂CH₂C(O)-).

Embodiment 5. The polymer conjugated lipid of any one of **embodiment 1 to embodiment 4,** wherein the lipid moiety [L] comprises at least one straight or branched, saturated or unsaturated alkyl chain comprising 6, 7, 8, 9, 10, 11, 12, 13, 14, 15, 16, 17, 18, 19, 20, 21, 22, 23, 24, 25, 26, 27, 28, 29, or 30 carbon atoms, preferably in the range of 10 to 20 carbon atoms, more preferably in the range of 12 to 18 carbon atoms, even more preferably 14, 16 or 18 carbon atoms, even more preferably 16 or 18 carbon atoms, most preferably 14 carbon atoms,
wherein all selections are independent of one another.

Embodiment 6. The polymer conjugated lipid of any one of **embodiment 1 to embodiment 5,** wherein the linker group [linker] is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), (-NHC(O)CH₂CH₂C(O)O-), -C(R5)=N-, - N=C(R⁵)-, -C(R⁵)=N-O-, -O-N=C(R⁵)-, -O-C(O)O-, -C(O)N(R⁵), -N(R⁵)C(O)-, - C(S)(NR⁵)-, (NR⁵)C(S)-, -N(R⁵)C(O)N(R⁵)-, -C(O)S-, -SC(O)-, -C(S)O-, -OC(S)-, - OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), and sulfonate esters, as well as combinations thereof, wherein R³, R⁴ and R⁵ are, independently H or alkyl (e.g. C₁-C₄ alkyl), preferably wherein the linker group [linker] is selected from the group consisting of (-NHC(O)CH2CH2C(O)-), a peptide bond or amid bond (-CO-NH-), (-NHC(O)CH2CH2C(O)O-), and -NH-CH2-.

In a further embodiment related to, but not limited to, multivalent linker groups (i.e. amido, succinamidyl (-NHC(O)CH₂CH₂C(O)NH-) and/or (-NHC(O)CH₂CH₂C(O)-), more than one lipid or alkyl chains are attached to said linker (e.g. -NHC(O)CH₂CH₂C(O)N[-L]₂).

Embodiment 7. The polymer conjugated lipid of any one of **embodiment 1 to embodiment 6,** wherein the linker group [linker] comprises an amide linker moiety, preferably an ester linker moiety, or wherein the linker group [linker] has the structure 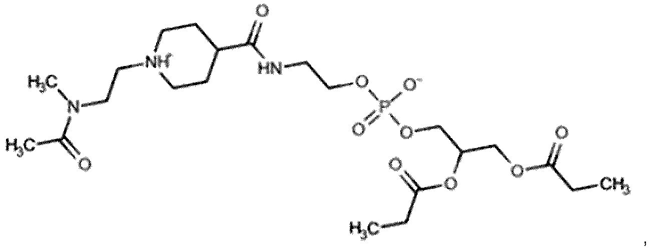 or 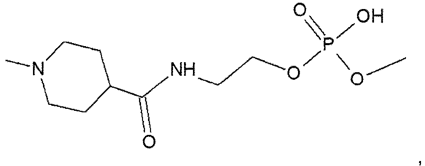 or preferably wherein the linker group comprises 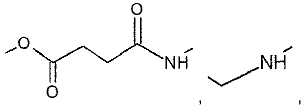 succinate, a peptide bond (-CO-NH-), an amine, or a secondary amine, more preferably wherein the linker group [linker] comprises (-NHC(O)CH₂CH₂C(O)-).

Embodiment 8. The polymer conjugated lipid of any one of **embodiment 1 to embodiment 7,** wherein the polymer conjugated lipid has the structure of
(i) 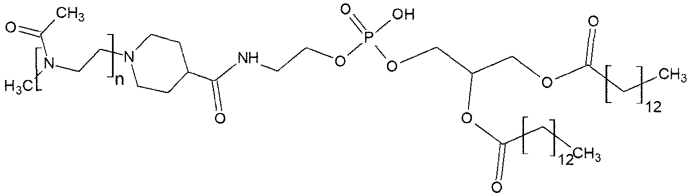 ("DMPE-PMOZ-v1")
   or preferably 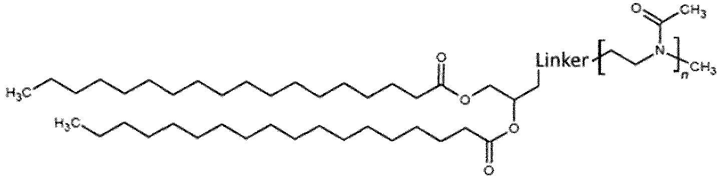 ; or 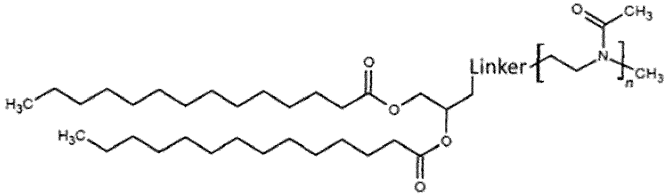 ; or 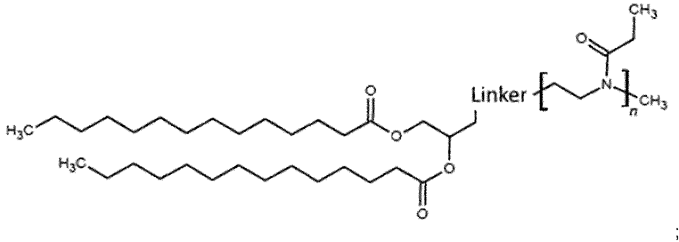 or 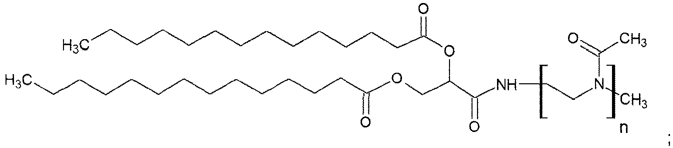 or 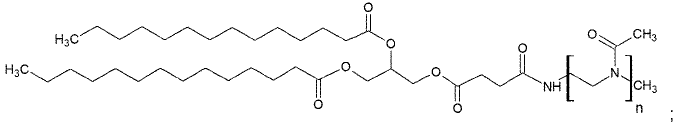 or 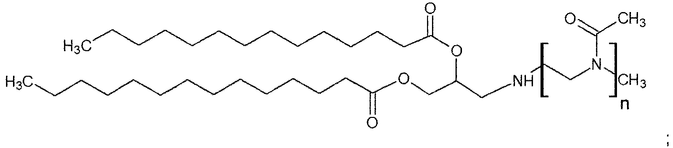 wherein the linker group [linker] is selected from any one of the linker groups as shown in **embodiment 6 or embodiment 7,** preferably the linker group [linker] comprising an ester moiety; or preferably
(ii) a "DMG-PMOZ" having the following structure: 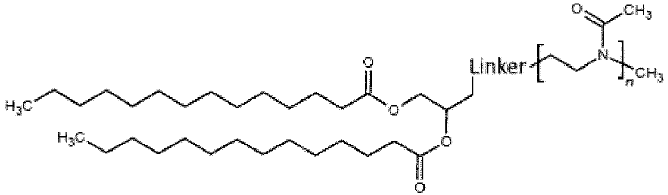 ("DMG-PMOZ"); or very preferably
(iii) "PMOZ 1", "PMOZ 2", "PMOZ 3", "PMOZ 5" or preferably "PMOZ 4" having the following structure 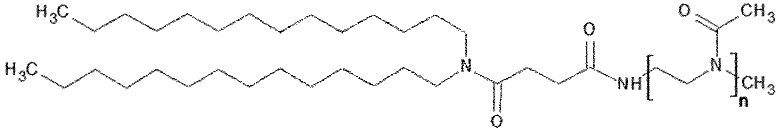 ["PMOZ 4"], preferably having 50 monomer repeats, i.e. 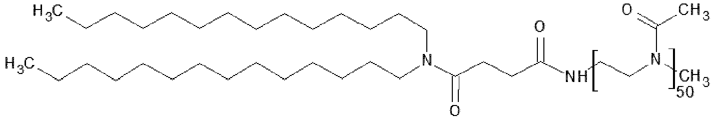 ["PMOZ 4" with 50 monomer repeats];
   whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa;
   most preferably wherein the polymer conjugated lipid of any one of **embodiment 1 to embodiment 7** is DMG-PMOZ with n having a mean value from 45 to 50, most preferably 50.

Embodiment 8.1 In a very preferred embodiment, the polymer conjugated lipid is "PMOZ 1", preferably whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa.

Embodiment 8.2 In a very preferred embodiment, the polymer conjugated lipid is "PMOZ 2", preferably whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa.

Embodiment 8.3 In a very preferred embodiment, the polymer conjugated lipid is "PMOZ 3", preferably whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa.

Embodiment 8.4 In a very preferred embodiment, the polymer conjugated lipid is "PMOZ 4", preferably whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa.

Embodiment 8.5 In a very preferred embodiment, the polymer conjugated lipid is "PMOZ 5", preferably whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa.

Embodiment 9. A lipid nanoparticle comprising a homopolymer moiety comprising at least one polyoxazoline (POZ) monomer unit 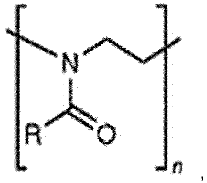
wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa,
preferably, wherein the homopolymer moiety comprising multiple monomer units comprises poly(2-methyl-2-oxazoline) (PMOZ), poly(2-ethyl-2-oxazoline) (PEOZ), poly(2-propyl-2-oxazoline) (PPOZ), poly(2-butyl-2-oxazoline) (PBOZ), poly(2-isopropyl-2-oxazoline) (PIPOZ), poly(2-methoxymethyl-2-oxazoline) (PMeOMeOx), or poly(2-dimethylamino-2-oxazoline) (PDMAOx),
more preferably the polymer conjugated lipid according to any one of **embodiment 1 to embodiment 8.**

Embodiment 10. The lipid nanoparticle of **embodiment 9,** wherein the lipid nanoparticle further comprises a cationic or ionizable lipid.

Embodiment 11. The lipid nanoparticle of **embodiment 9 to embodiment 10,** wherein the lipid nanoparticles
(i) do not comprise a polyethylene glycol-(PEG)-lipid conjugate or a conjugate of PEG and a lipid-like material, and preferably do not comprise PEG and/or
(ii) do not comprise a polymer conjugated lipid according to any one of **embodiment 1 to embodiment 8** comprising a sulphur group (-S-), a terminating nucleophile, and/or being covalently coupled to a biologically active ingredient being a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA.

Embodiment 11.1 The polymer conjugated lipid according to any one of **embodiment 1 to embodiment 8** not comprising a sulphur group (-S-).

Embodiment 11.2 The polymer conjugated lipid according to any one of **embodiment 1 to embodiment 8** not comprising a terminating nucleophile.

Embodiment 11.3 The polymer conjugated lipid according to any one of **embodiment 1 to embodiment 8** not being covalently coupled to a biologically active ingredient being a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA.

Embodiment 12. The lipid nanoparticle of any one of **embodiment 9 to embodiment 11,** wherein the cationic or ionizable lipid preferably carries a net positive charge at physiological pH, more preferably wherein the cationic or ionizable lipid comprises a tertiary nitrogen group or quaternary nitrogen group.

Embodiment 13. The lipid nanoparticle of any one of **embodiment 9 to embodiment 12,** wherein the lipid nanoparticle further comprises a phospholipid, wherein preferably the phospholipid is a zwitterionic compound selected from, but not limited to the group of 1,2-diphytanoyl-sn-glycero-3-phosphoethanolamine (DPhyPE; 1,2-di-(3,7,11,15-tetramethylhexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; dioleoylphosphatidylcholine), 1,2-Dipalmitoyl-sn-glycero-3-phosphocholine (DPPC; dipalmitoylphosphatidylcholine), 1,2-dioleoyl-sn-glycero-3-phosphoethanolamine (DOPE), phosphatidylethanolamines, distearoylphosphatidylcholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyloleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Dimyristoyl-sn-glycero-3-phosphoethanolamine (DMPE), 1,2-Dilinoleoyl-sn-glycero-3-phosphoethanolamine (DLoPE), distearoyl-phosphatidylethanolamine (DSPE), 1-Palmitoyl-2-oleoyl-sn-glycero-3-phosphoethanolamine (POPE), 1,2-Dilauroyl-sn-glycero-3-phosphoethanolamine (DLPE), 16-O-monomethylphosphoethanolamine, 16-O-dimethyl phosphatidylethanolamine, 1,2-Dierucoyl-sn-glycero-3-phosphoethanolamine (DEPE), 18-1-trans phosphatidylethanolamine, 1-stearoyl-2-oleoylphosphatidyethanolamine (SOPE), 1,2-Disqualeoyl-sn-glycero-3-phosphoethanolamine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodium salt), 1-O-hexadecanyl-2-O-(9Z-octadecenyl)-sn-glycero-3-phosphoethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesterylhemisuccinoyl-sn-glycero-3-phosphocholine (PChemsPC), 1,2-dicholesterylhemisuccinoyl-sn-glycero-3-phosphocholine (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine), preferably wherein the phospholipid is DSPC or DPhyPE.

Embodiment 14. The lipid nanoparticle of any one of **embodiment 9 to embodiment 13,** wherein the lipid nanoparticle further comprises a sterol or steroid, preferably selected from the group consisting of cholesterol, cholesteryl hemisuccinate (CHEMS) and a derivate thereof, preferably wherein the lipid nanoparticle further comprises cholesterol.

Embodiment 15. The lipid nanoparticle of any one of **embodiment 9 to embodiment 14,** wherein preferably the lipid nanoparticle comprises
(i) an amount of 1, 2, 3, 4, 5, 6, 7, 8, 9, or 10 mol% of the polymer conjugated lipid of any one of **embodiment 1 to embodiment 8;**
(ii) preferably an amount of 5 mol% of the polymer conjugated lipid of any one of **embodiment 1 to embodiment 8,**
(iii) more preferably an amount of 2.5 mol% of the polymer conjugated lipid of any one of **embodiment 1 to embodiment 8,** or
(iv) also preferably an amount of 1.7 mol% of the polymer conjugated lipid of any one of **embodiment 1 to embodiment 8**
based upon a mol-percentage of the composition of 100% of all lipid components or excipients.

Embodiment 16. The lipid nanoparticle of any one of **embodiment 9 to embodiment 15,** wherein the polymer conjugated lipid is a PMOZ-lipid according to any one of **embodiment 1 to embodiment 8.**

Embodiment 17. The lipid nanoparticle of any one of **embodiment 9 to embodiment 16,** wherein the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
(v) 47.4 mol% cationic or ionizable lipid, preferably one of the ionizable lipid structures of C1 to C27, more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;**
(vi) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8;**
(vii) 47.4 mol% formula III-3 (((4-hydroxybutyl)azanediyl)bis(hexane-6,1-diyl)bis(2-hexyldecanoate)), 40.9 mol% cholesterol, 10 mol% DSPC, and 1.7 mol% of the polymer conjugated lipid of any one of **embodiment 1 to embodiment 8;**
(viii) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% 2-[(PMOZ)]ₙ-N,N-ditetradecylacetamide]; and
(ix) 47.4 mol% formula III-3 (((4-hydroxybutyl)azanediyl)bis(hexane-6,1-diyl)bis(2-hexyldecanoate)), 40.9 mol% cholesterol, 10 mol% DSPC and 1.7 mol% 2-[(PMOZ)]ₙ-N,N-ditetradecylacetamide],
wherein n has a mean value ranging from about 45 to about 55, preferably n is about 50 or wherein n is selected such that the polymer moiety has an average molecular weight of about 4.2 kDa to about 4.4 kDa, or most preferably about 4.3 kDa.

Embodiment 17.1 The lipid nanoparticle of any one of **embodiment 9 to embodiment 16,** wherein the lipid nanoparticle comprises excipients selected from ratios selected from the group consisting of (i) about 48.5 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), about 38.9 mol% cholesterol, about 11.1 mol% neutral lipid and about 1.5 mol% of the polymer conjugated lipid of any one of **embodiment 1 to embodiment 8;**

Embodiment 17.2 The lipid nanoparticle of any one of **embodiment 9 to embodiment 16,** wherein the lipid nanoparticle comprises 59 mol% C24, 28.5 mol% cholesterol, 10 mol% DPhyPE and 2.5 mol% "PMOZ 4".

Embodiment 18. The lipid nanoparticle of any one of **embodiment 9 to embodiment 17,** wherein the polymer conjugated lipid of **embodiment 1 to embodiment 8** inhibits aggregation of the lipid nanoparticles.

Embodiment 19. The lipid nanoparticle of any one of **embodiment 9 to embodiment 18,** further comprising a biologically active ingredient.

Embodiment 20. The lipid nanoparticle of **embodiment 19,** wherein the biologically active ingredient is a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA; or any combination thereof, preferably wherein the biologically active ingredient is chemically modified mRNA or chemically unmodified mRNA, more preferably wherein the biologically active ingredient is chemically unmodified mRNA.

Embodiment 21. The lipid nanoparticle of any one of **embodiment 9 to embodiment 20,** wherein the mRNA is associated with the lipid nanoparticle, preferably wherein the mRNA is encapsulated in the lipid nanoparticle.

Embodiment 22. The lipid nanoparticle of any one of **embodiment 9 to embodiment 21,** wherein the lipid nanoparticles comprise the mRNA
(i) at an amount such as to achieve an N/P ratio in the range of about 1 to about 20, preferably about 2 to about 15, more preferably about 3 to about 10, even more preferably about 4 to about 9, most preferably about 6;
(ii) at an amount such as to achieve an N/P ratio in the range of about 5 to about 20, more preferably about 10 to about 18, even more preferably about 12 to about 16, most preferably about 14;
(iii) at an amount such as to achieve a lipid : mRNA weight ratio in the range of 20 to 60, preferably from about 3 to about 15, 5 to about 13, about 50 to about 70, about 4 to about 8 or from about 7 to about 11; or
(iv) at an amount such as to achieve an N/P ratio in the range of about 6 for a lipid nanoparticle according to **embodiment 17.**

Embodiment 23. The lipid nanoparticle of any one of **embodiment 9 to embodiment 22,** wherein the lipid nanoparticle is a sterile solid composition for reconstitution with a sterile liquid carrier, and wherein the lipid nanoparticle further comprises one or more inactive ingredients selected from pH-modifying agents, bulking agents, stabilizers, non-ionic surfactants and antioxidants, and wherein the sterile liquid carrier is an aqueous carrier.

Embodiment 24. The lipid nanoparticle of any one of **embodiment 9 to embodiment 23,** wherein the lipid nanoparticle is a sterile liquid composition, and wherein the lipid nanoparticles have a mean hydrodynamic diameter as determined by dynamic laser scattering from about 50 nm to about 300 nm, or from about 60 nm to about 250 nm, or from about 60 nm to about 200 nm, or from about 70 to 200 nm, or from about 75 nm to about 160, or from about 85 nm to about 140 nm, or from about 90 nm to about 130 nm, or from about 50 nm to about 120 nm.

Embodiment 25. The lipid nanoparticle of any one of **embodiment 9 to embodiment 24,** wherein the lipid nanoparticles exhibit a zeta potential in the range of -50 mV to +50 mV.

Embodiment 25.1 The lipid nanoparticle of any one of **embodiment 9 to embodiment 24,** wherein the lipid nanoparticles exhibit a zeta potential in the range of -5 mV to +5 mV.

Embodiment 26. The lipid nanoparticle of any one of **embodiment 9 to embodiment 25,** wherein the mRNA compound is a mono-, bi-, or multicistronic mRNA.

Embodiment 27. The lipid nanoparticle of any one **embodiment 9 to embodiment 26,** wherein the mRNA compound comprises at least one chemical modification.

Embodiment 28. The lipid nanoparticle of **embodiment 27,** wherein the chemical modification is selected from the group consisting of base modifications, sugar modifications, backbone modifications and lipid modifications, preferably wherein the chemical modification is a base modification, more preferably wherein the base modification preferably is selected from the group consisting of pseudouridine (psi or Ψ), N1-methylpseudouridine (N1MPU, N1Mpsi or N1Mψ), 1-ethylpseudouracil, 2-thiouracil (s2U), 4-thiouracil, 5-methylcytosine, 5-methyluracil, 5-methoxyuracil, and any combination thereof.

Embodiment 29. The lipid nanoparticle of any one of **embodiment 9 to embodiment 28,** wherein the mRNA compound comprises a coding region encoding a peptide or protein, wherein the coding region exhibits a sequence modification.

Embodiment 30. The lipid nanoparticle of **embodiment 29,** wherein the sequence modification is selected from a G/C content modification, a codon modification, a codon optimization or a C-optimization of the sequence; preferably wherein, compared with the coding region of the corresponding wild-type mRNA, the
- G/C content of the coding region is increased;
- C content of the coding region is increased;
- codon usage in the coding region is adapted to the human codon usage; and/or
- codon adaptation index (CAI) is increased or maximized in the coding region.

Embodiment 31. The lipid nanoparticle of any one of **embodiment 9 to embodiment 30,** wherein the mRNA compound further comprises
a) a 5'-CAP structure, preferably m7GpppN, more preferably CAP1 or m7G(5')ppp(5')(2'OMeA)pG;
b) optionally at least one miRNA sequence, preferably wherein the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof;
c) at least one 5'-UTR element;
d) a coding sequence;
e) at least one 3'-UTR element;
f) at least one poly(A) sequence;
g) at least one poly(C) sequence;
or any combinations of these.

Embodiment 32. The lipid nanoparticle of any one of **embodiment 9 to embodiment 31,** wherein the least one coding RNA comprises a 5'-CAP structure, preferably m7G, CAP0, CAP1, CAP2, a modified CAP0 or a modified CAP1 structure.

Embodiment 33. The lipid nanoparticle of any one of **embodiment 9 to embodiment 32,** wherein the at least one coding RNA comprises at least one heterologous 5'-UTR and/or at least one heterologous 3'-UTR, preferably wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes; and/or preferably wherein the at least one heterologous 3'-UTR comprises a nucleic acid sequence derived from a 3'-UTR of a gene selected from PSMB3, ALB7, alpha-globin, CASP1, COX6B1, GNAS, NDUFA1 and RPS9, or from a homolog, a fragment or a variant of any one of these genes.

Embodiment 34. The lipid nanoparticle of any one of **embodiment 9 to embodiment 33,** wherein the at least one coding RNA comprises a (i) HSD17B4 5'-UTR and a PSMB3 3'-UTR or (ii) a RPL32 5'-UTR and an ALB7 3'-UTR, preferably a mutated alpha-globin 3'-UTR (SEQ ID NO:11/12), more preferably a HSD17B4 5'-UTR (SEQ ID NO:21/22) and a PSMB3 3'-UTR (SEQ ID NO:19/20).

Embodiment 35. The lipid nanoparticle of any one of **embodiment 9 to embodiment 34,** comprising the following elements in the 5' to 3' direction:
a) a 5'-CAP structure, preferably selected from the group consisting of m7G(5'), m7G(5')ppp(5')(2'OMeA)pG and m7G(5')ppp(5')(2'OMeG)pG;
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, 24, 26, or a homolog, a fragment or a variant thereof, most preferably according to SEQ ID NO:22 (HSD17B4);
c) at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8, 10, 12, 14, 16, 18, 20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18, or a homolog, a fragment or a variant thereof, most preferably according to SEQ ID NO:20 (PSMB3);
e) optionally, at least one poly(A) sequence, preferably consisting of 10 to 200, 10 to 100, 40 to 80, or 50 to 70 adenosine nucleotides;
f) optionally, at least one poly(C) sequence, preferably consisting of 10 to 200, 10 to 100, 20 to 70, 20 to 60 or 10 to 40 cytosine nucleotides; and
g) optionally, at least one histone stem-loop, preferably comprising the RNA sequence according to SEQ ID NO:4.

Embodiment 35.1 The lipid nanoparticle of any one of **embodiment 9 to embodiment 34,** comprising the following elements in the 5' to 3' direction:
a) a 5'-CAP structure, preferably selected from the group consisting of m7G(5'), m7G(5')ppp(5')(2'OMeA)pG and m7G(5')ppp(5')(2'OMeG)pG;
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, 24, 26, or a homolog, a fragment or a variant thereof, most preferably according to SEQ ID NO:22 (HSD17B4);
c) at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8, 10, 12, 14, 16, 18, 20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18, or a homolog, a fragment or a variant thereof, most preferably according to SEQ ID NO:20 (PSMB3);
e) optionally a histone stem-loop preferably comprising the RNA sequence according to SEQ ID NO:4;
f) optionally, at least one poly(A) sequence, preferably consisting of 10 to 200, 10 to 100, 40 to 80, or 50 to 70 adenosine nucleotides, preferably 100 adenosine nucleotides.

Embodiment 36. The lipid nanoparticle of any one of **embodiment 9 to embodiment 35,** wherein the biologically active ingredient is
(a) an mRNA comprising at least one coding sequence encoding a peptide or protein, or a fragment or variant thereof, wherein the peptide or protein is an antigen, wherein the antigen preferably is derived from pathogenic antigens, tumor antigens, allergenic antigens or autoimmune self-antigens, or a fragment or variant thereof; or
(b) an mRNA comprising at least one coding sequence encoding a therapeutic protein, or a fragment or variant thereof, wherein the therapeutic protein is selected from the group consisting of
   (i) therapeutic proteins for use in enzyme replacement therapy for the treatment of metabolic, endocrine or amino acid disorders or for use in replacing an absent, deficient or mutated protein;
   (ii) therapeutic proteins for use in the treatment of blood disorders, diseases of the circulatory system, diseases of the respiratory system, infectious diseases or immune deficiencies;
   (iii) therapeutic proteins for use in the treatment of cancer or tumor diseases;
   (iv) therapeutic proteins for use in hormone replacement therapy;
   (v) therapeutic proteins for use in reprogramming somatic cells into pluri- or omnipotent stem cells;
   (vi) therapeutic proteins for use as adjuvant or immunostimulation;
   (vii) therapeutic proteins being a therapeutic antibody;
   (viii) therapeutic proteins being a gene editing agent; and
   (ix) therapeutic proteins for use in treating or preventing a liver disease selected from the group consisting of liver fibrosis, liver cirrhosis and liver cancer.

Embodiment 37. The lipid nanoparticle of **embodiment 36 sub-item (a),** wherein the at least one coding sequence encoding a pathogenic antigen is selected from the group consisting of a bacterial, viral, fungal and protozoal antigen.

Embodiment 38. The lipid nanoparticle of **embodiment 37,** wherein the at least one coding sequence encoding a pathogenic antigen
(i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
(ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
(iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof (e.g. receptor binding domain (RBD), critical neutralisation domain (CND)); and/or
(iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation.

Embodiment 39. The lipid nanoparticle of any one of **embodiment 9 to embodiment 38** for use
(i) in the treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition; and/or
(ii) for use in enzyme replacement therapy for the treatment of metabolic or endocrine disorders or for use in replacing an absent, deficient or mutated protein.

Embodiment 40. The lipid nanoparticle of any one of **embodiment 9 to embodiment 39** for use in the treatment or prophylaxis of infectious diseases.

Embodiment 41. The lipid nanoparticle of **embodiment 9 or embodiment 40** comprising at least one coding RNA, wherein said at least one coding RNA comprises at least one coding sequence encoding at least one peptide or protein for use in treatment or prevention of a disease, disorder or condition, wherein said lipid nanoparticle is administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, intraocular, periocular, periodontal, retrobulbar, subarachnoid, subconjunctival, suprachoroidal injection, infusion, implantation, nasal, buccal, sublingual, otic or auricular, ophthalmic, conjunctival, vaginal, rectal, intracervical, endosinusial, laryngeal, oropharyngeal, ureteral, urethral administration, more preferably said lipid nanoparticle is administered intramuscularly, intravenously, intradermally, subcutaneously, intratumorally, intranasally, or by inhalation, most preferably intramuscularly, to a subject in need thereof.

Embodiment 42. A kit or kit of parts, comprising any one of the lipid nanoparticle of **embodiment 9 to embodiment 41,** optionally comprising a liquid vehicle for solubilizing, and, optionally, technical instructions providing information on administration and dosage of the components.

Embodiment 43. The lipid nanoparticle of any one of **embodiment 9 to embodiment 41** or the kit or kit of parts of **embodiment 42** for use in *in vivo* drug delivery, preferably for use in delivering a nucleic acid, preferably a mRNA.

Embodiment 44. The lipid nanoparticle of any one of **embodiment 9 to embodiment 41** or the kit or kit of parts of **embodiment 43** for use as a medicament.

Embodiment 45. The lipid nanoparticle for use as a medicament according to **embodiment 44,** wherein the medicament is for the prevention, prophylaxis, treatment and/or amelioration of a disease selected from infectious diseases including viral, bacterial or protozoological infectious diseases, cancer or tumor diseases, liver diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and diseases associated with a peptide or protein deficiency.

Embodiment 46. The lipid nanoparticle for use as a medicament according to **embodiment 44 or embodiment 45,** wherein the medicament is a vaccine composition.

Embodiment 47. A vaccine composition comprising a lipid nanoparticle of any one of **embodiment 9 to embodiment 46** or a kit or kit of parts of **embodiment 42** for use as a medicament, and/or for prevention, prophylaxis, treatment and/or amelioration of a disease selected from infectious diseases including viral, bacterial or protozoological infectious diseases, cancer or tumor diseases.

Embodiment 48. A method of treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition comprising the steps:
a) providing a lipid nanoparticle of any one of **embodiment 9 to embodiment 45,** comprising a homopolymer moiety comprising at least one polyoxazoline (POZ) monomer, preferably the polymer conjugated lipid according to any one of **embodiment 1 to embodiment 8,** the vaccine composition of **embodiment 47,** or the kit or kit of parts of **embodiment 42;** and
b) applying or administering the mRNA, the lipid nanoparticle, the vaccine composition or the kit or kit of parts to a tissue or an organism.

Embodiment 49. A method for delivering mRNA encoding an antigen or a therapeutic peptide or protein to a subject, the method comprising administering to a subject a lipid nanoparticle of any one of embodiments 1 to 33, wherein the mRNA encodes an antigen or a therapeutic peptide or protein, and wherein delivering the mRNA to the subject is beneficial in treating or preventing a disease or disorder, preferably wherein the subject is a mammal, more preferably wherein the subject is a human.

Embodiment 50. The method according to any one of embodiments **embodiment 48 to embodiment 49,** wherein the mRNA, the lipid nanoparticle of any one of **embodiment 9 to embodiment 48,** the vaccine composition of **embodiment 47** or the kit or kit of parts of **embodiment 42** is administered to the tissue orto the organism by intravenous, intramuscular, subcutaneous, intradermal or intratumoral injection or any administration route as disclosed in any **preceding embodiment.**

Embodiment 51. A method of inducing an immune response in a subject, the method comprising administering to the subject the vaccine composition of **embodiment 47** in an amount effective to produce an antigen-specific immune response in the subject.

Embodiment 52. A pharmaceutical composition comprising a lipid nanoparticle of any one of **embodiment 9 to embodiment 48** or a kit or kit of parts of **embodiment 42** or the vaccine composition of **embodiment 47** for use in vaccination of a subject comprising an effective dose of mRNA encoding a virus antigen.

Embodiment 53. Use of a pharmaceutical composition according to **embodiment 52** or a kit or kit of parts according to **embodiment 42** for (i) inducing an immune response, for (ii) inducing an antigen specific T-cell response or preferably for (iii) inducing CD8+ T cells responses.

Embodiment 54. Use of the pharmaceutical composition of **embodiment 52** for the prophylaxis of an infectious disease or in the manufacture of a medicament for the prophylaxis of an infectious disease, wherein said medicament preferably is a vaccine composition.

Embodiment 55. A method for preventing, ameliorating or treating a disease or condition in a subject in need comprising administering to the subject a lipid nanoparticle of any one of **embodiment 9 to embodiment 48,** a pharmaceutical composition of **embodiment 52** or a kit or kit of parts of **embodiment 42.**

Embodiment 56. The method of any one of the **preceding method embodiments,** wherein administration of the lipid nanoparticle results in expression of the antigen encoded by mRNA in the lymphocytes of the subject.

Embodiment 57. A method of treating or preventing a disorder of any one of **embodiments 36, 39, 41, 45, 48, or 49,** wherein the disorder is an infection with coronavirus, or a disorder related to such an infection.

Embodiment 58. A method of treating or preventing a disorder of any one of **embodiments 36, 39, 41, 45, 48, or 49,** wherein the subject in need is a mammalian subject, preferably a human subject.

Embodiment 59. The method of any one of the **preceding method embodiments,** wherein the administration of the lipid nanoparticle results in an antigen specific antibody response, preferably wherein the antigen specific antibody response is measured by the presence of antigen-specific antibodies in serum.

Embodiment 60. The lipid nanoparticle of any one of **embodiment 9 to embodiment 16,** wherein the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;** and
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8.**

Embodiment 60.1 The lipid nanoparticle of any one of **embodiment 9 to embodiment 16,** wherein the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;**
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8;**
(vii) 47.4 mol% formula III-3 (((4-hydroxybutyl)azanediyl)bis(hexane-6,1-diyl)bis(2-hexyldecanoate)), 40.9 mol% cholesterol, 10 mol% DSPC, and 1.7 mol% of the polymer conjugated lipid of any one of **claim 1 to claim 8;**
(viii) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% 2-[(PMOZ)]n-N,N-ditetradecylacetamide];
(ix) 47.4 mol% formula III-3 (((4-hydroxybutyl)azanediyl)bis(hexane-6,1-diyl)bis(2-hexyldecanoate)), 40.9 mol% cholesterol, 10 mol% DSPC and 1.7 mol% 2-[(PMOZ)]n-N,N-ditetradecylacetamide]; and most preferably
(x) 59 mol% C24, 28.5 mol% cholesterol, 10 mol% DPhyPE and 2.5 mol% "PMOZ 4",
wherein n of the polymer-conjugated lipid has a mean value ranging from about 45 to about 55, preferably n is about 50 or wherein n is selected such that the polymer moiety has an average molecular weight of about 4.2 kDa to about 4.4 kDa, or most preferably about 4.3 kDa.

Embodiment 61. The lipid nanoparticle of any one of **embodiment 9 to embodiment 16 or embodiment 60,** wherein the lipid nanoparticle comprises a neutral lipid or phospholipid having at least one alkyl chain with a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₆, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).

Embodiment 62. The lipid nanoparticle of any one of **embodiment 9 to embodiment 16 or embodiment 60 to embodiment 61,** wherein the lipid nanoparticles comprise a neutral lipid or phospholipid having at least two alkyl chains, whereby each alkyl chain independently has a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₆, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).

Embodiment 63. The lipid nanoparticle of any one of **embodiment 9 to embodiment 16 or embodiment 60 to embodiment 62** or a lipid nanoparticle comprising a polymer conjugated lipid according to any one of **embodiment 1** to **embodiment 8,** wherein the lipid nanoparticle has a lower PDI and/or lower size as compared to a control lipid nanoparticle comprising a PEG-lipid instead of a polymer conjugated lipid according to any one of **embodiment 1** to **embodiment 8.**

### Second Set of Embodiments

All SEQ ID NO-references which are shown in the second set of embodiments are related to the SEQ ID NO from PCT application number PCT/EP2021/074342 or the subsequent patent application claiming the priority of PCT/EP2021/074342, the sequence listing of which is incorporated herein by reference.

Embodiment 1. A vaccine composition comprising
a) at least one nucleic acid encoding at least one antigen or fragment or variant thereof; and
b) a carrier composition, wherein the carrier composition comprises the phospholipid phosphatidylserine.

Embodiment 2. The vaccine composition according to Embodiment 1, wherein the at least one nucleic acid is not a tolerogenic nucleic acid; and/or wherein the at least one nucleic acid does not encode a tolerogenic polypeptide; and/or wherein the vaccine composition does not comprise an antigen or fragment or variant thereof; and/or wherein the vaccine composition comprises the at least one nucleic acid as the sole payload; and/or wherein the vaccine composition is not a tolerogenic composition.

Embodiment 3. The vaccine composition according to Embodiment 1 or 2, wherein the carrier composition at least partly encapsulates the at least one nucleic acid.

Embodiment 4. The vaccine composition according to any one of Embodiments 1 to 3, wherein the carrier composition encapsulates the at least one nucleic acid.

Embodiment 5. The vaccine composition according to any one of Embodiments 1 to 4, wherein the carrier composition comprises an inner surface and an outer surface facing the outside, wherein the phosphatidylserine is located at the outer surface of the carrier composition.

Embodiment 6. The vaccine composition according to Embodiment 5, wherein the hydrophilic head group of the phosphatidylserine is located at the outer surface of the carrier composition.

Embodiment 7. The vaccine composition according to any one of Embodiments 1 to 6, wherein the hydrophilic head group of the phosphatidylserine comprised in the carrier composition is accessible from the outside of the carrier composition.

Embodiment 8. The vaccine composition according to any one of Embodiments 1 to 7, wherein the phosphatidylserine is selected from the group consisting of DPhyPS, WT-PS, 16:0-PS, 14:0-PS, 10:0-PS, 6:0-PS, 18:1-PS DOPS, 18:1-Lyso PS and 18:0-Lyso PS.

Embodiment 9. The vaccine composition according to any one of Embodiments 1 to 8, wherein the carrier composition is a lipid nanoparticle composition.

Embodiment 10. The vaccine composition according to Embodiment 9, wherein the lipid nanoparticle composition further comprises
(i) a cationic or ionizable lipid; and/or
(ii) a steroid; and/or
(iii) a further phospholipid in addition to phosphatidylserine; and/or
(iv) a polymer conjugated lipid.

Embodiment 11. The vaccine composition according to Embodiment 9 or 10, wherein the lipid nanoparticle composition further comprises
(i) a cationic or ionizable lipid;
(ii) a steroid;
(iii) a further phospholipid in addition to phosphatidylserine; and
(iv) a polymer conjugated lipid.

Embodiment 12. The vaccine composition according to Embodiment 10 or 11, wherein the cationic or ionizable lipid carries a net positive charge at physiological pH, preferably wherein the cationic or ionizable lipid comprises a tertiary nitrogen group or quaternary nitrogen group, more preferably wherein the cationic or ionizable lipid is selected from the group consisting of HEXA1, HEXA2 and THIOETHER with the structures shown in Figure 1A, 1B and 1C, respectively.

Embodiment 13. The vaccine composition according to any one of Embodiments 10 to 12, wherein the steroid is selected from the group consisting of cholesterol, cholesteryl hemisuccinate (CHEMS) and a derivate thereof, preferably wherein the steroid is cholesterol.

Embodiment 14. The vaccine composition according to any one of Embodiments 10 to 13, wherein the further phospholipid is selected from the group consisting of 1,2-diphytanoyl-sn-glycero-3-phosphoethanolamine (DPhyPE; 1,2-di-(3,7,11,15-tetra¬methyl¬hexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; dioleoyl-phosphatidyl¬choline), 1,2-Di-palmitoyl-sn-glycero-3-phosphocholine (DPPC; dipalmitoylphosphatidylcholine), 1,2-dioleoyl-sn-glycero-3-phospho¬¬ethanolamine (DOPE), phosphatidylethanolamines, distearoyl¬phosphatidyl¬cholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyl¬oleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Di¬myristoyl-sn-glycero-3-phospho-ethanol¬amine (DMPE), 1,2-Dilinoleoyl-sn-glycero-3-phosphoethanolamine (DLoPE), distearoyl-phosphatidyl¬ethanol¬amine (DSPE), 1-Palmitoyl-2-oleoyl-sn-glycero-3-phosphoethanolamine (POPE), 1,2-Di-lauroyl-sn-glycero-3-phosphoethanolamine (DLPE), 16-O-monomethylphosphoethanolamine, 16-O-dimethyl phosphatidy¬l¬ethanolamine, 1,2-Dierucoyl-sn-glycero-3-phosphoethanolamine (DEPE), 18-1-trans phosphatidyl¬¬¬ethanolamine, 1-stearoyl-2-oleoylphosphatidyethanolamine (SOPE), 1,2-Disqualeoyl-sn-glycero-3-phospho¬ethanol¬amine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodium salt), 1-O-hexadecanyl-2-O-(9Z-octadecenyl)-sn-glycero-3-phospho-ethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesteryl¬hemisuccinoyl-sn-glycero-3-phosphocholine (PChemsPC), 1,2-dicholesterylhemisuccinoyl-sn-glycero-3-phospho-choline (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine), preferably wherein the further phospholipid is DPhyPE; and wherein the phospholipid, preferably DPhyPE, is optionally present in combination with a phospholipid having at least two alkyl chains, wherein each alkyl chain independently has a length of preferably C6, C7, C8, C9, or C10, more preferably a length of C6, C7, or C8, most preferably a length of C7, further most preferably a phospholipid selected from the group consisting of DHPC (1,2-diheptanoyl-sn-glycero-3-phosphocholine), 05:0 PC (1,2-dipentanoyl-sn-glycero-3-phosphocholine), 04:0 PC (1,2-dibutyryl-sn-glycero-3-phosphocholine), 06:0 PC (1,2-dihexanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), with DHPC being most preferred as the optionally present phospholipid having at least two alkyl chains.

Embodiment 15. The vaccine composition according to any one of Embodiments 10 to 14, wherein the polymer conjugated lipid is a polymer conjugated lipid as described above or below, preferably a lipid selected from PMOZ 1, PMOZ 2, PMOZ 3, PMOZ 4 and PMOZ 5, wherein n has a mean value ranging from 2 to 200, ranging from about 40 to about 80, preferably from about 45 to about 70, more preferably from about 50 to about 60, or most preferably n having a mean value of about 50.

Embodiment 16. The vaccine composition according to any one of Embodiments 11 to 15, wherein the composition comprises excipients in a ratio selected from the group consisting of
(a-i) the cationic lipid at an amount of 30-70 mol%; the steroid at an amount of 20-50 mol%; the phospholipid at an amount of 5-25 mol%; and the polymer conjugated lipid at an amount of 0.5-5 mol%;
(a-ii) the cationic lipid at an amount of 40-60 mol%; the steroid at an amount of 20-40 mol%; the phospholipid at an amount of 10-20 mol%; and the polymer conjugated lipid at an amount of 1-2 mol%;
(a-iii) the cationic lipid of Embodiment 12 at an amount of 30-70 mol%; the steroid of Embodiment 13 at an amount of 20-50 mol%; the phospholipid phosphatidylserine and the phospholipid of Embodiment 14 at an amount of 5-25 mol%; and the polymer conjugated lipid of Embodiment 15 at an amount of 0.5-5 mol%; and
(a-iv) the cationic lipid of Embodiment 12 at an amount of 40-60 mol%; the steroid of Embodiment 13 at an amount of 20-40 mol%; the phospholipid phosphatidylserine and the phospholipid of Embodiment 14 at an amount of 10-20 mol%; and the polymer conjugated lipid of Embodiment 15 at an amount of 1-2 mol%; preferably the composition comprising excipients in a ratio selected from the group consisting of
(b-i) the cationic lipid at an amount of 59 mol%; the steroid at an amount of 29.3 mol%; the phospholipid at an amount of 10 mol%; and the polymer conjugated lipid at an amount of 1.7 mol%;
(b-ii) the cationic lipid at an amount of 58 mol%; the steroid at an amount of 29.3 mol%; the phospholipid at an amount of 11 mol%; and the polymer conjugated lipid at an amount of 1.7 mol%;
(b-iii) the cationic lipid at an amount of 49 mol%; the steroid at an amount of 29.3 mol%; the phospholipid at an amount of 20 mol%; and the polymer conjugated lipid at an amount of 1.7 mol%;
(b-iv) the cationic lipid of Embodiment 12 at an amount of 59 mol%; the steroid of Embodiment 13 at an amount of 29.3 mol%; the phospholipid phosphatidylserine and the phospholipid of Embodiment 14 at an amount of 10 mol%; and the polymer conjugated lipid of Embodiment 15 at an amount of 1.7 mol%;
(b-v) the cationic lipid of Embodiment 12 at an amount of 58 mol%; the steroid of Embodiment 13 at an amount of 29.3 mol%; the phospholipid phosphatidylserine and the phospholipid of Embodiment 14 at an amount of 11 mol%; and the polymer conjugated lipid of Embodiment 15 at an amount of 1.7 mol%; and
(b-vi) the cationic lipid of Embodiment 12 at an amount of 49 mol%; the steroid of Embodiment 13 at an amount of 29.3 mol%; the phospholipid phosphatidylserine and the phospholipid of Embodiment 14 at an amount of 20 mol%; and the polymer conjugated lipid of Embodiment 15 at an amount of 1.7 mol%;
more preferably the composition comprising excipients in a ratio selected from the group consisting of (c-i) the cationic lipid of Embodiment 12 at an amount of 59 mol%; the steroid of Embodiment 13 at an amount of 29.3 mol%; the phospholipid phosphatidylserine at an amount of 5 mol% and DPhyPE at an amount of 5 mol%; and the polymer conjugated lipid of Embodiment 15 at an amount of 1.7 mol%;
(c-ii) the cationic lipid of Embodiment 12 at an amount of 59 mol%; the steroid of Embodiment 13 at an amount of 29.3 mol%; the phospholipid phosphatidylserine at an amount of 2 mol% and DPhyPE at an amount of 8 mol%; and the polymer conjugated lipid of Embodiment 15 at an amount of 1.7 mol%;
(c-iii) the cationic lipid of Embodiment 12 at an amount of 58 mol%; the steroid of Embodiment 13 at an amount of 29.3 mol%; the phospholipid phosphatidylserine at an amount of 5 mol%, DPhyPE at an amount of 5 mol% and DHPC at an amount of 1 mol%; and the polymer conjugated lipid of Embodiment 15 at an amount of 1.7 mol%;
(c-iv) the cationic lipid of Embodiment 12 at an amount of 49 mol%; the steroid of Embodiment 13 at an amount of 29.3 mol%; the phospholipid phosphatidylserine at an amount of 5 mol%, DPhyPE at an amount of 5 mol% and DHPC at an amount of 10 mol%; and the polymer conjugated lipid of Embodiment 15 at an amount of 1.7 mol%.

Embodiment 17. The vaccine composition according to any one of the preceding Embodiments, wherein the at least one nucleic acid is DNA or RNA.

Embodiment 18. The vaccine composition according to Embodiment 17, wherein the at least one nucleic acid is RNA, preferably mRNA comprising a coding sequence encoding the at least one antigen or fragment or variant thereof and optionally a coding sequence encoding at least one self-amplifying enzyme.

Embodiment 19. The vaccine composition according to Embodiment 18, wherein the lipid nanoparticles comprise the mRNA
(i) at an amount such as to achieve an N/P ratio in the range of 10 to 20, preferably about 2 to about 15, more preferably about 3 to about 10, even more preferably about 4 to about 9, most preferably about 6; or
(ii) at an amount such as to achieve an N/P ratio in the range of about 5 to about 20, more preferably about 10 to about 18, even more preferably about 12 to about 16, most preferably about 14; and/or
(iii) at an amount such as to achieve a lipid : mRNA weight ratio in the range of about 20 to about 60, preferably from about 3 to about 15, about 5 to about 13, about 4 to about 8 or from about 7 to about 11; and/or wherein the lipid nanoparticles have a mean hydrodynamic diameter as determined by dynamic laser scattering from about 50nm to about 300nm, or from about 60nm to about 250nm, or from about 60nm to about 200nm, or from about 70nm to 200nm, or from about 75nm to about 160nm, or from about 90nm to about 140nm, or from about 100nm to about 140nm; and/or
wherein the lipid nanoparticles exhibit a zeta potential in the range of -50 mV to +50 mV, preferably in the range of -25 mV to +25 mV, more preferably in the range of -10 mV to +10 mV, most preferably in the range of -5 mV to +5 mV.

Embodiment 20. The vaccine composition according to Embodiment 18 or 19, wherein the mRNA is a mono-, bi-, or multicistronic mRNA.

Embodiment 21. The vaccine composition according to any one of Embodiments 18 to 20, wherein the mRNA comprises at least one chemical modification.

Embodiment 22. The vaccine composition according to Embodiment 21, wherein the chemical modification is selected from the group consisting of base modifications, sugar modifications, backbone modifications and lipid modifications, preferably wherein the chemical modification is a base modification, more preferably wherein the base modification preferably is selected from the group consisting of pseudouridine (psi or ψ), N1-methylpseudouridine (N1MPU, N1Mpsi or N1Mψ), 1-ethylpseudouracil, 2-thiouracil (s2U), 4-thiouracil, 5-methylcytosine, 5-methyluracil, 5-methoxyuracil, and any combination thereof.

Embodiment 23. The vaccine composition according to any one of Embodiments 18 to 22, wherein the coding sequence exhibits a sequence modification.

Embodiment 24. The vaccine composition according to Embodiment 23, wherein the sequence modification is selected from a G/C content modification, a codon modification, a codon optimization or a C-optimization of the sequence; preferably wherein, compared with the coding sequence of the corresponding wild-type mRNA, the
a) G/C content of the coding sequence is increased;
b) C content of the coding sequence is increased;
c) codon usage in the coding sequence is adapted to the human codon usage; and/or
d) codon adaptation index (CAI) is increased or maximised in the coding sequence.

Embodiment 25. The vaccine composition according to any one of Embodiments 18 to 24, wherein the mRNA further comprises
a) a 5'-CAP structure, preferably m7GpppN, more preferably CAP1 or m7G(5')ppp(5')(2'OMeA)pG;
b) at least one miRNA binding site sequence, preferably wherein the microRNA binding site is for a microRNA selected from the group consisting of a miR-126, miR-142, miR-144, miR-146, miR-150, miR-155, miR-16, miR-21, miR-223, miR-24, miR-27, miR-26a binding site, preferably a miR-122 or miR-142 binding site, or any combination of the aforementioned miRNA binding sites thereof;
c) at least one 5'-UTR element;
d) at least one 3'-UTR element;
e) at least one poly(A) sequence;
f) at least one poly(C) sequence;
g) optionally, a histone stem-loop selected from SEQ ID NO:3 or 4;
h) optionally, a 3'-terminal sequence element selected from SEQ ID NO:41-70;
or any combinations of these.

Embodiment 26. The vaccine composition according to any one of Embodiments 18 to 25, wherein the mRNA comprises a 5'-CAP structure, preferably m7G, CAP0, CAP1, CAP2, a modified CAP0 or a modified CAP1 structure.

Embodiment 27. The vaccine composition according to Embodiment 25, wherein the at least one coding RNA comprises at least one heterologous 5'-UTR and/or at least one heterologous 3'-UTR, preferably wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes; and/or preferably wherein the at least one heterologous 3'-UTR comprises a nucleic acid sequence derived from a 3'-UTR of a gene selected from PSMB3, ALB/albumin, alpha-globin, CASP1 (preferably SEQ ID NO:81 (DNA) or SEQ ID NO:82 (RNA)), COX6B1 (preferably SEQ ID NO:83 (DNA) or SEQ ID NO:84 (RNA)), GNAS (preferably SEQ ID NO:85 (DNA) or SEQ ID NO:86 (RNA)), NDUFA1 (preferably SEQ ID NO:87 (DNA) or SEQ ID NO:88 (RNA)) and RPS9 (preferably SEQ ID NO:79 (DNA) or SEQ ID NO:80 (RNA)), or from a homolog, a fragment or a variant of any one of these genes.

Embodiment 28. The vaccine composition according to Embodiment 27, wherein the at least one coding RNA comprises a (i) HSD17B4 5'-UTR and a PSMB3 3'-UTR or (ii) a RPL32 5'-UTR and an ALB/albumin 3'-UTR, preferably a mutated alpha-globin 3'-UTR (SEQ ID NO:11, 12), more preferably a HSD17B4 5'-UTR (SEQ ID NO:21, 22) and a PSMB3 3'-UTR (SEQ ID NO:19, 20).

Embodiment 29. The vaccine composition according to any one of Embodiments 18 to 24, wherein the mRNA comprises the following elements in the 5' to 3' direction:
a) a 5'-CAP structure, preferably selected from the group consisting of m7G(5'), m7G(5')ppp(5')(2'OMeA) and m7G(5')ppp(5')(2'OMeG);
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, SEQ ID NO:24, SEQ ID NO:26 or preferably SEQ ID NO:77/78 (SLC7A3) or SEQ ID NO:75/76 (RPL31), or a homolog, a fragment or a variant thereof; most preferably according to SEQ ID NO:22 (HSD17B4);
c) the at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8 or SEQ ID NO:10, 12, 14, 16, 18, or preferably SEQ ID NO:20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18 (ALB/albumin) or preferably SEQ ID NO:79/80 (RPS9), or a homolog, a fragment or a variant thereof; most preferably according to SEQ ID NO:20 (PSMB3);
e) optionally, at least one poly(A) sequence, preferably consisting of 10 to 200, 10 to 100, 40 to 80, or 50 to 70 adenosine nucleotides, more preferably at least 70 adenosine nucleotides, even more preferably about 100 adenosine nucleotides;
f) optionally, at least one poly(C) sequence, preferably consisting of 10 to 200, 10 to 100, 20 to 70, 20 to 60 or 10 to 40 cytosine nucleotides; and
g) optionally, at least one histone stem-loop, preferably comprising the RNA sequence according to SEQ ID NO:4.

Embodiment 30. The vaccine composition according to any one of the preceding Embodiments, wherein the antigen is derived from a pathogenic antigen, a tumour antigen, an allergenic antigen or an autoimmune self-antigen.

Embodiment 31. The vaccine composition according to Embodiment 30, wherein the pathogenic antigen is selected from the group consisting of a bacterial antigen, a viral antigen, a fungal antigen and a protozoal antigen.

Embodiment 32. The vaccine composition according to Embodiment 30 or 31, wherein the pathogenic antigen
(i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus (RABV), Respiratory Syncytial virus (RSV), Rhinovirus, Rotavirus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
(ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
(iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof; and/or
(iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation.

Embodiment 33. A pharmaceutical composition comprising the vaccine composition according to any one of Embodiments 30 to 32 and a pharmaceutically acceptable carrier, diluent or excipient, preferably wherein the pharmaceutical composition is a sterile solid composition for reconstitution with a sterile liquid carrier, and wherein the composition further comprises one or more inactive ingredients selected from pH-modifying agents, bulking agents, stabilizers, non-ionic surfactants and antioxidants, and wherein the sterile liquid carrier is an aqueous carrier.

Embodiment 34. The vaccine composition according to any one of Embodiments 30 to 32 or the pharmaceutical composition according to Embodiment 33 for use in the treatment or prophylaxis of infectious diseases; cancer or tumor diseases, disorders or conditions; liver diseases selected from the group consisting of liver fibrosis, liver cirrhosis and liver cancer; allergies; or autoimmune disease, disorder or condition; in a subject.

Embodiment 35. The vaccine composition according to Embodiment 32 or a pharmaceutical composition comprising the vaccine composition according to Embodiment 32 for use in the treatment or prophylaxis of infectious diseases including viral, bacterial or protozoological infectious diseases in a subject.

Embodiment 36. The vaccine composition and the pharmaceutical composition for use according to Embodiment 34 or 35, wherein the vaccine composition or pharmaceutical composition is administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, intraocular, periocular, periodontal, retrobulbar, subarachnoid, subconjunctival, suprachoroidal injection, infusion, implantation, nasal, buccal, sublingual, otic or auricular, ophthalmic, conjunctival, vaginal, rectal, intracervical, endosinusial, laryngeal, oropharyngeal, ureteral, urethral administration, more preferably said lipid nanoparticle is administered intramuscularly, intravenously, intradermally, subcutaneously, intratumorally, intranasally, or by inhalation to a subject, preferably via local or locoregional injection or infusion to a subject.

Embodiment 37. A kit or kit of parts, comprising the vaccine composition according to any one of Embodiments 30 to 32 or the pharmaceutical composition according to Embodiment 33, optionally comprising a liquid vehicle for solubilizing, and, optionally, technical instructions providing information on administration and dosage of the components.

Embodiment 38. A method of treatment or prophylaxis of infectious diseases; cancer or tumor diseases, disorders or conditions; liver diseases selected from the group consisting of liver fibrosis, liver cirrhosis and liver cancer; allergies; or autoimmune disease, disorder or condition; in a subject comprising the steps:
a) providing the vaccine composition of any one of Embodiments 30 to 32 or the pharmaceutical composition according to Embodiment 33 or the kit or kit of parts according to Embodiment 37; and
b) applying or administering the vaccine composition or the pharmaceutical composition or the kit or kit of parts to a tissue or an organism of the subject.

Embodiment 39. A method of inducing an immune response in a subject, the method comprising administering to the subject the vaccine composition of any one of Embodiments 1 to 32 or the pharmaceutical composition of Embodiment 33 in an amount effective to produce an antigen-specific immune response in the subject.

Embodiment 40. A method of targeting a vaccine composition comprising a) at least one nucleic acid, preferably mRNA, encoding at least one antigen or fragment or variant thereof; and b) a carrier composition, preferably a lipid nanoparticle composition, to antigen-presenting cells including dendritic cells and macrophages, and/or to the spleen, the method comprising administering to the subject the vaccine composition of any one of Embodiments 1 to 32 or the pharmaceutical composition of Embodiment 33.

Embodiment 41. Use of a vaccine composition of any one of Embodiments 1 to 32 or the pharmaceutical composition according to Embodiment 33 or the kit or kit of parts according to Embodiment 37 for (i) inducing an immune response, for (ii) inducing an antigen specific T-cell response, preferably for (iii) inducing CD8+ T cells responses, and/or for (iv) targeting the vaccine composition or the pharmaceutical composition to antigen-presenting cells, including dendritic cells and macrophages, and/or to the spleen, in a subject.

Embodiment 42. Use of phosphatidylserine in the carrier composition of a vaccine composition comprising a) at least one nucleic acid, preferably mRNA, encoding at least one antigen or fragment or variant thereof; and b) a carrier composition, preferably a lipid nanoparticle composition, for targeting the vaccine composition to antigen-presenting cells, including dendritic cells and macrophages, and/or to the spleen, in a subject.

Embodiment 43. The vaccine composition or the pharmaceutical composition for use according to Embodiment 34 or 35, the method according to Embodiment 38, 39 or 40, or the use according to Embodiment 41 or 42, wherein the subject is a mammalian subject, preferably a human subject.

### Third Set of Embodiments

Embodiment 1. A polymer conjugated lipid according to formula (I):

[P]-[linker]-[L] formula (I)

or a pharmaceutically acceptable salt, prodrug, tautomer or stereoisomer thereof, wherein
- [P]: is a homopolymer moiety comprising at least one polyoxazoline (POZ) monomer unit 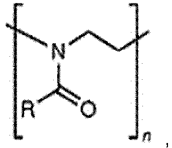 wherein R is C1-9 alkyl or C2-9 alkenyl, preferably C1 or C2 alkyl, and n has a mean value ranging from 2 to 200, preferably from 20 to 100, more preferably from 24 to 26 or 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa
- [linker]: is an optional linker group, and
- [L]: is a lipid moiety.

Embodiment 2. The polymer conjugated lipid of **embodiment 1,** wherein [P] is a heteropolymer moiety or homopolymer moiety comprising multiple monomer units selected from the group consisting of
poly(2-methyl-2-oxazoline) (PMOZ) 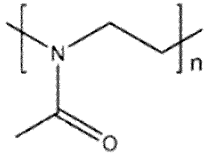 ,
poly(2-ethyl-2-oxazoline) (PEOZ) 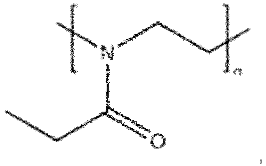
poly(2-propyl-2-oxazoline) (PPOZ) 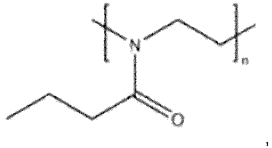
poly(2-butyl-2-oxazoline) (PBOZ) 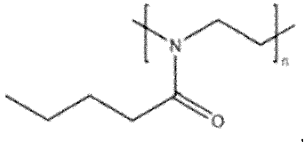
poly(2-isopropyl-2-oxazoline) (PIPOZ) 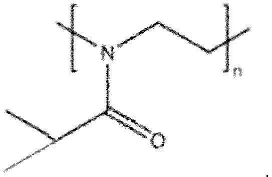
poly(2-methoxymethyl-2-oxazoline) (PMeOMeOx), and
poly(2-dimethylamino-2-oxazoline) (PDMAOx),

preferably wherein [P] is a homopolymer moiety comprising multiple PMOZ or PEOZ monomer units, more preferably wherein [P] comprises or preferably consists of multiple PMOZ monomer units,
wherein
   (i) n has a mean value ranging from 2 to 200, preferably from 20 to 100, more preferably from 24 to 26 or 45 to 50 or wherein
   (ii) n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 3. The polymer conjugated lipid of any one of **embodiment 1 to embodiment 2,** wherein the polymer conjugated lipid is selected from the group consisting of a POZ-monoacylglycerol conjugate, POZ-diacylglycerol conjugate, a POZ-dialkyloxypropyl conjugate, a POZ-steroid or POZ-sterol conjugate, a POZ-phospholipid conjugate, a POZ-ceramide conjugate, and a mixture thereof.

Embodiment 4. The polymer conjugated lipid of any one of **embodiment 1 to embodiment 3,** wherein the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain,
wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH- ), succinyl (-(O)CCH₂CH₂C(O)-), succinamidyl (-NHC(O)CH₂CH₂C(O)NH-), (-NHC(O)CH₂CH₂C(O)-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl).

Embodiment 4.1 The polymer conjugated lipid of any one of **embodiment 1 to embodiment 3,** wherein
(i) the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain,
   wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH- ), succinyl (-(O)CCH₂CH₂C(O)-), succinamidyl (-NHC(O)CH₂CH₂C(O)NH-), (-NHC(O)CH₂CH₂C(O)-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl), or
(ii) the lipid moiety [L] comprises ditetradecylamin, preferably wherein the linker group [linker] is (-NHC(O)CH₂CH₂C(O)-).

Embodiment 5. The polymer conjugated lipid of any one of **embodiment 1 to embodiment 4,** wherein the lipid moiety [L] comprises at least one straight or branched, saturated or unsaturated alkyl chain comprising 6, 7, 8, 9, 10, 11, 12, 13, 14, 15, 16, 17, 18, 19, 20, 21, 22, 23, 24, 25, 26, 27, 28, 29, or 30 carbon atoms, preferably in the range of 10 to 20 carbon atoms, more preferably in the range of 12 to 18 carbon atoms, even more preferably 14, 16 or 18 carbon atoms, even more preferably 16 or 18 carbon atoms, most preferably 14 carbon atoms,
wherein all selections are independent of one another.

Embodiment 6. The polymer conjugated lipid of any one of **embodiment 1 to embodiment 5,** wherein the linker group [linker] is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), (-NHC(O)CH₂CH₂C(O)O-), -C(R5)=N-, - N=C(R⁵)-, -C(R⁵)=N-O-, -O-N=C(R⁵)-, -O-C(O)O-, -C(O)N(R⁵), -N(R⁵)C(O)-, - C(S)(NR⁵)-, (NR⁵)C(S)-, -N(R⁵)C(O)N(R⁵)-, -C(O)S-, -SC(O)-, -C(S)O-, -OC(S)-, - OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), and sulfonate esters, as well as combinations thereof, wherein R³, R⁴ and R⁵ are, independently H or alkyl (e.g. C₁-C₄ alkyl), preferably wherein the linker group [linker] is selected from the group consisting of (-NHC(O)CH2CH2C(O)-), a peptide bond or amid bond (-CO-NH-), (-NHC(O)CH2CH2C(O)O-), and -NH-CH2-.

In a further embodiment related to, but not limited to, multivalent linker groups (i.e. amido, succinamidyl (-NHC(O)CH₂CH₂C(O)NH-) and/or (-NHC(O)CH₂CH₂C(O)-), more than one lipid or alkyl chains are attached to said linker (e.g. -NHC(O)CH₂CH₂C(O)N[-L]₂).

Embodiment 7. The polymer conjugated lipid of any one of **embodiment 1 to embodiment 6,** wherein the linker group [linker] comprises an amide linker moiety, preferably an ester linker moiety, or wherein the linker group [linker] has the structure 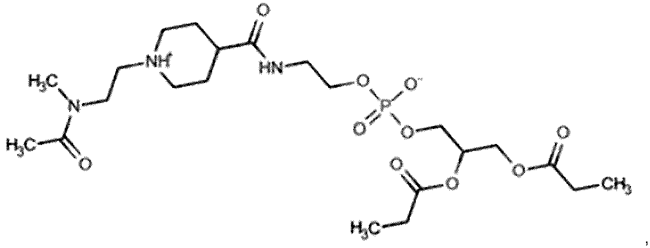 or 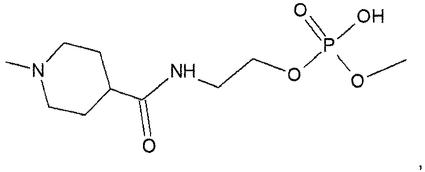 or preferably wherein the linker group comprises 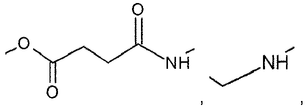 succinate, a peptide bond (-CO-NH-), an amine, or a secondary amine, more preferably wherein the linker group [linker] comprises (-NHC(O)CH₂CH₂C(O)-).

Embodiment 8. The polymer conjugated lipid of any one of embodiment 1 to embodiment 7, wherein the polymer conjugated lipid has the structure of
(i) 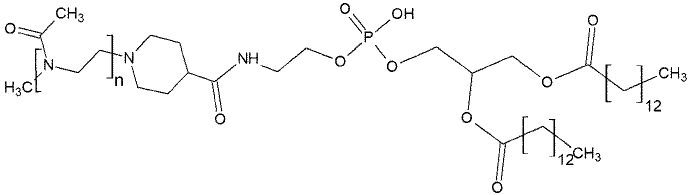 ("DMPE-PMOZ-v1")
   or preferably 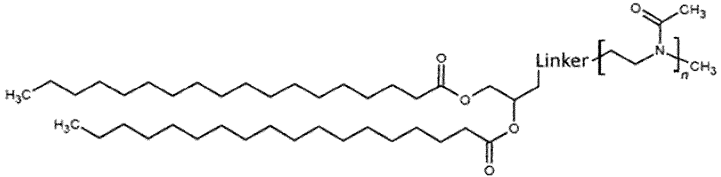 ; or 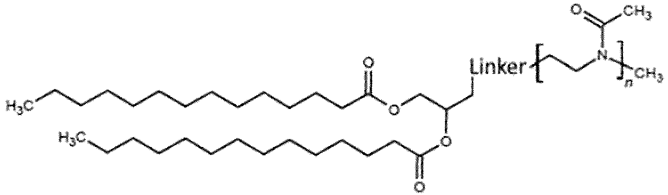 ; or 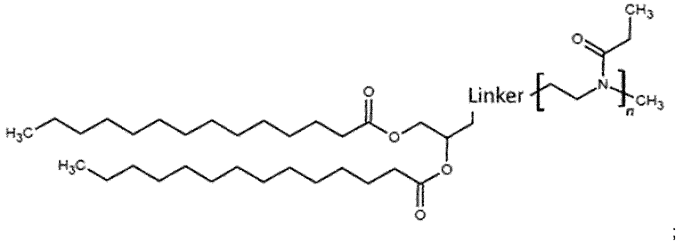 or 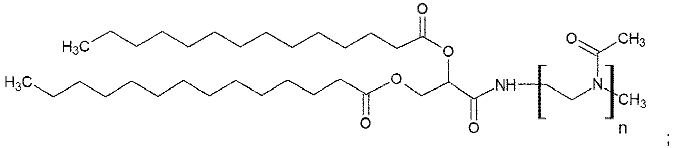 or 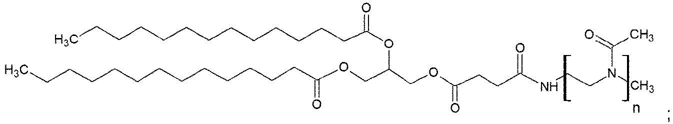 or 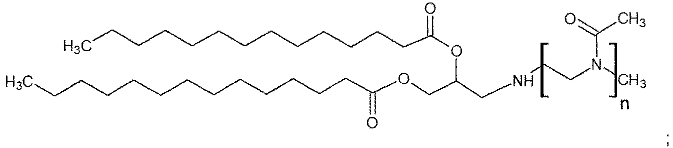 wherein the linker group [linker] is selected from any one of the linker groups as shown in **embodiment 6 or embodiment** 7, preferably the linker group [linker] comprising an ester moiety; or preferably
(ii) a "DMG-PMOZ" having the following structure: 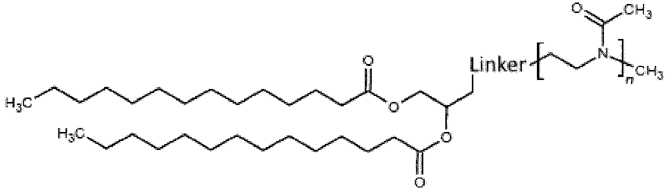 ("DMG-PMOZ"); or very preferably
(iii) "PMOZ 1", "PMOZ 2", "PMOZ 3", "PMOZ 5" or preferably "PMOZ 4" having the following structure 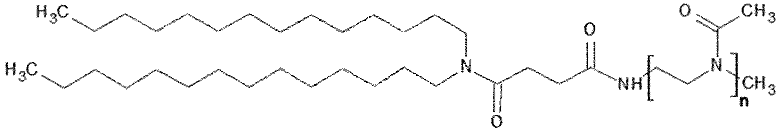 ["PMOZ 4"], preferably having 50 monomer repeats, i.e. 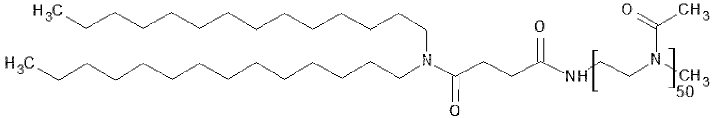 ["PMOZ 4" with 50 monomer repeats];
   whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa;
   most preferably wherein the polymer conjugated lipid of any one of **embodiment 1 to embodiment 7** is DMG-PMOZ with n having a mean value from 45 to 50.

Embodiment 8.1 In a very preferred embodiment, the polymer conjugated lipid is "PMOZ 1", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.2 In a very preferred embodiment, the polymer conjugated lipid is "PMOZ 2", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.3 In a very preferred embodiment, the polymer conjugated lipid is "PMOZ 3", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.4 In a very preferred embodiment, the polymer conjugated lipid is "PMOZ 4", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.5 In a very preferred embodiment, the polymer conjugated lipid is "PMOZ 5", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 9. A lipid nanoparticle comprising a lipid comprising at least one polyoxazoline (POZ) monomer unit 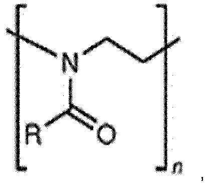 wherein R is C1-9 alkyl or C2-9 alkenyl, preferably C1 or C2 alkyl, and n has a mean value ranging from 2 to 200, preferably from 20 to 100, more preferably from 24 to 26 or 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa,
preferably, wherein the homopolymer moiety comprising multiple monomer units comprises poly(2-methyl-2-oxazoline) (PMOZ), poly(2-ethyl-2-oxazoline) (PEOZ), poly(2-propyl-2-oxazoline) (PPOZ), poly(2-butyl-2-oxazoline) (PBOZ), poly(2-isopropyl-2-oxazoline) (PIPOZ), poly(2-methoxymethyl-2-oxazoline) (PMeOMeOx), or poly(2-dimethylamino-2-oxazoline) (PDMAOx),
more preferably the polymer conjugated lipid according to any one of **embodiment 1 to embodiment 8.**

Embodiment 10. The lipid nanoparticle of **embodiment 9,** wherein the lipid nanoparticle further comprises a cationic or ionizable lipid.

Embodiment 11. The lipid nanoparticle of **embodiment 9 to embodiment 10,** wherein the lipid nanoparticles
(i) do not comprise a polyethylene glycol-(PEG)-lipid conjugate or a conjugate of PEG and a lipid-like material, and preferably do not comprise PEG and/or
(ii) do not comprise a polymer conjugated lipid according to any one of **embodiment 1 to embodiment 8** comprising a sulphur group (-S-), a terminating nucleophile, and/or being covalently coupled to a biologically active ingredient being a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA.

Embodiment 12. The lipid nanoparticle of any one of **embodiment 9 to embodiment 11,** wherein the cationic or ionizable lipid preferably carries a net positive charge at physiological pH, more preferably wherein the cationic or ionizable lipid comprises a tertiary nitrogen group or quaternary nitrogen group.

Embodiment 13. The lipid nanoparticle of any one of **embodiment 9 to embodiment 12,** wherein the lipid nanoparticle further comprises a phospholipid, wherein preferably the phospholipid is a zwitterionic compound selected from, but not limited to the group of 1,2-diphytanoyl-sn-glycero-3-phosphoethanolamine (DPhyPE; 1,2-di-(3,7,11,15-tetramethylhexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; dioleoylphosphatidylcholine), 1,2-Dipalmitoyl-sn-glycero-3-phosphocholine (DPPC; dipalmitoylphosphatidylcholine), 1,2-dioleoyl-sn-glycero-3-phosphoethanolamine (DOPE), phosphatidylethanolamines, distearoylphosphatidylcholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyloleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Dimyristoyl-sn-glycero-3-phosphoethanolamine (DMPE), 1,2-Dilinoleoyl-sn-glycero-3-phosphoethanolamine (DLoPE), distearoyl-phosphatidylethanolamine (DSPE), 1-Palmitoyl-2-oleoyl-sn-glycero-3-phosphoethanolamine (POPE), 1,2-Dilauroyl-sn-glycero-3-phosphoethanolamine (DLPE), 16-O-monomethylphosphoethanolamine, 16-O-dimethyl phosphatidylethanolamine, 1,2-Dierucoyl-sn-glycero-3-phosphoethanolamine (DEPE), 18-1-trans phosphatidylethanolamine, 1-stearoyl-2-oleoylphosphatidyethanolamine (SOPE), 1,2-Disqualeoyl-sn-glycero-3-phosphoethanolamine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodiumsalt), 1-O-hexadecanyl-2-O-(9Z-octadecenyl)-sn-glycero-3-phosphoethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesterylhemisuccinoyl-sn-glycero-3-phosphocholine (PChemsPC), 1,2-dicholesterylhemisuccinoyl-sn-glycero-3-phosphocholine (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine), preferably wherein the phospholipid is DSPC or DPhyPE.

Embodiment 14. The lipid nanoparticle of any one of **embodiment 9 to embodiment 13,** wherein the lipid nanoparticle further comprises a sterol or steroid, preferably selected from the group consisting of cholesterol, cholesteryl hemisuccinate (CHEMS) and a derivate thereof, preferably wherein the lipid nanoparticle further comprises cholesterol.

Embodiment 15. The lipid nanoparticle of any one of **embodiment 9 to embodiment 14,** wherein preferably the lipid nanoparticle comprises
(i) an amount of 1, 2, 3, 4, 5, 6, 7, 8, 9, or 10 mol% of the polymer conjugated lipid of any one of **embodiment 1 to embodiment 8;**
(ii) preferably an amount of 5 mol% of the polymer conjugated lipid of any one of **embodiment 1 to embodiment 8,**
(iii) more preferably an amount of 2.5 mol% of the polymer conjugated lipid of any one of **embodiment 1 to embodiment 8,** or
(iv) also preferably an amount of 1.7 mol% of the polymer conjugated lipid of any one of **embodiment 1 to embodiment 8**
based upon a mol-percentage of the composition of 100% of all lipid components or excipients.

Embodiment 16. The lipid nanoparticle of any one of **embodiment 9 to embodiment 15,** wherein the polymer conjugated lipid is a PMOZ-lipid according to any one of **embodiment 1 to embodiment 8.**

Embodiment 17. The lipid nanoparticle of any one of **embodiment 9 to embodiment 16,** wherein the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
(v) 47.4 mol% cationic or ionizable lipid, preferably one of the ionizable lipid structures of C1 to C27, more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;**
(vi) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8;**
(vii) 47.4 mol% formula III-3 (((4-hydroxybutyl)azanediyl)bis(hexane-6,1-diyl)bis(2-hexyldecanoate)), 40.9 mol% cholesterol, 10 mol% DSPC, and 1.7 mol% of the polymer conjugated lipid of any one of **embodiment 1 to embodiment 8;**
(viii) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% 2-[(PMOZ)]ₙ-N,N-ditetradecylacetamide]; and
(ix) 47.4 mol% formula III-3 (((4-hydroxybutyl)azanediyl)bis(hexane-6,1-diyl)bis(2-hexyldecanoate)), 40.9 mol% cholesterol, 10 mol% DSPC and 1.7 mol% 2-[(PMOZ)]ₙ-N,N-ditetradecylacetamide],
wherein n has a mean value ranging from 2 to 200, preferably from 20 to 100, more preferably from 24 to 26 or 45 to 50 or wherein n is selected such that the polymer moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 17.1 The lipid nanoparticle of any one of **embodiment 9 to embodiment 16,** wherein the lipid nanoparticle comprises excipients selected from ratios selected from the group consisting of (i) about 48.5 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), about 38.9 mol% cholesterol, about 11.1 mol% neutral lipid and about 1.5 mol% of the polymer conjugated lipid of any one of **embodiment 1 to embodiment 8;**

Embodiment 18. The lipid nanoparticle of any one of **embodiment 9 to embodiment 17,** wherein the polymer conjugated lipid of **embodiment 1 to embodiment 8** inhibits aggregation of the lipid nanoparticles.

Embodiment 19. The lipid nanoparticle of any one of **embodiment 9 to embodiment 18,** further comprising a biologically active ingredient.

Embodiment 20. The lipid nanoparticle of **embodiment 19,** wherein the biologically active ingredient is a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA; or any combination thereof, preferably wherein the biologically active ingredient is chemically modified mRNA or chemically unmodified mRNA, more preferably wherein the biologically active ingredient is chemically unmodified mRNA.

Embodiment 21. The lipid nanoparticle of any one of **embodiment 9 to embodiment 20,** wherein the mRNA is associated with the lipid nanoparticle, preferably wherein the mRNA is encapsulated in the lipid nanoparticle.

Embodiment 22. The lipid nanoparticle of any one of **embodiment 9 to embodiment 21,** wherein the lipid nanoparticles comprise the mRNA
(i) at an amount such as to achieve an N/P ratio in the range of about 1 to about 20, preferably about 2 to about 15, more preferably about 3 to about 10, even more preferably about 4 to about 9, most preferably about 6;
(ii) at an amount such as to achieve an N/P ratio in the range of about 5 to about 20, more preferably about 10 to about 18, even more preferably about 12 to about 16, most preferably about 14;
(iii) at an amount such as to achieve a lipid : mRNA weight ratio in the range of 20 to 60, preferably from about 3 to about 15, 5 to about 13, about 50 to about 70, about 4 to about 8 or from about 7 to about 11; or
(iv) at an amount such as to achieve an N/P ratio in the range of about 6 for a lipid nanoparticle according to **embodiment 17.**

Embodiment 23. The lipid nanoparticle of any one of **embodiment 9 to embodiment 22,** wherein the lipid nanoparticle is a sterile solid composition for reconstitution with a sterile liquid carrier, and wherein the lipid nanoparticle further comprises one or more inactive ingredients selected from pH-modifying agents, bulking agents, stabilizers, non-ionic surfactants and antioxidants, and wherein the sterile liquid carrier is an aqueous carrier.

Embodiment 24. The lipid nanoparticle of any one of **embodiment 9 to embodiment 23,** wherein the lipid nanoparticle is a sterile liquid composition, and wherein the lipid nanoparticles have a mean hydrodynamic diameter as determined by dynamic laser scattering from about 50 nm to about 300 nm, or from about 60 nm to about 250 nm, or from about 60 nm to about 200 nm, or from about 70 to 200 nm, or from about 75 nm to about 160, or from about 85 nm to about 140 nm, or from about 90 nm to about 130 nm, or from about 50 nm to about 120 nm.

Embodiment 25. The lipid nanoparticle of any one of **embodiment 9 to embodiment 24,** wherein the lipid nanoparticles exhibit a zeta potential in the range of -50 mV to +50 mV, preferably in the range of -5 mV to +5 mV.

Embodiment 26. The lipid nanoparticle of any one of **embodiment 9 to embodiment 25,** wherein the mRNA compound is a mono-, bi-, or multicistronic mRNA.

Embodiment 27. The lipid nanoparticle of any one **embodiment 9 to embodiment 26,** wherein the mRNA compound comprises at least one chemical modification.

Embodiment 28. The lipid nanoparticle of **embodiment 27,** wherein the chemical modification is selected from the group consisting of base modifications, sugar modifications, backbone modifications and lipid modifications, preferably wherein the chemical modification is a base modification, more preferably wherein the base modification preferably is selected from the group consisting of pseudouridine (psi or ψ), N1-methylpseudouridine (N1MPU, N1Mpsi or N1Mψ), 1-ethylpseudouracil, 2-thiouracil (s2U), 4-thiouracil, 5-methylcytosine, 5-methyluracil, 5-methoxyuracil, and any combination thereof.

Embodiment 29. The lipid nanoparticle of any one of **embodiment 9 to embodiment 28,** wherein the mRNA compound comprises a coding region encoding a peptide or protein, wherein the coding region exhibits a sequence modification.

Embodiment 30. The lipid nanoparticle of **embodiment 29,** wherein the sequence modification is selected from a G/C content modification, a codon modification, a codon optimization or a C-optimization of the sequence; preferably wherein, compared with the coding region of the corresponding wild-type mRNA, the
- G/C content of the coding region is increased;
- C content of the coding region is increased;
- codon usage in the coding region is adapted to the human codon usage; and/or
- codon adaptation index (CAI) is increased or maximized in the coding region.

Embodiment 31. The lipid nanoparticle of any one of **embodiment 9 to embodiment 30,** wherein the mRNA compound further comprises
a) a 5'-CAP structure, preferably m7GpppN, more preferably CAP1 or m7G(5')ppp(5')(2'OMeA)pG;
b) optionally at least one miRNA sequence, preferably wherein the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof;
c) at least one 5'-UTR element;
d) a coding sequence;
e) at least one 3'-UTR element;
f) at least one poly(A) sequence;
g) at least one poly(C) sequence;
or any combinations of these.

Embodiment 32. The lipid nanoparticle of any one of **embodiment 9 to embodiment 31,** wherein the least one coding RNA comprises a 5'-CAP structure, preferably m7G, CAPO, CAP1, CAP2, a modified CAPO or a modified CAP1 structure.

Embodiment 33. The lipid nanoparticle of any one of **embodiment 9 to embodiment 32,** wherein the at least one coding RNA comprises at least one heterologous 5'-UTR and/or at least one heterologous 3'-UTR, preferably wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes; and/or preferably wherein the at least one heterologous 3'-UTR comprises a nucleic acid sequence derived from a 3'-UTR of a gene selected from PSMB3, ALB7, alpha-globin, CASP1, COX6B1, GNAS, NDUFA1 and RPS9, or from a homolog, a fragment or a variant of any one of these genes.

Embodiment 34. The lipid nanoparticle of any one of **embodiment 9 to embodiment 33,** wherein the at least one coding RNA comprises a (i) HSD17B4 5'-UTR and a PSMB3 3'-UTR or (ii) a RPL32 5'-UTR and an ALB7 3'-UTR, preferably a mutated alpha-globin 3'-UTR (SEQ ID NO:11/12), more preferably a HSD17B4 5'-UTR (SEQ ID NO:21/22) and a PSMB3 3'-UTR (SEQ ID NO:19/20).

Embodiment 35. The lipid nanoparticle of any one of **embodiment 9 to embodiment 34,** comprising the following elements in the 5' to 3' direction:
a) a 5'-CAP structure, preferably selected from the group consisting of m7G(5'), m7G(5')ppp(5')(2'OMeA)pG and m7G(5')ppp(5')(2'OMeG)pG;
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, 24, 26, or a homolog, a fragment or a variant thereof, most preferably according to SEQ ID NO:22 (HSD17B4);
c) at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8, 10, 12, 14, 16, 18, 20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18, or a homolog, a fragment or a variant thereof, most preferably according to SEQ ID NO:20 (PSMB3);
e) optionally, at least one poly(A) sequence, preferably consisting of 10 to 200, 10 to 100, 40 to 80, or 50 to 70 adenosine nucleotides;
f) optionally, at least one poly(C) sequence, preferably consisting of 10 to 200, 10 to 100, 20 to 70, 20 to 60 or 10 to 40 cytosine nucleotides; and
g) optionally, at least one histone stem-loop, preferably comprising the RNA sequence according to SEQ ID NO:4.

Embodiment 35.1 The lipid nanoparticle of any one of **embodiment 9 to embodiment 34,** comprising the following elements in the 5' to 3' direction:
a) a 5'-CAP structure, preferably selected from the group consisting of m7G(5'), m7G(5')ppp(5')(2'OMeA)pG and m7G(5')ppp(5')(2'OMeG)pG;
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, 24, 26, or a homolog, a fragment or a variant thereof, most preferably according to SEQ ID NO:22 (HSD17B4);
c) at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8, 10, 12, 14, 16, 18, 20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18, or a homolog, a fragment or a variant thereof, most preferably according to SEQ ID NO:20 (PSMB3);
e) optionally a histone stem-loop preferably comprising the RNA sequence according to SEQ ID NO:4;
f) optionally, at least one poly(A) sequence, preferably consisting of 10 to 200, 10 to 100, 40 to 80, or 50 to 70 adenosine nucleotides, preferably 100 adenosine nucleotides.

Embodiment 36. The lipid nanoparticle of any one of **embodiment 9 to embodiment 35,** wherein the biologically active ingredient is
(a) an mRNA comprising at least one coding sequence encoding a peptide or protein, or a fragment or variant thereof, wherein the peptide or protein is an antigen, wherein the antigen preferably is derived from pathogenic antigens, tumor antigens, allergenic antigens or autoimmune self-antigens, or a fragment or variant thereof; or
(b) an mRNA comprising at least one coding sequence encoding a therapeutic protein, or a fragment or variant thereof, wherein the therapeutic protein is selected from the group consisting of
   (i) therapeutic proteins for use in enzyme replacement therapy for the treatment of metabolic, endocrine or amino acid disorders or for use in replacing an absent, deficient or mutated protein;
   (ii) therapeutic proteins for use in the treatment of blood disorders, diseases of the circulatory system, diseases of the respiratory system, infectious diseases or immune deficiencies;
   (iii) therapeutic proteins for use in the treatment of cancer or tumor diseases;
   (iv) therapeutic proteins for use in hormone replacement therapy;
   (v) therapeutic proteins for use in reprogramming somatic cells into pluri- or omnipotent stem cells;
   (vi) therapeutic proteins for use as adjuvant or immunostimulation;
   (vii) therapeutic proteins being a therapeutic antibody;
   (viii) therapeutic proteins being a gene editing agent; and
   (ix) therapeutic proteins for use in treating or preventing a liver disease selected from the group consisting of liver fibrosis, liver cirrhosis and liver cancer.

Embodiment 37. The lipid nanoparticle of **embodiment 36 sub-item (a),** wherein the at least one coding sequence encoding a pathogenic antigen is selected from the group consisting of a bacterial, viral, fungal and protozoal antigen.

Embodiment 38. The lipid nanoparticle of **embodiment 37,** wherein the at least one coding sequence encoding a pathogenic antigen
(i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
(ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
(iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof (e.g. receptor binding domain (RBD), critical neutralisation domain (CND)); and/or
(iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation.

Embodiment 39. The lipid nanoparticle of any one of **embodiment 9 to embodiment 38** for use
(i) in the treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition; and/or
(ii) for use in enzyme replacement therapy for the treatment of metabolic or endocrine disorders or for use in replacing an absent, deficient or mutated protein.

Embodiment 40. The lipid nanoparticle of any one of **embodiment 9 to embodiment 39** for use in the treatment or prophylaxis of infectious diseases.

Embodiment 41. The lipid nanoparticle of **embodiment 9 or embodiment 40** comprising at least one coding RNA, wherein said at least one coding RNA comprises at least one coding sequence encoding at least one peptide or protein for use in treatment or prevention of a disease, disorder or condition, wherein said lipid nanoparticle is administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, intraocular, periocular, periodontal, retrobulbar, subarachnoid, subconjunctival, suprachoroidal injection, infusion, implantation, nasal, buccal, sublingual, otic or auricular, ophthalmic, conjunctival, vaginal, rectal, intracervical, endosinusial, laryngeal, oropharyngeal, ureteral, urethral administration, more preferably said lipid nanoparticle is administered intramuscularly, intravenously, intradermally, subcutaneously, intratumorally, intranasally, or by inhalation, most preferably intramuscularly, to a subject in need thereof.

Embodiment 42. A kit or kit of parts, comprising any one of the lipid nanoparticle of **embodiment 9 to embodiment 41,** optionally comprising a liquid vehicle for solubilizing, and, optionally, technical instructions providing information on administration and dosage of the components.

Embodiment 43. The lipid nanoparticle of any one of **embodiment 9 to embodiment 41** or the kit or kit of parts of **embodiment 42** for use in *in vivo* drug delivery, preferably for use in delivering a nucleic acid, preferably a mRNA.

Embodiment 44. The lipid nanoparticle of any one of **embodiment 9 to embodiment 41** or the kit or kit of parts of **embodiment 43** for use as a medicament.

Embodiment 45. The lipid nanoparticle for use as a medicament according to **embodiment 44,** wherein the medicament is for the prevention, prophylaxis, treatment and/or amelioration of a disease selected from infectious diseases including viral, bacterial or protozoological infectious diseases, cancer or tumor diseases, liver diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and diseases associated with a peptide or protein deficiency.

Embodiment 46. The lipid nanoparticle for use as a medicament according to **embodiment 44 or embodiment 45,** wherein the medicament is a vaccine composition.

Embodiment 47. A vaccine composition comprising a lipid nanoparticle of any one of **embodiment 9 to embodiment 46** or a kit or kit of parts of **embodiment 42** for use as a medicament, and/or for prevention, prophylaxis, treatment and/or amelioration of a disease selected from infectious diseases including viral, bacterial or protozoological infectious diseases, cancer or tumor diseases.

Embodiment 48. A method of treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition comprising the steps:
a) providing a lipid nanoparticle of any one of **embodiment 9 to embodiment 45,** comprising a homopolymer moiety comprising at least one polyoxazoline (POZ) monomer, preferably the polymer conjugated lipid according to any one of **embodiment 1 to embodiment 8,** the vaccine composition of **embodiment 47,** or the kit or kit of parts of **embodiment 42;** and
b) applying or administering the mRNA, the lipid nanoparticle, the vaccine composition or the kit or kit of parts to a tissue or an organism.

Embodiment 49. A method for delivering mRNA encoding an antigen or a therapeutic peptide or protein to a subject, the method comprising administering to a subject a lipid nanoparticle of any one of embodiments 1 to 33, wherein the mRNA encodes an antigen or a therapeutic peptide or protein, and wherein delivering the mRNA to the subject is beneficial in treating or preventing a disease or disorder, preferably wherein the subject is a mammal, more preferably wherein the subject is a human.

Embodiment 50. The method according to any one of embodiments **embodiment 48 to embodiment 49,** wherein the mRNA, the lipid nanoparticle of any one of **embodiment 9 to embodiment 48,** the vaccine composition of **embodiment 47** or the kit or kit of parts of **embodiment 42** is administered to the tissue or to the organism by intravenous, intramuscular, subcutaneous, intradermal or intratumoral injection or any administration route as disclosed in any **preceding embodiment.**

Embodiment 51. A method of inducing an immune response in a subject, the method comprising administering to the subject the vaccine composition of **embodiment 47** in an amount effective to produce an antigen-specific immune response in the subject.

Embodiment 52. A pharmaceutical composition comprising a lipid nanoparticle of any one of **embodiment 9 to embodiment 48** or a kit or kit of parts of **embodiment 42** or the vaccine composition of **embodiment 47** for use in vaccination of a subject comprising an effective dose of mRNA encoding a virus antigen.

Embodiment 53. Use of a pharmaceutical composition according to **embodiment 52** or a kit or kit of parts according to **embodiment 42** for (i) inducing an immune response, for (ii) inducing an antigen specific T-cell response or preferably for (iii) inducing CD8+ T cells responses.

Embodiment 54. Use of the pharmaceutical composition of **embodiment 52** for the prophylaxis of an infectious disease or in the manufacture of a medicament for the prophylaxis of an infectious disease, wherein said medicament preferably is a vaccine composition.

Embodiment 55. A method for preventing, ameliorating or treating a disease or condition in a subject in need comprising administering to the subject a lipid nanoparticle of any one of **embodiment 9 to embodiment 48,** a pharmaceutical composition of **embodiment 52** or a kit or kit of parts of **embodiment 42.**

Embodiment 56. The method of any one of the **preceding method embodiments,** wherein administration of the lipid nanoparticle results in expression of the antigen encoded by mRNA in the lymphocytes of the subject.

Embodiment 57. A method of treating or preventing a disorder of any one of **embodiments 36, 39, 41, 45, 48, or 49,** wherein the disorder is an infection with coronavirus, or a disorder related to such an infection.

Embodiment 58. A method of treating or preventing a disorder of any one of **embodiments 36, 39, 41, 45, 48, or 49,** wherein the subject in need is a mammalian subject, preferably a human subject.

Embodiment 59. The method of any one of the **preceding method embodiments,** wherein the administration of the lipid nanoparticle results in an antigen specific antibody response, preferably wherein the antigen specific antibody response is measured by the presence of antigen-specific antibodies in serum.

Embodiment 60. The lipid nanoparticle of any one of **embodiment 9 to embodiment 16,** wherein the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;** and
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8.**

Embodiment 61. The lipid nanoparticle of any one of **embodiment 9 to embodiment 16 or embodiment 60,** wherein the lipid nanoparticle comprises a neutral lipid or phospholipid having at least one alkyl chain with a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₈, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).

Embodiment 62. The lipid nanoparticle of any one of **embodiment 9 to embodiment 16 or embodiment 60 to embodiment 61,** wherein the lipid nanoparticles comprise a neutral lipid or phospholipid having at least two alkyl chains, whereby each alkyl chain independently has a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₆, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).

Embodiment 63. The lipid nanoparticle of any one of **embodiment 9 to embodiment 16 or embodiment 60 to embodiment 62** or a lipid nanoparticle comprising a polymer conjugated lipid according to any one of **embodiment 1** to **embodiment 8,** wherein the lipid nanoparticle has a lower PDI and/or lower size as compared to a control lipid nanoparticle comprising a PEG-lipid instead of a polymer conjugated lipid according to any one of **embodiment 1** to **embodiment 8.**

### Fourth Set of Embodiments

All SEQ ID NO-references which are shown in the fourth set of embodiments are related to the SEQ ID NO from PCT application number PCT/EP2021/074342 or the subsequent patent application claiming the priority of PCT/EP2021/074342, the sequence listing of which is incorporated herein by reference.

Embodiment 1. A vaccine composition comprising
a) at least one nucleic acid encoding at least one antigen or fragment or variant thereof; and
b) a carrier composition, wherein the carrier composition comprises the phospholipid phosphatidylserine, wherein the amount of the phosphatidylserine is not more than 9 mol%, preferably not more than 5 mol%, of the total molar amount of all lipidic excipients in the carrier composition.

Embodiment 2. The vaccine composition according to embodiment 1, wherein the at least one nucleic acid is not a tolerogenic nucleic acid; and/or wherein the at least one nucleic acid does not encode a tolerogenic polypeptide; and/or wherein the vaccine composition does not comprise an antigen or fragment or variant thereof; and/or wherein the vaccine composition comprises the at least one nucleic acid as the sole payload; and/or wherein the vaccine composition is not a tolerogenic composition.

Embodiment 3. The vaccine composition according to embodiment 1 or 2, wherein the carrier composition at least partly encapsulates the at least one nucleic acid.

Embodiment 4. The vaccine composition according to any one of embodiments 1 to 3, wherein the carrier composition encapsulates the at least one nucleic acid.

Embodiment 5. The vaccine composition according to any one of embodiments 1 to 4, wherein the carrier composition comprises an inner surface and an outer surface facing the outside, wherein the phosphatidylserine is located at the outer surface of the carrier composition.

Embodiment 6. The vaccine composition according to embodiment 5, wherein the hydrophilic head group of the phosphatidylserine is located at the outer surface of the carrier composition.

Embodiment 7. The vaccine composition according to any one of embodiments 1 to 6, wherein the hydrophilic head group of the phosphatidylserine comprised in the carrier composition is accessible from the outside of the carrier composition.

Embodiment 8. The vaccine composition according to any one of embodiments 1 to 7, wherein the phosphatidylserine is selected from the group consisting of DPhyPS, WT-PS, 16:0-PS, 14:0-PS, 10:0-PS, 6:0-PS, 18:1-PS DOPS, 18:1-Lyso PS and 18:0-Lyso PS.

Embodiment 9. The vaccine composition according to any one of embodiments 1 to 8, wherein the carrier composition is a lipid nanoparticle composition.

Embodiment 10. The vaccine composition according to embodiment 9, wherein the lipid nanoparticle composition further comprises
(i) a cationic or ionizable lipid; and/or
(ii) a steroid; and/or
(iii) a further phospholipid in addition to phosphatidylserine, preferably DPhyPE; and/or
(iv) a polymer conjugated lipid.

Embodiment 11. The vaccine composition according to embodiment 9 or 10, wherein the lipid nanoparticle composition further comprises
(i) a cationic or ionizable lipid;
(ii) a steroid;
(iii) a further phospholipid in addition to phosphatidylserine, preferably DPhyPE; and
(iv) a polymer conjugated lipid.

Embodiment 12. The vaccine composition according to embodiment 10 or 11, wherein the cationic or ionizable lipid carries a net positive charge at physiological pH, preferably wherein the cationic or ionizable lipid comprises a tertiary nitrogen group or quaternary nitrogen group, more preferably wherein the cationic or ionizable lipid is selected from the group consisting of HEXA1, HEXA2 and THIOETHER with the structures shown in Figure 1A, 1B and 1C, respectively.

Embodiment 13. The vaccine composition according to any one of embodiments 10 to 12, wherein the steroid is selected from the group consisting of cholesterol, cholesteryl hemisuccinate (CHEMS) and a derivate thereof, preferably wherein the steroid is cholesterol.

Embodiment 14. The vaccine composition according to any one of embodiments 10 to 13, wherein the further phospholipid is selected from the group consisting of 1,2-diphytanoyl-sn-glycero-3-phosphoethanolamine (DPhyPE; 1,2-di-(3,7,11,15-tetramethylhexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; dioleoylphosphatidylcholine), 1,2-Dipalmitoyl-sn-glycero-3-phosphocholine (DPPC; dipalmitoylphosphatidylcholine), 1,2-dioleoyl-sn-glycero-3-phosphoethanolamine (DOPE), phosphatidylethanolamines, distearoylphosphatidylcholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyloleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Dimyristoyl-sn-glycero-3-phosphoethanolamine (DMPE), 1,2-Dilinoleoyl-sn-glycero-3-phosphoethanolamine (DLoPE), distearoyl-phosphatidylethanolamine (DSPE), 1-Palmitoyl-2-oleoyl-sn-glycero-3-phosphoethanolamine (POPE), 1,2-Dilauroyl-sn-glycero-3-phosphoethanolamine (DLPE), 16-O-monomethylphosphoethanolamine, 16-O-dimethyl phosphatidylethanolamine, 1,2-Dierucoyl-sn-glycero-3-phosphoethanolamine (DEPE), 18-1-trans phosphatidylethanolamine, 1-stearoyl-2-oleoylphosphatidyethanolamine (SOPE), 1,2-Disqualeoyl-sn-glycero-3-phosphoethanolamine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodium salt), 1-O-hexadecanyl-2-O-(9Z-octadecenyl)-sn-glycero-3-phospho-ethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesterylhemisuccinoyl-sn-glycero-3-phosphocholine (PChemsPC), 1,2-dicholesterylhemisuccinoyl-sn-glycero-3-phosphocholine (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine),
preferably wherein the further phospholipid is DPhyPE; and wherein the phospholipid, preferably DPhyPE, is optionally present in combination with a phospholipid having at least two alkyl chains, wherein each alkyl chain independently has a length of preferably C6, C7, C8, C9, or C10, more preferably a length of C6, C7, or C8, most preferably a length of C7, further most preferably a phospholipid selected from the group consisting of DHPC (1,2-diheptanoyl-sn-glycero-3-phosphocholine), 05:0 PC (1,2-dipentanoyl-sn-glycero-3-phosphocholine), 04:0 PC (1,2-dibutyryl-sn-glycero-3-phosphocholine), 06:0 PC (1,2-dihexanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), with DHPC being most preferred as the optionally present phospholipid having at least two alkyl chains.

Embodiment 15. The vaccine composition according to any one of embodiments 10 to 14, wherein the polymer conjugated lipid is a pegylated lipid or a PMOZ-lipid.

Embodiment 16. The vaccine composition according to any one of embodiments 11 to 15, wherein the composition comprises excipients in a ratio selected from the group consisting of
(a-i) the cationic lipid at an amount of 30-70 mol%; the steroid at an amount of 20-50 mol%; the phospholipid at an amount of 5-25 mol%; and the polymer conjugated lipid at an amount of 0.5-5 mol%;
(a-ii) the cationic lipid at an amount of 40-60 mol%; the steroid at an amount of 20-40 mol%; the phospholipid at an amount of 10-20 mol%; and the polymer conjugated lipid at an amount of 1-2 mol%;
(a-iii) the cationic lipid of embodiment 12 at an amount of 30-70 mol%; the steroid of embodiment 13 at an amount of 20-50 mol%; the phospholipid phosphatidylserine and the phospholipid of embodiment 14 at an amount of 5-25 mol%; and the polymer conjugated lipid of embodiment 15 at an amount of 0.5-5 mol%; and
(a-iv) the cationic lipid of embodiment 12 at an amount of 40-60 mol%; the steroid of embodiment 13 at an amount of 20-40 mol%; the phospholipid phosphatidylserine and the phospholipid of embodiment 14 at an amount of 10-20 mol%; and the polymer conjugated lipid of embodiment 15 at an amount of 1-2 mol%; preferably the composition comprising excipients in a ratio selected from the group consisting of
(b-i) the cationic lipid at an amount of 59 mol%; the steroid at an amount of 29.3 mol%; the phospholipid at an amount of 10 mol%; and the polymer conjugated lipid at an amount of 1.7 mol%;
(b-ii) the cationic lipid at an amount of 58 mol%; the steroid at an amount of 29.3 mol%; the phospholipid at an amount of 11 mol%; and the polymer conjugated lipid at an amount of 1.7 mol%;
(b-iii) the cationic lipid at an amount of 49 mol%; the steroid at an amount of 29.3 mol%; the phospholipid at an amount of 20 mol%; and the polymer conjugated lipid at an amount of 1.7 mol%;
(b-iv) the cationic lipid of embodiment 12 at an amount of 59 mol%; the steroid of embodiment 13 at an amount of 29.3 mol%; the phospholipid phosphatidylserine and the phospholipid of embodiment 14 at an amount of 10 mol%; and the polymer conjugated lipid of embodiment 15 at an amount of 1.7 mol%;
(b-v) the cationic lipid of embodiment 12 at an amount of 58 mol%; the steroid of embodiment 13 at an amount of 29.3 mol%; the phospholipid phosphatidylserine and the phospholipid of embodiment 14 at an amount of 11 mol%; and the polymer conjugated lipid of embodiment 15 at an amount of 1.7 mol%; and
(b-vi) the cationic lipid of embodiment 12 at an amount of 49 mol%; the steroid of embodiment 13 at an amount of 29.3 mol%; the phospholipid phosphatidylserine and the phospholipid of embodiment 14 at an amount of 20 mol%; and the polymer conjugated lipid of embodiment 15 at an amount of 1.7 mol%;
more preferably the composition comprising excipients in a ratio selected from the group consisting of (c-i) the cationic lipid of embodiment 12 at an amount of 59 mol%; the steroid of embodiment 13 at an amount of 29.3 mol%; the phospholipid phosphatidylserine at an amount of 5 mol% and DPhyPE at an amount of 5 mol%; and the polymer conjugated lipid of embodiment 15 at an amount of 1.7 mol%;
(c-ii) the cationic lipid of embodiment 12 at an amount of 59 mol%; the steroid of embodiment 13 at an amount of 29.3 mol%; the phospholipid phosphatidylserine at an amount of 2 mol% and DPhyPE at an amount of 8 mol%; and the polymer conjugated lipid of embodiment 15 at an amount of 1.7 mol%;
(c-iii) the cationic lipid of embodiment 12 at an amount of 58 mol%; the steroid of embodiment 13 at an amount of 29.3 mol%; the phospholipid phosphatidylserine at an amount of 5 mol%, DPhyPE at an amount of 5 mol% and DHPC at an amount of 1 mol%; and the polymer conjugated lipid of embodiment 15 at an amount of 1.7 mol%;
(c-iv) the cationic lipid of embodiment 12 at an amount of 49 mol%; the steroid of embodiment 13 at an amount of 29.3 mol%; the phospholipid phosphatidylserine at an amount of 5 mol%, DPhyPE at an amount of 5 mol% and DHPC at an amount of 10 mol%; and the polymer conjugated lipid of embodiment 15 at an amount of 1.7 mol%.

Embodiment 17. The vaccine composition according to any one of the preceding embodiments, wherein the at least one nucleic acid is DNA or RNA.

Embodiment 18. The vaccine composition according to embodiment 17, wherein the at least one nucleic acid is RNA, preferably mRNA comprising a coding sequence encoding the at least one antigen or fragment or variant thereof and optionally a coding sequence encoding at least one self-amplifying enzyme.

Embodiment 19. The vaccine composition according to embodiment 18, wherein the lipid nanoparticles comprise the mRNA
(i) at an amount such as to achieve an N/P ratio in the range of 10 to 20, preferably about 2 to about 15, more preferably about 3 to about 10, even more preferably about 4 to about 9, most preferably about 6; or
(ii) at an amount such as to achieve an N/P ratio in the range of about 5 to about 20, more preferably about 10 to about 18, even more preferably about 12 to about 16, most preferably about 14; and/or
(iii) at an amount such as to achieve a lipid : mRNA weight ratio in the range of about 20 to about 60, preferably from about 3 to about 15, about 5 to about 13, about 4 to about 8 or from about 7 to about 11; and/or

wherein the lipid nanoparticles have a mean hydrodynamic diameter as determined by dynamic laser scattering from about 50nm to about 300nm, or from about 60nm to about 250nm, or from about 60nm to about 200nm, or from about 70nm to 200nm, or from about 75nm to about 160nm, or from about 90nm to about 140nm, or from about 100nm to about 140nm; and/or
wherein the lipid nanoparticles exhibit a zeta potential in the range of -50 mV to +50 mV.

Embodiment 20. The vaccine composition according to embodiment 18 or 19, wherein the mRNA is a mono-, bi-, or multicistronic mRNA.

Embodiment 21. The vaccine composition according to any one of embodiments 18 to 20, wherein the mRNA comprises at least one chemical modification.

Embodiment 22. The vaccine composition according to embodiment 21, wherein the chemical modification is selected from the group consisting of base modifications, sugar modifications, backbone modifications and lipid modifications, preferably wherein the chemical modification is a base modification, more preferably wherein the base modification preferably is selected from the group consisting of pseudouridine (psi or ψ), N1-methylpseudouracil (N1MPU, N1Mpsi or N1Mψ), 1-ethylpseudouracil, 2-thiouracil (s2U), 4-thiouracil, 5-methylcytosine, 5-methyluracil, 5-methoxyuracil, and any combination thereof.

Embodiment 23. The vaccine composition according to any one of embodiments 18 to 22, wherein the coding sequence exhibits a sequence modification.

Embodiment 24. The vaccine composition according to embodiment 23, wherein the sequence modification is selected from a G/C content modification, a codon modification, a codon optimization or a C-optimization of the sequence; preferably wherein, compared with the coding sequence of the corresponding wild-type mRNA, the
a) G/C content of the coding sequence is increased;
b) C content of the coding sequence is increased;
c) codon usage in the coding sequence is adapted to the human codon usage; and/or
d) codon adaptation index (CAI) is increased or maximised in the coding sequence.

Embodiment 25. The vaccine composition according to any one of embodiments 18 to 24, wherein the mRNA further comprises
a) a 5'-CAP structure, preferably m7GpppN, more preferably CAP1 or m7G(5')ppp(5')(2'OMeA)pG;
b) at least one miRNA binding site sequence, preferably wherein the microRNA binding site is for a microRNA selected from the group consisting of a miR-126, miR-142, miR-144, miR-146, miR-150, miR-155, miR-16, miR-21, miR-223, miR-24, miR-27, miR-26a binding site, preferably a miR-122 or miR-142 binding site, or any combination of the aforementioned miRNA binding sites thereof;
c) at least one 5'-UTR element;
d) at least one 3'-UTR element;
e) at least one poly(A) sequence;
f) at least one poly(C) sequence;
g) optionally, a histone stem-loop selected from SEQ ID NO:3 or 4;
h) optionally, a 3'-terminal sequence element selected from SEQ ID NO:41-70;
or any combinations of these.

Embodiment 26. The vaccine composition according to any one of embodiments 18 to 25, wherein the mRNA comprises a 5'-CAP structure, preferably m7G, CAP0, CAP1, CAP2, a modified CAP0 or a modified CAP1 structure.

Embodiment 27. The vaccine composition according to embodiment 25, wherein the at least one coding RNA comprises at least one heterologous 5'-UTR and/or at least one heterologous 3'-UTR, preferably wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes; and/or preferably wherein the at least one heterologous 3'-UTR comprises a nucleic acid sequence derived from a 3'-UTR of a gene selected from PSMB3, ALB/albumin, alpha-globin, CASP1 (preferably SEQ ID NO:81 (DNA) or SEQ ID NO:82 (RNA)), COX6B1 (preferably SEQ ID NO:83 (DNA) or SEQ ID NO:84 (RNA)), GNAS (preferably SEQ ID NO:85 (DNA) or SEQ ID NO:86 (RNA)), NDUFA1 (preferably SEQ ID NO:87 (DNA) or SEQ ID NO:88 (RNA)) and RPS9 (preferably SEQ ID NO:79 (DNA) or SEQ ID NO:80 (RNA)), or from a homolog, a fragment or a variant of any one of these genes.

Embodiment 28. The vaccine composition according to embodiment 27, wherein the at least one coding RNA comprises a (i) HSD17B4 5'-UTR and a PSMB3 3'-UTR or (ii) a RPL32 5'-UTR and an ALB/albumin 3'-UTR, preferably a mutated alpha-globin 3'-UTR (SEQ ID NO:11, 12), more preferably a HSD17B4 5'-UTR (SEQ ID NO:21, 22) and a PSMB3 3'-UTR (SEQ ID NO:19, 20).

Embodiment 29. The vaccine composition according to any one of embodiments 18 to 24, wherein the mRNA comprises the following elements in the 5' to 3' direction:
a) a 5'-CAP structure, preferably selected from the group consisting of m7G(5'), m7G(5')ppp(5')(2'OMeA) and m7G(5')ppp(5')(2'OMeG);
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, SEQ ID NO:24, SEQ ID NO:26 or preferably SEQ ID NO:77/78 (SLC7A3) or SEQ ID NO:75/76 (RPL31), or a homolog, a fragment or a variant thereof; most preferably according to SEQ ID NO:22 (HSD17B4);
c) the at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8 or SEQ ID NO:10, 12, 14, 16, 18, or preferably SEQ ID NO:20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18 (ALB/albumin) or preferably SEQ ID NO:79/80 (RPS9), or a homolog, a fragment or a variant thereof; most preferably according to SEQ ID NO:20 (PSMB3);
e) optionally, at least one poly(A) sequence, preferably consisting of 10 to 200, 10 to 100, 40 to 80, or 50 to 70 adenosine nucleotides, more preferably at least 70 adenosine nucleotides, even more preferably about 100 adenosine nucleotides;
f) optionally, at least one poly(C) sequence, preferably consisting of 10 to 200, 10 to 100, 20 to 70, 20 to 60 or 10 to 40 cytosine nucleotides; and
g) optionally, at least one histone stem-loop, preferably comprising the RNA sequence according to SEQ ID NO:4.

Embodiment 30. The vaccine composition according to any one of the preceding embodiments, wherein the antigen is derived from a pathogenic antigen, a tumour antigen, an allergenic antigen or an autoimmune self-antigen.

Embodiment 31. The vaccine composition according to embodiment 30, wherein the pathogenic antigen is selected from the group consisting of a bacterial antigen, a viral antigen, a fungal antigen and a protozoal antigen.

Embodiment 32. The vaccine composition according to embodiment 30 or 31, wherein the pathogenic antigen
(i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus (RABV), Respiratory Syncytial virus (RSV), Rhinovirus, Rotavirus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
(ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
(iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof; and/or
(iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation.

Embodiment 33. A pharmaceutical composition comprising the vaccine composition according to any one of embodiments 30 to 32 and a pharmaceutically acceptable carrier, diluent or excipient, preferably wherein the pharmaceutical composition is a sterile solid composition for reconstitution with a sterile liquid carrier, and wherein the composition further comprises one or more inactive ingredients selected from pH-modifying agents, bulking agents, stabilizers, non-ionic surfactants and antioxidants, and wherein the sterile liquid carrier is an aqueous carrier.

Embodiment 34. The vaccine composition according to any one of embodiments 30 to 32 or the pharmaceutical composition according to embodiment 33 for use in the treatment or prophylaxis of infectious diseases; cancer or tumor diseases, disorders or conditions; liver diseases selected from the group consisting of liver fibrosis, liver cirrhosis and liver cancer; allergies; or autoimmune disease, disorder or condition; in a subject.

Embodiment 35. The vaccine composition according to embodiment 32 or a pharmaceutical composition comprising the vaccine composition according to embodiment 32 for use in the treatment or prophylaxis of infectious diseases including viral, bacterial or protozoological infectious diseases in a subject.

Embodiment 36. The vaccine composition and the pharmaceutical composition for use according to embodiment 34 or 35, wherein the vaccine composition or pharmaceutical composition is administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, periocular, periodontal, retrobulbar, subarachnoid, subconjunctival, suprachoroidal injection, infusion, implantation, nasal, buccal, sublingual, otic or auricular, ophthalmic, conjunctival, vaginal, rectal, intracervical, endosinusial, laryngeal, oropharyngeal, ureteral, urethral administration, more preferably said lipid nanoparticle is administered intramuscularly, intravenously, intradermally, subcutaneously, intratumorally, intranasally, or by inhalation to a subject, preferably via local or locoregional injection or infusion to a subject.

Embodiment 37. A kit or kit of parts, comprising the vaccine composition according to any one of embodiments 30 to 32 or the pharmaceutical composition according to embodiment 33, optionally comprising a liquid vehicle for solubilizing, and, optionally, technical instructions providing information on administration and dosage of the components.

Embodiment 38. A method of treatment or prophylaxis of infectious diseases; cancer or tumor diseases, disorders or conditions; liver diseases selected from the group consisting of liver fibrosis, liver cirrhosis and liver cancer; allergies; or autoimmune disease, disorder or condition; in a subject comprising the steps:
a) providing the vaccine composition of any one of embodiments 30 to 32 or the pharmaceutical composition according to embodiment 33 or the kit or kit of parts according to embodiment 37; and
b) applying or administering the vaccine composition or the pharmaceutical composition or the kit or kit of parts to a tissue or an organism of the subject.

Embodiment 39. A method of inducing an immune response in a subject, the method comprising administering to the subject the vaccine composition of any one of embodiments 1 to 32 or the pharmaceutical composition of embodiment 33 in an amount effective to produce an antigen-specific immune response in the subject.

Embodiment 40. A method of targeting a vaccine composition comprising a) at least one nucleic acid, preferably mRNA, encoding at least one antigen or fragment or variant thereof; and b) a carrier composition, preferably a lipid nanoparticle composition, to antigen-presenting cells including dendritic cells and macrophages, and/or to the spleen, the method comprising administering to the subject the vaccine composition of any one of embodiments 1 to 32 or the pharmaceutical composition of embodiment 33.

Embodiment 41. Use of a vaccine composition of any one of embodiments 1 to 32 or the pharmaceutical composition according to embodiment 33 or the kit or kit of parts according to embodiment 37 for (i) inducing an immune response, for (ii) inducing an antigen specific T-cell response, preferably for (iii) inducing CD8+ T cells responses, and/or for (iv) targeting the vaccine composition or the pharmaceutical composition to antigen-presenting cells, including dendritic cells and macrophages, and/or to the spleen, in a subject.

Embodiment 42. Use of phosphatidylserine in a vaccine of any one of the above embodiments or in a carrier composition of any one of the above embodiments comprising a) at least one nucleic acid, preferably mRNA, encoding at least one antigen or fragment or variant thereof; and b) a carrier composition, preferably a lipid nanoparticle composition, for targeting the vaccine composition to antigen-presenting cells, including dendritic cells and macrophages, and/or to the spleen, in a subject.

Embodiment 43. The vaccine composition or the pharmaceutical composition for use according to embodiment 34 or 35, the method according to embodiment 38, 39 or 40, or the use according to embodiment 41 or 42, wherein the subject is a mammalian subject, preferably a human subject.

Embodiment 44. A vaccine composition or carrier composition comprising
a) at least one nucleic acid encoding at least one antigen or fragment or variant thereof; and
b) a carrier composition, wherein the carrier composition comprises the phospholipid 1,2-diheptanoyl-sn-glycero-3-phosphocholine (DHPC).

Embodiment 45. Use of DHPC in the carrier composition of a vaccine composition comprising a) at least one nucleic acid, preferably mRNA, encoding at least one antigen or fragment or variant thereof; and b) a vaccine or carrier composition, preferably a lipid nanoparticle composition, for targeting the vaccine composition to antigen-presenting cells, including dendritic cells and macrophages, and/or to the spleen, in a subject.

Embodiment 46. A vaccine composition or the pharmaceutical composition comprising DHPC for use according to embodiment 34 or 35, the method according to embodiment 38, 39 or 40, or the use according to embodiment 41 or 42, wherein the subject is a mammalian subject, preferably a human subject.

### Fifth Set of Embodiments

Embodiment 1. A polymer conjugated lipid according to formula (I):

[P]-[linker]-[L] formula (I)

or a pharmaceutically acceptable salt, prodrug, tautomer or stereoisomer thereof, wherein
[P] is a heteropolymer moiety or homopolymer moiety, preferably a homopolymer moiety, comprising at least one polyoxazoline (POZ) monomer unit 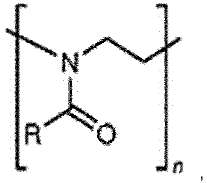 wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.4 kDa, or most preferably about 4.3 kDa
[linker] is an optional linker group, and
[L] is a lipid moiety.

Embodiment 2. The polymer conjugated lipid of **embodiment 1,** wherein [P] is a heteropolymer moiety or homopolymer moiety comprising multiple monomer units selected from the group consisting of
poly(2-methyl-2-oxazoline) (PMOZ) 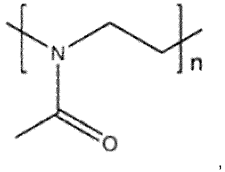
poly(2-ethyl-2-oxazoline) (PEOZ) 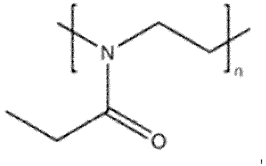
poly(2-propyl-2-oxazoline) (PPOZ) 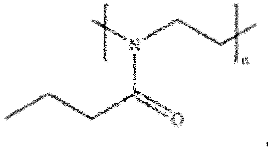
poly(2-butyl-2-oxazoline) (PBOZ) 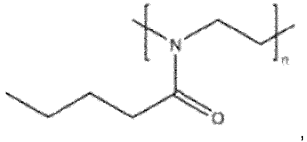
poly(2-isopropyl-2-oxazoline) (PIPOZ) 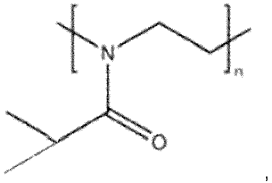
poly(2-methoxymethyl-2-oxazoline) (PMeOMeOx), and
poly(2-dimethylamino-2-oxazoline) (PDMAOx),

preferably wherein [P] is a homopolymer moiety comprising multiple PMOZ or PEOZ monomer units, more preferably wherein [P] comprises or preferably consists of multiple PMOZ monomer units,
wherein
   (i) n has a mean value ranging from about 45 to about 55, preferably n is about 50 or wherein
   (ii) n is selected such that the [P] moiety has an average molecular weight of about 4.2 kDa to about 4.4 kDa, or most preferably about 4.3 kDa.

Embodiment 3. The polymer conjugated lipid of any one of **embodiment 1 to embodiment 2,** wherein the polymer conjugated lipid is selected from the group consisting of a POZ-monoacylglycerol conjugate, POZ-diacylglycerol conjugate, a POZ-dialkyloxypropyl conjugate, a POZ-steroid or POZ-sterol conjugate, a POZ-phospholipid conjugate, a POZ-ceramide conjugate, and a mixture thereof.

Embodiment 4. The polymer conjugated lipid of any one of **embodiment 1 to embodiment 3,** wherein
(i) the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain,
   wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, -C(O)(CR³R⁴)C(O)O-, or-OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl), or
(ii) the lipid moiety [L] comprises ditetradecylamin, preferably wherein the linker group [linker] is (-NHC(O)CH₂CH₂C(O)-).

Embodiment 5. The polymer conjugated lipid of any one of **embodiment 1 to embodiment 4,** wherein the lipid moiety [L] comprises at least one straight or branched, saturated or unsaturated alkyl chain comprising 6, 7, 8, 9, 10, 11, 12, 13, 14, 15, 16, 17, 18, 19, 20, 21, 22, 23, 24, 25, 26, 27, 28, 29, or 30 carbon atoms, preferably in the range of 10 to 20 carbon atoms, more preferably in the range of 12 to 18 carbon atoms, even more preferably 14, 16 or 18 carbon atoms, even more preferably 16 or 18 carbon atoms, most preferably 14 carbon atoms, wherein all selections are independent of one another.

Embodiment 6. The polymer conjugated lipid of any one of **embodiment 1 to embodiment 5,** wherein the linker group [linker] is selected from the group consisting of but not limited to a pH-sensitive moiety, a peptide or amid bond (-CO-NH-), an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., - C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), (-NHC(O)CH₂CH₂C(O)O-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, - SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), and sulfonate esters, as well as combinations thereof, wherein R³, R⁴ and R⁵ are, independently H or alkyl (e.g. C₁-C₄ alkyl), preferably wherein the linker group [linker] is selected from the group consisting of (-NHC(O)CH2CH2C(O)-), a peptide bond or amid bond (-CO-NH-), (-NHC(O)CH2CH2C(O)O-), and -NH-CH2-.

Embodiment 7. The polymer conjugated lipid of any one of **embodiment 1 to embodiment** 6, wherein the linker group [linker] comprises an amide linker moiety, preferably an ester linker moiety, or wherein the linker group [linker] has the structure 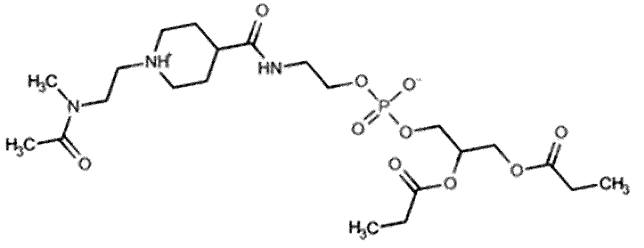 , or 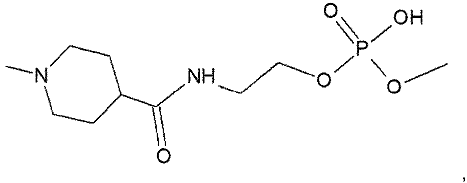 or preferably wherein the linker group comprises 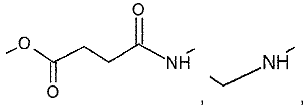 succinate, a peptide or amid bond (-CO-NH-), an amine, or a secondary amine, more preferably wherein the linker group [linker] comprises (-NHC(O)CH₂CH₂C(O)-).

Embodiment 8. The polymer conjugated lipid of any one of **embodiment 1 to embodiment 7,** wherein the polymer conjugated lipid has the structure of
(i) 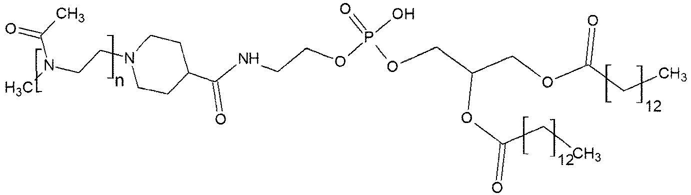 ("DM PE-PMOZ-v1")
   or preferably 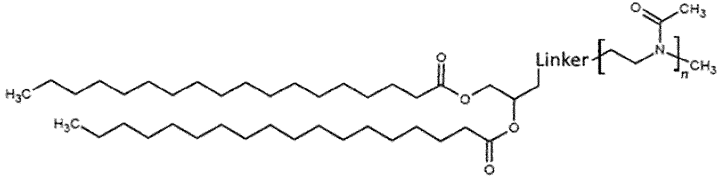 ; or 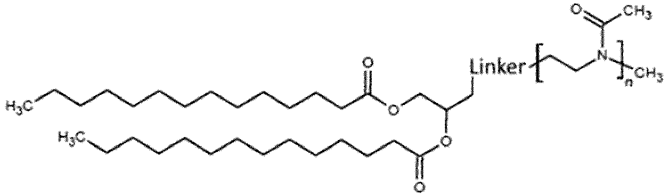 ; or 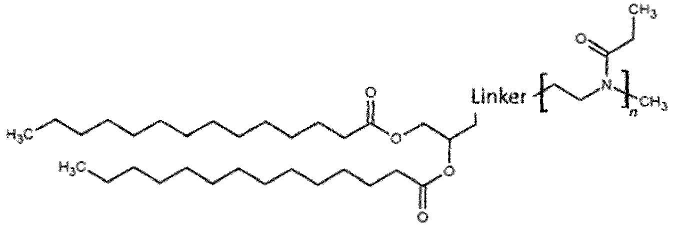 or 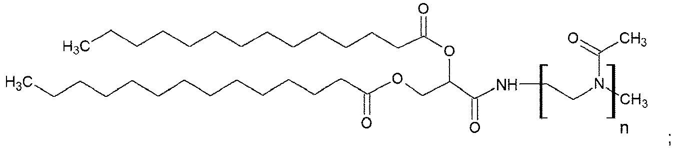 or 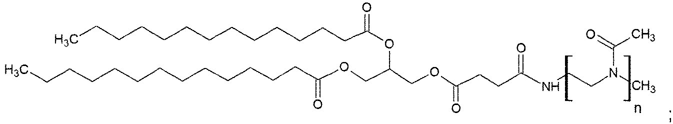 or 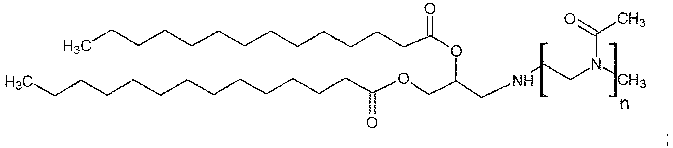 wherein the linker group [linker] is selected from any one of the linker groups as shown in **embodiment 6 or embodiment 7,** preferably the linker group [linker] comprising an ester moiety; or preferably
(ii) a "DMG-PMOZ" having the following structure: 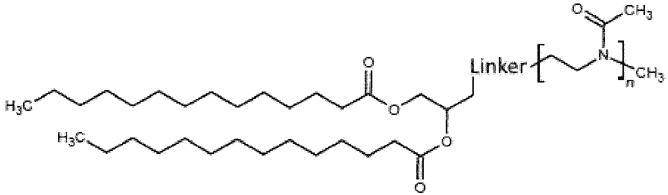 ("DMG-PMOZ"); or

very preferably the polymer conjugated lipid is selected from the group consisting of "PMOZ 1", "PMOZ 2", "PMOZ 3", "PMOZ 4" and "PMOZ 5";
whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 4.2 kDa to about 4.4 kDa, or most preferably about 4.3 kDa;
most preferably wherein the polymer conjugated lipid of any one of **embodiment 1 to embodiment 7** is DMG-PMOZ with n having a mean value from 45 to 50, most preferably 50.

Embodiment 8.1 In a very preferred embodiment, the polymer conjugated lipid is "PMOZ 1", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.2 In a very preferred embodiment, the polymer conjugated lipid is "PMOZ 2", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.3 In a very preferred embodiment, the polymer conjugated lipid is "PMOZ 3", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.4 In a very preferred embodiment, the polymer conjugated lipid is "PMOZ 4", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.5 In a very preferred embodiment, the polymer conjugated lipid is "PMOZ 5", preferably whereby n has a mean value ranging from 2 to 200, preferably from 20 to 100, more preferably from 24 to 26, even more preferably about 100, or further even more preferably from 45 to 50 or wherein n is selected such that the [P] moiety has an average molecular weight of 1.5 to 22 kDa, more preferably of 2 to 19 kDa, even more preferably of about 7.5 kDa or of about 15 kDa, preferably from 1 to 15 kDa, more preferably of 2 to 12.5 kDa, more preferably of about 5 kDa or of about 10 kDa, even more preferably of about 2 kDa to 2.5 kDa or of about 4 kDa to 5 kDa.

Embodiment 8.6 In a very preferred embodiment, n for the polymer conjugated lipid of the invention is selected such that the [P] moiety has an average molecular weight 5 kDa.

Embodiment 8.7 In a very preferred embodiment, n for the polymer conjugated lipid of the invention is selected such that the [P] moiety has an average molecular weight 4 kDa.

Embodiment 8.8 In a very preferred embodiment, n for the polymer conjugated lipid of the invention is selected such that the [P] moiety has an average molecular weight 4.2 kDa.

Embodiment 8.9 In a very preferred embodiment, n for the polymer conjugated lipid of the invention is selected such that the [P] moiety has an average molecular weight 4.3 kDa.

Embodiment 8.10 In a very preferred embodiment, n for the polymer conjugated lipid of the invention is selected such that the [P] moiety has an average molecular weight 4.4 kDa.

Embodiment 8.11 In a very preferred embodiment, n for the polymer conjugated lipid of the invention is selected such that the [P] moiety has an average molecular weight 4.5 kDa.

Embodiment 9. A lipid nanoparticle comprising a homopolymer moiety comprising at least one polyoxazoline (POZ) monomer unit 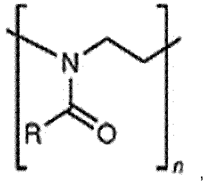 wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa,
preferably, wherein the homopolymer moiety comprising multiple monomer units comprises poly(2-methyl-2-oxazoline) (PMOZ), poly(2-ethyl-2-oxazoline) (PEOZ), poly(2-propyl-2-oxazoline) (PPOZ), poly(2-butyl-2-oxazoline) (PBOZ), poly(2-isopropyl-2-oxazoline) (PIPOZ), poly(2-methoxymethyl-2-oxazoline) (PMeOMeOx), or poly(2-dimethylamino-2-oxazoline) (PDMAOx),
more preferably the polymer conjugated lipid according to any one of **embodiment 1 to embodiment** 8.

Embodiment 10. The lipid nanoparticle of **embodiment 9,** wherein the lipid nanoparticle further comprises a cationic or ionizable lipid.

Embodiment 11. The lipid nanoparticle of **embodiment 9 to embodiment 10,** wherein the lipid nanoparticles
(i) do not comprise a polyethylene glycol-(PEG)-lipid conjugate or a conjugate of PEG and a lipid-like material, and preferably do not comprise PEG and/or
(ii) do not comprise a polymer conjugated lipid according to any one of **embodiment 1 to embodiment 8** comprising a sulphur group (-S-), a terminating nucleophile, and/or being covalently coupled to a biologically active ingredient being a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA.

Embodiment 12. The lipid nanoparticle of any one of **embodiment 9 to embodiment 11,** wherein the cationic or ionizable lipid preferably carries a net positive charge at physiological pH, more preferably wherein the cationic or ionizable lipid comprises a tertiary nitrogen group or quaternary nitrogen group.

Embodiment 13. The lipid nanoparticle of any one of **embodiment 9 to embodiment 12,** wherein the lipid nanoparticle further comprises a phospholipid, wherein preferably the phospholipid is a zwitterionic compound selected from, but not limited to the group of 1,2-diphytanoyl-sn-glycero-3-phosphoethanolamine (DPhyPE; 1,2-di-(3,7,11,15-tetramethylhexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; dioleoylphosphatidylcholine), 1,2-Dipalmitoyl-sn-glycero-3-phosphocholine (DPPC; dipalmitoylphosphatidylcholine), 1,2-dioleoyl-sn-glycero-3-phosphoethanolamine (DOPE), phosphatidylethanolamines, distearoylphosphatidylcholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyloleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Dimyristoyl-sn-glycero-3-phosphoethanolamine (DMPE), 1,2-Dilinoleoyl-sn-glycero-3-phosphoethanolamine (DLoPE), distearoyl-phosphatidylethanolamine (DSPE), 1-Palmitoyl-2-oleoyl-sn-glycero-3-phosphoethanolamine (POPE), 1,2-Dilauroyl-sn-glycero-3-phosphoethanolamine (DLPE), 16-O-monomethylphosphoethanolamine, 16-O-dimethyl phosphatidylethanolamine, 1,2-Dierucoyl-sn-glycero-3-phosphoethanolamine (DEPE), 18-1-trans phosphatidylethanolamine, 1-stearoyl-2-oleoylphosphatidyethanolamine (SOPE), 1,2-Disqualeoyl-sn-glycero-3-phosphoethanolamine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodium salt), 1-O-hexadecanyl-2-O-(9Z-octadecenyl)-sn-glycero-3-phosphoethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesterylhemisuccinoyl-sn-glycero-3-phosphocholine (PChemsPC), 1,2-dicholesterylhemisuccinoyl-sn-glycero-3-phosphocholine (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine), preferably wherein the phospholipid is DSPC or DPhyPE.

Embodiment 14. The lipid nanoparticle of any one of **embodiment 9 to embodiment 13,** wherein the lipid nanoparticle further comprises a sterol or steroid, preferably selected from the group consisting of cholesterol, cholesteryl hemisuccinate (CHEMS) and a derivate thereof, preferably wherein the lipid nanoparticle further comprises cholesterol.

Embodiment 15. The lipid nanoparticle of any one of **embodiment 9 to embodiment 14,** wherein preferably the lipid nanoparticle comprises
(i) an amount of 1, 2, 3, 4, 5, 6, 7, 8, 9, or 10 mol% of the polymer conjugated lipid of any one of **embodiment 1 to embodiment 8;**
(ii) preferably an amount of 5 mol% of the polymer conjugated lipid of any one of **embodiment 1 to embodiment 8,**
(iii) more preferably an amount of 2.5 mol% of the polymer conjugated lipid of any one of **embodiment 1 to embodiment 8,** or
(iv) also preferably an amount of 1.7 mol% of the polymer conjugated lipid of any one of **embodiment 1 to embodiment 8**
based upon a mol-percentage of the composition of 100% of all lipid components or excipients.

Embodiment 16. The lipid nanoparticle of any one of **embodiment 9 to embodiment 15,** wherein the polymer conjugated lipid is a PMOZ-lipid according to any one of **embodiment 1 to embodiment 8.**

Embodiment 17. The lipid nanoparticle of any one of **embodiment 9 to embodiment 16,** wherein the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
(v) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;**
(vi) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8;**
(vii) 47.4 mol% formula III-3 (((4-hydroxybutyl)azanediyl)bis(hexane-6,1-diyl)bis(2-hexyldecanoate)), 40.9 mol% cholesterol, 10 mol% DSPC, and 1.7 mol% of the polymer conjugated lipid of any one of **embodiment 1 to embodiment 8;**
(viii) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% 2-[(PMOZ)]n-N,N-ditetradecylacetamide]; and
(ix) 47.4 mol% formula III-3 (((4-hydroxybutyl)azanediyl)bis(hexane-6,1-diyl)bis(2-hexyldecanoate)), 40.9 mol% cholesterol, 10 mol% DSPC and 1.7 mol% 2-[(PMOZ)]n-N,N-ditetradecylacetamide],
wherein n has a mean value ranging from about 45 to about 55, preferably n is about 50 or wherein n is selected such that the polymer moiety has an average molecular weight of about 4.2 kDa to about 4.4 kDa, or most preferably about 4.3 kDa.

Embodiment 18. The lipid nanoparticle of any one of **embodiment 9 to embodiment 17,** wherein the polymer conjugated lipid of **embodiment 1 to embodiment 8** inhibits aggregation of the lipid nanoparticles.

Embodiment 19. The lipid nanoparticle of any one of **embodiment 9 to embodiment 18,** further comprising a biologically active ingredient.

Embodiment 20. The lipid nanoparticle of **embodiment 19,** wherein the biologically active ingredient is a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA; or any combination thereof, preferably wherein the biologically active ingredient is chemically modified mRNA or chemically unmodified mRNA, more preferably wherein the biologically active ingredient is chemically unmodified mRNA.

Embodiment 21. The lipid nanoparticle of any one of **embodiment 9 to embodiment 20,** wherein the mRNA is associated with the lipid nanoparticle, preferably wherein the mRNA is encapsulated in the lipid nanoparticle.

Embodiment 22. The lipid nanoparticle of any one of **embodiment 9 to embodiment 21,** wherein the lipid nanoparticles comprise the mRNA
(i) at an amount such as to achieve an N/P ratio in the range of about 1 to about 20, preferably about 2 to about 15, more preferably about 3 to about 10, even more preferably about 4 to about 9, most preferably about 6;
(ii) at an amount such as to achieve an N/P ratio in the range of about 5 to about 20, more preferably about 10 to about 18, even more preferably about 12 to about 16, most preferably about 14;
(iii) at an amount such as to achieve a lipid : mRNA weight ratio in the range of 20 to 60, preferably from about 3 to about 15, 5 to about 13, about 50 to about 70, about 4 to about 8 or from about 7 to about 11; or
(iv) at an amount such as to achieve an N/P ratio in the range of about 6 for a lipid nanoparticle according to **embodiment 17.**

Embodiment 23. The lipid nanoparticle of any one of **embodiment 9 to embodiment 22,** wherein the lipid nanoparticle is a sterile solid composition for reconstitution with a sterile liquid carrier, and wherein the lipid nanoparticle further comprises one or more inactive ingredients selected from pH-modifying agents, bulking agents, stabilizers, non-ionic surfactants and antioxidants, and wherein the sterile liquid carrier is an aqueous carrier.

Embodiment 24. The lipid nanoparticle of any one of **embodiment 9 to embodiment 23,** wherein the lipid nanoparticle is a sterile liquid composition, and wherein the lipid nanoparticles have a mean hydrodynamic diameter as determined by dynamic laser scattering from about 50 nm to about 300 nm, or from about 60 nm to about 250 nm, or from about 60 nm to about 200 nm, or from about 70 to 200 nm, or from about 75 nm to about 160, or from about 85 nm to about 140 nm, or from about 90 nm to about 130 nm, or from about 50 nm to about 120 nm.

Embodiment 25. The lipid nanoparticle of any one of **embodiment 9 to embodiment 24,** wherein the lipid nanoparticles exhibit a zeta potential in the range of -50 mV to +50 mV, preferably in the range of -25 mV to +25 mV, more preferably in the range of -10 mV to +10 mV, most preferably in the range of -5 mV to +5 mV.

Embodiment 26. The lipid nanoparticle of any one of **embodiment 9 to embodiment 25,** wherein the mRNA compound is a mono-, bi-, or multicistronic mRNA.

Embodiment 27. The lipid nanoparticle of any one **embodiment 9 to embodiment 26,** wherein the mRNA compound comprises at least one chemical modification.

Embodiment 28. The lipid nanoparticle of **embodiment 27,** wherein the chemical modification is selected from the group consisting of base modifications, sugar modifications, backbone modifications and lipid modifications, preferably wherein the chemical modification is a base modification, more preferably wherein the base modification preferably is selected from the group consisting of pseudouridine (psi or ψ), N1-methylpseudouridine (N1MPU, N1Mpsi or N1Mψ), 1-ethylpseudouracil, 2-thiouracil (s2U), 4-thiouracil, 5-methylcytosine, 5-methyluracil, 5-methoxyuracil, and any combination thereof.

Embodiment 29. The lipid nanoparticle of any one of **embodiment 9 to embodiment 28,** wherein the mRNA compound comprises a coding region encoding a peptide or protein, wherein the coding region exhibits a sequence modification.

Embodiment 30. The lipid nanoparticle of **embodiment 29,** wherein the sequence modification is selected from a G/C content modification, a codon modification, a codon optimization or a C-optimization of the sequence; preferably wherein, compared with the coding region of the corresponding wild-type mRNA, the
- G/C content of the coding region is increased;
- C content of the coding region is increased;
- codon usage in the coding region is adapted to the human codon usage; and/or
- codon adaptation index (CAI) is increased or maximized in the coding region.

Embodiment 31. The lipid nanoparticle of any one of **embodiment 9 to embodiment 30,** wherein the mRNA compound further comprises
a) a 5'-CAP structure, preferably m7GpppN, more preferably CAP1 or m7G(5')ppp(5')(2'OMeA)pG;
b) optionally at least one miRNA sequence, preferably wherein the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof;
c) at least one 5'-UTR element;
d) a coding sequence;
e) at least one 3'-UTR element;
f) at least one poly(A) sequence;
g) at least one poly(C) sequence;
or any combinations of these.

Embodiment 32. The lipid nanoparticle of any one of **embodiment 9 to embodiment 31,** wherein the least one coding RNA comprises a 5'-CAP structure, preferably m7G, CAP0, CAP1, CAP2, a modified CAP0 or a modified CAP1 structure.

Embodiment 33. The lipid nanoparticle of any one of **embodiment 9 to embodiment 32,** wherein the at least one coding RNA comprises at least one heterologous 5'-UTR and/or at least one heterologous 3'-UTR, preferably wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes; and/or preferably wherein the at least one heterologous 3'-UTR comprises a nucleic acid sequence derived from a 3'-UTR of a gene selected from PSMB3, ALB7, alpha-globin, CASP1, COX6B1, GNAS, NDUFA1 and RPS9, or from a homolog, a fragment or a variant of any one of these genes.

Embodiment 34. The lipid nanoparticle of any one of **embodiment 9 to embodiment 33,** wherein the at least one coding RNA comprises a (i) HSD17B4 5'-UTR and a PSMB3 3'-UTR or (ii) a RPL32 5'-UTR and an ALB7 3'-UTR, preferably a mutated alpha-globin 3'-UTR (SEQ ID NO:11/12), more preferably a HSD17B4 5'-UTR (SEQ ID NO:21/22) and a PSMB3 3'-UTR (SEQ ID NO:19/20).

Embodiment 35. The lipid nanoparticle of any one of **embodiment 9 to embodiment 34,** comprising the following elements in the 5' to 3' direction:
a) a 5'-CAP structure, preferably selected from the group consisting of m7G(5'), m7G(5')ppp(5')(2'OMeA)pG and m7G(5')ppp(5')(2'OMeG)pG;
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, 24, 26, or a homolog, a fragment or a variant thereof, most preferably according to SEQ ID NO:22 (HSD17B4);
c) at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8, 10, 12, 14, 16, 18, 20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18, or a homolog, a fragment or a variant thereof, most preferably according to SEQ ID NO:20 (PSMB3);
e) optionally, at least one poly(A) sequence, preferably consisting of 10 to 200, 10 to 100, 40 to 80, or 50 to 70 adenosine nucleotides;
f) optionally, at least one poly(C) sequence, preferably consisting of 10 to 200, 10 to 100, 20 to 70, 20 to 60 or 10 to 40 cytosine nucleotides; and
g) optionally, at least one histone stem-loop, preferably comprising the RNA sequence according to SEQ ID NO:4.

Embodiment 36. The lipid nanoparticle of any one of **embodiment 9 to embodiment 35,** wherein the biologically active ingredient is
(a) an mRNA comprising at least one coding sequence encoding a peptide or protein, or a fragment or variant thereof, wherein the peptide or protein is an antigen, wherein the antigen preferably is derived from pathogenic antigens, tumor antigens, allergenic antigens or autoimmune self-antigens, or a fragment or variant thereof; or
(b) an mRNA comprising at least one coding sequence encoding a therapeutic protein, or a fragment or variant thereof, wherein the therapeutic protein is selected from the group consisting of
   (i) therapeutic proteins for use in enzyme replacement therapy for the treatment of metabolic, endocrine or amino acid disorders or for use in replacing an absent, deficient or mutated protein;
   (ii) therapeutic proteins for use in the treatment of blood disorders, diseases of the circulatory system, diseases of the respiratory system, infectious diseases or immune deficiencies;
   (iii) therapeutic proteins for use in the treatment of cancer or tumor diseases;
   (iv) therapeutic proteins for use in hormone replacement therapy;
   (v) therapeutic proteins for use in reprogramming somatic cells into pluri- or omnipotent stem cells;
   (vi) therapeutic proteins for use as adjuvant or immunostimulation;
   (vii) therapeutic proteins being a therapeutic antibody;
   (viii) therapeutic proteins being a gene editing agent; and
   (ix) therapeutic proteins for use in treating or preventing a liver disease selected from the group consisting of liver fibrosis, liver cirrhosis and liver cancer.

Embodiment 37. The lipid nanoparticle of **embodiment 36 subitem (a),** wherein the at least one coding sequence encoding a pathogenic antigen is selected from the group consisting of a bacterial, viral, fungal and protozoal antigen.

Embodiment 38. The lipid nanoparticle of **embodiment 37,** wherein the at least one coding sequence encoding a pathogenic antigen
(i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
(ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
(iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof (e.g. receptor binding domain (RBD), critical neutralisation domain (CND)); and/or
(iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation.

Embodiment 39. The lipid nanoparticle of any one of **embodiment 9 to embodiment 38** for use
(i) in the treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition; and/or
(ii) for use in enzyme replacement therapy for the treatment of metabolic or endocrine disorders or for use in replacing an absent, deficient or mutated protein.

Embodiment 40. The lipid nanoparticle of any one of **embodiment 9 to embodiment 39** for use in the treatment or prophylaxis of infectious diseases.

Embodiment 41. The lipid nanoparticle of **embodiment 9 or embodiment 40** comprising at least one coding RNA, wherein said at least one coding RNA comprises at least one coding sequence encoding at least one peptide or protein for use in treatment or prevention of a disease, disorder or condition, wherein said lipid nanoparticle is administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, intraocular, periocular, periodontal, retrobulbar, subarachnoid, subconjunctival, suprachoroidal injection, infusion, implantation, nasal, buccal, sublingual, otic or auricular, ophthalmic, conjunctival, vaginal, rectal, intracervical, endosinusial, laryngeal, oropharyngeal, ureteral, urethral administration, more preferably said lipid nanoparticle is administered intramuscularly, intravenously, intradermally, subcutaneously, intratumorally, intranasally, or by inhalation, most preferably intramuscularly, to a subject in need thereof.

Embodiment 42. A kit or kit of parts, comprising any one of the lipid nanoparticle of **embodiment 9 to embodiment 41,** optionally comprising a liquid vehicle for solubilizing, and, optionally, technical instructions providing information on administration and dosage of the components.

Embodiment 43. The lipid nanoparticle of any one of **embodiment 9 to embodiment 41** or the kit or kit of parts of **embodiment 42** for use in *in vivo* drug delivery, preferably for use in delivering a nucleic acid, preferably a mRNA.

Embodiment 44. The lipid nanoparticle of any one of **embodiment 9 to embodiment 41** or the kit or kit of parts of **embodiment 43** for use as a medicament.

Embodiment 45. The lipid nanoparticle for use as a medicament according to **embodiment 44,** wherein the medicament is for the prevention, prophylaxis, treatment and/or amelioration of a disease selected from infectious diseases including viral, bacterial or protozoological infectious diseases, cancer or tumor diseases, liver diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and diseases associated with a peptide or protein deficiency.

Embodiment 46. The lipid nanoparticle for use as a medicament according to **embodiment 44 or embodiment 45,** wherein the medicament is a vaccine composition.

Embodiment 47. A vaccine composition comprising a lipid nanoparticle of any one of **embodiment 9 to embodiment 46** or a kit or kit of parts of **embodiment 42** for use as a medicament, and/or for prevention, prophylaxis, treatment and/or amelioration of a disease selected from infectious diseases including viral, bacterial or protozoological infectious diseases, cancer or tumor diseases.

Embodiment 48. A method of treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition comprising the steps:
a) providing a lipid nanoparticle of any one of **embodiment 9 to embodiment 45,** comprising a homopolymer moiety comprising at least one polyoxazoline (POZ) monomer, preferably the polymer conjugated lipid according to any one of **embodiment 1 to embodiment 8,** the vaccine composition of **embodiment 47,** or the kit or kit of parts of **embodiment 42;** and
b) applying or administering the mRNA, the lipid nanoparticle, the vaccine composition or the kit or kit of parts to a tissue or an organism.

Embodiment 49. A method for delivering mRNA encoding an antigen or a therapeutic peptide or protein to a subject, the method comprising administering to a subject a lipid nanoparticle of any one of embodiments 1 to 33, wherein the mRNA encodes an antigen or a therapeutic peptide or protein, and wherein delivering the mRNA to the subject is beneficial in treating or preventing a disease or disorder, preferably wherein the subject is a mammal, more preferably wherein the subject is a human.

Embodiment 50. The method according to any one of embodiments **embodiment 48 to embodiment 49,** wherein the mRNA, the lipid nanoparticle of any one **of embodiment 9 to embodiment 48,** the vaccine composition of **embodiment 47** or the kit or kit of parts of **embodiment 42** is administered to the tissue or to the organism by intravenous, intramuscular, subcutaneous, intradermal or intratumoral injection or any administration route as disclosed in any **preceding embodiment.**

Embodiment 51. A method of inducing an immune response in a subject, the method comprising administering to the subject the vaccine composition of **embodiment 47** in an amount effective to produce an antigen-specific immune response in the subject.

Embodiment 52. A pharmaceutical composition comprising a lipid nanoparticle of any one of **embodiment 9 to embodiment 48** or a kit or kit of parts of **embodiment 42** or the vaccine composition of **embodiment 47** for use in vaccination of a subject comprising an effective dose of mRNA encoding a virus antigen.

Embodiment 53. Use of a pharmaceutical composition according to **embodiment 52** or a kit or kit of parts according to **embodiment 42** for (i) inducing an immune response, for (ii) inducing an antigen specific T-cell response or preferably for (iii) inducing CD8+ T cells responses.

Embodiment 54. Use of the pharmaceutical composition of **embodiment 52** for the prophylaxis of an infectious disease or in the manufacture of a medicament for the prophylaxis of an infectious disease, wherein said medicament preferably is a vaccine composition.

Embodiment 55. A method for preventing, ameliorating or treating a disease or condition in a subject in need comprising administering to the subject a lipid nanoparticle of any one of **embodiment 9 to embodiment 48,** a pharmaceutical composition of **embodiment 52** or a kit or kit of parts of **embodiment 42.**

Embodiment 56. The method of any one of the **preceding method embodiments,** wherein administration of the lipid nanoparticle results in expression of the antigen encoded by mRNA in the lymphocytes of the subject.

Embodiment 57. A method of treating or preventing a disorder of any one of **embodiments 36, 39, 41, 45, 48, or 49,** wherein the disorder is an infection with coronavirus, or a disorder related to such an infection.

Embodiment 58. A method of treating or preventing a disorder of any one of **embodiments 36, 39, 41, 45, 48, or 49,** wherein the subject in need is a mammalian subject, preferably a human subject.

Embodiment 59. The method of any one of the **preceding method embodiments,** wherein the administration of the lipid nanoparticle results in an antigen specific antibody response, preferably wherein the antigen specific antibody response is measured by the presence of antigen-specific antibodies in serum.

Embodiment 60. The lipid nanoparticle of any one of **embodiment 9 to embodiment 16,** wherein the lipid nanoparticle comprises excipients selected from ratios selected from the group consisting of
(i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
(ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
(iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
(iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;** and
(vi) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8.**

Embodiment 61. The lipid nanoparticle of any one of **embodiment 9 to embodiment 16 or embodiment 60,** wherein the lipid nanoparticle comprises a neutral lipid or phospholipid having at least one alkyl chain with a length of Cs, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₆, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).

Embodiment 62. The lipid nanoparticle of any one of **embodiment 9 to embodiment 16 or embodiment 60 to embodiment 61,** wherein the lipid nanoparticles comprise a neutral lipid or phospholipid having at least two alkyl chains, whereby each alkyl chain independently has a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₆, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).

Embodiment 63. The lipid nanoparticle of any one of **embodiment 9 to embodiment 16 or embodiment 60 to embodiment 62** or a lipid nanoparticle comprising a polymer conjugated lipid according to any one of **embodiment 1** to **embodiment 8,** wherein the lipid nanoparticle has a lower PDI and/or lower size as compared to a control lipid nanoparticle comprising a PEG-lipid instead of a polymer conjugated lipid according to any one of **embodiment 1** to **embodiment 8.**

### Sixth Set of Embodiments

Embodiment 1. A polymer conjugated lipid according to formula (I):

[P]-[linker]-[L] formula (I)

or a pharmaceutically acceptable salt, prodrug, tautomer or stereoisomer thereof, wherein
- [P]: is a homopolymer moiety, comprising at least one polyoxazoline (POZ) monomer unit 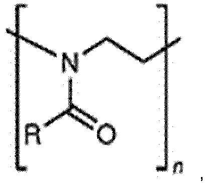 wherein R is C1 alkyl, and n has a mean value ranging from about 45 to about 55, preferably n is about 50;
- [linker]: is an optional linker group, and
- [L]: is a lipid moiety.

Embodiment 2. The polymer conjugated lipid of **embodiment 1**, wherein [P] is a homopolymer moiety comprising multiple monomer units selected from the group consisting of
poly(2-methyl-2-oxazoline) (PMOZ) 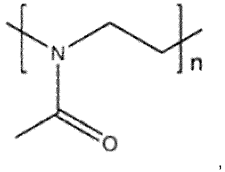 wherein
(i) n has a mean value ranging from about 45 to about 55, preferably n is about 50.

Embodiment 3. The polymer conjugated lipid of any one of **embodiment 1 to embodiment 2,** wherein the polymer conjugated lipid is selected from the group consisting of a POZ-monoacylglycerol conjugate, POZ-diacylglycerol conjugate, a POZ-dialkyloxypropyl conjugate, a POZ-steroid or POZ-sterol conjugate, a POZ-phospholipid conjugate, a POZ-ceramide conjugate, and a mixture thereof.

Embodiment 4. The polymer conjugated lipid of any one of **embodiment 1 to embodiment 3,** wherein
(i) the lipid moiety [L] comprises two straight saturated alkyl chains.

Embodiment 5. The polymer conjugated lipid of any one of **embodiment 1 to embodiment 4,** wherein the lipid moiety [L] comprises at least one straight or branched, saturated or unsaturated alkyl chain comprising 14 carbon atoms.

Embodiment 6. The polymer conjugated lipid of any one of **embodiment 1 to embodiment 5,** wherein the linker group [linker] is succinamidyl (-NHC(O)CH₂CH₂C(O)NH-).

Embodiment 7. The polymer conjugated lipid of any one of **embodiment 1 to embodiment 6,** wherein the linker group [linker] has the structure

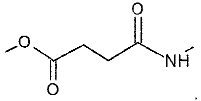

Embodiment 8. The polymer conjugated lipid of any one of **embodiment 1 to embodiment** 7, wherein the polymer conjugated lipid has the structure of "PMOZ 4", whereby n of "PMOZ 4" has a mean value ranging from 30 to 70, more preferably from 40 to 60, even more preferably from 45 to 50, most preferably n has a mean value of 50.

Embodiment 9. A lipid nanoparticle comprising a polymer conjugated lipid according to any one of **embodiment 1 to embodiment 8.**

Embodiment 10. The lipid nanoparticle of **embodiment 9,** wherein the lipid nanoparticle further comprises a cationic or ionizable lipid.

Embodiment 11. The lipid nanoparticle of **embodiment 9 to embodiment 10,** wherein the lipid nanoparticles
(i) do not comprise a polyethylene glycol-(PEG)-lipid conjugate or a conjugate of PEG and a lipid-like material, and preferably do not comprise PEG and
(ii) do not comprise a polymer conjugated lipid according to any one of **embodiment 1 to embodiment 8** comprising a sulphur group (-S-) and/or a terminating nucleophile, and/or being covalently coupled to a nucleic acid compound.

Embodiment 12. The lipid nanoparticle of any one of **embodiment 9 to embodiment 11,** wherein the cationic or ionizable lipid preferably carries a net positive charge at physiological pH, more preferably wherein the cationic or ionizable lipid comprises a tertiary nitrogen group or quaternary nitrogen group.

Embodiment 13. The lipid nanoparticle of any one of **embodiment 9 to embodiment 12,** wherein the lipid nanoparticle further comprises a phospholipid, preferably DPhyPE.

Embodiment 14. The lipid nanoparticle of any one of **embodiment 9 to embodiment 13,** wherein the lipid nanoparticle further comprises a sterol or steroid, preferably cholesterol.

Embodiment 15. The lipid nanoparticle of any one of **embodiment 9 to embodiment 14,** wherein preferably the lipid nanoparticle comprises an amount of 2,5 mol% of the polymer conjugated lipid of any one of **embodiment 1 to embodiment 8** based upon a mol-percentage of the composition of 100% of all lipid components or excipients.

Embodiment 16. The lipid nanoparticle of any one of **embodiment 15,** wherein the polymer conjugated lipid is a PMOZ-lipid according to any one of **embodiment 8.**

Embodiment 17. The lipid nanoparticle of any one of **embodiment 9 to embodiment 16,** wherein the lipid nanoparticle comprises 59 mol% cationic or ionizable lipid, preferably C24, 28.5 mol% cholesterol, 10 mol% neutral lipid and 2.5 mol% of the polymer conjugated lipid of any one of **embodiment 1 to embodiment 8.**

Embodiment 18. The lipid nanoparticle of any one of **embodiment 9 to embodiment 17,** wherein the polymer conjugated lipid of **embodiment 1 to embodiment 8** inhibits aggregation of the lipid nanoparticles.

Embodiment 19. The lipid nanoparticle of any one of **embodiment 9 to embodiment 18,** further comprising a biologically active ingredient.

Embodiment 20. The lipid nanoparticle of **embodiment 19,** wherein the biologically active ingredient is chemically modified or unmodified messenger RNA (mRNA) comprising at least one coding sequence.

Embodiment 20.1. The lipid nanoparticle of **embodiment 19,** wherein the biologically active ingredient is chemically modified messenger RNA (mRNA) comprising at least one coding sequence.

Embodiment 20.2 The lipid nanoparticle of **embodiment 19,** wherein the biologically active ingredient is unmodified messenger RNA (mRNA) comprising at least one coding sequence.

Embodiment 21. The lipid nanoparticle of any one of **embodiment 9 to embodiment 20,** wherein the mRNA is associated with the lipid nanoparticle, preferably wherein the mRNA is encapsulated in the lipid nanoparticle.

Embodiment 22. The lipid nanoparticle of any one of **embodiment 9 to embodiment 21,** wherein the lipid nanoparticles comprise the mRNA at an amount such as to achieve an N/P ratio in the range of about 5 to about 20, more preferably about 10 to about 18, even more preferably about 12 to about 16, most preferably about 14.

Embodiment 23. The lipid nanoparticle of any one of **embodiment 9 to embodiment 22,** wherein the lipid nanoparticle is a sterile solid composition for reconstitution with a sterile liquid carrier, and wherein the lipid nanoparticle further comprises one or more inactive ingredients selected from pH-modifying agents, bulking agents, stabilizers, non-ionic surfactants and antioxidants, and wherein the sterile liquid carrier is an aqueous carrier.

Embodiment 24. The lipid nanoparticle of any one of **embodiment 9 to embodiment 23,** wherein the lipid nanoparticle is a sterile liquid composition, and wherein the lipid nanoparticles have a mean hydrodynamic diameter as determined by dynamic laser scattering from about 50 nm to about 300 nm, or from about 60 nm to about 250 nm, or from about 60 nm to about 200 nm, or from about 70 to 200 nm, or from about 75 nm to about 160, or from about 85 nm to about 140 nm, or from about 90 nm to about 130 nm, or from about 50 nm to about 120 nm.

Embodiment 25. The lipid nanoparticle of any one of **embodiment 9 to embodiment 24,** wherein the lipid nanoparticles exhibit a zeta potential in the range of -5 mV to +5 mV.

Embodiment 26. The lipid nanoparticle of any one of **embodiment 9 to embodiment 25,** wherein the mRNA compound is a mono-, bi-, or multicistronic mRNA.

Embodiment 27. The lipid nanoparticle of any one **embodiment 9 to embodiment 26,** wherein the mRNA compound comprises at least one chemical modification.

Embodiment 28. The lipid nanoparticle of **embodiment 27,** wherein the chemical modification is N1-methylpseudouridine (N1MPU, N1Mpsi or N1Mψ),

Embodiment 29. The lipid nanoparticle of any one of **embodiment 9 to embodiment 28,** wherein the mRNA compound comprises a coding region encoding a peptide or protein, wherein the coding region exhibits a sequence modification.

Embodiment 30. The lipid nanoparticle of **embodiment 29,** wherein the sequence modification is selected from a G/C content modification, a codon modification, a codon optimization or a C-optimization of the sequence; preferably wherein, compared with the coding region of the corresponding wild-type mRNA, the
- G/C content of the coding region is increased;
- C content of the coding region is increased;
- codon usage in the coding region is adapted to the human codon usage; and/or
- codon adaptation index (CAI) is increased or maximized in the coding region.

Embodiment 31. The lipid nanoparticle of any one of **embodiment 9 to embodiment 30,** wherein the mRNA compound further comprises
a) a 5'-CAP structure, preferably m7GpppN, more preferably CAP1 or m7G(5')ppp(5')(2'OMeA)pG;
b) optionally at least one miRNA sequence, preferably wherein the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof;
c) at least one 5'-UTR element;
d) a coding sequence;
e) at least one 3'-UTR element;
f) at least one poly(A) sequence;
g) at least one poly(C) sequence;
or any combinations of these.

Embodiment 32. The lipid nanoparticle of any one of **embodiment 9 to embodiment 31,** wherein the least one coding RNA comprises a 5'-CAP structure, preferably a CAP1 structure, more preferably CleanCap^{™} or respectively m7G(5')ppp(5')(2'OMeA)pG.

Embodiment 33. The lipid nanoparticle of any one of **embodiment 9 to embodiment 32,** wherein the at least one coding RNA comprises at least one heterologous 5'-UTR and/or at least one heterologous 3'-UTR, preferably wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes; and/or preferably wherein the at least one heterologous 3'-UTR comprises a nucleic acid sequence derived from a 3'-UTR of a gene selected from PSMB3, ALB7, alpha-globin, CASP1, COX6B1, GNAS, NDUFA1 and RPS9, or from a homolog, a fragment or a variant of any one of these genes.

Embodiment 34. The lipid nanoparticle of any one of **embodiment 9 to embodiment 33,** wherein the at least one coding RNA comprises a (i) HSD17B4 5'-UTR and a PSMB3 3'-UTR or (ii) a RPL32 5'-UTR and an ALB7 3'-UTR, preferably a mutated alpha-globin 3'-UTR (SEQ ID NO:11/12), more preferably a HSD17B4 5'-UTR (SEQ ID NO:21/22) and a PSMB3 3'-UTR (SEQ ID NO:19/20).

Embodiment 35. The lipid nanoparticle of any one of **embodiment 9 to embodiment 34,** comprising the following elements in the 5' to 3' direction:
a) a 5'-CAP structure, preferably selected from the group consisting of m7G(5'), m7G(5')ppp(5')(2'OMeA)pG and m7G(5')ppp(5')(2'OMeG)pG;
b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, 24, 26, or a homolog, a fragment or a variant thereof, most preferably according to SEQ ID NO:22 (HSD17B4);
c) at least one coding sequence;
d) a 3'-UTR element comprising a nucleic acid sequence derived from an α-globin gene, said nucleic acid sequence preferably comprising an RNA sequence that corresponds to the nucleic acid sequence according to SEQ ID NO:6, 8, 10, 12, 14, 16, 18, 20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18, or a homolog, a fragment or a variant thereof, most preferably according to SEQ ID NO:20 (PSMB3);
e) optionally, at least one poly(A) sequence, preferably consisting of 10 to 200, 10 to 100, 40 to 80, or 50 to 70 adenosine nucleotides;
f) optionally, at least one poly(C) sequence, preferably consisting of 10 to 200, 10 to 100, 20 to 70, 20 to 60 or 10 to 40 cytosine nucleotides; and
g) optionally, at least one histone stem-loop, preferably comprising the RNA sequence according to SEQ ID NO:4.

Embodiment 36. The lipid nanoparticle of any one of **embodiment 9 to embodiment 35,** wherein the biologically active ingredient is
(a) an mRNA comprising at least one coding sequence encoding a peptide or protein, or a fragment or variant thereof, wherein the peptide or protein is an antigen, wherein the antigen preferably is derived from pathogenic antigens, tumor antigens, allergenic antigens or autoimmune self-antigens, or a fragment or variant thereof; or
(b) an mRNA comprising at least one coding sequence encoding a therapeutic protein, or a fragment or variant thereof, wherein the therapeutic protein is selected from the group consisting of
   (i) therapeutic proteins for use in enzyme replacement therapy for the treatment of metabolic, endocrine or amino acid disorders or for use in replacing an absent, deficient or mutated protein;
   (ii) therapeutic proteins for use in the treatment of blood disorders, diseases of the circulatory system, diseases of the respiratory system, infectious diseases or immune deficiencies;
   (iii) therapeutic proteins for use in the treatment of cancer or tumor diseases;
   (iv) therapeutic proteins for use in hormone replacement therapy;
   (v) therapeutic proteins for use in reprogramming somatic cells into pluri- or omnipotent stem cells;
   (vi) therapeutic proteins for use as adjuvant or immunostimulation;
   (vii) therapeutic proteins being a therapeutic antibody;
   (viii) therapeutic proteins being a gene editing agent; and
   (ix) therapeutic proteins for use in treating or preventing a liver disease selected from the group consisting of liver fibrosis, liver cirrhosis and liver cancer.

Embodiment 37. The lipid nanoparticle of **embodiment 36 subitem (a),** wherein the at least one coding sequence encoding a pathogenic antigen is selected from the group consisting of a bacterial, viral, fungal and protozoal antigen.

Embodiment 38. The lipid nanoparticle of **embodiment 37,** wherein the at least one coding sequence encoding a pathogenic antigen
(i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus (EBOV), Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
(ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
(iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof (e.g. receptor binding domain (RBD), critical neutralisation domain (CND)); and/or
(iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation.

Embodiment 39. The lipid nanoparticle of any one of **embodiment 9 to embodiment 38** for use
(i) in the treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition; and/or
(ii) for use in enzyme replacement therapy for the treatment of metabolic or endocrine disorders or for use in replacing an absent, deficient or mutated protein.

Embodiment 40. The lipid nanoparticle of any one of **embodiment 9 to embodiment 39** for use in the treatment or prophylaxis of infectious diseases.

Embodiment 41. The lipid nanoparticle of **embodiment 9 or embodiment 40** comprising at least one coding RNA, wherein said at least one coding RNA comprises at least one coding sequence encoding at least one peptide or protein for use in treatment or prevention of a disease, disorder or condition, wherein said lipid nanoparticle is administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, intraocular, periocular, periodontal, retrobulbar, subarachnoid, subconjunctival, suprachoroidal injection, infusion, implantation, nasal, buccal, sublingual, otic or auricular, ophthalmic, conjunctival, vaginal, rectal, intracervical, endosinusial, laryngeal, oropharyngeal, ureteral, urethral administration, more preferably said lipid nanoparticle is administered intramuscularly, intravenously, intradermally, subcutaneously, intratumorally, intranasally, or by inhalation, most preferably intramuscularly, to a subject in need thereof.

Embodiment 42. A kit or kit of parts, comprising any one of the lipid nanoparticle of **embodiment 9 to embodiment 41,** optionally comprising a liquid vehicle for solubilizing, and, optionally, technical instructions providing information on administration and dosage of the components.

Embodiment 43. The lipid nanoparticle of any one of **embodiment 9 to embodiment 41** or the kit or kit of parts of **embodiment 42** for use in *in vivo* drug delivery, preferably for use in delivering a nucleic acid, preferably a mRNA.

Embodiment 44. The lipid nanoparticle of any one of **embodiment 9 to embodiment 41** or the kit or kit of parts of **embodiment 43** for use as a medicament.

Embodiment 45. The lipid nanoparticle for use as a medicament according to **embodiment 44,** wherein the medicament is for the prevention, prophylaxis, treatment and/or amelioration of a disease selected from infectious diseases including viral, bacterial or protozoological infectious diseases, cancer or tumor diseases, liver diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and diseases associated with a peptide or protein deficiency.

Embodiment 46. The lipid nanoparticle for use as a medicament according to **embodiment 44 or embodiment 45,** wherein the medicament is a vaccine composition.

Embodiment 47. A vaccine composition comprising a lipid nanoparticle of any one of **embodiment 9 to embodiment 46** or a kit or kit of parts of **embodiment 42** for use as a medicament, and/or for prevention, prophylaxis, treatment and/or amelioration of a disease selected from infectious diseases including viral, bacterial or protozoological infectious diseases, cancer or tumor diseases.

Embodiment 48. A method of treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition comprising the steps:
a) providing a lipid nanoparticle of any one of **embodiment 9 to embodiment 45,** comprising a homopolymer moiety comprising at least one polyoxazoline (POZ) monomer, preferably the polymer conjugated lipid according to any one of **embodiment 1 to embodiment 8,** the vaccine composition of **embodiment 47,** or the kit or kit of parts of **embodiment 42;** and
b) applying or administering the mRNA, the lipid nanoparticle, the vaccine composition or the kit or kit of parts to a tissue or an organism.

Embodiment 49. A method for delivering mRNA encoding an antigen or a therapeutic peptide or protein to a subject, the method comprising administering to a subject a lipid nanoparticle of any one of embodiments 1 to 33, wherein the mRNA encodes an antigen or a therapeutic peptide or protein, and wherein delivering the mRNA to the subject is beneficial in treating or preventing a disease or disorder, preferably wherein the subject is a mammal, more preferably wherein the subject is a human.

Embodiment 50. The method according to any one of embodiments **embodiment 48 to embodiment 49,** wherein the mRNA, the lipid nanoparticle of any one **of embodiment 9 to embodiment 48,** the vaccine composition of **embodiment 47** or the kit or kit of parts of **embodiment 42** is administered to the tissue orto the organism by intravenous, intramuscular, subcutaneous, intradermal or intratumoral injection or any administration route as disclosed in any **preceding embodiment.**

Embodiment 51. A method of inducing an immune response in a subject, the method comprising administering to the subject the vaccine composition of **embodiment 47** in an amount effective to produce an antigen-specific immune response in the subject.

Embodiment 52. A pharmaceutical composition comprising a lipid nanoparticle of any one of **embodiment 9 to embodiment 48** or a kit or kit of parts of **embodiment 42** or the vaccine composition of **embodiment 47** for use in vaccination of a subject comprising an effective dose of mRNA encoding a virus antigen.

Embodiment 53. Use of a pharmaceutical composition according to **embodiment 52** or a kit or kit of parts according to **embodiment 42** for (i) inducing an immune response, for (ii) inducing an antigen specific T-cell response or preferably for (iii) inducing CD8+ T cells responses.

Embodiment 54. Use of the pharmaceutical composition of **embodiment 52** for the prophylaxis of an infectious disease or in the manufacture of a medicament for the prophylaxis of an infectious disease, wherein said medicament preferably is a vaccine composition.

Embodiment 55. A method for preventing, ameliorating or treating a disease or condition in a subject in need comprising administering to the subject a lipid nanoparticle of any one of **embodiment 9 to embodiment 48,** a pharmaceutical composition of **embodiment 52** or a kit or kit of parts of **embodiment 42.**

Embodiment 56. The method of any one of the **preceding method embodiments,** wherein administration of the lipid nanoparticle results in expression of the antigen encoded by mRNA in the lymphocytes of the subject.

Embodiment 57. A method of treating or preventing a disorder of any one of **embodiments 36, 39, 41, 45, 48, or 49,** wherein the disorder is an infection with coronavirus, or a disorder related to such an infection.

Embodiment 58. A method of treating or preventing a disorder of any one of **embodiments 36, 39, 41, 45, 48, or 49,** wherein the subject in need is a mammalian subject, preferably a human subject.

Embodiment 59. The method of any one of the **preceding method embodiments,** wherein the administration of the lipid nanoparticle results in an antigen specific antibody response, preferably wherein the antigen specific antibody response is measured by the presence of antigen-specific antibodies in serum.

Embodiment 60. The lipid nanoparticle of any one of **embodiment 9 to embodiment 16,** wherein the lipid nanoparticle comprises 59 mol% cationic or ionizable lipid, preferably C24, 28.5 mol% cholesterol, 10 mol% neutral lipid and 2.5 mol% of the polymer conjugated lipid of any one of **embodiment 1 to embodiment 8,** preferably "PMOZ 4".

Embodiment 61. The lipid nanoparticle of any one of **embodiment 9 to embodiment 16 or embodiment 60,** wherein the lipid nanoparticle comprises 07:0 PC (DHPC, 1,2-diheptanoyl-sn-glycero-3-phosphocholine).

Embodiment 63. The lipid nanoparticle of any one of **embodiment 9 to embodiment 16 or embodiment 60 to embodiment 61** or a lipid nanoparticle comprising a polymer conjugated lipid according to any one of **embodiment 1** to **embodiment 8,** wherein the lipid nanoparticle has a lower PDI and/or lower size as compared to a control lipid nanoparticle comprising a PEG-lipid instead of a polymer conjugated lipid according to any one of **embodiment 1** to **embodiment 8.**

Embodiment 63.1 A lipid nanoparticle comprising a polymer conjugated lipid according to any one of **embodiment 1** to **embodiment 8,** wherein the lipid nanoparticle has a lower PDI and/or lower size upon (i) freezing and thawing or (ii) freeze-drying (lypophilizing) and reconstitution, as compared to a control lipid nanoparticle comprising a PEG-lipid instead said polymer conjugated lipid according to any one of **embodiment 1** to **embodiment 8.**

Embodiment 64. A method of making a frozen lipid nanoparticle of any one of **embodiment 9 to embodiment 16 or embodiment 60 to embodiment 62** or a lipid nanoparticle comprising a polymer conjugated lipid according to any one of **embodiment 1** to **embodiment 8,** wherein the lipid nanoparticle upon thawing has a lower PDI and/or lower size as compared to a control lipid nanoparticle comprising a PEG-lipid instead of a polymer conjugated lipid according to any one of **embodiment 1** to **embodiment 8.**

Embodiment 65. A method of making a lyophilized lipid nanoparticle of any one of **embodiment 9 to embodiment 16 or embodiment 60 to embodiment 62** or a lipid nanoparticle comprising a polymer conjugated lipid according to any one of **embodiment 1** to **embodiment 8,** wherein the lipid nanoparticle upon reconstitution has a lower PDI and/or lower size as compared to a control lipid nanoparticle comprising a PEG-lipid instead of a polymer conjugated lipid according to any one of **embodiment 1** to **embodiment 8.**

Embodiment 66. An improved lyophilization process for the preparation of lyophilized lipid nanoparticles of any one of **embodiment 9 to embodiment 16 or embodiment 60 to embodiment 62,** said process comprising the step of using a polymer conjugated lipid according to any one of **embodiment 1** to **embodiment 8** as excipient instead of a PEG-lipid, wherein the lipid nanoparticle upon reconstitution has a lower PDI and/or lower size as compared to a control lipid nanoparticle comprising a PEG-lipid instead of a polymer conjugated lipid according to any one of **embodiment 1** to **embodiment 8.**

Embodiment 67. A vaccine composition, comprising a lipid nanoparticle of any one of **embodiment 9 to embodiment 16 or embodiment 60 to embodiment 62** or a polymer conjugated lipid according to any one of **embodiment 1** to **embodiment 8.**

Embodiment 68. A vaccine composition or a lipid nanoparticle of any one of the preceding embodiments comprising a polymer-conjugated lipid according to any of the preceding embodiments, wherein the formulation has an increase in LNP mean size of about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less after one or more freeze/thaw cycles as compared to that prior to freeze/thaw cycles.

Embodiment 69. A vaccine composition or a lipid nanoparticle of any one of the preceding embodiments comprising a polymer-conjugated lipid according to any of the preceding embodiments, wherein the formulation has an increase in LNP mean size of about 10%, 9%, 8%, 7%, 6%, 5%, 4%, 3%, 2%, 1%, or less after one or more freeze/thaw cycles as compared to that prior to freeze/thaw cycles.

Embodiment 70. A vaccine composition or a lipid nanoparticle of any one of the preceding embodiments comprising a polymer-conjugated lipid according to any of the preceding embodiments, wherein the formulation has an increase in LNP mean size of about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less after lyophilization as compared to that prior to lyophilization.

Embodiment 71. A vaccine composition or a lipid nanoparticle of any one of the preceding embodiments comprising a polymer-conjugated lipid according to any of the preceding embodiments, wherein the formulation has an increase in LNP mean size of about 10%, 9%, 8%, 7%, 6%, 5%, 4%, 3%, 2%, 1%, or less after lyophilization as compared to that prior to lyophilization.

Embodiment 72. A vaccine composition or a lipid nanoparticle of any one of the preceding embodiments comprising a polymer-conjugated lipid according to any of the preceding embodiments, wherein the formulation has an increase in LNP mean size of about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less after dilution as compared to that prior to dilution.

Embodiment 73. A vaccine composition or a lipid nanoparticle of any one of the preceding embodiments comprising a polymer-conjugated lipid according to any of the preceding embodiments, wherein the formulation has an increase in LNP mean size of about 10%, 9%, 8%, 7%, 6%, 5%, 4%, 3%, 2%, 1%, or less after dilution as compared to that prior to dilution.

Embodiment 74. A vaccine composition or a lipid nanoparticle of any one of the preceding embodiments comprising a polymer-conjugated lipid according to any of the preceding embodiments, wherein the encapsulation efficiency of the formulation is substantially the same after storage at about 4 °C or lower for at least one month.

Embodiment 75. A vaccine composition or a lipid nanoparticle of any one of the preceding embodiments comprising a polymer-conjugated lipid according to any of the preceding embodiments, wherein the LNP mean size of the LNPs is substantially the same after storage at about 4 °C or lower for at least one month.

Embodiment 76. Any polymer conjugated lipid of the previous embodiments, wherein said polymer conjugated lipid does not comprise a polyethylene glycol-(PEG)-moiety or residue; and/or does not comprise a sulphur group (-S-); and/or a terminating nucleophile.

Embodiment 77. Any polymer conjugated lipid of the previous embodiments, wherein said polymer conjugated lipid does not comprise a polyethylene glycol-(PEG)-moiety or residue.

Embodiment 78. Any polymer conjugated lipid of the previous embodiments, wherein said polymer conjugated lipid does not comprise a sulphur group (-S-).

Embodiment 79. Any polymer conjugated lipid of the previous embodiments, wherein said polymer conjugated lipid does not comprise a terminating nucleophile.

Embodiment 80. Any polymer conjugated lipid of the previous embodiments, wherein said polymer conjugated lipid does not comprise a sulphur group (-S-); and a terminating nucleophile.

### EXAMPLES

In the following section, particular examples illustrating various embodiments and aspects of the invention are presented. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the claims as disclosed herein.

### Example 1: Generation of RNA constructs

The following RNA constructs which are described in the following were used in the experiments:
i) RNA encoding circumsporozoite protein (PfCSP mRNA);
ii) RNA encoding Rabies Virus Glycoprotein (RABV-G mRNA);
iii) RNA encoding Photinus pyralis luciferase (PpLuc mRNA); and
iv) RNA encoding a Coronavirus spike protein as disclosed in SEQ ID NO:89 (CVnCoV according to WO2021156267 or PMID 34794169).

To prepare the RNA constructs, DNA sequences encoding the desired proteins as listed above (i.e. PfCSP, RABV-G and PpLuc) were prepared and used for subsequent RNA in vitro transcription, wherein the DNA sequences were prepared by optionally modifying the wild type coding sequence (CDS) by introducing a GC optimized CDS. Sequences were introduced into a plasmid vector comprising the motifs described below (UTR sequences, a stretch of adenosines, optionally a histone stem-loop structure, and optionally a stretch of 30 cytosines). The obtained plasmid DNA was transformed and propagated in bacteria using common protocols. Subsequently, plasmid DNA was extracted, purified, enzymatically linearized using a restriction enzyme and then used for DNA dependent RNA *in vitro* transcription using T7 RNA polymerase in the presence of a nucleotide mixture (ATP/GTP/CTP/UTP) and a CAP analogue (e.g., m7GpppG, CleanCap^{™} or respectively m7G(5')ppp(5')(2'OMeA)pG or m7G(5')ppp(5')(2'OMeG)pG)) under suitable buffer conditions. The obtained RNA was purified using a RP-HPLC (PureMessenger^{®}; according to WO2008077592) and used for further experimentation. The obtained mRNA was enzymatically polyadenylated using a commercial polyadenylation kit.

The details of the RNA constructs were as follows:
Ad i): PfCSP mRNA comprised a 5'-UTR from HSD17B4 (SEQ ID NO:21, SEQ ID NO:22), a 3'-UTR from PSMB3 (SEQ ID NO:19, SEQ ID NO:20), 64x adenosine at the 3'-terminal end (polyA-tail); 5 nucleotides, 30x cytosine at the 3'-terminal end (poly-C-tail), a histone stem-loop and 5 additional nucleotides. The mRNA was further enzymatically capped using ScriptCap^{™} m7G Capping System (CellScript, Madison, WI, USA) according to manufacturer's instructions and enzymatically polyadenylated using a commercial polyadenylation kit, resulting in SEQ ID NO:38;
Ad ii): RABV-G mRNA comprised mCap, a muag 3'-UTR (SEQ ID NO:11); 64x adenosine at the 3'-terminal end (polyA-tail); 5 nucleotides, 30x cytosine at the 3'-terminal end (polyC-tail), a histone stem-loop and 5 additional nucleotides (SEQ ID NO:32);
Ad iii): PpLuc mRNA comprised a 5'-Cap (CleanCap AG) - HSD17B4 5'-UTR - GC-optimized Photinus pyralis luciferase CDS/coding sequence/ORF - PSMB3 3'-UTR - histone stem-loop - polyA100 (SEQ ID NO:71);
Ad iv): CVnCoV mRNA comprised a GC-enriched open reading frame (ORF) encoding spike glycoprotein of SARS-CoV-2 with NCBI Reference Sequence NC_045512.2, GenBank accession number YP_009724390.1 encoding full length S featuring K986P and V987P mutations - a muag 3'-UTR (SEQ ID NO:11); 64x adenosine at the 3'-terminal end (polyA-tail); 5 nucleotides, 30x cytosine at the 3'-terminal end (polyC-tail), a histone stem-loop and 5 additional nucleotides (SEQ ID NO:89)

### Example 2: Synthesis of lipids and preparation of the LNPs

In this working example, different HEXA and HEAD lipids as shown in **Table Ex-1** were synthesized according to general protocols of Mercachem B.V. (Symeres, Groningen, The Netherlands, custom synthesis, C24-C27), or ChiroBlock GmbH (Bitterfeld-Wolfen, Germany, C1-C22) where indicated.

**Table Ex-1: Overview of the synthesized HEXA and HEAD lipids**

| **Lipids No. / Compound name** | **Compound name*** [Tail-Linker-Head]** | **Ester structure** | **Lipid type** |
|---|---|---|---|
| C2 | HEXA-C5DE-PipSS | diester | HEXA lipid |
| C24 | VitE-C4DE-Piperidine-Thioether (or short herein "THIOETHER") | diester | HEAD lipid |
| C25 | VitE-C4DE-Pip-C3SS or VitE-C4DE-Piperidine-C3SS (or short herein "C3SS") | diester | HEAD lipid |
| C26 | HEXA-C5DE-inverted-PipSS or HEXA-C5DE-inverted-Piperidine SS | diester | HEXA lipid |
| C27 | HEXA-C5DE-Pip-C3 thioether or HEXA-C5DE-piperidine-C3 thioether | diester | HEXA lipid |

| | | | |
|---|---|---|---|
| ***see comments to **Table 1** | | | |

The name of the lipids is derived as follows: the basic name consists of three parts: "Tail-Linker-Head". If the tail is "HEXA" it is referred to a hexyldecanoic acid (Hexyl-1-decanol). If the tail is VitE, it is referred to a Vitamin E-moiety. The nomenclature of the linker is as follows: e.g. "C5DE" refers to a C₅ group comprising a diester linker (C5 = 5 Carbon atoms of the linker; diester), "C4DE" refers to a C₄ group comprising a diester linker (C4 = 4 Carbon atoms of the linker; diester). The reference to the head "PipSS" indicates piperidine residues connected via a disulfide bridge, wherein "PipSS" here specifically relates to a structure with 2 carbon atoms between the piperidine ring and the disulfide bridge. "Thioether" refers to a head comprising a thioether ("-S-"). C3 thioether refers to a head group comprising a structure with 3 carbon atoms between the piperidine ring and the thioether bridge.

Purity and structural identity of the HEXA lipids was confirmed by nuclear magnetic resonance spectroscopy (H-NMR, 500.13 MHz) and mass spectrometry (electrospray ionization-ESI or atmospheric pressure chemical ionization-APCI, via direct injection). All lipids mentioned herein were successfully synthesised with good purity. Purities of the products were confirmed by HPLC analysis with a TFA buffer on a C4 column with ELSD detection. Mass analysis was done by HPLC on a C18 column and the structures were confirmed by ¹H NMR analysis.

### Example 2.1: Synthesis and structure of the lipid "HEXA-C5DE-PipSS" (cationic lipid from GN02)

HEXA-C5DE-PipSS was synthesized according to general protocols of ChiroBlock GmbH (Bitterfeld-Wolfen, Germany), wherein the structure of HEXA-C5DE-PipSS is as follows:

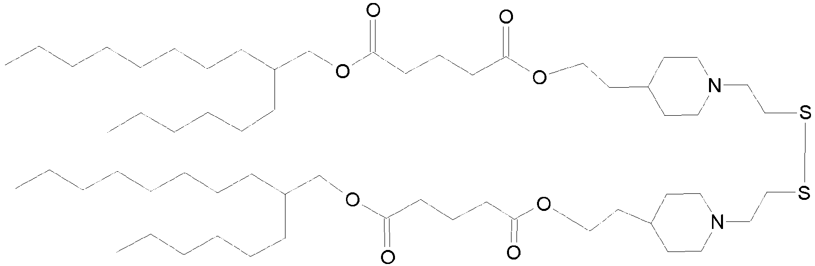

HEXA-C5DE-PipSS was synthesized as follows:

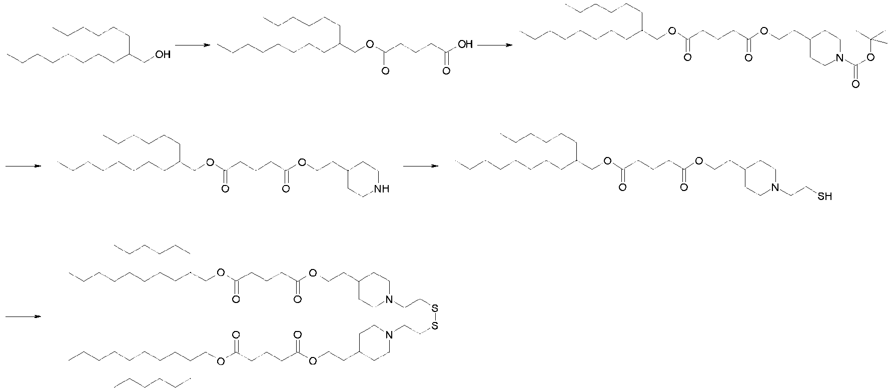

### Example 2.1.1

Synthesis of 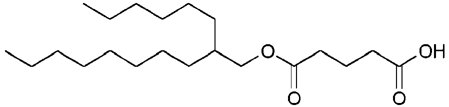 5-(2-hexyldecoxy)-5-oxo-pentanoic acid

To a solution of 2-Hexyl-1-decanol (150 g) and glutaric anhydride (74.13 g) 1000 ml of dry dichloromethane dimethylaminopyridine (90.71 g) is added and the reaction mixture is stirred for 65 hours under nitrogen at room temperature. The white precipitate that has formed is filtered off and discharged. The filtrate is concentrated in vacuum and mixed with 200 ml of petrol ether for 40 minutes resulting in a white suspension. The precipitate is filtered off and the filtrate concentrated. The crude is partitioned between 300 ml 1N hydrochloric acid and 500 ml of ethyl acetate. The organic phase is separated, washed with 500 ml of water and dried over anhydrous sodium sulphate. The sodium sulphate is filtered off and the solvent evaporated in vacuum. The crude residue is purified by flash chromatography on silica eluting with a gradient dichloromethane → dichloromethane:methanol 90:10. Fractions containing the product are combined and concentrated to give the pure target compound as a yellow oil (123.9 g, 56.1% yield).

### Example 2.1.2

Synthesis of 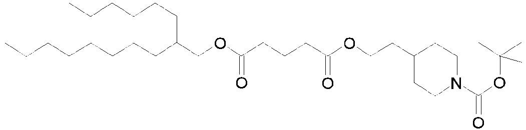 O1-[2-(1-tert-butoxycarbonyl-4-piperidyl)ethyl] O5-(2-hexyldecyl) pentanedioate

The product from Example 2.1.1 (52.6 g) and tert-Butyl 4-(2-hydroxyethyl)piperidine-1-carboxylate (37.2 g) are dissolved in 600 ml of dichloromethane at room temperature giving a clear yellow solution. N,N'-Dicyclohexylcarbodiimid (48.6 g) is added and the reaction mixture is stirred at room temperature for 22 hours. More N,N'-Dicyclohexylcarbodiimid (15.2 g) is added and the mixture stirred at room temperature for another 42 hours. The white precipitate that has formed is filtered off and washed with a small volume of petrol ether. The combined filtrates are concentrated in vacuum and the residue purified by flash chromatography on silica with a solvent gradient from pure petrol ether to petrol ether:ethylacetate 90:10. The pure fractions of the product are combined and concentrated to give the target compound as an oil (32.8 g, 39.2% yield).

### Example 2.1.3

Synthesis of 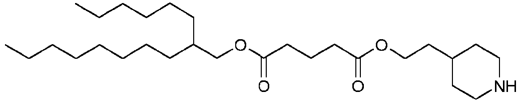 O5-(2-hexyldecyl) O1-[2-(4-piperidyl)ethyl] pentanedioate

The product from Example 2.1.2 (32.8 g) is dissolved in 1000 ml of dichloromethane at room temperature. The solution is cooled in an ice bath and trifluoroacetic acid (35.6 ml) is added slowly at ~0°C. The mixture is allowed to warm up to room temperature and stirred overnight. The mixture is washed with saturated sodium hydrogen carbonate solution and the aqueous phase is back-extracted with dichloromethane. The combined organic solutions are washed with brine, dried over anhydrous sodium sulphate, filtered and concentrated to give the target compound as a yellow oil (27.15 g, quantitative yield). The product was used without further purification in the next step.

### Example 2.1.4

Synthesis of 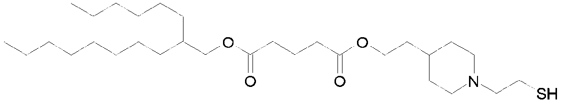 O5-(2-hexyldecyl) O1-[2-[1-(2-sulfanylethyl)-4-piperidyl]ethyl] pentanedioate

The crude product from Example 2.1.3 (1.37 g) is dissolved in 10 ml of dry toluene. N,N-diisopropylethylamine (0.533 ml) is added at room temperature resulting in a clear solution. The mixture is transferred to a pressure vial and 0.7 ml of ethylene sulphide is added. The vial is sealed and heated in an oil bath at 65°C overnight. After cooling to room temperature, the complex reaction mixture is concentrated and used as obtained in the subsequent step.

### Example 2.1.5

Synthesis of 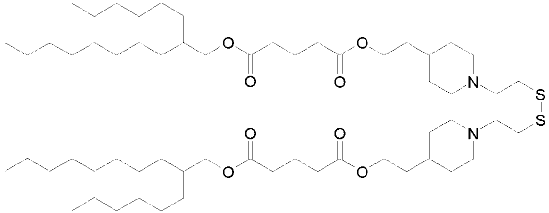 O1-[2-[1-[2-[2-[4-[2-[5-(2-hexyldecoxy)-5-oxo-pentanoyl]oxyethyl]-1-piperidyl]ethyldisulfanyl]ethyl]-4-piperidyl]ethyl] O5-(2-hexyldecyl) pentanedioate

The crude product mixture from Example 2.1.4 is dissolved in 15 ml acetonitrile. A solution of iodine in acetonitrile:water 9:1 is added drop wise at room temperature while stirring until a brown colour remains. The reaction mixture is concentrated and taken up in ethylacetate. This solution is washed subsequently with sodium hydrogen carbonate solution and sodium thiosulphate solution. The organic phase is dried over anhydrous sodium sulphate, filtered and concentrated in vacuum. The target compound is isolated by flash chromatography on silica, eluting with a gradient chloroform → chloroform:methanol 80:20. The respective fractions are combined and the solvents are evaporated to provide the pure target compound as a yellow oil (562 mg, 22% yield over two steps).

¹H-NMR-data (500 MHz, CDCl3): 4.11 ppm (4H), 3.98 ppm (4H), 3.15-2.5 ppm (12H), 2.37 (8H), 2.17-1.84 ppm (8H), 1.81-1.5 ppm (10H), 1.49-1.08 ppm (54H), 0.88 (12H)

### Example 2.2: Synthesis of VitE-C4DE-Piperidine-Thioether (compound C24)

For the synthesis of VitE-C4DE-Piperidine-Thioether (short: "THIOETHER"), coupling of dicarboxylic acid derivative 3 and 4-piperidineethanol gave bisamide 4 in 86%. Subsequent reduction with LiAlH₄ afforded 5 in 24% after purification by flash chromatography. Final coupling with commercially available Vitamin E derivative 6 gave VitE-C4DE-Piperidine-Thioether in 52%, which was purified by extraction between heptane and MeCN, followed by flash chromatography. 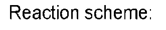 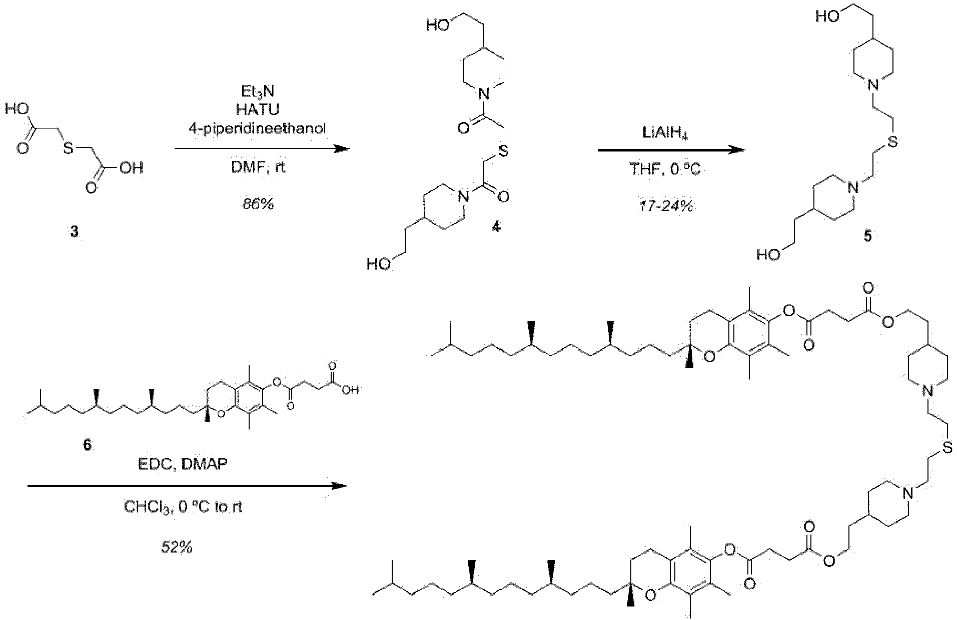
¹H NMR data THIOETHER or VitE-C4DE-Piperidine-thioether
¹H NMR (400 MHz, C₆D₆) δ 4.16 (4H), 2.85- 2.67 (12H), 2.64-.52 (8H), 2.44 (4H), 2.35 (6H), 2.23 (12H), 1.84 (4H), 1.64 (16H), 1.55- 1.17 (50H), 1.04 (24H).

### Example 2.3: Synthesis of VitE-C4DE-Piperidine-C3SS (compound C25)

The route towards "VitE-C4DE-Piperidine-C3SS" (short: "C3SS") started with dimerization of 7, which afforded 8 in quantitative yield. Mesylation gave 9 in 70% after purification by flash chromatography. Coupling with 4-piperdineethanol yielded 10 in 33%. Subsequent coupling with 6 afforded VitE-C4DE-Piperidine-C3SS in 51% after extraction and flash purification. 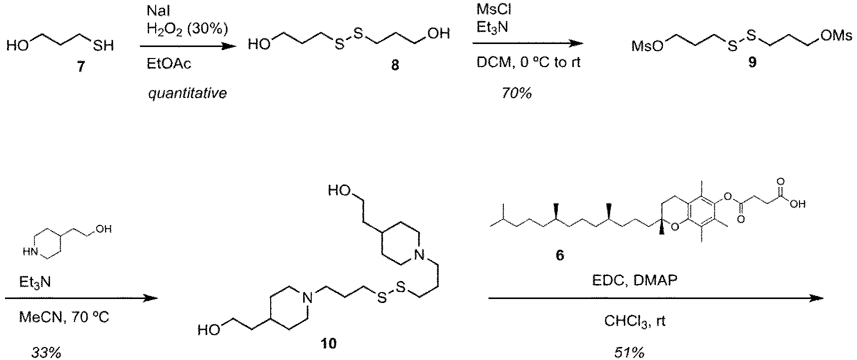 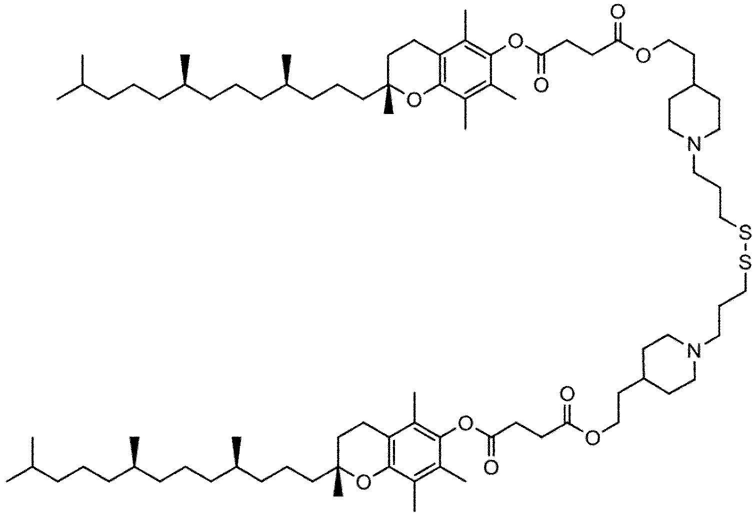
¹H NMR data C3SS or VitE-C4DE-Piperidine-C3SS
¹H NMR (400 MHz, C₆D₆) δ 4.17 (4H), 2.85- 2.71 (12H), 2.63-2.52 (4H), 2.45 (4H), 2.34 (10H), 2.23 (12H), 1.92 (4H), 1.79 (4H), 1.73- 1.15 (68H), 1.01 (24H).

### Example 2.4: Synthesis of HEXA-C5DE-inverted-PipSS (compound C26)

For the synthesis of target HEXA-C5DE-inverted-PipSS alkylation of potassium thioacetate with tosylate 11 afforded thioacetate derivative 12 in a quantitative yield. Formation of disulfide derivative 13 was achieved with aqueous NaOH in MeOH and after purification 13 was obtained in 80%. Boc-deprotection with HCl gave 14 in quantitative yield. Direct alkylation with 2-bromoethanol to compound 16 failed. Therefore reductive alkylation of 14 with aldehyde 19 was carried out and afforded TBDMS-protected diol 16' in 37-45%. Subsequent deprotection with TBAF gave clean conversion to 16. Because the product could not be separated completely from residual TBA-salts, it was used in the final coupling with carboxylic acid 21 as such. This carboxylic acid was prepared by treatment of alcohol 20 with glutaric anhydride and afforded 21 in 34-40%. The final coupling of 21 and 16 was successfully tested and gave HEXA-C5DE-inverted-PipSS in 43% and desired purity. 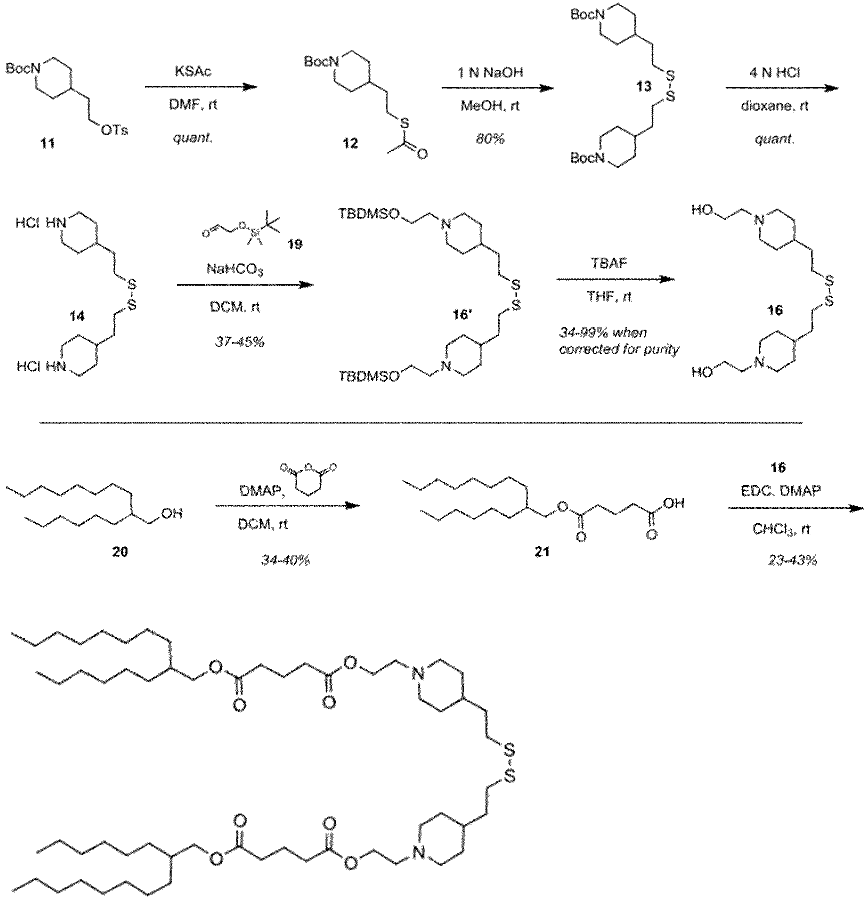
¹H NMR data HEXA-C5DE-inverted-PipSS
¹H NMR (400 MHz, C₆D₆) δ 4.28- 4.14 (8H), 2.83 (4H), 2.70- 2.61 (4H), 2.48 (4H), 2.31 (8H), 2.03 (4H), 1.88 (4H), 1.76- 1.61 (6H), 1.57- 1.20 (58H), 1.03 (12H).

### Example 2.5: Synthesis of "HEXA-C5DE-Pip-C3 thioether" or "HEXA-C5DE-piperidine-C3 thioether" (compound C27)

The route towards target HEXA-C5DE-Pip-C3 thioether started with mesylation of diol 22, which provided bismesylated derivative 23 in 99%. Subsequent substitution gave bisamine 24 in 68%. The final esterification with carboxylic acid 21 gave HEXA-C5DE-Pip-C3 thioether in 23% after extraction and flash chromatography. 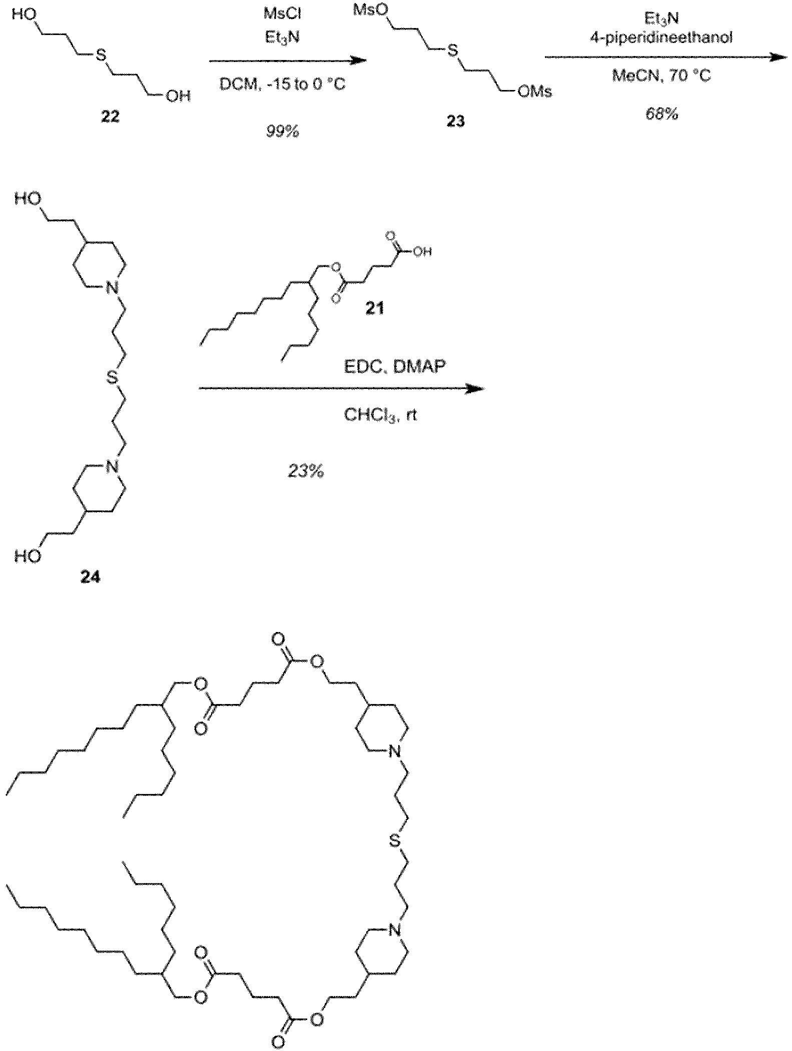
¹H NMR data HEXA-C5DE-Pip-C3 thioether (HEXA-C5DE-piperidine-C3 thioether)
¹H NMR (400 MHz, C₆D₆) δ 4.21- 4.08 (8H), 2.84 (4H), 2.62 (4H), 2.41 (4H), 2.31 (8H), 2.04 (4H), 1.84 (8H), 1.71 (2H), 1.59- 1.21 (62H), 1.03 (12H).

### Example 2.6: Synthesis of "PMOZ 4" and "PMOZ 2"

"PMOZ 2" as used herein throughout the examples section has the structure of:

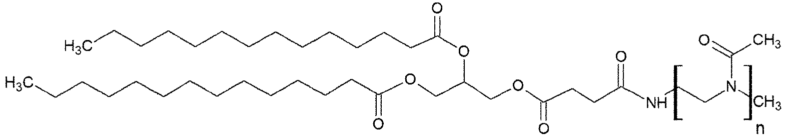

["PMOZ 2"] with n = 50 i.e. having 50 monomer repeats.

"PMOZ 4" as used herein throughout in the examples section has the structure of:

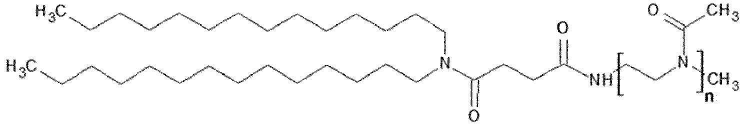

["PMOZ 4"] with n = 50 i.e. having 50 monomer repeats.

Summarized, "PMOZ 2" was synthesized as follows:

### Step 1 (synthesis of PMOZ-polymer):

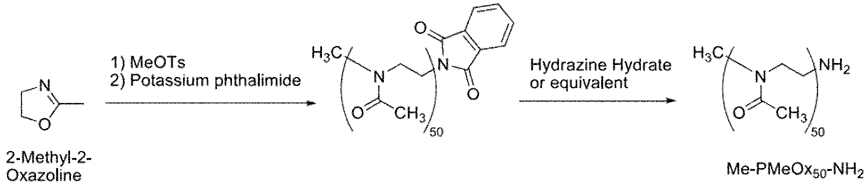

Thereafter, 1.0 g of DMG (1) was reacted with succinic anhydride (2) to afford 848 mg (71%) of ester (3). 344 mg of this material was reacted with 1.92 g of PMOz polymer (4) from step 1:

### Step 2:

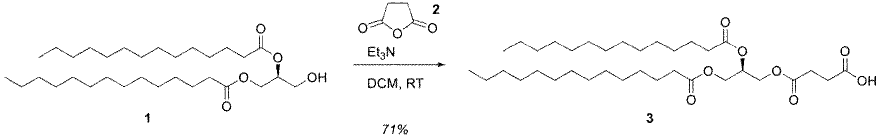

### Step 3:

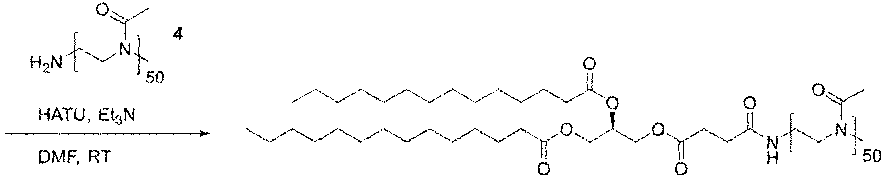

Summarized, "PMOZ 4" was synthesized as follows:

### Step 1 (synthesis of PMOZ-polymer):

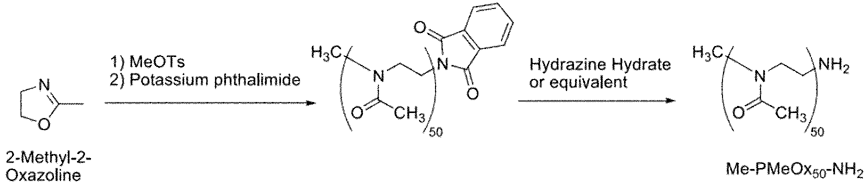

Thereafter, 0.5 g of di(tetradecyl)amine (1) was reacted with succinic anhydride (2) to afford 563 mg (90%) of amide (3). 312 mg of this material was reacted with 2.10 g of PMOz polymer (4) from step 1.

### Step 2:

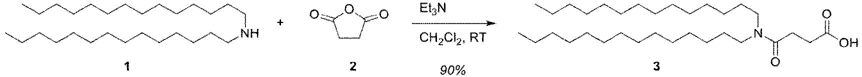

### Step 3:

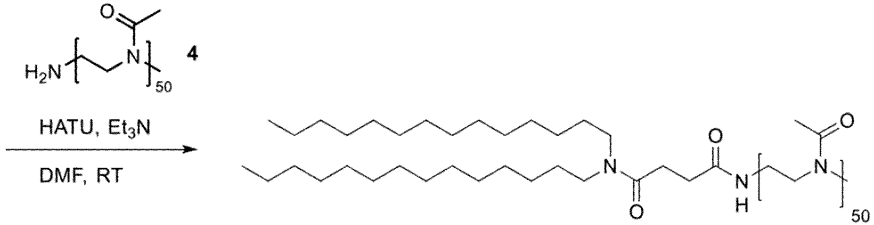

For all lipids, purity and structural identity of the lipids were confirmed by nuclear magnetic resonance spectroscopy (H-NMR, 500.13 MHz) and mass spectrometry (electrospray ionization-ESI or atmospheric pressure chemical ionization-APCI, via direct injection).

### Example 3: Preparation of LNPs using the NanoAssemblr^{™} microfluidic system

The LNPs were prepared using the NanoAssemblr^{™} microfluidic system (Precision NanoSystems Inc., Vancouver, BC) according to standard protocols which enables controlled, bottom-up, molecular self-assembly of nanoparticles via custom-engineered microfluidic mixing chips that enable millisecond mixing of nanoparticle components at a nanolitre scale.

In the present examples, the cationic lipid HEXA-C5DE-PipSS was used for preparation of lipid nanoparticle compositions. Furthermore, cholesterol (Avanti Polar Lipids; Alabaster, AL), the neutral lipid / phospholipid "DPhyPE" (Avanti Polar Lipids; Alabaster, AL) and as polymer conjugated lipid
- DMPE-PMOZ-v1 (Iris Biotech, Marktredwitz, Germany), DMPE-PMOZ-v1 being 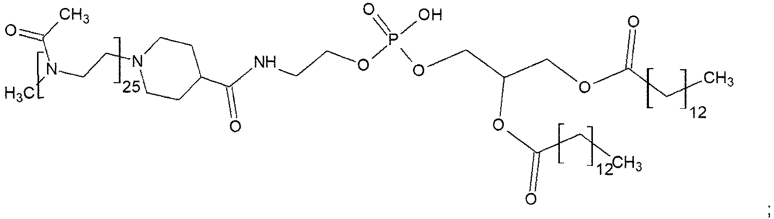 and
- for comparison, DMG-PEG 2000 (NOF Corporation, Tokyo, Japan) or DSG-PEG 2000 (NOF Corporation, Tokyo, Japan) were used as indicated.

Finally, in specific formulations, the additional phospholipid "07:0 PC" also referred to as "DHPC" was used. The structure of (07:0) PC (DHPC; 1,2-diheptanoyl-sn-glycero-3-phosphocholine) from Avanti is as follows:

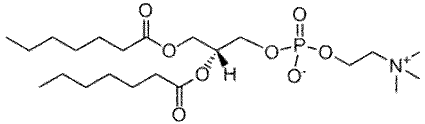

In the context of the working examples and also the disclosure of the invention, if only "DMG-PEG" / "DSG-PEG" or "DMG-mPEG" / "DSG-mPEG" is indicated, reference is made herein to 1,2-dimyristoyl-rac-glycero-3-methoxypolyethylene glycol-2000 (DMG-(m)PEG 2000) and distearoyl-rac-glycerol-PEG 2000 (DSG-(m)PEG 2000), respectively.

The lipids were solubilized in alcoholic solution (ethanol) according to standard procedures. The corresponding lipid nanoparticle compositions are detailed in **Table Ex-2A and Table Ex-2B.**

In detail, LNPs were prepared by mixing appropriate volumes of lipid stock solutions in ethanol buffer with an aqueous phase (50 mM sodium acetate, pH 4.0) containing appropriate amounts of mRNA as indicated herein; cholesterol, phospholipid and polymer conjugated lipid: 20 mg/ml in EtOH, cationic lipids, except for GN01: 20 mg/ml in EtOH, GN01-lipid: 30 mg/ml in tert-butanol and lipids C24, C25, C26 and C27 were also solubilized in 30 mg/ml t-butanol and added to the ethanol premix of lipids.

Briefly, mRNA as indicated in the following working examples, was diluted to 0.05 to 0.2 mg/ml in 50 mM acetate buffer, pH 4. Syringe pumps were installed into inlet parts of the NanoAssemblr^{™} (Precision NanoSystems Inc., Vancouver, BC) and used to mix the ethanolic lipid solution with the mRNA aqueous solution at a ratio of about 1:5 to 1:3 (vol/vol) with total flow rates from about 14 ml/min to about 18 ml/min.

The ethanol was then removed and the external buffer replaced with PBS/sucrose buffer (pH 7.4, 75 mM NaCl, 10 mM phosphate, 150 mM sucrose) by dialysis (Slide-A-Lyzer^{™} Dialysis Cassettes, ThermoFisher). Finally, the lipid nanoparticles were filtered through a 0.2 µm pore sterile filter. Lipid nanoparticle particle diameter size was from about 90 nm to about 140 nm as determined by quasi-elastic light scattering using a Malvern Zetasizer Nano (Malvern Instruments Ltd.; Malvern, UK). For other cationic lipid compounds mentioned in the present specification, the formulation process is similar. For all LNPs, the ethanol was then removed and buffer replaced by 10 mM PBS, pH 7.4 comprising 9% sucrose.

**Table Ex-2A: Summary / overview of lipid nanoparticle compositions of the working examples: reference to the cationic lipids as disclosed in Table Ex-1 and Table 1 or the specification above is made herein**

| Name of LNP formulation / composition No. | Excipients [cationic lipid : steroid : neutral lipid + optional further excipient : polymer conjugated lipid] | mol-percentages for excipients [mol%] |
|---|---|---|
| GN01 | C23 : Cholesterol : DPhyPE : DMG-PEG 2000 | 59 : 29.3 : 10 : 1.7 |
| GN01-PMOZ-10% | C23 : Cholesterol : DPhyPE : DMG-PMOZ | 49:31:10:10 |
| GN01-PMOZ-2.5% | C23 : Cholesterol : DPhyPE : DMG-PMOZ | 59 : 28.5 : 10 : 2.5 |
| GN02 | HEXA-C5DE-PipSS : Cholesterol : DPhyPE : DMG-PEG 2000 | 59 : 29.3 : 10 : 1.7 |
| GN02-PMOZ | HEXA-C5DE-PipSS : Cholesterol : DPhyPE : DMG-PMOZ | 59 : 29.3 : 10 : 1.7 |
| LNP-C | state of the art cationic lipid : Cholesterol : DSPC : state of the art PEG-lipid comprising mPEG2000 | 47.4 : 40.9 : 10 : 1.7 |
| | | |
| LNP19A | C24 : Chol : DPhyPE : DMG-PMOZ | **59** : 29.3 : 10 : 1.7 |
| LNP20A | C25 : Chol : DPhyPE : DMG-PMOZ | 59 : 29.3 : 10 : 1.7 |
| LNP21A | C13 : Chol : DPhyPE : DMG-PMOZ | 59 : 29.3 : 10 : 1.7 |
| LNP22A | C16 : Chol : DPhyPE : DMG-PMOZ | 59 : 29.3 : 10 : 1.7 |
| LNP23A | C17 : Chol : DPhyPE : DMG-PMOZ | 59 : 29.3 : 10 : 1.7 |
| LNP24A | C18 : Chol : DSPC : DMG-PMOZ | 59 : 29.3 : 10 : 1.7 |
| LNP25A | C26 : Chol : DPhyPE : DMG-PMOZ | 59 : 29.3 : 10 : 1.7 |
| LNP26A | C27 : Chol : DPhyPE : DMG-PMOZ | 59 : 29.3 : 10 : 1.7 |
| | | |
| LNP19B | C24 : Chol : DPhyPE : DMG-PMOZ | 59 : 28.5 : 10 : 2.5 |
| LNP20B | C25 : Chol : DPhyPE : DMG-PMOZ | 59 : 28.5 : 10 : 2.5 |
| LNP21B | C13 : Chol : DPhyPE : DMG-PMOZ | 59 : 28.5 : 10 : 2.5 |
| LNP22B | C16 : Chol : DPhyPE : DMG-PMOZ | 59 : 28.5 : 10 : 2.5 |
| LNP23B | C17 : Chol : DPhyPE : DMG-PMOZ | 59 : 28.5 : 10 : 2.5 |
| LNP24B | C18 : Chol : DSPC : DMG-PMOZ | 59 : 28.5 : 10 : 2.5 |
| LNP25B | C26 : Chol : DPhyPE : DMG-PMOZ | 59 : 28.5 : 10 : 2.5 |
| LNP26B | C27 : Chol : DPhyPE : DMG-PMOZ | 59 : 28.5 : 10 : 2.5 |
| | | |
| LNP27 | C2 : Chol : DPhyPE : DMPE-PMOZ-v1 | 59 : 29.3 : 10 : 1.7 |
| LNP28 | C2 : Chol : DSPC + (07:0) PC (DHPC) : DMPE-PMOZ-v1 | 59 : 28.3 : 10+1 : 1.7 |
| LNP29 | C2 : Chol : DSPC + (07:0) PC (DHPC) : DMPE-PMOZ-v1 | 49 : 29.3 : 10+10 : 1.7 |
| | | |
| LNP27B | C2 : Chol : DPhyPE : DMG-PMOZ | 59 : 29.3 : 10 : 1.7 |
| LNP28B | C2 : Chol : DSPC + (07:0) PC (DHPC) : DMG-PMOZ | 59 : 28.3 : 10+1 : 1.7 |
| LNP29B | C2 : Chol : DSPC + (07:0) PC (DHPC) : DMG-PMOZ | 49 : 29.3 : 10+10 : 1.7 |

**Table Ex-2B: Further summary / overview of lipid nanoparticle compositions of the working examples; reference to the cationic lipids as disclosed in Table Ex-1 and Table 1 or the specification above is made herein**

| Name of LNP formulation / composition No. | Excipients [cationic lipid : steroid : neutral lipid + optional further excipient : polymer conjugated lipid] | mol-percentages for excipients [mol%] |
|---|---|---|
| LNP18C (GN02 + 1% DHPC) | C2 : Chol : DPhyPE+(07:0) PC (DHPC) : DMG-PMOZ | 59 : 28.3 : 10+1 : 1.7 |
| LNP18D (GN02 + 10% DHPC) | C2 : Chol : DPhyPE+(07:0) PC (DHPC) : DMG-PMOZ | 49 : 29.3 : 10+10 : 1.7 |
| LNP18E (GN02 control) | C2 : Chol : DPhyPE : DMG-PMOZ | 59 : 29.3 : 10 : 1.7 |
| | | |
| LNP19C (C24 + 1% DHPC) | C24 : Chol : DPhyPE + (07:0) PC (DHPC) : DMG-PMOZ | 58 : 28.5 : 10+1 : 2.5 |
| LNP19D (C24 + 10% DHPC) | C24 : Chol : DPhyPE + (07:0) PC (DHPC) : DMG-PMOZ | 49 : 28.5 : 10+10 : 2.5 |
| LNP19E (C24 + 0% DHPC) | C24 : Chol : DPhyPE : DMG-PMOZ | 59 : 28.5 : 10 : 2.5 |
| LNP19F (C24 + 0% DHPC) | C24 : Chol : DPhyPE : DMG-PMOZ | 49 : 38.5 : 10 : 2.5 |

Summarized, as described above,
- "GN02" resembles a lipid nanoparticle comprising 59 mol% cationic lipid HEXA-C5DE-PipSS, 29.3 mol% cholesterol as steroid, 10 mol% DPhyPE as neutral lipid / phospholipid and 1.7 mol% DMG-PEG 2000 as polymer conjugated lipid. For "GN02", the N/P (lipid to mRNA mol ratio) preferably was 14 and the total lipid/mRNA mass ratio preferably is about 40;
- "GN02-PMOZ" resembles a lipid nanoparticle comprising 59 mol% cationic lipid HEXA-C5DE-PipSS, 29.3 mol% cholesterol as steroid, 10 mol% DPhyPE as neutral lipid / phospholipid and 1.7 mol% DMG-PMOZ as polymer conjugated lipid. For "GN02-PMOZ", the N/P (lipid to mRNA mol ratio) preferably is about 14 and the total lipid/mRNA mass ratio preferably is about 40.

### Example 3.1: Biophysical characterization of lipid nanoparticle compositions of PMOZ-LNPs

Each LNP used in the working examples of the present invention was characterized in terms of particle size, zeta potential, encapsulation efficiency / %-encapsulation (EE), and RNA content (basically corresponding to mRNA content within the context of the present invention).

The mean diameter and zeta potential of the LNPs after dialysis was as determined by dynamic light scattering and Laser Doppler Microelectrophoresis, respectively using a Malvern Zetasizer Nano (Malvern Instruments Ltd.; Malvern, UK). Encapsulation efficiency (EE [%]) was calculated by the following equation: %-encapsulation = (Ft - Fi)/Ft x 100; whereby Fi = free unencapsulated RNA as determined by addition of RiboGreen (Molecular Probes, Eugene, OR, USA) to the LNP aliquot and Ft = total content RNA content measured by adding RiboGreen (Molecular Probes, Eugene, OR, USA) to fluorescence value = Fi) to an aliquot of lysed LNP achieved by incubation with 0.25% Triton X-100.

### Results:

Sizes of LNPs comprising P(M)OZ-lipids instead of PEG-lipids were comparable (data not shown). I.e. particle sizes for LNPs comprising DMG-PMOZ did not vary significantly when different amounts of DMG-PMOZ were introduced (1.7 mol%; 2.5 mol%, 5 mol%, 7.5 mol%, 10 mol%; data not shown). The zeta potential was in accordance with typical neutral values (-7 to +7 mV, or preferably from -7 to 0 mV) and LNPs showed a high encapsulation efficiency (EE%) of about 90% (data not shown).

### Example 4: Effect of the presence of PMOZ-lipids in LNPs on organ-targeting

Firefly luciferase PpLuc mRNA (SEQ ID NO:71) was used as a reporter to analyze tissue distribution and expression levels after 6 h in different organs after intravenous injection. Therefore, PpLuc was formulated in state of the art LNPs comprising 59 mol% SS-33/4PE-15 as cationic lipid, 10 mol% DPhyPE, as polymer conjugated lipid
- 1.7 mol% PEG-lipid DMG-PEG 2000; or
- 5 or 10 mol% PMOZ-lipid,
and 29.3 mol% Cholesterol for LNPs comprising 1.7 mol% PEG-lipid DMG-PEG 2000 or respectively 24.3 mol% or 19.3 mol% for LNPs comprising 5 or 10 mol% PMOZ-lipid.

The formulations shown below in **Table Ex-3** were applied to female Balb/c mice on day 0 intravenously (i.v.) with doses of PpLuc RNA or buffer, respectively (100 µl injection volume each). Biodistribution was analyzed 6 h after administration by organ collection and organ analysis. Furthermore, serum samples were taken 14 days before the application and at the time point indicated below. Cytokine and chemokine levels in the sera of the animals were analyzed via CBA assay and IFN-alpha ELISA. Animals were treated with a 20 µg of PpLuc mRNA, or buffer, respectively.

**Table Ex-3: Experimental setup**

| **Group** | **No. of mice** | **Injection of** | **GN01-based Formulation** | **dose** |
|---|---|---|---|---|
| 1 (LNP 1) | 5 | PpLuc mRNA | 1.7% DMG-PEG 2000 | 20 µg |
| 2 (LNP 2) | 5 | PpLuc mRNA | 5.0% PMOZ-DMPE | 20 µg |
| 3 (LNP 3) | 5 | PpLuc mRNA | 10% PMOZ-DMPE | 20 µg |
| 4 (Buffer) | 5 | Buffer | --- | --- |

PpLuc expression was determined by Luciferase assay in the tissue lysates prepared according to standard methods after the organ collection. The following organs were analyzed: i) liver; ii) lungs; iii) spleen; iv) heart; v) kidneys; vi) ovaries; and vii) Inguinal lymph nodes (ing. LN).

### Results:

LNPs comprising a PMOZ-lipid as polymer conjugated lipid surprisingly did not have a negative effect on the efficacy of the LNPs, even a positive effect is apparent for spleen and lymph nodes luciferase values. As shown herein above, the inventors surprisingly found that PMOZ-LNPs performed very well when compared to standard PEG-LNPs. The organ distribution profile can be regarded as generally similar when comparing PEG-LNPs and PMOZ-LNPs (see **Figure 1****).**

### Example 5: Effect of the presence of PMOZ-lipids in LNPs on RABV-G vaccination using intramuscular administration

To analyse immunogenicity of LNPs comprising PMOZ-lipids instead of PEG-lipids, RABV-G (Rabies virus glycoprotein) mRNA was produced according to the procedures described above, yielding a RABV-G mRNA comprising mCap, a muag-3'-UTR; 64x adenosine at the 3'-terminal end (polyA-tail); 5 nucleotides, 30x cytosine at the 3'-terminal end (polyC-tail), histone stem-loop and 5 additional nucleotides (SEQ ID NO:32).

Resulting RABV-G mRNA was formulated in LNPs for comparison of LNPs comprising a PEG-lipid or, respectively, a PMOZ-lipid (GN01 comprising a DMG-PEG 2000 PEG-lipid versus GN01-PMOZ comprising a PMOZ-lipid).

Composition and formulation details are shown herein below in **Table Ex-4.**

**Table Ex-4: Composition and formulation details**

| **Group /Set** | **Formulation designation** | **mRNA dose [µg]** | **Cationic lipid [mol%]** | **Chol [mol%]** | **DSPC [mol%]** | **Conjugated lipid [mol%]** | **No. of mice** | **Route / volume** | **Dosing [day]** |
|---|---|---|---|---|---|---|---|---|---|
| 1 | GN01-PMOZ 10% | 1 | C23, 59% | 21 | 10 | 10 [PMOZ-DMPE-v1] | 8 | i.m. / 1x25 µl | 0, 21 |
| | GN01-DMG-PEG 2000 | 1 | C23, 59% | 29.3 | 10 | 1.7 [DMG-PEG 2000] | 8 | i.m. / 1x25 µl | 0, 21 |
| PBS | / | / | / | / | / | / | 5 | i.m. / 1x25 µl | 0, 21 |

7 weeks old female Balb/C mice (n=8) were injected intramuscularly (M. tibialis) at day 0 and day 21 with the formulations and buffer-control according to **Table Ex-4** comprising above described RABV-G mRNA.

For determining the levels of antibody against the rabies virus in serum, a classical virus neutralization test was performed (Fluorescent Antibody Virus Neutralization (FAVN) assay).

Accordingly, 28 days after the first mRNA administration, mice were sacrificed and blood and organ samples (liver) were collected for further analysis, i.e. for Virus neutralizing antibodies (VNA) analysis via FAVN assay. For said immunogenicity assays, the VNT was measured as described before, i.e. anti-rabies virus neutralizing titers (VNTs) in serum were analyzed by the Eurovir^{®} Hygiene-Labor GmbH, Germany, using the FAVN assay and the Standard Challenge Virus CVS-11 according to WHO protocol.

### Results:

Again, PMOZ-LNPs performed very well when compared to the standard controls, i.e. for all LNPs very high levels of Rabies VNTs were measured: **Figure 2** shows that already a single i.m. immunization with only 1 µg of mRNA formulated in PMOZ-LNPs induced very robust VNTs well above the protective titer of 0.5 IU/ml in all animals. A similar experiment which was performed with 2.5 mol% instead of 1.7 mol% PMOZ-lipid, decreasing the cholesterol amount by 0.8 mol% to sum up to 100 mol%, led to similar results (results not shown).

### Example 6: Analysis of size and PDI of LNPs using the NanoAssemblr^{™} microfluidic system and analysis of LNP stability upon freeze-thaw

Similar to Example 3, LNPs were prepared using the NanoAssemblr^{™} microfluidic system (Precision NanoSystems Inc., Vancouver, BC) according to standard protocols which enables controlled, bottom-up, molecular self-assembly of nanoparticles via custom-engineered microfluidic mixing chips that enable millisecond mixing of nanoparticle components at a nanolitre scale. The lipids were solubilized in alcoholic solution (ethanol) according to standard procedures. The corresponding lipid nanoparticle compositions are detailed in **Table Ex-4A.** In detail, LNPs were prepared by mixing appropriate volumes of lipid stock solutions in ethanol buffer with an aqueous phase (50 mM sodium acetate, pH 4.0) containing appropriate amounts of RABV-G (Rabies virus glycoprotein) mRNA (mRNA was produced according to the procedures described above, yielding a RABV-G mRNA comprising mCap, a muag-3'-UTR; 64x adenosine at the 3'-terminal end (polyA-tail); 5 nucleotides, 30x cytosine at the 3'-terminal end (polyC-tail), histone stem-loop and 5 additional nucleotides (SEQ ID NO:32)). Briefly, mRNA as indicated in the following working examples, was diluted to 0.05 to 0.2 mg/ml in 50 mM acetate buffer, pH 4. Syringe pumps were installed into inlet parts of the NanoAssemblr^{™} (Precision NanoSystems Inc., Vancouver, BC) and used to mix the ethanolic lipid solution with the mRNA aqueous solution at a ratio of about 1:5 to 1:3 (vol/vol) with total flow rates from about 14 ml/min to about 18 ml/min. The ethanol was then removed and the external buffer replaced with PBS/sucrose buffer (pH 7.4, 75 mM NaCl, 10mM phosphate, 150 mM sucrose) by dialysis (Slide-A-Lyzer^{™} Dialysis Cassettes, ThermoFisher). Finally, the lipid nanoparticles were filtered through a 0.2 µm pore sterile filter. Lipid nanoparticle particle diameter size was from about 90 nm to about 140 nm as determined by quasi-elastic light scattering using a Malvern Zetasizer Nano (Malvern Instruments Ltd.; Malvern, UK). For other cationic lipid compounds mentioned in the present specification, the formulation process is similar. For all LNPs, the ethanol was then removed and buffer replaced by 10 mM PBS, pH 7.4 comprising 9% sucrose.

**Table Ex-4A: Summary / overview of lipid nanoparticle compositions of working examples 6; reference to the cationic lipids as disclosed in Table Ex-1 and Table 1 or the specification above is made herein**

| Name of LNP formulation / composition No. | Excipients [cationic lipid : steroid : neutral lipid + optional further excipient : polymer conjugated lipid] | mol-percentages for excipients [mol%] |
|---|---|---|
| Formulation 1 | VitE-C4DE-Piperidine-Thioether : Cholesterol : DPhyPE : "PMOZ 4" with 50 monomer repeats | 59 : 29.3 : 10 : 1.7 |
| Formulation 2 | VitE-C4DE-Piperidine-Thioether : Cholesterol : DPhyPE : "PMOZ 4" with 50 monomer repeats | 59 : 28.5 : 10 : 2.5 |
| Formulation 3 | VitE-C4DE-Piperidine-Thioether : Cholesterol : DPhyPE : "PMOZ 5" with 50 monomer repeats | 59 : 29.3 : 10 : 1.7 |
| Formulation 4 | VitE-C4DE-Piperidine-Thioether : Cholesterol : DPhyPE : "PMOZ 5" with 50 monomer repeats | 59 : 28.5 : 10 : 2.5 |
| LNP-control 1 | VitE-C4DE-Piperidine-Thioether : Cholesterol : DPhyPE : DMG-PEG2000 | 59 : 29.3 : 10 : 1.7 |
| LNP-control 2 | VitE-C4DE-Piperidine-Thioether : Cholesterol : DPhyPE : DMG-PEG2000 | 59 : 28.5 : 10 : 2.5 |

LNPs as shown in Table Ex-4A were prepared fresh with an LNP concentration of 0.1 g/l. Consequently, fresh LNPs were diluted to different LNP concentrations (0.027 g/l and 0.01 g/l). Physiochemical characteristics were measured to compare PMOZ-LNPs with PEG-LNPs (PDI and size measurements).

For further analyzing the LNP stability after freeze/thaw, LNP formulations were also frozen and thawed. These thermal Stress studies were performed on the formulated LNPs at concentrations of 0.27 g/l (10 mM Phosphate pH 7.4, 75 mM NaCl, 150 mM Sucrose) stored in standard 2R glass vials. For one freeze-thawing cycle, LNPs were submitted to a freezer at -80°C, and removed seven days later from the freezer to thaw at room-temperature. Thereafter, again, physiochemical characteristics were measured to compare PMOZ-LNPs with PEG-LNPs (PDI and Zetasizer size measurements).

Data from different experiments can be found in **Table Ex-5 to Table Ex-8.**

**Table Ex-5: Freeze/Thaw (F/T) results part I**

| | **Before F/T** | | **After F/T (1 freeze-thaw cycle = FTC)** | | | |
|---|---|---|---|---|---|---|
| LNP | Particle size average (nm) | Polydispersity index | Particle size average (nm) | | Polydispersity index | |
| LNP-control 1 | 57.6 | 0.149 | 66.4 | +15% | 0.233 | +56% |
| LNP-control 2 | 62.2 | 0.227 | 74.4 | +20% | 0.371 | +63% |
| Formulation 1 | 50.4 | 0.09 | 51.9 | +3% | 0.140 | +56% |
| Formulation 2 | 51.6 | 0.204 | 53.0 | +3% | 0.227 | +11% |

**Table Ex-6: Freeze/Thaw (F/T) results part II**

| | **Before F/T** | | **After F/T (1 FTC)** | | | |
|---|---|---|---|---|---|---|
| LNP | Particle size average (nm) | Polydispersity index | Particle size average (nm) | | Polydispersity index | |
| LNP-control 1 | 98 | 0.183 | 112 | +15% | 0.171 | -7% |
| Formulation 1 | 86 | 0.163 | 93 | +8% | 0.160 | -2% |
| Formulation 2 | 70 | 0.146 | 80 | +15% | 0.196 | +34% |
| Formulation 3 | 87 | 0.122 | 87 | +0% | 0.139 | +14% |
| Formulation 4 | 65 | 0.147 | 75 | +16% | 0.200 | +36% |

**Table Ex-7: Freeze/Thaw (F/T) results part III**

| | **Before F/T** | | **After F/T (1 FTC)** | | | |
|---|---|---|---|---|---|---|
| LNP | Particle size average (nm) | Polydispersity index | Particle size average (nm) | | Polydispersity index | |
| LNP-control 1 | 108 | 0.178 | 117 | +8% | 0.150 | -15% |
| Formulation 1 | 88 | 0.080 | 90 | +2% | 0.106 | +32% |
| Formulation 2 | 73 | 0.116 | 76 | +4% | 0.151 | +30% |
| Formulation 3 | 89 | 0.163 | 93 | +4% | 0.144 | -11% |
| Formulation 4 | 71 | 0.148 | 75 | +6% | 0.183 | +24% |

**Table Ex-8: Freeze/Thaw (F/T) results part IV with dilutions (LNPs from Table Ex-8 were prepared with a different cargo, i.e. R9515 with SEQ ID NO:89)**

| | 0.27 g/L 0FTC | 0.027 g/L 1FTC | 0.01 g/L 1FTC |
|---|---|---|---|
| LNP-control 1 | 118 | 128 | 127.6 |
| Formulation 1 | 89 | 89.8 | 88.1 |
| Formulation 2 | 75.1 | 76.7 | 75.1 |
| Formulation 3 | 92.5 | 93.4 | 92 |
| Formulation 4 | 75.2 | 77.2 | 74.8 |

Results: in detail, when measuring size and PDI of LNP-formulations and comparing these with both control LNP-formulations, the following surprising and beneficial characteristics of the PMOZ-comprising LNPs were found:
- a disadvantageous increase of size and PDI was found for PEG-LNPs upon freeze/thaw (F/T) - the increase of size and PDI was not found, or respectively not found that pronounced, for LNPs comprising PMOZ as conjugated lipid;
- further, for dilutions, also a disadvantageous increase of size and PDI was found for PEG-LNPs, i.e. when PEG- and PMOZ-LNPs were diluted to different concentrations (0.27 g/l, 0.1 g/l, 0.027 g/l, and 0.01 g/l), size and PDI of PEG-LNPs increased - said increase of size and PDI upon dilution was not found, or respectively not found that pronounced, for LNPs comprising PMOZ as conjugated lipid (Table Ex-8).

In sum, PEG-LNPs had an increasing size and greater PDI when being frozen and thawed and/or diluted, while PMOZ-LNPs behaved superior over PEG-LNPs when it came to size (smaller) and PDI (narrower). In cases, in which PMOZ-LNPs show an increase of size in a similar range as PEG-LNPs, PMOZ-LNPs still were smaller than PEG-LNPs, which would be favorable as findings in that field indicate that smaller particles are more immunogenic.

### Example 7: Further data on effect of the presence of PMOZ-lipids in LNPs on RABV-G vaccination using intramuscular administration

To further analyse immunogenicity of LNPs comprising PMOZ-lipids instead of PEG-lipids, RABV-G (Rabies virus glycoprotein) mRNA was produced according to the procedures described above, yielding a RABV-G mRNA comprising mCap, a muag-3'-UTR; 64x adenosine at the 3'-terminal end (polyA-tail); 5 nucleotides, 30x cytosine at the 3'-terminal end (polyC-tail), histone stem-loop and 5 additional nucleotides (SEQ ID NO:32).

Resulting RABV-G mRNA was formulated in LNPs for comparison of LNPs comprising a PEG-lipid or, respectively, a PMOZ-lipid (GN01 comprising a DMG-PEG 2000 PEG-lipid versus GN01-PMOZ comprising a PMOZ-lipid). Composition and formulation details are shown herein below in **Table Ex-9.**

**Table Ex-9: Composition and formulation details for Example 7**

| **Group/ Set** | **Formulation designation** | **mRNA dose [µg]** | **Cationic lipid [mol%]** | **Chol [mol%]** | **DPhyP E [mol%]** | **Conjugated lipid [mol%]** | **No. of mice** | **Route / volume** | **Dosing [day]** |
|---|---|---|---|---|---|---|---|---|---|
| 1 | CV01 "PEG-DMG2000" 1.7% | 1 | C24, 59% | 29.3 | 10 | 1.7 [DMG-PEG 2000] | 5 | i.m. / 1x25 µl | 0, 21 |
| 2 | CV01 "PMOZ 4" 2.5% | 1 | C24, 59% | 28.5 | 10 | 2.5 "PMOZ 4" (with with 50 monomer repeats (n=50)) | 5 | i.m. / 1x25 µl | 0, 21 |
| 3 | CV01 "PMOZ 2" 2.5% | 1 | C24, 59% | 28.5 | | 2.5 "PMOZ 5" (with with 50 monomer repeats (n=50)) | 5 | i.m. / 1x25 µl | 0, 21 |
| Buffer (PBS) | / | / | / | / | / | / | 5 | i.m. / 1x25 µl | 0, 21 |

7 weeks old female Balb/C mice (n=8) were injected intramuscularly (M. tibialis) at day 0 and day 21 with the formulations and buffer-control according to **Table Ex-9** comprising above described RABV-G mRNA. For determining the levels of antibody against the rabies virus in serum, a classical virus neutralization test was performed (Fluorescent Antibody Virus Neutralization (FAVN) assay).

Accordingly, 28 days after the first mRNA administration, mice were sacrificed and blood and organ samples (liver) were collected for further analysis, i.e. for Virus neutralizing antibodies (VNA) analysis via FAVN assay. For said immunogenicity assays, the VNT was measured as described before, i.e. anti-rabies virus neutralizing titers (VNTs) in serum were analyzed by the Eurovir^{®} Hygiene-Labor GmbH, Germany, using the FAVN assay and the Standard Challenge Virus CVS-11 according to WHO protocol.

### Results:

Again, PMOZ-LNPs performed very well when compared to the standard controls, i.e. for all LNPs very high levels of Rabies VNTs were measured: **Figure 3** shows that already a single i.m. immunization with only 1 µg of mRNA formulated in PMOZ-LNPs induced very robust VNTs well above the protective titer of 0.5 IU/ml in all animals. After boost vaccination **(****Figure 4****),** this effect also was observed. Summarized, LNPs comprising "PMOZ 4", i.e. comprising the inventive preferred PMOZ lipid "PMOZ 4", outperformed all other LNPs LNPs by inducing the highest VNTs at both d21 and d28.

### Example 8: Effect of the presence of PMOZ-lipids in LNPs on cytokines

To analyze the effect of the presence of PMOZ-lipids in LNPs on cytokines, Firefly luciferase PpLuc mRNA (SEQ ID NO:71) was used for intravenous injection. Therefore, PpLuc was formulated in LNPs according to Table Ex-10

**Table Ex-10: Composition and formulation details for Example 8**

| **Group (Bar in** **Figure 5****)** | **Lipids (mol%)** | | | | **N:P ratio** |
|---|---|---|---|---|---|
| | **Ionizable lipid C24** | **Cholesterol** | **DPhyPE** | **polymer-conjugated lipid** | |
| 1 (DMG) | 59 | 29.3 | 10 | 2.5 "PMOZ 2" (with with 50 monomer repeats (n=50)) | 14 |
| 2 (Amide) | 59 | 28.5 | 10 | 2.5 "PMOZ 4" (with with 50 monomer repeats (n=50)) | 14 |
| 3 (mPEG DMG) | 59 | 29.3 | 10 | 1.7 DMG-PEG2000 | 14 |
| 4 (buffer) | ---- | | | | |

The formulations shown below in **Table Ex-10** were applied to female Balb/c mice on day 0 intravenously (i.v.) with doses of PpLuc RNA or buffer, respectively (100 µl injection volume each) according to the experimental setup as shown in Table Ex-11. Animals were treated with 2 µg or 20 µg of PpLuc mRNA, or buffer, respectively, depending on the cytokine assay. Serum samples were taken 14 days before the application and 4 h after injection. Cytokine levels in the sera of the animals were analyzed via IFN-alpha or IFN-gamma standard assay.

**Table Ex-11: Experimental setup**

| **Group** | **Group name in** **Figure 5** | **No. of mice** | **Injection of** | **dose (see x-axis** **Figure 5****)** |
|---|---|---|---|---|
| 1 (LNP 1) | 1 (DMG) | 5 | PpLuc mRNA | 2 µg or 20 µg |
| 2 (LNP 2) | 2 (Amide) | 5 | PpLuc mRNA | 2 µg or 20 µg |
| 3 (LNP 3) | 3 (mPEG DMG) | 5 | PpLuc mRNA | 2 µg or 20 µg |
| 4 (Buffer) | 4 (buffer) | 5 | Buffer | - |

### Results:

PpLuc expression was comparable for all groups (not shown). However, cytokine levels, as apparent from figure 5A and 5B were strongly reduced for LNPs comprising the inventive PMOZ-lipids when compared to LNPs comprising PEG-lipids which may be of advantage for Molecular Therapy (mRNA replacement therapy) approaches.

### Example P1 [prophetic]: Effect of the presence of short phospholipids and PMOZ-lipids in LNPs on RABV-G vaccination using intramuscular administration

To analyse the immunogenicity of LNPs according to **Table Ex-2B,** RABV-G (Rabies virus glycoprotein) mRNA was produced according to the procedures described above, yielding a RABV-G mRNA comprising mCap, a muag 3'-UTR; 64x adenosine at the 3'-terminal end (polyA-tail); 5 nucleotides, 30x cytosine at the 3'-terminal end (polyC-tail), a histone stem-loop and 5 additional nucleotides (SEQ ID NO:32).

7 weeks old female Balb/C mice (n=8 for each group of **Table Ex-P1)** are injected intramuscularly at day 0 and day 21 with the formulations according to **Table Ex-2B** comprising above described RABV-G mRNA.

**Table Ex-P1: Composition and formulation details; further reference is made to descriptions under Table Ex-2B where the mol%-ratios of the compositions are described**

| **Group** | **Name of** GN02-based **LNP formulation** / **composition** | **mRNA Dose [µg]** | **Route / volume** | **Dosing [day]** |
|---|---|---|---|---|
| 1 | LNP18C | 5 | i.m. / 1x25 µl | 0, 21 |
| 2 | LNP18D | 5 | i.m. / 1x25 µl | 0, 21 |
| 3 | LNP18E (GN02 control) | 5 | i.m. / 1x25 µl | 0, 21 |
| 4 | PBS only | / | i.m. / 1x25 µl | 0, 21 |

For determining the levels of antibody against the rabies virus in serum, a classical virus neutralization test is performed (Fluorescent Antibody Virus Neutralization (FAVN) assay) for the groups of **Table Ex-P1.**

28 days after the first mRNA administration, mice are sacrificed and blood and organ samples (spleen) are collected for further analysis. In this regard, rabies virus glycoprotein (RABV-G)-specific cellular responses in splenocyte samples obtained in this step are measured as RABV-G-specific T cell activation. This is analyzed by intracellular cytokine staining and subsequent analysis by flow cytometry according to standard protocols as follows: splenocytes are stimulated with a RABV-G peptide cocktail in the presence of anti-CD107a (Biolegend, San Diego, USA) and anti-CD28 (BD Biosciences, San Jose, USA). Unstimulated splenocytes are treated the same way but were not supplemented with the peptide cocktail. Additional controls are splenocytes stimulated with PMA/ionomycin (no anti-CD28; PMA and ionomycin from Sigma-Aldrich; Merck KGaA, Darmstadt, Germany) (positive control) and splenocytes which are left unstained by fluorophore-conjugated antibodies (negative control). After the stimulation procedure, splenocytes are stained with surface and intracellular, fluorophore-conjugated antibodies and analysed by flow cytometry.

Serum samples are also taken on day 21 prior to the boost, wherein the serum samples at day 21 are analyzed for Virus neutralizing antibodies (VNA) analysis via FAVN assay. Further, serum samples are taken 18h after first application of the formulation for an early analysis of cytokine levels in the serum of mice immunized with RABV-G-encoding mRNA formulated with LNPs.

For said immunogenicity assays, the VNT is measured as described before, i.e. anti-rabies virus neutralizing titers (VNTs) in serum are analyzed by the Eurovir^{®} Hygiene-Labor GmbH, Germany, using the FAVN assay and the Standard Challenge Virus CVS-11 according to WHO protocol.

A CBA assay is performed with serum samples drawn from mice 18 h after immunization with LNP-formulated antigen-encoding mRNA with the following cytokines/chemokines included in the array: MIG, MCP-1, MIP-1α, MIP-1β, RANTES, IL-12p70, IL-6, TNF, IL-1β, IFN-gamma. Furthermore, the level of IFN-alpha in the serum is determined by ELISA according to standard protocols.

Furthermore, for the groups of **Table Ex-P1,** the spleen samples taken at day 28 are re-stimulated with a RABV-G peptide library and assayed for the T cell response (CD4 and CD8), i.e. CD4 T cell immune response (IFN-gamma/TNF-alpha producing CD4 T cells) and CD8 T cell immune response (IFN-gamma/TNF-alpha producing CD8 T cells and CD107+ IFN-gamma producing CD8 T cells); induction of antigen-specific T cells is determined using intracellular cytokine staining (ICS). Assays are performed as described before.

### Example P2 [prophetic]: Formulation and biophysical characterization of different lipid nanoparticle compositions

The LNPs as shown in **Table Ex-P2** are formulated in accordance with the procedure shown in **Example 3.** mRNA which is encapsulated is indicated herein below.

Characterization of the LNPs as shown in **Table Ex-P2** and **Table Ex-2A, Table Ex-2B** and **Table Ex-4** is carried out as set out in **Example 3.1** to determine encapsulation efficiency, particle size, and zeta potential. Further, the pKₐ is measured. pKₐ is the negative base 10 logarithm of the acid dissociation constant (Kₐ) of a solution, i.e. pKₐ = -log(Kₐ). The pKₐ value is measured according to standard proceedings with fluorescent dye 2-p-toluidinylnaphthalene-6-sulphonate (TNS).

**Table Ex-P2: Formulation summary of HEAD lipids and controls**

| **Group** | **Name of LNP formulation / composition designation** | **Excipients [cationic lipid as disclosed in Table Ex-1 (or respectively Table 1): steroid : neutral lipid (optionally two as indicated) : polymer conjugated lipid]** | **mol-percentages for excipients [mol%]** |
|---|---|---|---|
| 1 | LNP28P-1.7 | VitE-C4DE-Piperidine-Thioether: Chol : DPhyPE : DMG-PMOZ | 59 : 29.3 : 10 : 1.7 |
| 2 | LNP29P-1.7 | VitE-C4DE-Pip-C3SS : Chol : DPhyPE : DMG-PMOZ | 59 : 29.3 : 10 : 1.7 |
| 3 | LNP30P-1.7 | VitE-C4DE-Piperidine-Thioether: Chol : DPhyPE + (07:0) PC (DHPC) : DMG-PMOZ | 58 : 29.3 : 10+1: 1.7 |
| 4 | LNP31P-1.7 | VitE-C4DE-Piperidine-Thioether: Chol : DPhyPE + (07:0) PC (DHPC) DMG-PMOZ | 49 : 29.3 : 10+10 : 1.7 |
| 5 | LNP32P-1.7 | VitE-C4DE-Piperidine-Thioether: Chol : DPhyPE : DMG-PMOZ | 49 : 39.3 : 10 : 1.7 |
| 6 | LNP28P-2.5 | VitE-C4DE-Piperidine-Thioether: Chol : DPhyPE : DMG-PMOZ | 59 : 28.5 : 10 : 2.5 |
| 7 | LNP29P-2.5 | VitE-C4DE-Pip-C3SS : Chol : DPhyPE : DMG-PMOZ | 59 : 28.5 : 10 : 2.5 |
| 8 | LNP30P-2.5 | VitE-C4DE-Piperidine-Thioether: Chol : DPhyPE + (07:0) PC (DHPC) : DMG-PMOZ | 58 : 28.5 : 10+1 : 2.5 |
| 9 | LNP31 P-2.5 | VitE-C4DE-Piperidine-Thioether: Chol : DPhyPE + (07:0) PC (DHPC) DMG-PMOZ | 49 : 28.5 : 10+10 : 2.5 |
| 10 | LNP32P-2.5 | VitE-C4DE-Piperidine-Thioether : Chol : DPhyPE : DMG-PMOZ | 49 : 38.5 : 10 : 2.5 |
| 11 | LNP28P-5 | VitE-C4DE-Piperidine-Thioether: Chol : DPhyPE : DMG-PMOZ | 59 : 26 : 10 : 5 |
| 12 | LNP29P-5 | VitE-C4DE-Pip-C3SS : Chol : DPhyPE : DMG-PMOZ | 59 : 26 : 10 : 5 |
| 13 | LNP30P-5 | VitE-C4DE-Piperidine-Thioether : Chol : DPhyPE + (07:0) PC (DHPC) : DMG-PMOZ | 58 : 26 : 10+1 : 5 |
| 14 | LNP31P-5 | VitE-C4DE-Piperidine-Thioether: Chol : DPhyPE + (07:0) PC (DHPC) DMG-PMOZ | 49 : 26 : 10+10 : 5 |
| 15 | LNP32P-5 | VitE-C4DE-Piperidine-Thioether : Chol : DPhyPE : DMG-PMOZ | 49 : 36 : 10 : 5 |
| 16 | LNP-C1 | state of the art cationic lipid : Chol : DSPC : DMG-PMOZ | 47.4 : 40.9 : 10 : 1.7 |
| 17 | LNP-C2 | state of the art cationic lipid : Chol : DSPC : DMG-PEG2000 | 47.4 : 40.9 : 10 : 1.7 |
| 18 | LNP-C3 | state of the art cationic lipid : Chol : DSPC : DMG-PMOZ | 47.4 : 40.1 : 10 : 2.5 |
| 19 | LNP-C4 | state of the art cationic lipid : Chol : DSPC : DMG-PEG2000 | 47.4 : 40.1 : 10 : 2.5 |

To analyse the immunogenicity of the LNPs shown in **Table Ex-P1,**
- RABV-G (Rabies virus glycoprotein) mRNA is produced according to the procedures described above, yielding a RABV-G mRNA comprising mCap, a muag 3'-UTR; 64x adenosine at the 3'-terminal end (polyA-tail); 5 nucleotides, 30x cytosine at the 3'-terminal end (polyC-tail), a histone stem-loop and 5 additional nucleotides (SEQ ID NO:32). 7 weeks old female Balb/C mice (n=8) are injected intramuscularly at day 0 and day 21 with the formulations according to **Table Ex-P1** (each 25 µl, comprising 1 µg mRNA each) comprising above described RABV-G mRNA. A control group (n=8) receives a buffer injection i.m. (25 µl).
- PfCSP mRNA comprising a 5'-UTR from HSD17B4, a 3'-UTR from PSMB3, 64x adenosine at the 3'-terminal end (polyA-tail); 5 nucleotides, 30x cytosine at the 3'-terminal end (poly-C-tail), a histone stem-loop and 5 additional nucleotides is produced according to the procedure described above. The mRNA is further enzymatically capped using ScriptCap^{™} m7G Capping System (CellScript, Madison, WI, USA) according to the manufacturer's instructions and enzymatically polyadenylated using a commercial polyadenylation kit, resulting in SEQ ID NO:38. The LNP formulations according to **Table Ex-P1** (each 25 µl, comprising 1 µg mRNA each), are applied to 7 weeks old female Balb/C mice (n=8) which are injected intramuscularly at day 0 and day 21. A control group (n=8) receives a buffer injection i.m. (25 µl). Serum samples are taken at day 21 and day 35 for ELISA.

### Example P3 [prophetic]: Analysis of response to LNP formulated PfCSP-mRNA of Example P2

### Determination of specific humoral immune responses by ELISA:

ELISA is performed using malaria [NANP]₇ peptide or C-terminus peptides for coating. Coated plates are incubated using respective serum dilutions, and binding of specific antibodies to the respective malaria [NANP]₇ or C-terminus peptide are detected using biotinylated isotype specific anti-mouse antibodies followed by streptavidin-HRP (horse radish peroxidase) with Amplex^{™} Red Reagent as substrate. Endpoint titers of antibodies (total IgG) directed against the malaria [NANP]₇ or C-terminus peptide are measured by ELISA on day 35 post prime.

### Intracellular cytokine staining:

Splenocytes from vaccinated mice are isolated on day 35 according to a standard protocol known in the art. Briefly, isolated spleens are grinded through a cell strainer and washed in PBS/1%FBS followed by red blood cell lysis. After an extensive washing step with PBS/1% FBS, splenocytes are seeded into 96-well plates (2x10⁶ cells per well). Cells are stimulated with a mixture of PfCSP peptides (1 µg/ml) in the presence of 2.5 µg/ml of an anti-CD28 antibody (BD Biosciences) and a protein transport inhibitor for 6 h at 37°C. After stimulation, cells are washed and stained for intracellular cytokines using the Cytofix/Cytoperm^{™} reagent (BD Biosciences) according to the manufacturer's instructions. The following antibodies are used for staining: Thy1.2-FITC (1:100), CD8-PE-Cy7 (1:200), TNF-PE (1:100), IFNγ-APC (1:100) (eBioscience), CD4-BD Horizon V450 (1:200) (BD Biosciences) and incubated with Fcγ-block diluted 1:100. Aqua Dye is used to distinguish live/dead cells (Invitrogen). Cells are acquired using a BD FACS Canto II flow cytometer (Becton Dickinson). Flow cytometry data is analyzed using FlowJo software (Tree Star, Inc.).

LNP formulated PfCSP mRNA vaccine induces strong, humoral immune responses in mice.

### Example P4 [prophetic]: Analysis of response to LNP formulated RABV-G-mRNA of Example P2

For determining the levels of antibody against the rabies virus in serum, a classical virus neutralization test is performed (Fluorescent Antibody Virus Neutralization (FAVN) assay). Accordingly, 35 days after the first mRNA administration, mice are sacrificed and blood and organ samples (liver) are collected for further analysis, i.e. for Virus neutralizing antibodies (VNA) analysis via FAVN assay. For said immunogenicity assays, the VNT is measured as described before, i.e. anti-rabies virus neutralizing titers (VNTs) in serum are analyzed by the Eurovir^{®} Hygiene-Labor GmbH, Germany, using the FAVN assay and the Standard Challenge Virus CVS-11 according to WHO protocol.

Furthermore, liver samples are taken for analysis of T cell response (CD4 and CD8), i.e. CD4 T cell immune response (IFN-gamma/TNF-alpha producing CD4 T cells) and CD8 T cell immune response (IFN-gamma/TNF-alpha producing CD8 T cells and CD107+ IFN-gamma producing CD8 T cells) are assessed; induction of antigen-specific T cells is determined using intracellular cytokine staining (ICS). Assays are performed as described before.

Already a single i.m. immunization with induces very robust VNTs well above the protective titer of 0.5 IU/ml in all animals at day 35 after prime vaccination.

### Example P5 [prophetic]: In vivo effect of polymer conjugated lipid (PMOZ-lipid) component after intradermal and intramuscular injections in mice

To analyse the effect of the disclosed PMOZ-components in LNPs *in vivo,* the LNPs from **Tables Ex-2, Ex-3 and Ex-4** are used analogously in an in vivo setting. PpLuc or HsEPO mRNA are used as cargo. For in vivo analysis, six to eight weeks old Balb/C mice (5 mice per group) are injected with 0.5 mg/kg LNP formulated hEpo. One group is administered with PpLuc or hEpo LNP-formulated mRNA via intradermal route and another group is administered with PpLuc or hEpo LNP-formulated via the intramuscular route.

### Example P6 [prophetic]: Effect of PMOZ-lipids in LNPs on RABV-G vaccination using intramuscular administration

To analyse the immunogenicity of LNPs according to **Table Ex-P6,** RABV-G (Rabies virus glycoprotein) mRNA was produced according to the procedures described above, yielding a RABV-G mRNA comprising mCap, a muag 3'-UTR; 64x adenosine at the 3'-terminal end (polyA-tail); 5 nucleotides, 30x cytosine at the 3'-terminal end (polyC-tail), a histone stem-loop and 5 additional nucleotides (SEQ ID NO:32).

7 weeks old female Balb/C mice (n=8 for each group of **Table Ex-P6)** are injected intramuscularly at day 0 and day 21 with the formulations according to **Table Ex-2B** comprising above described RAV-G mRNA (i.m. / 1x25 µl, dosing day 0 and 21 prime/boost).

**Table Ex-P6: Formulation summary of HEAD lipids and controls (LNP-C1-4)**

| **Group** | **Name of LNP formulation / composition designation** | **Excipients [cationic lipid as disclosed in Table Ex-1 (or respectively Table 1): steroid : neutral lipid (optionally two as indicated) : polymer conjugated lipid]** | **mol-percentages for excipients [mol%]** |
|---|---|---|---|
| I-1 | LNP I-1 | VitE-C4DE-Piperidine-Thioether : Chol : DPhyPE : PMOZ-DMG lipid comprising 5K PMOZ | 59 : 29.3 : 10 : 1.7 |
| I-2 | LNP I-2 | VitE-C4DE-Piperidine-Thioether : Chol : DPhyPE : PMOZ-DMG lipid comprising 5K PEOZ | 59 : 29.3 : 10 : 1.7 |
| I-3 | LNP I-3 | VitE-C4DE-Piperidine-Thioether : Chol : DPhyPE : PEG-DMG | 59 : 29.3 : 10 : 1.7 |
| I-4 | LNP I-4 | VitE-C4DE-Piperidine-Thioether : Chol : DPhyPE : "PMOZ 1" | 59 : 28.5 : 10 : 2.5 |
| I-5 | LNP I-5 | VitE-C4DE-Piperidine-Thioether : Chol : DPhyPE : "PMOZ 2" | 59 : 28.5 : 10 : 2.5 |
| I-6 | LNP I-6 | VitE-C4DE-Piperidine-Thioether : Chol : DPhyPE : "PMOZ 3" | 59 : 28.5 : 10 : 2.5 |
| I-7 | LNP I-7 | VitE-C4DE-Piperidine-Thioether : Chol : DPhyPE : "PMOZ 4" | 59 : 28.5 : 10 : 2.5 |
| I-8 | LNP I-8 | VitE-C4DE-Piperidine-Thioether : Chol : DPhyPE : "PMOZ 5" | 59 : 28.5 : 10 : 2.5 |
| I-9 | LNP I-9 | VitE-C4DE-Piperidine-Thioether : Chol : DPhyPE : "PMOZ 1" | 59 : 29.3 : 10 : 1.7 |
| I-10 | LNP I-10 | VitE-C4DE-Piperidine-Thioether : Chol : DPhyPE : "PMOZ 2" | 59 : 29.3 : 10 : 1.7 |
| I-11 | LNP I-11 | VitE-C4DE-Piperidine-Thioether : Chol : DPhyPE : "PMOZ 3" | 59 : 29.3 : 10 : 1.7 |
| I-12 | LNP I-12 | VitE-C4DE-Piperidine-Thioether : Chol : DPhyPE : "PMOZ 4" | 59 : 29.3 : 10 : 1.7 |
| I-13 | LNP I-13 | VitE-C4DE-Piperidine-Thioether : Chol : DPhyPE : "PMOZ 5" | 59 : 29.3 : 10 : 1.7 |
| II-1 | LNP II-1 | VitE-C4DE-Piperidine-Thioether : Chol : DPhyPE : "PMOZ 1" | 47.4 : 40.1 : 10 : 2.5 |
| II-2 | LNP II-2 | VitE-C4DE-Piperidine-Thioether : Chol : DPhyPE : "PMOZ 2" | 47.4 : 40.1 : 10 : 2.5 |
| II-3 | LNP II-3 | VitE-C4DE-Piperidine-Thioether : Chol : DPhyPE : "PMOZ 3" | 47.4 : 40.1 : 10 : 2.5 |
| II-4 | LNP II-4 | VitE-C4DE-Piperidine-Thioether : Chol : DPhyPE : "PMOZ 4" | 47.4 : 40.1 : 10 : 2.5 |
| II-5 | LNP II-5 | VitE-C4DE-Piperidine-Thioether : Chol : DPhyPE : "PMOZ 5" | 47.4 : 40.1 : 10 : 2.5 |
| II-6 | LNP II-6 | VitE-C4DE-Piperidine-Thioether : Chol : DPhyPE : PEG-lipid (i.e. "PMOZ 1", wherein PMOZ-moiety has been replaced by PEG2000) | 47.4 : 40.9 : 10 : 1.7 |

For determining the levels of antibody against the rabies virus in serum, a classical virus neutralization test is performed (Fluorescent Antibody Virus Neutralization (FAVN) assay) for the groups of **Table Ex-P6.**

28 days after the first mRNA administration, mice are sacrificed and blood and organ samples (spleen) are collected for further analysis. In this regard, rabies virus glycoprotein (RABV-G)-specific cellular responses in splenocyte samples obtained in this step are measured as RABV-G-specific T cell activation. This is analyzed by intracellular cytokine staining and subsequent analysis by flow cytometry according to standard protocols as follows: splenocytes are stimulated with a RABV-G peptide cocktail in the presence of anti-CD107a (Biolegend, San Diego, USA) and anti-CD28 (BD Biosciences, San Jose, USA). Unstimulated splenocytes are treated the same way but were not supplemented with the peptide cocktail. Additional controls are splenocytes stimulated with PMA/ionomycin (no anti-CD28; PMA and ionomycin from Sigma-Aldrich; Merck KGaA, Darmstadt, Germany) (positive control) and splenocytes which are left unstained by fluorophore-conjugated antibodies (negative control). After the stimulation procedure, splenocytes are stained with surface and intracellular, fluorophore-conjugated antibodies and analysed by flow cytometry.

Serum samples are also taken on day 21 prior to the boost, wherein the serum samples at day 21 are analyzed for Virus neutralizing antibodies (VNA) analysis via FAVN assay. Further, serum samples are taken 18 h after first application of the formulation for an early analysis of cytokine levels in the serum of mice immunized with RABV-G-encoding mRNA formulated with LNPs.

For said immunogenicity assays, the VNT is measured as described before, i.e. anti-rabies virus neutralizing titers (VNTs) in serum are analyzed by the Eurovir^{®} Hygiene-Labor GmbH, Germany, using the FAVN assay and the Standard Challenge Virus CVS-11 according to WHO protocol.

A CBA assay is performed with serum samples drawn from mice 18 h after immunization with LNP-formulated antigen-encoding mRNA with the following cytokines/chemokines included in the array: MIG, MCP-1, MIP-1α, MIP-1β, RANTES, IL-12p70, IL-6, TNF, IL-1β, IFN-gamma. Furthermore, the level of IFN-alpha in the serum is determined by ELISA according to standard protocols.

Furthermore, for the groups of **Table Ex-P6,** the spleen samples taken at day 28 are re-stimulated with a RABV-G peptide library and assayed for the T cell response (CD4 and CD8), i.e. CD4 T cell immune response (IFN-gamma/TNF-alpha producing CD4 T cells) and CD8 T cell immune response (IFN-gamma/TNF-alpha producing CD8 T cells and CD107+ IFN-gamma producing CD8 T cells); induction of antigen-specific T cells is determined using intracellular cytokine staining (ICS). Assays are performed as described before.

A set of embodiments of the present invention relates to:
1. A polymer conjugated lipid according to formula (I):

   [P]-[linker]-[L] formula (I)

   or a pharmaceutically acceptable salt, prodrug, tautomer or stereoisomer thereof, wherein
   [P] is a heteropolymer moiety or homopolymer moiety, preferably a homopolymer moiety, comprising at least one polyoxazoline (POZ) monomer unit 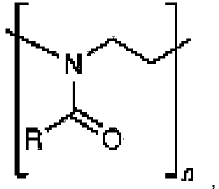 wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.4 kDa, or most preferably about 4.3 kDa
   [linker] is an optional linker group, and
   [L] is a lipid moiety.
2. The polymer conjugated lipid of **embodiment 1,** wherein [P] is a heteropolymer moiety or homopolymer moiety comprising multiple monomer units selected from the group consisting of
   poly(2-methyl-2-oxazoline) (PMOZ) 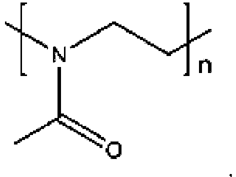
   poly(2-ethyl-2-oxazoline) (PEOZ) 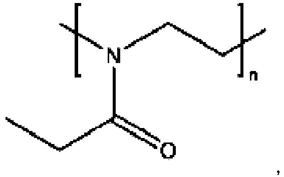
   poly(2-propyl-2-oxazoline) (PPOZ) 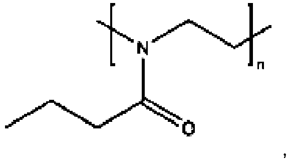
   poly(2-butyl-2-oxazoline) (PBOZ) 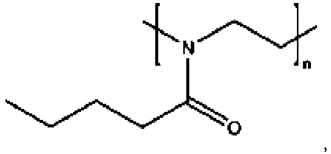
   poly(2-isopropyl-2-oxazoline) (PIPOZ) 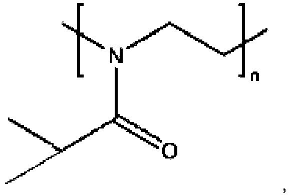
   poly(2-methoxymethyl-2-oxazoline) (PMeOMeOx), and
   poly(2-dimethylamino-2-oxazoline) (PDMAOx),

   preferably wherein [P] is a homopolymer moiety comprising multiple PMOZ or PEOZ monomer units, more preferably wherein [P] comprises or preferably consists of multiple PMOZ monomer units,
   wherein
      (i) n has a mean value ranging from about 45 to about 55, preferably n is about 50 or wherein
      (ii) n is selected such that the [P] moiety has an average molecular weight of about 3 kDa to about 6 kDa, preferably an average molecular weight of about 4.2 kDa to about 4.4 kDa, or most preferably about 4.3 kDa.
3. The polymer conjugated lipid of any one of **embodiment 1 to embodiment 2,** wherein the polymer conjugated lipid is selected from the group consisting of a POZ-monoacylglycerol conjugate, POZ-diacylglycerol conjugate, a POZ-dialkyloxypropyl conjugate, a POZ-steroid or POZ-sterol conjugate, a POZ-phospholipid conjugate, a POZ-ceramide conjugate, and a mixture thereof.
4. The polymer conjugated lipid of any one of **embodiment 1 to embodiment 3,** wherein
   (i) the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain,
      wherein the alkyl chain is optionally interrupted by one or more biodegradable group(s) and/or optionally comprises one terminal biodegradable group, wherein the biodegradable group is selected from the group consisting of but not limited to a pH-sensitive moiety, an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., -C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, -SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), cyclic compound, heterocyclic compound, piperidine, pyrazine, pyridine, piperazine, and sulfonate esters, as well as combinations thereof, wherein R³, R⁴ and R⁵ are, independently H or alkyl (e.g. C₁-C₄ alkyl), or
   (ii) the lipid moiety [L] comprises ditetradecylamin, preferably wherein the linker group [linker] is (-NHC(O)CH₂CH₂C(O)-).
5. The polymer conjugated lipid of any one of **embodiment 1 to embodiment 4,** wherein the lipid moiety [L] comprises at least one, preferably two, straight or branched, saturated or unsaturated alkyl chain comprising 6, 7, 8, 9, 10, 11, 12, 13, 14, 15, 16, 17, 18, 19, 20, 21, 22, 23, 24, 25, 26, 27, 28, 29, or 30 carbon atoms, preferably in the range of 10 to 20 carbon atoms, more preferably in the range of 12 to 18 carbon atoms, even more preferably 14, 16 or 18 carbon atoms, even more preferably 16 or 18 carbon atoms, most preferably 14 carbon atoms,
   wherein all selections are independent of one another.
6. The polymer conjugated lipid of any one of **embodiment 1 to embodiment 5,** wherein the linker group [linker] is selected from the group consisting of but not limited to a pH-sensitive moiety, a peptide or amid bond (-CO-NH-), an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime (e.g., - C(H)=N-O- or -O- N=C(H)-), carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), (-NHC(O)CH₂CH₂C(O)O-), -C(R5)=N-, -N=C(R⁵)-, -C(R⁵)=N-O-, -O-N=C(R⁵)-, -O-C(O)O-, -C(O)N(R⁵), -N(R⁵)C(O)-, -C(S)(NR⁵)-, (NR⁵)C(S)-, -N(R⁵)C(O)N(R⁵)-, -C(O)S-, - SC(O)-, -C(S)O-, -OC(S)-, -OSi(R⁵)₂O-, -C(O)(CR³R⁴)C(O)O-, or -OC(O)(CR³R⁴)C(O)-, carbonate (-OC(O)O-), nitrogen (N), succinoyl, succinate, phosphate esters (-O-(O)POH-O-), and sulfonate esters, as well as combinations thereof, wherein R³, R⁴ and R⁵ are, independently H or alkyl (e.g. C₁-C₄ alkyl), preferably wherein the linker group [linker] is selected from the group consisting of (-NHC(O)CH2CH2C(O)-), a peptide bond or amid bond (-CO-NH-), (-NHC(O)CH2CH2C(O)O-), and -NH-CH2-.
7. The polymer conjugated lipid of any one of **embodiment 1 to embodiment** 6, wherein the linker group [linker] comprises an amide linker moiety, preferably an ester linker moiety, or wherein the linker group [linker] comprises 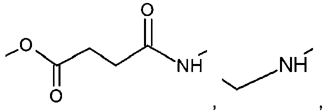 succinate, a peptide or amid bond (-CO-NH-), an amine, or a secondary amine, most preferably wherein the linker group [linker] comprises (-NHC(O)CH₂CH₂C(O)-).
8. The polymer conjugated lipid of any one of **embodiment 1 to embodiment 7,** wherein the polymer conjugated lipid is selected from the group consisting of "PMOZ 1", "PMOZ 2", "PMOZ 3", "PMOZ 4" and "PMOZ 5", most preferably the polymer conjugated lipid is "PMOZ 4";
   whereby n has a mean value ranging from 2 to 200, preferably from 20 to 100, more preferably from 24 to 26, even more preferably about 100, or further even more preferably from 45 to 50, most preferably 50 or wherein n is selected such that the [P] moiety has an average molecular weight of about 3 kDa to about 6 kDa, preferably has an average molecular weight of about 4.2 kDa to about 4.4 kDa, or most preferably about 4.3 kDa;
   most preferably wherein the polymer conjugated lipid of any one of **embodiment 1 to embodiment 7** is "PMOZ 4" with n having a mean value from 45 to 50, most preferably 50.
9. A lipid nanoparticle comprising a homopolymer moiety comprising at least one polyoxazoline (POZ) monomer unit 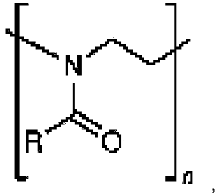
   wherein R is C₁-C₉ alkyl or C₂-C₉ alkenyl, preferably C₁ or C₂ alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa,
   preferably, wherein the homopolymer moiety comprising multiple monomer units comprises poly(2-methyl-2-oxazoline) (PMOZ), poly(2-ethyl-2-oxazoline) (PEOZ), poly(2-propyl-2-oxazoline) (PPOZ), poly(2-butyl-2-oxazoline) (PBOZ), poly(2-isopropyl-2-oxazoline) (PIPOZ), poly(2-methoxymethyl-2-oxazoline) (PMeOMeOx), or poly(2-dimethylamino-2-oxazoline) (PDMAOx),
   more preferably the polymer conjugated lipid according to any one of **embodiment 1 to embodiment 8.**
10. The lipid nanoparticle of **embodiment 9,** wherein the lipid nanoparticle further comprises a cationic or ionizable lipid.
11. The lipid nanoparticle of **embodiment 9 to embodiment 10,** wherein the lipid nanoparticles
   (i) do not comprise a polyethylene glycol-(PEG)-lipid conjugate or a conjugate of PEG and a lipid-like material, and preferably do not comprise PEG; and/or
   (ii) do not comprise a polymer conjugated lipid according to any one of **embodiment 1 to embodiment 8** comprising a sulphur group (-S-), a terminating nucleophile, and/or being covalently coupled to a biologically active ingredient being a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA.
12. The lipid nanoparticle of any one of **embodiment 9 to embodiment 11,** wherein the cationic or ionizable lipid preferably carries a net positive charge at physiological pH, more preferably wherein the cationic or ionizable lipid comprises a tertiary nitrogen group or quaternary nitrogen group.
13. The lipid nanoparticle of any one of **embodiment 9 to embodiment 12,** wherein the lipid nanoparticle further comprises a phospholipid, wherein preferably the phospholipid is a zwitterionic compound selected from, but not limited to the group of 1,2-diphytanoyl-sn-glycero-3-phosphoethanolamine (DPhyPE; 1,2-di-(3,7,11,15-tetramethylhexadecanoyl)-sn-glycero-3-phosphoethanolamine), 1,2-diphytanoyl-sn-glycero-3-phosphocholine (DPhyPC), 1,2-dioleoyl-sn-glycero-3-phosphocholine (DOPC; dioleoylphosphatidylcholine), 1,2-Dipalmitoyl-sn-glycero-3-phosphocholine (DPPC; dipalmitoylphosphatidylcholine), 1,2-dioleoyl-sn-glycero-3-phosphoethanolamine (DOPE), phosphatidylethanolamines, distearoylphosphatidylcholines, dioleoyl-phosphatidylethanolamine (DOPEA), 1,2-distearoyl-sn-glycero-3-phosphoethanolamine (DSPE), palmitoyloleoylphosphatidylcholine (POPC), palmitoyloleoyl-phosphatidylethanolamine (POPE), 1,2-Dipalmitoyl-sn-glycero-3-phosphoethanolamine (DPPE), dioleoyl-phosphatidylethanolamine 4-(N-maleimidomethyl)-cyclohexane-1-carboxylate (DOPE-mal), 1,2-Dimyristoyl-sn-glycero-3-phosphoethanolamine (DMPE), 1,2-Dilinoleoyl-sn-glycero-3-phosphoethanolamine (DLoPE), distearoyl-phosphatidylethanolamine (DSPE), 1-Palmitoyl-2-oleoyl-sn-glycero-3-phosphoethanolamine (POPE), 1,2-Dilauroyl-sn-glycero-3-phosphoethanolamine (DLPE), 16-O-monomethylphosphoethanolamine, 16-O-dimethyl phosphatidylethanolamine, 1,2-Dierucoyl-sn-glycero-3-phosphoethanolamine (DEPE), 18-1-trans phosphatidylethanolamine, 1-stearoyl-2-oleoylphosphatidyethanolamine (SOPE), 1,2-Disqualeoyl-sn-glycero-3-phosphoethanolamine (DSQPE), 1,2-dielaidoyl-sn-glycero-3-phosphoethanolamine (transDOPE), 1-Stearoyl-2-linoleoyl-sn-glycero-3-phosphoethanolamine (SLPE), 1-tridecanoyl-sn-glycero-3-phospho-L-serine (sodium salt), 1-oleoyl-2-hydroxy-sn-glycero-3-phospho-L-serine (sodium salt), 1-palmitoyl-2-oleoyl-sn-glycero-3-phospho-L-serine (sodium salt) (POPS), 1-1-stearoyl-2-oleoyl-sn-glycero-3-phospho-L-serine (sodium salt), 1,2-dioleoyl-sn-glycero-3-phospho-L-serine (sodium salt) (DOPS), 1,2-distearoyl-sn-glycero-3-phospho-L-serine (sodium salt), 1,2-diphytanoyl-sn-glycero-3-phospho-L-serine (sodium salt), 1-0-hexadecanyl-2-0-(9Z-octadecenyl)-sn-glycero-3-phosphoethanolamine, 1,2-distearoyl-sn-glycero-3-phosphatidylcholine or 1,2-distearoyl-sn-glycero-3-phosphocholine (DSPC), 1,2-di-O-phytanyl-sn-glycero-3-phosphoethanolamine, 1-palmitoyl-2-cholesterylhemisuccinoyl-sn-glycero-3-phosphocholine (PChemsPC), 1,2-dicholesterylhemisuccinoyl-sn-glycero-3-phosphocholine (DChemsPC), 2-((2,3-bis(oleoyloxy)propyl)dimethylammonio)ethyl hydrogen phosphate (DOCP), 2-((2,3-bis(oleoyloxy)propyl)dimtheylammonio)ethyl ethyl phosphate (DOCPe), and 1-O-octadecyl-2-O-methyl-sn-glycero-3-phosphocholine (Edelfosine), preferably wherein the phospholipid is DSPC or DPhyPE, most preferably DPhyPE.
14. The lipid nanoparticle of any one of **embodiment 9 to embodiment 13,** wherein the lipid nanoparticle further comprises a sterol or steroid, preferably selected from the group consisting of cholesterol, cholesteryl hemisuccinate (CHEMS) and a derivate thereof, preferably wherein the lipid nanoparticle further comprises cholesterol.
15. The lipid nanoparticle of any one of **embodiment 9 to embodiment 14,** wherein preferably the lipid nanoparticle comprises
   (i) an amount of 1, 2, 3, 4, 5, 6, 7, 8, 9, or 10 mol% of the polymer conjugated lipid of any one of **embodiment 1 to embodiment 8;**
   (ii) preferably an amount of 5 mol% of the polymer conjugated lipid of any one of **embodiment 1 to embodiment 8,**
   (iii) more preferably an amount of 2.5 mol% of the polymer conjugated lipid of any one of **embodiment 1 to embodiment 8;**
   based upon a mol-percentage of the composition of 100% of all lipid components or excipients.
16. The lipid nanoparticle of any one of **embodiment 9 to embodiment** 15, wherein the polymer conjugated lipid is a PMOZ-lipid according to any one of **embodiment 1 to embodiment 8.**
17. The lipid nanoparticle of any one of **embodiment 9 to embodiment 16,** wherein the lipid nanoparticle comprises excipients selected from ratios selected from the group consisting of
   (i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
   (ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
   (iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
   (iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
   (v) 47.4 mol% cationic or ionizable lipid, preferably one of the ionizable lipid structures of **C1 to C27,** more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;**
   (vi) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8;**
   (vii) 47.4 mol% formula III-3 (((4-hydroxybutyl)azanediyl)bis(hexane-6,1-diyl)bis(2-hexyldecanoate)), 40.9 mol% cholesterol, 10 mol% DSPC, and 1.7 mol% of the polymer conjugated lipid of any one of **embodiment 1 to embodiment 8;**
   (viii) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% 2-[(PMOZ)]n-N,N-ditetradecylacetamide];
   (ix) 47.4 mol% formula III-3 (((4-hydroxybutyl)azanediyl)bis(hexane-6,1-diyl)bis(2-hexyldecanoate)), 40.9 mol% cholesterol, 10 mol% DSPC and 1.7 mol% 2-[(PMOZ)]n-N,N-ditetradecylacetamide]; and most preferably
   (x) 59 mol% C24, 28.5 mol% cholesterol, 10 mol% DPhyPE and 2.5 mol% "PMOZ 4",
   wherein n of the polymer-conjugated lipid has a mean value ranging from about 45 to about 55, preferably n is about 50 or wherein n is selected such that the polymer moiety has an average molecular weight of about 4.2 kDa to about 4.4 kDa, or most preferably about 4.3 kDa.
18. The lipid nanoparticle of any one of **embodiment 9 to embodiment 17,** wherein the polymer conjugated lipid of **embodiment 1 to embodiment 8** inhibits aggregation of the lipid nanoparticles.
19. The lipid nanoparticle of any one of **embodiment 9 to embodiment 18,** further comprising a biologically active ingredient.
20. The lipid nanoparticle of **embodiment 19,** wherein the biologically active ingredient is a nucleic acid compound selected from the group consisting of RNA, an artificial mRNA, chemically modified or unmodified messenger RNA (mRNA) comprising at least one coding sequence, self-replicating RNA, circular RNA, viral RNA, and replicon RNA; or any combination thereof, more preferably wherein the biologically active ingredient is chemically modified mRNA or chemically unmodified mRNA.
21. The lipid nanoparticle of any one of **embodiment 9 to embodiment 20,** wherein the mRNA is associated with the lipid nanoparticle, preferably wherein the mRNA is encapsulated in the lipid nanoparticle.
22. The lipid nanoparticle of any one of **embodiment 9 to embodiment 21,** wherein the lipid nanoparticles comprise the mRNA at an amount such as to achieve an N/P ratio in the range of about 5 to about 20, more preferably about 10 to about 18, even more preferably about 12 to about 16, most preferably about 14.
23. The lipid nanoparticle of any one of **embodiment 9 to embodiment 22,** wherein the lipid nanoparticle is a sterile solid composition for reconstitution with a sterile liquid carrier, and wherein the lipid nanoparticle further comprises one or more inactive ingredients selected from pH-modifying agents, bulking agents, stabilizers, non-ionic surfactants and antioxidants, and wherein the sterile liquid carrier is an aqueous carrier.
24. The lipid nanoparticle of any one of **embodiment 9 to embodiment 23,** wherein the lipid nanoparticle is a sterile liquid composition, and wherein the lipid nanoparticles have a mean hydrodynamic diameter as determined by dynamic laser scattering from about 50 nm to about 300 nm, or from about 60 nm to about 250 nm, or from about 60 nm to about 200 nm, or from about 70 to 200 nm, or from about 75 nm to about 160, or from about 85 nm to about 140 nm, or from about 90 nm to about 130 nm, or from about 50 nm to about 120 nm.
25. The lipid nanoparticle of any one of **embodiment 9 to embodiment 24,** wherein the lipid nanoparticles exhibit a zeta potential in the range of -50 mV to +50 mV, preferably in the range of -25 mV to +25 mV, more preferably in the range of -10 mV to +10 mV, most preferably in the range of -5 mV to +5 mV.
26. The lipid nanoparticle of any one of **embodiment 9 to embodiment 25,** wherein the mRNA compound is a mono-, bi-, or multicistronic mRNA.
27. The lipid nanoparticle of any one **embodiment 9 to embodiment 26,** wherein the mRNA compound comprises at least one chemical modification.
28. The lipid nanoparticle of **embodiment 27,** wherein the chemical modification is selected from the group consisting of base modifications, sugar modifications, backbone modifications and lipid modifications, preferably wherein the chemical modification is a base modification, more preferably wherein the base modification preferably is selected from the group consisting of pseudouridine (psi or ψ), N1-methylpseudouridine (N1MPU, N1Mpsi or N1Mψ), 1-ethylpseudouracil, 2-thiouracil (s2U), 4-thiouracil, 5-methylcytosine, 5-methyluracil, 5-methoxyuracil, and any combination thereof.
29. The lipid nanoparticle of any one of **embodiment 9 to embodiment 28,** wherein the mRNA compound comprises a coding region encoding a peptide or protein, wherein the coding region exhibits a sequence modification.
30. The lipid nanoparticle of **embodiment 29,** wherein the sequence modification is selected from a G/C content modification, a codon modification, a codon optimization or a C-optimization of the sequence; preferably wherein, compared with the coding region of the corresponding wild-type mRNA, the
   - G/C content of the coding region is increased;
   - C content of the coding region is increased;
   - codon usage in the coding region is adapted to the human codon usage; and/or
   - codon adaptation index (CAI) is increased or maximized in the coding region.
31. The lipid nanoparticle of any one of **embodiment 9 to embodiment 30,** wherein the mRNA compound further comprises
   a) a 5'-CAP structure, preferably m7GpppN, more preferably CAP1 or m7G(5')ppp(5')(2'OMeA)pG;
   b) optionally at least one miRNA sequence, preferably wherein the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof;
   c) at least one 5'-UTR element;
   d) a coding sequence;
   e) at least one 3'-UTR element;
   f) at least one poly(A) sequence;
   g) at least one poly(C) sequence;
   or any combinations of these.
32. The lipid nanoparticle of any one of **embodiment 9 to embodiment 31,** wherein the least one coding RNA comprises a 5'-CAP structure, preferably m7G, CAP0, CAP1, CAP2, a modified CAP0 or a modified CAP1 structure.
33. The lipid nanoparticle of any one of **embodiment 9 to embodiment 32,** wherein the at least one coding RNA comprises at least one heterologous 5'-UTR and/or at least one heterologous 3'-UTR, preferably wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes; and/or preferably wherein the at least one heterologous 3'-UTR comprises a nucleic acid sequence derived from a 3'-UTR of a gene selected from PSMB3, ALB7, alpha-globin, CASP1, COX6B1, GNAS, NDUFA1 and RPS9, or from a homolog, a fragment or a variant of any one of these genes.
34. The lipid nanoparticle of any one of **embodiment 9 to embodiment 33,** wherein the at least one coding RNA comprises a (i) HSD17B4 5'-UTR and a PSMB3 3'-UTR or (ii) a RPL32 5'-UTR and an ALB7 3'-UTR, preferably a mutated alpha-globin 3'-UTR (SEQ ID NO:11/12), more preferably a HSD17B4 5'-UTR (SEQ ID NO:21/22) and a PSMB3 3'-UTR (SEQ ID NO:19/20).
35. The lipid nanoparticle of any one of **embodiment 9 to embodiment 34,** comprising the following elements in the 5' to 3' direction:
   a) a 5'-CAP structure, preferably selected from the group consisting of m7G(5'), m7G(5')ppp(5')(2'OMeA)pG and m7G(5')ppp(5')(2'OMeG)pG;
   b) a 5'-UTR element comprising a nucleic acid sequence derived from the 5'-UTR of a TOP gene, said nucleic acid sequence preferably comprising an RNA sequence that corresponds to the nucleic acid sequence according to SEQ ID NO:22, 24, 26, or a homolog, a fragment or a variant thereof, most preferably according to SEQ ID NO:22 (HSD17B4);
   c) at least one coding sequence;
   d) a 3'-UTR element comprising a nucleic acid sequence derived from an α-globin gene, said nucleic acid sequence preferably comprising an RNA sequence that corresponds to the nucleic acid sequence according to SEQ ID NO:6, 8, 10, 12, 14, 16, 18, 20, or a homolog, a fragment or a variant thereof; and/or a 3'-UTR element comprising a nucleic acid sequence derived from an albumin gene, said nucleic acid sequence preferably comprising an RNA sequence that corresponds to the nucleic acid sequence according to SEQ ID NO:18, or a homolog, a fragment or a variant thereof, most preferably according to SEQ ID NO:20 (PSMB3);
   e) optionally, at least one poly(A) sequence, preferably consisting of 10 to 200, 10 to 100, 40 to 80, or 50 to 70 adenosine nucleotides;
   f) optionally, at least one poly(C) sequence, preferably consisting of 10 to 200, 10 to 100, 20 to 70, 20 to 60 or 10 to 40 cytosine nucleotides; and
   g) optionally, at least one histone stem-loop, preferably comprising the RNA sequence according to SEQ ID NO:4.
36. The lipid nanoparticle of any one of **embodiment 9 to embodiment 35,** wherein the biologically active ingredient is
   (a) an mRNA comprising at least one coding sequence encoding a peptide or protein, or a fragment or variant thereof, wherein the peptide or protein is an antigen, wherein the antigen preferably is derived from pathogenic antigens, tumor antigens, allergenic antigens or autoimmune self-antigens, or a fragment or variant thereof; or
   (b) an mRNA comprising at least one coding sequence encoding a therapeutic protein, or a fragment or variant thereof, wherein the therapeutic protein is selected from the group consisting of
      (i) therapeutic proteins for use in enzyme replacement therapy for the treatment of metabolic, endocrine or amino acid disorders or for use in replacing an absent, deficient or mutated protein;
      (ii) therapeutic proteins for use in the treatment of blood disorders, diseases of the circulatory system, diseases of the respiratory system, infectious diseases or immune deficiencies;
      (iii) therapeutic proteins for use in the treatment of cancer or tumor diseases;
      (iv) therapeutic proteins for use in hormone replacement therapy;
      (v) therapeutic proteins for use in reprogramming somatic cells into pluri- or omnipotent stem cells;
      (vi) therapeutic proteins for use as adjuvant or immunostimulation;
      (vii) therapeutic proteins being a therapeutic antibody;
      (viii) therapeutic proteins being a gene editing agent; and
      (ix) therapeutic proteins for use in treating or preventing a liver disease selected from the group consisting of liver fibrosis, liver cirrhosis and liver cancer.
37. The lipid nanoparticle of **embodiment 36 subitem (a),** wherein the at least one coding sequence encoding a pathogenic antigen is selected from the group consisting of a bacterial, viral, fungal and protozoal antigen.
38. The lipid nanoparticle of **embodiment 37,** wherein the at least one coding sequence encoding a pathogenic antigen
   (i) is derived from a SARS coronavirus 2 (SARS-CoV-2), nCoV-2019 coronavirus, SARS coronavirus (SARS-CoV), Bunyavirales virus, Cytomegalovirus (CMV), Dengue viruses (DENV-1, DENV-2, DENV-3 and DENV-4), Ebola virus, Epstein-Barr virus (EBV), Flavivirus, Hepatitis B virus (HBV), Herpes simplex virus (HSV), Human immunodeficiency virus (HIV), Human metapneumovirus (HMPV), Human Papilloma virus (HPV), Human parainfluenza viruses (HPIV), Influenza virus, extraintestinal pathogenic E. coli (ExPEC), Lassa mammarenavirus (LASV), MERS coronavirus, Mycobacterium tuberculosis, Nipah virus, Norovirus, Rabies virus, Respiratory Syncytial virus (RSV), Rhinovirus, Rota virus, Vaccinia virus, Yellow Fever virus (YFV), Zika virus (ZIKV), Chlamydia trachomatis (i.e. bacterium chlamydia causing chlamydia), or Malaria parasite (e.g. Plasmodium falciparum, Plasmodium vivax, Plasmodium malariae, or Plasmodium ovale); and/or
   (ii) is derived from a structural protein, an accessory protein, or a replicase protein from a SARS coronavirus 2 (SARS-CoV-2), nCoV-2019 coronavirus, SARS coronavirus (SARS-CoV), or an immunogenic fragment or immunogenic variant of any of these; and/or
   (iii) is derived from a spike protein (S), an envelope protein (E), a membrane protein (M) or a nucleocapsid protein (N) from a SARS coronavirus 2 (SARS-CoV-2), nCoV-2019 coronavirus, SARS coronavirus (SARS-CoV), or an immunogenic fragment or immunogenic variant of any of these, preferably wherein the spike protein (S) comprises or consists of spike protein fragment S1 or spike protein fragment S2, more preferably spike protein fragment S1, or an immunogenic fragment or immunogenic variant thereof (e.g. receptor binding domain (RBD), critical neutralisation domain (CND)); and/or
   (iv) is derived from a pre-fusion stabilized spike protein (S) (S_stab) from a SARS coronavirus 2 (SARS-CoV-2), nCoV-2019 coronavirus, SARS coronavirus (SARS-CoV) comprising at least one pre-fusion stabilizing mutation.
39. The lipid nanoparticle of any one of **embodiment 9 to embodiment 38** for use
   (i) in the treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition; and/or
   (ii) for use in enzyme replacement therapy for the treatment of metabolic or endocrine disorders or for use in replacing an absent, deficient or mutated protein.
40. The lipid nanoparticle of any one of **embodiment 9 to embodiment 39** for use in the treatment or prophylaxis of infectious diseases.
41. The lipid nanoparticle of **embodiment 9 or embodiment 40** comprising at least one coding RNA, wherein said at least one coding RNA comprises at least one coding sequence encoding at least one peptide or protein for use in treatment or prevention of a disease, disorder or condition, wherein said lipid nanoparticle is administered via local or locoregional injection, infusion or implantation, in particular intradermal, subcutaneous, intramuscular, intracameral, subconjunctival, suprachoroidal injection, subretinal, subtenon, retrobulbar, topical, posterior juxtascleral administration, or intrapulmonal inhalation, interstitial, locoregional, intravitreal, intratumoral, intralymphatic, intranodal, intra-articular, intrasynovial, periarticular, intraperitoneal, intra-abdominal, intracardial, intralesional, intrapericardial, intraventricular, intrapleural, perineural, intrathoracic, epidural, intradural, peridural, intrathecal, intramedullary, intracerebral, intracavernous, intracorporus cavernosum, intraprostatic, intratesticular, intracartilaginous, intraosseous, intradiscal, intraspinal, intracaudal, intrabursal, intragingival, intraovarian, intrauterine, intraocular, periocular, periodontal, retrobulbar, subarachnoid, subconjunctival, suprachoroidal injection, infusion, implantation, nasal, buccal, sublingual, otic or auricular, ophthalmic, conjunctival, vaginal, rectal, intracervical, endosinusial, laryngeal, oropharyngeal, ureteral, urethral administration, more preferably said lipid nanoparticle is administered intramuscularly, intravenously, intradermally, subcutaneously, intratumorally, intranasally, or by inhalation, most preferably intramuscularly, to a subject in need thereof.
42. A kit or kit of parts, comprising any one of the lipid nanoparticle of **embodiment 9 to embodiment 41,** optionally comprising a liquid vehicle for solubilizing, and, optionally, technical instructions providing information on administration and dosage of the components.
43. The lipid nanoparticle of any one of **embodiment 9 to embodiment 41** or the kit or kit of parts of **embodiment 42** for use in *in vivo* drug delivery, preferably for use in delivering a nucleic acid, preferably a mRNA.
44. The lipid nanoparticle of any one of **embodiment 9 to embodiment 41** or the kit or kit of parts of **embodiment 43** for use as a medicament.
45. The lipid nanoparticle for use as a medicament according to **embodiment 44,** wherein the medicament is for the prevention, prophylaxis, treatment and/or amelioration of a disease selected from infectious diseases including viral, bacterial or protozoological infectious diseases, cancer or tumor diseases, liver diseases, autoimmune diseases, allergies, monogenetic diseases including hereditary diseases, genetic diseases in general, diseases which have a genetic inherited background and which are typically caused by a defined gene defect and are inherited according to Mendel's laws; cardiovascular diseases, neuronal diseases, diseases of the respiratory system, diseases of the digestive system, diseases of the skin, musculoskeletal disorders, disorders of the connective tissue, neoplasms, immune deficiencies, endocrine, nutritional and metabolic diseases, eye diseases, ear diseases and diseases associated with a peptide or protein deficiency.
46. The lipid nanoparticle for use as a medicament according to **embodiment 44 or embodiment 45,** wherein the medicament is a vaccine composition.
47. A vaccine composition comprising a lipid nanoparticle of any one of **embodiment 9 to embodiment 46** or a kit or kit of parts of **embodiment 42** for use as a medicament, and/or for prevention, prophylaxis, treatment and/or amelioration of a disease selected from infectious diseases including viral, bacterial or protozoological infectious diseases, cancer or tumor diseases.
48. A method of treatment or prophylaxis of infectious diseases; cancer or tumor diseases, disorders or conditions; liver diseases selected from the group consisting of liver fibrosis, liver cirrhosis and liver cancer; allergies; or autoimmune disease; disorder or condition comprising the steps:
   a) providing a lipid nanoparticle of any one of **embodiment 9 to embodiment 45,** comprising a homopolymer moiety comprising at least one polyoxazoline (POZ) monomer, preferably the polymer conjugated lipid according to any one of **embodiment 1 to embodiment 8,** the vaccine composition of **embodiment 47,** or the kit or kit of parts of **embodiment 42;** and
   b) applying or administering the mRNA, the lipid nanoparticle, the vaccine composition or the kit or kit of parts to a tissue or an organism.
49. A method for delivering mRNA encoding an antigen or a therapeutic peptide or protein to a subject, the method comprising administering to a subject a lipid nanoparticle of any one of embodiments 1 to 33, wherein the mRNA encodes an antigen or a therapeutic peptide or protein, and wherein delivering the mRNA to the subject is beneficial in treating or preventing a disease or disorder, preferably wherein the subject is a mammal, more preferably wherein the subject is a human.
50. The method according to any one of embodiments **embodiment 48 to embodiment 49,** wherein the mRNA, the lipid nanoparticle of any one of **embodiment 9 to embodiment 48,** the vaccine composition of **embodiment 47** or the kit or kit of parts of **embodiment 42** is administered to the tissue or to the organism by intravenous, intramuscular, subcutaneous, intradermal or intratumoral injection or any administration route as disclosed in any **preceding embodiment.**
51. A method of inducing an immune response in a subject, the method comprising administering to the subject the vaccine composition of **embodiment 47** in an amount effective to produce an antigen-specific immune response in the subject.
52. A pharmaceutical composition comprising a lipid nanoparticle of any one of **embodiment 9 to embodiment 48** or a kit or kit of parts of **embodiment 42** or the vaccine composition of **embodiment 47** for use in vaccination of a subject comprising an effective dose of mRNA encoding a virus antigen.
53. Use of a pharmaceutical composition according to **embodiment 52** or a kit or kit of parts according to **embodiment 42** for (i) inducing an immune response, for (ii) inducing an antigen specific T-cell response or preferably for (iii) inducing CD8+ T cells responses.
54. Use of the pharmaceutical composition of **embodiment 52** for the prophylaxis of an infectious disease or in the manufacture of a medicament for the prophylaxis of an infectious disease, wherein said medicament preferably is a vaccine composition.
55. A method for preventing, ameliorating or treating a disease or condition in a subject in need comprising administering to the subject a lipid nanoparticle of any one of **embodiment 9 to embodiment 48,** a pharmaceutical composition of **embodiment 52** or a kit or kit of parts of **embodiment 42.**
56. The method of any one of the **preceding method embodiments,** wherein administration of the lipid nanoparticle results in expression of the antigen encoded by mRNA in the lymphocytes of the subject.
57. A method of treating or preventing a disorder of any one of **embodiments 36, 39, 41, 45, 48, or 49,** wherein the disorder is an infection with coronavirus, or a disorder related to such an infection.
58. A method of treating or preventing a disorder of any one of **embodiments 36, 39, 41, 45, 48, or 49,** wherein the subject in need is a mammalian subject, preferably a human subject.
59. The method of any one of the **preceding method embodiments,** wherein the administration of the lipid nanoparticle results in an antigen specific antibody response, preferably wherein the antigen specific antibody response is measured by the presence of antigen-specific antibodies in serum.
60. The lipid nanoparticle of any one of **embodiment 9 to embodiment 16,** wherein the lipid nanoparticle comprises excipients selected from ratios selected from the group consisting of
   (i) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% neutral lipid and 1.7 mol% of the polymer conjugated lipid of any one of **embodiment 1 to embodiment 8;**
   (ii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.5 mol% cholesterol, 10 mol% neutral lipid and 2.5 mol% of the polymer conjugated lipid of any one of **embodiment 1 to embodiment 8;**
   (iii) 59 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 28.3 mol% cholesterol, 10 mol% DSPC or DPhyPE, preferably DPhyPE, 1 mol% DHPC, and 2.5 mol% of the polymer conjugated lipid of any one of **embodiment 1 to embodiment 8;**
   (iv) 49 mol% cationic or ionizable lipid, preferably one of the ionizable lipid structures of C1 to C24, more preferably the ionizable lipid structure C24 or formula III-3 ((4-hydroxybutyl)azanediyl)bis (hexane-6,1-diyl)bis(2-hexyldecanoate)), 29.3 mol% cholesterol, 10 mol% DSPC or DPhyPE, preferably DPhyPE, 10 mol% DHPC, and 2.5 mol% of the polymer conjugated lipid of any one of **embodiment 1 to embodiment 8;**
   (vi) 47.4 mol% cationic or ionizable lipid, preferably one of the ionizable lipid structures of **C1 to** C27, more preferably the ionizable lipid structure C24 or formula III-3 (((4-hydroxybutyl)azanediyl)bis(hexane-6,1-diyl)bis(2-hexyldecanoate)), 40.9 mol% cholesterol, 10 mol% DSPC or DPhyPE, preferably DPhyPE, and 1.7 mol% of the polymer conjugated lipid of any one of **embodiment 1 to embodiment 8;**
   (vi) 47.4 mol% cationic or ionizable lipid, preferably one of the ionizable lipid structures of **C1 to** C27, more preferably the ionizable lipid structure C24 or formula III-3 (((4-hydroxybutyl)azanediyl)bis(hexane-6,1-diyl)bis(2-hexyldecanoate)), 40.1 mol% cholesterol, 10 mol% DSPC and 2.5 mol% of the polymer conjugated lipid of any one of **embodiment 1 to embodiment 8;**
   (vii) 47.4 mol% formula III-3 (((4-hydroxybutyl)azanediyl)bis(hexane-6,1-diyl)bis(2-hexyldecanoate)), 40.9 mol% cholesterol, 10 mol% DSPC, and 1.7 mol% of the polymer conjugated lipid of any one of **embodiment 1 to embodiment 8;**
   (viii) 47.4 mol% formula III-3 (((4-hydroxybutyl)azanediyl)bis(hexane-6,1-diyl)bis(2-hexyldecanoate)), 40.1 mol% cholesterol, 10 mol% DSPC and 2.5 mol% 2-[(PMOZ)]n-N,N-ditetradecylacetamide];
   (ix) 47.4 mol% formula III-3 (((4-hydroxybutyl)azanediyl)bis(hexane-6,1-diyl)bis(2-hexyldecanoate)), 40.9 mol% cholesterol, 10 mol% DSPC and 1.7 mol% 2-[(PMOZ)]n-N,N-ditetradecylacetamide]; and most preferably
   (x) 59 mol% C24, 28.5 mol% cholesterol, 10 mol% DPhyPE and 2.5 mol% "PMOZ 4",
   wherein n of the polymer-conjugated lipid has a mean value ranging from about 45 to about 55, preferably n is about 50 or wherein n is selected such that the polymer moiety has an average molecular weight of about 4.2 kDa to about 4.4 kDa, or most preferably about 4.3 kDa.
61. The lipid nanoparticle of any one of **embodiment 9 to embodiment 16 or embodiment 60,** wherein the lipid nanoparticle comprises a neutral lipid or phospholipid having at least one alkyl chain with a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₆, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).
62. The lipid nanoparticle of any one of **embodiment 9 to embodiment 16 or embodiment 60 to embodiment 61,** wherein the lipid nanoparticles comprise a neutral lipid or phospholipid having at least two alkyl chains, whereby each alkyl chain independently has a length of C₅, C₆, C₇, C₈, C₉, C₁₀, C₁₁, C₁₂, C₁₃ or C₁₄, preferably with a length of C₆, C₇, C₈, C₉, or C₁₀, more preferably with a length of C₆, C₇, C₈, most preferably with a length of C₇, or further most preferably wherein the lipid nanoparticle comprises a combination of two neutral lipids wherein the combination comprises a neutral lipid or phospholipid having at least two alkyl chains, whereby each alkyl chain independently has a length of preferably C₆, C₇, C₈, C₉, or C₁₀, more preferably with a length of C₆, C₇, C₈, most preferably with a length of C₇, further most preferably a phospholipid selected from the group consisting of 05:0 PC (1,2-dipentanoyl-sn-glycero-3-phosphocholine), 04:0 PC (1,2-dibutyryl-sn-glycero-3-phosphocholine), 06:0 PC (DHPC, 1,2-dihexanoyl-sn-glycero-3-phosphocholine), 07:0 PC (DHPC, 1,2-diheptanoyl-sn-glycero-3-phosphocholine), 08:0 PC (1,2-dioctanoyl-sn-glycero-3-phosphocholine), and 09:0 PC (1,2-dinonanoyl-sn-glycero-3-phosphocholine), preferably 07:0 PC (DHPC, 1,2-diheptanoyl-sn-glycero-3-phosphocholine).
63. A lipid nanoparticle comprising a polymer conjugated lipid according to any one of **embodiment 1** to **embodiment 8,** wherein the lipid nanoparticle has a lower PDI and/or lower size upon (i) freezing and thawing or (ii) freeze-drying (lypophilizing) and reconstitution, as compared to a control lipid nanoparticle comprising a PEG-lipid instead said polymer conjugated lipid according to any one of **embodiment 1** to **embodiment 8.**
64. A method of making a frozen lipid nanoparticle of any one of **embodiment 9 to embodiment 16 or embodiment 60 to embodiment 62** or a lipid nanoparticle comprising a polymer conjugated lipid according to any one of **embodiment 1** to **embodiment 8,** wherein the lipid nanoparticle upon thawing has a lower PDI and/or lower size as compared to a control lipid nanoparticle comprising a PEG-lipid instead of a polymer conjugated lipid according to any one of **embodiment 1** to **embodiment 8.**
65. A method of making a lyophilized lipid nanoparticle of any one of **embodiment 9 to embodiment 16 or embodiment 60 to embodiment 62** or a lipid nanoparticle comprising a polymer conjugated lipid according to any one of **embodiment 1** to **embodiment 8,** wherein the lipid nanoparticle upon reconstitution has a lower PDI and/or lower size as compared to a control lipid nanoparticle comprising a PEG-lipid instead of a polymer conjugated lipid according to any one of **embodiment 1** to **embodiment 8.**
66. An improved lyophilization process for the preparation of lyophilized lipid nanoparticles of any one of **embodiment 9 to embodiment 16 or embodiment 60 to embodiment 62,** said process comprising the step of using a polymer conjugated lipid according to any one of **embodiment 1** to **embodiment 8** as excipient instead of a PEG-lipid, wherein the lipid nanoparticle upon reconstitution has a lower PDI and/or lower size as compared to a control lipid nanoparticle comprising a PEG-lipid instead of a polymer conjugated lipid according to any one of **embodiment 1** to **embodiment 8.**
67. A vaccine composition, comprising a lipid nanoparticle of any one of **embodiment 9 to embodiment 16 or embodiment 60 to embodiment 62** or a polymer conjugated lipid according to any one of **embodiment 1 to embodiment 8.**
68. A vaccine composition or a lipid nanoparticle of any one of the preceding embodiments comprising a polymer-conjugated lipid according to any of the preceding embodiments, wherein the formulation has an increase in LNP mean size of about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less after one or more freeze/thaw cycles as compared to that prior to freeze/thaw cycles.
69. A vaccine composition or a lipid nanoparticle of any one of the preceding embodiments comprising a polymer-conjugated lipid according to any of the preceding embodiments, wherein the formulation has an increase in LNP mean size of about 10%, 9%, 8%, 7%, 6%, 5%, 4%, 3%, 2%, 1%, or less after one or more freeze/thaw cycles as compared to that prior to freeze/thaw cycles.
70. A vaccine composition or a lipid nanoparticle of any one of the preceding embodiments comprising a polymer-conjugated lipid according to any of the preceding embodiments, wherein the formulation has an increase in LNP mean size of about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less after lyophilization as compared to that prior to lyophilization.
71. A vaccine composition or a lipid nanoparticle of any one of the preceding embodiments comprising a polymer-conjugated lipid according to any of the preceding embodiments, wherein the formulation has an increase in LNP mean size of about 10%, 9%, 8%, 7%, 6%, 5%, 4%, 3%, 2%, 1%, or less after lyophilization as compared to that prior to lyophilization.
72. A vaccine composition or a lipid nanoparticle of any one of the preceding embodiments comprising a polymer-conjugated lipid according to any of the preceding embodiments, wherein the formulation has an increase in LNP mean size of about 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less after dilution as compared to that prior to dilution.
73. A vaccine composition or a lipid nanoparticle of any one of the preceding embodiments comprising a polymer-conjugated lipid according to any of the preceding embodiments, wherein the formulation has an increase in LNP mean size of about 10%, 9%, 8%, 7%, 6%, 5%, 4%, 3%, 2%, 1%, or less after dilution as compared to that prior to dilution.
74. A vaccine composition or a lipid nanoparticle of any one of the preceding embodiments comprising a polymer-conjugated lipid according to any of the preceding embodiments, wherein the encapsulation efficiency of the formulation is substantially the same after storage at about 4 °C or lower for at least one month.
75. A vaccine composition or a lipid nanoparticle of any one of the preceding embodiments comprising a polymer-conjugated lipid according to any of the preceding embodiments, wherein the LNP mean size of the LNPs is substantially the same after storage at about 4 °C or lower for at least one month.

## Claims

1. A polymer conjugated lipid according to formula (I):
[P]-[linker]-[L]
or a pharmaceutically acceptable salt, prodrug, tautomer or stereoisomer thereof, wherein
[P] is a heteropolymer moiety or homopolymer moiety, preferably a homopolymer moiety, comprising at least one polyoxazoline (POZ) monomer unit 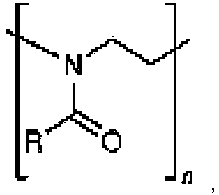
wherein R is C1-9 alkyl or C2-9 alkenyl, preferably C1 or C2 alkyl, and n has a mean value ranging from about 45 to about 55, preferably n is about 50 or wherein n is selected such that the [P] moiety has an average molecular weight of about 4.2 kDa to about 4.4 kDa, or most preferably about 4.3 kDa,
[linker] is an optional linker group, and
[L] is a lipid moiety.

2. The polymer conjugated lipid of claim 1, wherein [P] is a heteropolymer moiety or homopolymer moiety comprising multiple monomer units selected from the group consisting of poly(2-methyl-2-oxazoline) (PMOZ), poly(2-ethyl-2-oxazoline) (PEOZ), poly(2-propyl-2-oxazoline) (PPOZ), poly(2-butyl-2-oxazoline) (PBOZ), poly(2-isopropyl-2-oxazoline) (PIPOZ), poly(2-methoxymethyl-2-oxazoline) (PMeOMeOx), and poly(2-dimethylamino-2-oxazoline) (PDMAOx),
preferably wherein [P] is a homopolymer moiety comprising multiple PMOZ or PEOZ monomer units, more preferably wherein [P] comprises or preferably consists of multiple PMOZ monomer units,
wherein (i) n has a mean value ranging from about 45 to about 55, preferably n is about 50 or wherein (ii) n is selected such that the [P] moiety has an average molecular weight of about 4.2 kDa to about 4.4 kDa, or most preferably about 4.3 kDa.

3. The polymer conjugated lipid of any one of claim 1 to claim 2, wherein
(i) the lipid moiety [L] comprises at least one straight or branched, saturated or unsaturated alkyl chain containing from 6 to 30 carbon atoms, preferably wherein the lipid moiety [L] comprises at least one straight or branched saturated alkyl chain, or
(ii) the lipid moiety [L] comprises ditetradecylamin.

4. The polymer conjugated lipid of any one of claim 1 to claim 3, wherein the lipid moiety [L] comprises at least one, preferably two, straight or branched, saturated or unsaturated alkyl chain comprising 6, 7, 8, 9, 10, 11, 12, 13, 14, 15, 16, 17, 18, 19, 20, 21, 22, 23, 24, 25, 26, 27, 28, 29, or 30 carbon atoms, preferably in the range of 10 to 20 carbon atoms, more preferably in the range of 12 to 18 carbon atoms, even more preferably 14, 16 or 18 carbon atoms, even more preferably 16 or 18 carbon atoms, most preferably 14 carbon atoms.

5. The polymer conjugated lipid of any one of claim 1 to claim 4, wherein the linker group [linker] is selected from the group consisting of but not limited to a pH-sensitive moiety, a peptide or amid bond (-CO-NH-), an alkyl or alkenyl moiety (C₁₋₉ alkyl or C₂₋₉ alkenyl), a zwitterionic linker, non-ester containing linker moieties and ester-containing linker moieties (-C(O)O- or -OC(O)-), amido (-C(O)NH-), disulfide (-S-S-), carbonyl (-C(O)-), ether (-O-), thioether (-S-), oxime, carbamate (-NHC(O)O-), urea (-NHC(O)NH-), succinyl (-(O)CCH₂CH₂C(O)-), succinamidyl (-NHC(O)CH₂CH₂C(O)NH-), (-NHC(O)CH₂CH₂C(O)-), (-NHC(O)CH₂CH₂C(O)O-), carbonate (-OC(O)O-), nitrogen (N), succinoyl, succinate, phosphate esters (-O-(O)POH-O-), and sulfonate esters, as well as combinations thereof.

6. The polymer conjugated lipid of claim 5, preferably wherein the linker group [linker] is selected from the group consisting of (-NHC(O)CH2CH2C(O)-), a peptide bond or amid bond (-CO-NH-), (-NHC(O)CH2CH2C(O)O-), and -NH-CH2-.

7. The polymer conjugated lipid of any one of claim 1 to claim 6, wherein the lipid moiety [L] comprises ditetradecylamin, preferably wherein the linker group [linker] is (-NHC(O)CH₂CH₂C(O)-).

8. The polymer conjugated lipid of any one of claim 1 to claim 7, wherein the linker group [linker] comprises an amide linker moiety, preferably an ester linker moiety.

9. The polymer conjugated lipid of any one of claim 1 to claim 8, wherein the linker group [linker] comprises preferably a succinate linker moiety.

10. The polymer conjugated lipid of any one of claim 1 to claim 9, wherein the polymer conjugated lipid does not comprise a polyethylene glycol-(PEG)-moiety or residue.

11. The polymer conjugated lipid of any one of claim 1 to claim 10, wherein the polymer conjugated lipid does not comprise a sulphur group (-S-).

12. The polymer conjugated lipid of any one of claim 1 to claim 11, wherein the polymer conjugated lipid does not comprise a terminating nucleophile.

13. The polymer conjugated lipid of any one of claim 1 to claim 12, wherein the polymer conjugated lipid is selected from the group consisting of "PMOZ 1", "PMOZ 2", "PMOZ 3", "PMOZ 4" and "PMOZ 5".

14. The polymer conjugated lipid of claim 13, wherein n has a mean value ranging from about 45 to about 55, preferably n is about 50.
